(19) Europäisches Patentamt
European Patent Office
Office européen des brevets

(11) EP 3 760 627 A1

(12) **EUROPEAN PATENT APPLICATION**
published in accordance with Art. 153(4) EPC

(43) Date of publication:
**06.01.2021 Bulletin 2021/01**

(21) Application number: **19760584.3**

(22) Date of filing: **28.02.2019**

(51) Int Cl.:
*C07D 417/12* (2006.01) *A01N 43/80* (2006.01)
*A01N 43/828* (2006.01) *A01P 3/00* (2006.01)

(86) International application number:
**PCT/JP2019/007874**

(87) International publication number:
**WO 2019/168112 (06.09.2019 Gazette 2019/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.02.2018 JP 2018035759**
**29.06.2018 JP 2018124041**

(71) Applicant: **Hokko Chemical Industry Co., Ltd.**
**Tokyo 103-8341 (JP)**

(72) Inventors:
- **MORITA Motoaki**
**Atsugi-shi, Kanagawa 243-0023 (JP)**
- **YOSHII Atsushi**
**Atsugi-shi, Kanagawa 243-0023 (JP)**
- **KUZUMA Seiichi**
**Atsugi-shi, Kanagawa 243-0023 (JP)**
- **MORIYA Yuka**
**Atsugi-shi, Kanagawa 243-0023 (JP)**
- **SAITOH Junko**
**Atsugi-shi, Kanagawa 243-0023 (JP)**
- **MURAKAMI Hideyuki**
**Atsugi-shi, Kanagawa 243-0023 (JP)**

(74) Representative: **Reitstötter Kinzebach Patentanwälte**
**Sternwartstrasse 4**
**81679 München (DE)**

# (54) IMIDE DERIVATIVE AND BACTERICIDE CONTAINING SAME AS ACTIVE INGREDIENT

(57) The present invention provides an imide derivative represented by the following formula (1):

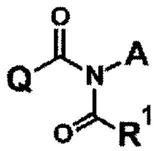

(1)

wherein $R^1$ is substituted by a substituent such as an isothiazolyl group, Q is substituted by a substituent such as a thiophenyl group, and A is substituted by a substituent such as a pyrazolyl group, and an agricultural and horticultural fungicide offering an excellent fungicidal effect in agricultural and horticultural situations, which is characterized by containing the imide derivative as an active ingredient.

EP 3 760 627 A1

## Description

TECHNICAL FIELD

The present invention relates to an imide derivative exhibiting a high control effect against various pathogenic fungi harmful for cultivation of crops and having both high safety to crops and excellent environmental friendliness, and an agricultural and horticultural fungicide using the imide derivative as an active ingredient.

BACKGROUND ART

Conventionally, in the field of agriculture and horticulture, although many agricultural and horticultural fungicides intended to control various diseases have been developed and are used in practice, these are not always satisfied in terms of efficacy, spectrum, residual effectiveness, *etc.* As well, the societal requirements such as reduction in a number of applications or an application amount are not sufficiently fulfilled.

In addition, appearance of pathogenic fungi gained resistance to conventional general-purpose agricultural and horticultural fungicides also becomes a problem. Although there are agricultural chemicals to which pathogenic fungi have not yet gained resistance, these generally require a large application amount or a large number of applications and are therefore disadvantageous in view of environmental pollution, *etc.* Accordingly, it is keenly demanded to develop a novel agricultural and horticultural fungicide exhibiting a sufficient control effect with a small dosage also on various pathogenic fungi that have gained resistance to conventional fungicides, and moreover, having a low adverse environmental impact.

Furthermore, the enhancement of productivity of important crops is indispensable for solving the food crisis caused by world population growth which is expected to come near future. In order to realize stable food supply, pathogenic fungi acting as a barrier at the cultivation of crops need to be controlled economically and efficiently, and in turn, development of a novel agricultural and horticultural fungicide capable of offering a solution becomes increasingly important.

Heretofore, imide derivatives exhibiting fungicidal activity are known. The acyl group on an amide nitrogen of the compounds described in Patent Literatures 1 to 6 is different from that of the present invention.

CITATION LIST

PATENT LITERATURE

Patent Literature 1: WO2002/059086
Patent Literature 2: JP-A-H08-176112
Patent Literature 3: WO2005/042480
Patent Literature 4: WO2005/074686
Patent Literature 5: WO2007/068375
Patent Literature 6: JP-A-S53-132536

SUMMARY OF INVENTION

TECHNICAL PROBLEM

An object of the present invention is to provide an excellent agricultural and horticultural fungicide.

SOLUTION TO PROBLEM

As a result of many intensive studies to attain the object above, the present inventors have found that an imide derivative represented by the following formula (1) exhibits excellent fungicidal activity in agricultural and horticultural situations, and have accomplished the present invention.

Accordingly, a first aspect of the present application relates to an imide derivative represented by the following formula (1) (in the present description, sometimes referred to as "the compound of the present invention").

A second aspect of the present application relates to an agricultural and horticultural fungicide characterized by containing the imide derivative represented by formula (1) as an active ingredient.

More specifically, the above-described problems can be solved by the following configurations.

[1] An imide derivative represented by formula (1):

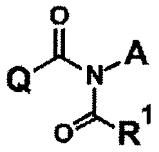
(1)

wherein in formula (1), $R^1$ represents (1a) to (1j) shown below:

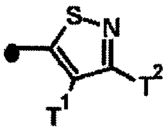
(1 a)
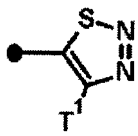
(1 b)
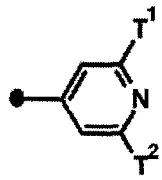
(1 c)

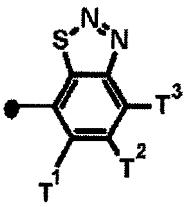
(1 d)
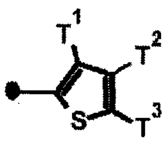
(1 e)
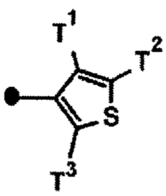
(1 f)

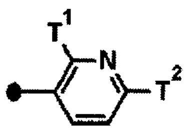
(1 g)
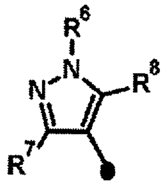
(1 h)
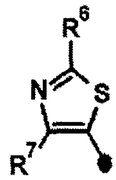
(1 i)

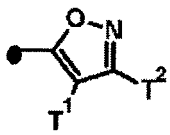
(1 j)

wherein each of $T^1$, $T^2$ and $T^3$ represents a hydrogen atom, a halogen atom, a C1-C6 alkyl group, or a C1-C6 haloalkyl group; $T^1$, $T^2$ and $T^3$ may be the same or different; each of $R^6$ and $R^7$ represents a hydrogen atom, a C1-C6 alkyl group, or a C1-C6 haloalkyl group; and $R^8$ represents a hydrogen atom, a halogen atom, a C1-C6 alkyl group, or a C1-C6 haloalkyl group,
A represents (1k) to (1n) shown below:

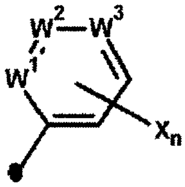
(1 k)
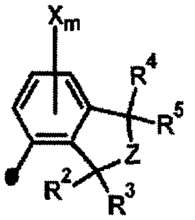
(1 l)
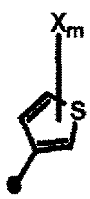
(1 m)

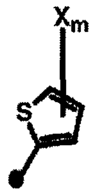

(1 n)

wherein X in formula (1k), (11), (1m), or (1n) represents a hydrogen atom, a halogen atom, a C1-C16 alkyl group which may be substituted (the group may be monosubstituted or polysubstituted by the same or different groups selected from the group consisting of a C1-C6 trialkylsilyl group wherein the alkyl groups may be the same or different, and a cyano group), a C1-C16 haloalkyl group, a C2-C16 alkenyl group which may be substituted (the group may be monosubstituted or polysubstituted by the same or different halogen atoms), a C2-C16 haloalkenyl group, a C2-C16 alkynyl group, a C2-C16 haloalkynyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkoxy C1-C6 alkyl group, a C1-C6 haloalkoxy C1-C6 alkyl group, a C1-C6 alkoxy C1-C6 alkoxy C1-C6 alkyl group, a C1-C6 alkylthio C1-C6 alkyl group, a C1-C6 haloalkylthio C1-C6 alkyl group, a C3-C6 cycloalkylthio C1-C6 alkyl group, a C1-C6 alkylsulfinyl C1-C6 alkyl group, a C1-C6 alkylsulfonyl C1-C6 alkyl group, a C1-C6 alkylamino C1-C6 alkyl group, a C1-C6 alkoxycarbonyl C1-C6 alkyl group, a C1-C6 alkylcarbonyl group, a C1-C6 haloalkylcarbonyl group, a C3-C6 cycloalkylcarbonyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a benzenecarbonyl group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), a C1-C6 alkylaminocarbonyl group, a di C1-C6 alkylaminocarbonyl group (the alkyl moieties of the group may be the same or different and may also combine with each other through an alkylene group to form a 3-membered ring, a 4-membered ring, a 5-membered ring, or a 6-membered ring), a C1-C6 alkylsulfonyl group, a C1-C6 haloalkylsulfonyl group, a C1-C6 alkylaminosulfonyl group, a di C1-C6 alkylaminosulfonyl group (the alkyl moieties of the group may be the same or different and may also combine with each other through an alkylene group to form a 3-membered ring, a 4-membered ring, a 5-membered ring, or a 6-membered ring), a C1-C6 alkoxycarbonyl group, a C7-C12 aralkyl group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), a heterocyclic C1-C6 alkyl group, a C1-C6 alkoxy group, a C1-C6 haloalkoxy group, a C1-C6 alkylthio group, C1-C6 haloalkylthio group, a C3-C6 cycloalkyloxy group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C3-C6 cycloalkyl C1-C6 alkoxy group, a C1-C6 alkylamino group, a di C1-C6 alkylamino group (the alkyl moieties of the group may be the same or different and may also combine with each other through an alkylene group to form a 3-membered ring, a 4-membered ring, a 5-membered ring, or a 6-membered ring), a C1-C6 alkylthio C1-C6 alkoxy group, a C1-C6 alkoxy C1-C6 alkoxy group, a phenyl group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), a heterocyclic ring which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), a phenoxy group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), a C7-C12 aralkyloxy group which may be substituted (the group may be monosubstituted or polysubstituted by the same or different groups selected from the group consisting of a halogen atom, a

cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), or a phenylthio group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group);

n in formula (1k) represents an integer of 1 to 5 and when n is an integer of 2 to 5, X may be the same or different;

$W^1$, $W^2$ and $W^3$ in formula (1k) all represent a carbon atom or represent two carbon atoms and one nitrogen atom whereinwhen any of $W^1$, $W^2$ and $W^3$ is a nitrogen atom, n cannot be 5;

m in formulae (11), (1m), and (1n) represents an integer of 1 to 3, and when m is an integer of 2 to 3, X may be the same or different;

each of $R^2$, $R^3$, $R^4$ and $R^5$ in formula (11) represents a hydrogen atom or a C1-C6 alkyl group; and

Z in formula (11) represents a methylene group or an oxygen atom, and

Q represents formula (1h), (1i), (1o), or (1p) shown below:

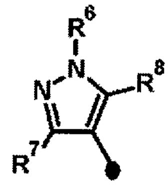 (1 h) 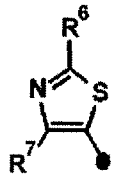 (1 i) 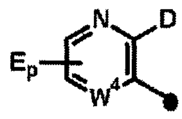 (1 o)

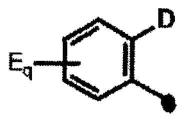 (1 p)

wherein each of $R^6$ and $R^7$ in formula (1h) or (1i) represents a hydrogen atom, a C1-C6 alkyl group, or a C1-C6 haloalkyl group, and $R^8$ in formula (1h) represents a hydrogen atom, a halogen atom, a C1-C6 alkyl group, or a C1-C6 haloalkyl group;

D in formula (1o) or (1p) represents a halogen atom, a C1-C6 alkyl group, or a C1-C6 haloalkyl group; E in formula (1o) or (1p) represents a hydrogen atom, a halogen atom, or a C1-C6 alkyl group; $W^4$ in formula (1o) represents a carbon atom or a nitrogen atom; and p represents an integer of 1 to 3 wherein when $W^4$ is a nitrogen atom, p cannot be 3, and when p is 2 to 3, E may be the same or different; and q represents an integer of 1 to 4, and when q is 2 to 4, E may be the same or different.

[2] The imide derivative according to [1], wherein

$R^1$ is formula (1a), (1b), (1c), (1d), (1i) or (1j) shown below:

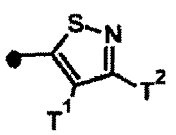 (1 a) 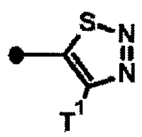 (1 b) 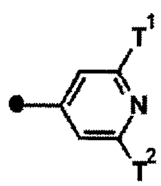 (1 c)

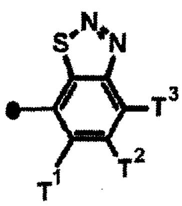 (1 d) 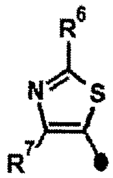 (1 i) 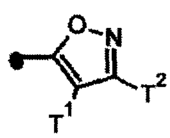 (1 j)

wherein each of $T^1$, $T^2$ and $T^3$ in formula (1a), (1b), (1c), (1d) or (1j) is a hydrogen atom, a halogen atom, a C1-C6 alkyl group, or a C1-C6 haloalkyl group wherein $T^1$, $T^2$ and $T^3$ may be the same or different,

$R^6$ in formula (1i) is a hydrogen atom or a C1-C6 alkyl group, and
$R^7$ in formula (1i) is a hydrogen atom, a C1-C6 alkyl group, or a C1-C6 haloalkyl group,
Q is formula (1h) shown below:

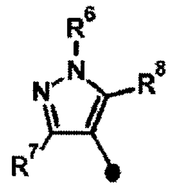
(1 h)

wherein $R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group, and
A is formula (1ka) shown below:

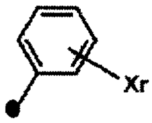
(1 k a)

wherein X in formula (1ka) is a hydrogen atom, a halogen atom, a C1-C8 alkyl group, a C1-C8 haloalkyl group, a C2-C8 alkenyl group which may be substituted (the group may be monosubstituted or polysubstituted by the same or different halogen atoms), a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkoxy C1-C6 alkyl group, a C1-C6 alkylthio C1-C6 alkyl group, a C1-C6 haloalkylthio C1-C6 alkyl group, a C1-C6 alkoxy group, a C1-C6 haloalkoxy group, a C1-C6 alkylthio group, a C1-C6 haloalkylthio group, a C3-C6 cycloalkyloxy group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkylamino group, a di C1-C6 alkylamino group (the alkyl moieties of the group may be the same or different and may also combine with each other through an alkylene group to form a 3-membered ring, a 4-membered ring, a 5-membered ring, or a 6-membered ring), a phenyl group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), or a phenoxy group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), and
r is an integer of 1 to 5 and when r is an integer of 2 to 5, X may be the same or different.

[3] The imide derivative according to [1], wherein
$R^1$ is formula (1a), (1b), (1c), (1d), (1i) or (1j) shown below:

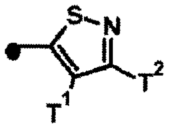
(1 a)
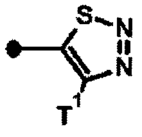
(1 b)
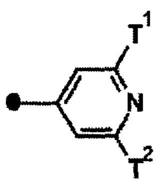
(1 c)

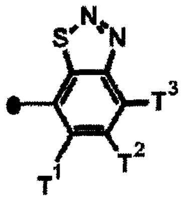
(1 d)
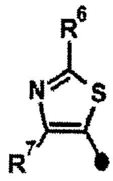
(1 i)
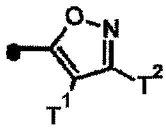
(1 j)

wherein each of $T^1$, $T^2$ and $T^3$ in formula (1a), (1b), (1c), (1d) or (1j) is a hydrogen atom, a halogen atom, a C1-C6 alkyl group, or a C1-C6 haloalkyl group wherein $T^1$, $T^2$ and $T^3$ may be the same or different,
$R^6$ in formula (1i) is a hydrogen atom or a C1-C6 alkyl group, and
$R^7$ in formula (1i) is a hydrogen atom, a C1-C6 alkyl group, or a C1-C6 haloalkyl group,
Q is formula (1h) shown below:

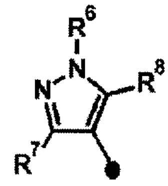
(1 h)

$R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group, and
A is formula (11) shown below:

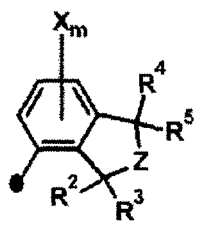
(1 l)

wherein X in formula (11) is a hydrogen atom, a halogen atom, or a C1-C6 alkyl group,
m is an integer of 1 to 3, and when m is an integer of 2 to 3, X may be the same or different,
each of $R^2$, $R^3$, $R^4$ and $R^5$ in formula (11) is a hydrogen atom or a C1-C6 alkyl group, and
Z is a methylene group or an oxygen atom.

[4] The imide derivative according to [1], wherein
$R^1$ is formula (1a), (1b), (1c), (1d), (1i) or (1j) shown below:

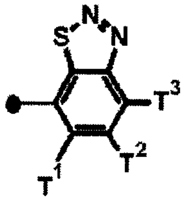
(1 d)
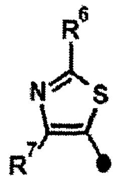
(1 i)
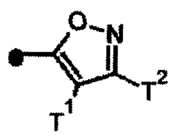
(1 j)

each of $T^1$, $T^2$ and $T^3$ in formula (1a), (1b), (1c), (1d) or (1j) is a hydrogen atom, a halogen atom, a C1-C6 alkyl group, or a C1-C6 haloalkyl group ($T^1$, $T^2$ and $T^3$ may be the same or different),
$R^6$ in formula (1i) is a hydrogen atom or a C1-C6 alkyl group, and
$R^7$ in formula (1i) is a hydrogen atom, a C1-C6 alkyl group, or a C1-C6 haloalkyl group,
Q is formula (1h) shown below:

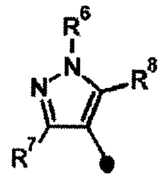

(1h)

$R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group, and
A is formula (1m) shown below:

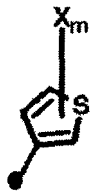

(1m)

X in formula (1m) is a hydrogen atom, a halogen atom, a C1-C8 alkyl group, a C1-C8 haloalkyl group, a C2-C8 alkenyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), or a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), and
m is an integer of 1 to 3, and when m is an integer of 2 to 3, X may be the same or different.

[5] The imide derivative according to [1], wherein
$R^1$ is formula (1a), (1b), (1c), (1d), (1i) or (1j) shown below:

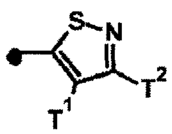 (1a) 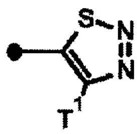 (1b) 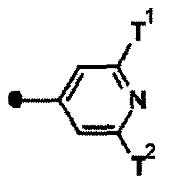 (1c)

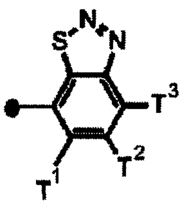 (1d) 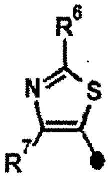 (1i) 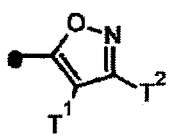 (1j)

wherein each of $T^1$, $T^2$ and $T^3$ in formula (1a), (1b), (1c), (1d) or (1j) is a hydrogen atom, a halogen atom, a C1-C6 alkyl group, or a C1-C6 haloalkyl group wherein $T^1$, $T^2$ and $T^3$ may be the same or different,
$R^6$ in formula (1i) is a hydrogen atom or a C1-C6 alkyl group, and
$R^7$ in formula (1i) is a hydrogen atom, a C1-C6 alkyl group, or a C1-C6 haloalkyl group,
Q is formula (1i) shown below:

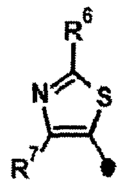 (1 i)

wherein $R^6$ in formula (1i) is a hydrogen atom or a C1-C6 alkyl group, and
$R^7$ in formula (1i) is a hydrogen atom, a C1-C6 alkyl group, or a C1-C6 haloalkyl group, and
A is formula (1ka) shown below:

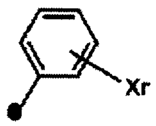 (1 k a)

wherein X in formula (1ka) is a hydrogen atom, a halogen atom, a C1-C8 alkyl group, a C1-C8 haloalkyl group, a C2-C8 alkenyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkoxy C1-C6 alkyl group, a C1-C6 alkylthio C1-C6 alkyl group, a C1-C6 haloalkylthio C1-C6 alkyl group, a C1-C6 alkoxy group, a C1-C6 haloalkoxy group, a C1-C6 alkylthio group, a C1-C6 haloalkylthio group, a C3-C6 cycloalkyloxy group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkylamino group, a di C1-C6 alkylamino group (the groups may be the same or different and may also combine with each other through an alkylene group to form a 3-membered ring, a 4-membered ring, a 5-membered ring, or a 6-membered ring), a phenyl group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group, wherein the case where Q and $R^1$ in formula (1) are formula (1i), $R^6$ in formula (1i) is a methyl group, and $R^7$ is a difluoromethyl group is excluded), or a phenoxy group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), and
r is an integer of 1 to 5 and when r is an integer of 2 to 5, X may be the same or different.

[6] The imide derivative according to [1], wherein
$R^1$ is formula (1a), (1b), (1c), (1d), (1i) or (1j) shown below:

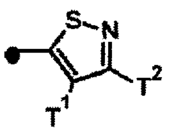 (1 a) 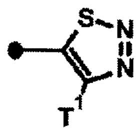 (1 b) 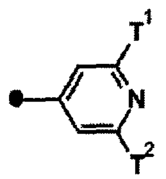 (1 c)

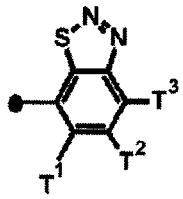 (1 d) 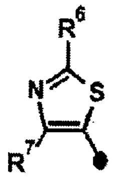 (1 i) 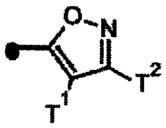 (1 j)

wherein each of $T^1$, $T^2$ and $T^3$ in formula (1a), (1b), (1c), (1d) or (1j) is a hydrogen atom, a halogen atom, a C1-C6 alkyl group, or a C1-C6 haloalkyl group wherein $T^1$, $T^2$ and $T^3$ may be the same or different,
$R^6$ in formula (1i) is a hydrogen atom or a C1-C6 alkyl group, and
$R^7$ in formula (1i) is a hydrogen atom, a C1-C6 alkyl group, or a C1-C6 haloalkyl group,
Q is formula (1i) shown below:

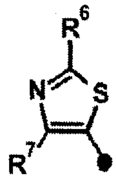 (1 i)

wherein $R^6$ in formula (1i) is a hydrogen atom or a C1-C6 alkyl group, and
$R^7$ in formula (1i) is a hydrogen atom, a C1-C6 alkyl group, or a C1-C6 haloalkyl group, and
A is formula (11) shown below:

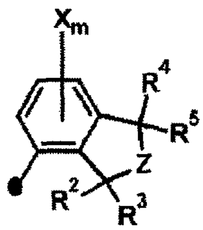 (1 l)

wherein X in formula (11) is a hydrogen atom, a halogen atom, or a C1-C6 alkyl group,
m is an integer of 1 to 3, and when m is an integer of 2 to 3, X may be the same or different,
each of $R^2$, $R^3$, $R^4$ and $R^5$ in formula (11) is a hydrogen atom or a C1-C6 alkyl group, and
Z is a methylene group or an oxygen atom.

[7] The imide derivative according to [1], wherein
$R^1$ is formula (1a), (1b), (1c), (1d), (1i) or (1j) shown below:

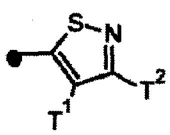 (1 a) 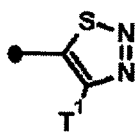 (1 b) 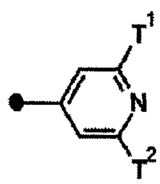 (1 c)

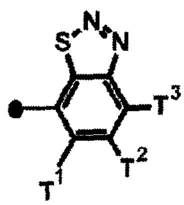 (1 d) 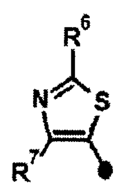 (1 i) 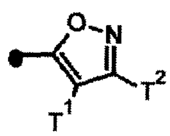 (1 j)

wherein each of $T^1$, $T^2$ and $T^3$ in formula (1a), (1b), (1c), (1d) or (1j) is a hydrogen atom, a halogen atom, a C1-C6 alkyl group, or a C1-C6 haloalkyl group wherein $T^1$, $T^2$ and $T^3$ may be the same or different,
$R^6$ in formula (1i) is a hydrogen atom or a C1-C6 alkyl group, and
$R^7$ in formula (1i) is a hydrogen atom, a C1-C6 alkyl group, or a C1-C6 haloalkyl group,
Q is formula (1i) shown below:

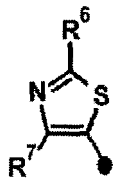 (1 i)

wherein $R^6$ in formula (1i) is a hydrogen atom or a C1-C6 alkyl group, and
$R^7$ in formula (1i) is a hydrogen atom, a C1-C6 alkyl group, or a C1-C6 haloalkyl group, and
A is formula (1m) shown below:

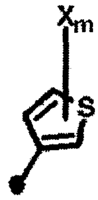 (1 m)

wherein X in formula (1m) is a hydrogen atom, a halogen atom, a C1-C8 alkyl group, a C1-C8 haloalkyl group, a C2-C8 alkenyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), or a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), and
m is an integer of 1 to 3, and when m is an integer of 2 to 3, X may be the same or different.

[8] The imide derivative according to [1], wherein
$R^1$ is formula (1a), (1b), (1c), (1d), (1i) or (1j) shown below:

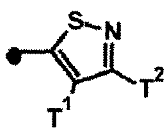 (1 a) 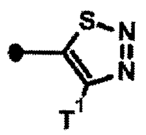 (1 b) 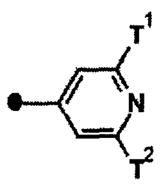 (1 c)

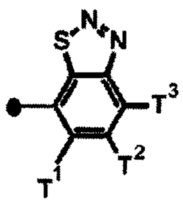 (1 d) 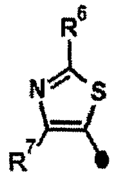 (1 i) 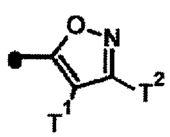 (1 j)

wherein each of $T^1$, $T^2$ and $T^3$ in formula (1a), (1b), (1c), (1d) or (1j) is a hydrogen atom, a halogen atom, a C1-C6 alkyl group, or a C1-C6 haloalkyl group wherein $T^1$, $T^2$ and $T^3$ may be the same or different,
$R^6$ in formula (1i) is a hydrogen atom or a C1-C6 alkyl group, and

$R^7$ in formula (1i) is a hydrogen atom, a C1-C6 alkyl group, or a C1-C6 haloalkyl group,
Q is formula (1o) shown below:

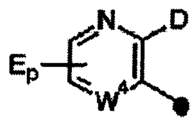
(1o)

wherein D in formula (1o) is a halogen atom, a C1-C6 alkyl group, or a C1-C6 haloalkyl group,
E in formula (1o) is a hydrogen atom, a halogen atom, or a C1-C6 alkyl group,
$W^4$ in formula (1o) is a carbon atom or a nitrogen atom, and
p is an integer of 1 to 3 wherein when $W^4$ is a nitrogen atom, p cannot be 3, and when p is 2 to 3, E may be the same or different,
A is formula (1ka) shown below:

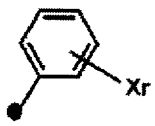
(1ka)

wherein X in formula (1ka) is a hydrogen atom, a halogen atom, a C1-C8 alkyl group, a C1-C8 haloalkyl group, a C2-C8 alkenyl group, a C3-C6 cycloalkyl group which may be substituted (group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkoxy C1-C6 alkyl group, a C1-C6 alkylthio C1-C6 alkyl group, a C1-C6 haloalkylthio C1-C6 alkyl group, a C1-C6 alkoxy group, a C1-C6 haloalkoxy group, a C1-C6 alkylthio group, a C1-C6 haloalkylthio group, a C3-C6 cycloalkyloxy group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkylamino group, a di C1-C6 alkylamino group (the groups may be the same or different and may also combine with each other through an alkylene group to form a 3-membered ring, a 4-membered ring, a 5-membered ring, or a 6-membered ring), a phenyl group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), or a phenoxy group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), and
r is an integer of 1 to 5 and when r is an integer of 2 to 5, X may be the same or different.

[9] The imide derivative according to [1], wherein
$R^1$ is formula (1a), (1b), (1c), (1d), (1i) or (1j) shown below:

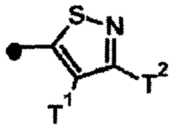
(1a)
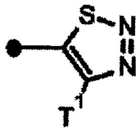
(1b)
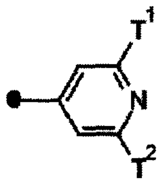
(1c)

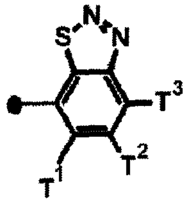 (1d) 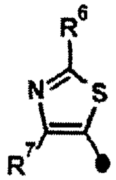 (1i) 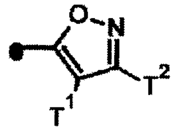 (1j)

wherein each of $T^1$, $T^2$ and $T^3$ in formula (1a), (1b), (1c), (1d) or (1j) is a hydrogen atom, a halogen atom, a C1-C6 alkyl group, or a C1-C6 haloalkyl group wherein $T^1$, $T^2$ and $T^3$ may be the same or different,
$R^6$ in formula (1i) is a hydrogen atom or a C1-C6 alkyl group, and
$R^7$ in formula (1i) is a hydrogen atom, a C1-C6 alkyl group, or a C1-C6 haloalkyl group,
Q is formula (1o) shown below:

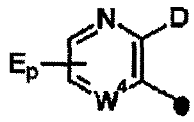 (1o)

wherein D in formula (1o) is a halogen atom, a C1-C6 alkyl group, or a C1-C6 haloalkyl group,
E in formula (1o) is a hydrogen atom, a halogen atom, or a C1-C6 alkyl group,
$W^4$ in formula (1o) is a carbon atom or a nitrogen atom, and
p is an integer of 1 to 3 wherein when $W^4$ is a nitrogen atom, p cannot be 3, and when p is 2 to 3, E may be the same or different, and
A is formula (11) shown below:

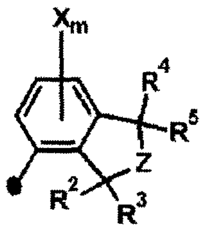 (1l)

wherein X in formula (11) is a hydrogen atom, a halogen atom, or a C1-C6 alkyl group,
m is an integer of 1 to 3, and when m is an integer of 2 to 3, X may be the same or different,
each of $R^2$, $R^3$, $R^4$ and $R^5$ in formula (11) is a hydrogen atom or a C1-C6 alkyl group, and
Z is a methylene group or an oxygen atom.

[10] The imide derivative according to [1], wherein
$R^1$ is formula (1a), (1b), (1c), (1d), (1i) or (1j) shown below:

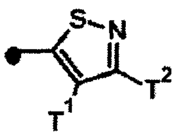 (1a) 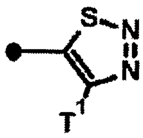 (1b) 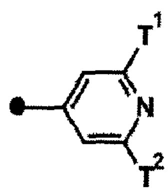 (1c)

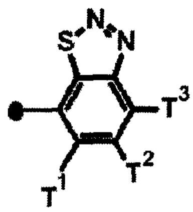 (1d) 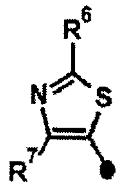 (1i) 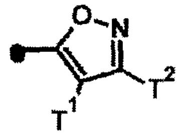 (1j)

wherein each of $T^1$, $T^2$ and $T^3$ in formula (1a), (1b), (1c), (1d) or (1j) is a hydrogen atom, a halogen atom, a C1-C6 alkyl group, or a C1-C6 haloalkyl group wherein $T^1$, $T^2$ and $T^3$ may be the same or different,
$R^6$ in formula (1i) is a hydrogen atom or a C1-C6 alkyl group, and
$R^7$ in formula (1i) is a hydrogen atom, a C1-C6 alkyl group, or a C1-C6 haloalkyl group,
Q is formula (1o) shown below:

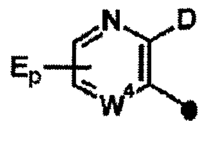 (1o)

wherein D in formula (1o) is a halogen atom, a C1-C6 alkyl group, or a C1-C6 haloalkyl group,
E in formula (1o) is a hydrogen atom, a halogen atom, or a C1-C6 alkyl group,
$W^4$ in formula (1o) is a carbon atom or a nitrogen atom, and
p is an integer of 1 to 3 wherein when $W^4$ is a nitrogen atom, p cannot be 3, and when p is 2 to 3, E may be the same or different, and
A is formula (1m) shown below:

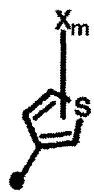 (1m)

wherein X in formula (1m) is a hydrogen atom, a halogen atom, a C1-C8 alkyl group, a C1-C8 haloalkyl group, a C2-C8 alkenyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), or a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), and
m is an integer of 1 to 3, and when m is an integer of 2 to 3, X may be the same or different.

[11] The imide derivative according to [1], wherein
$R^1$ is formula (1a), (1b), (1c), (1d), (1i) or (1j) shown below:

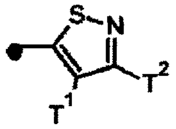 (1a) 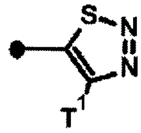 (1b) 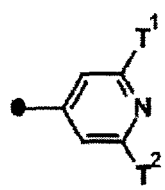 (1c)

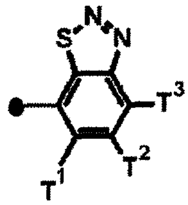 (1 d) 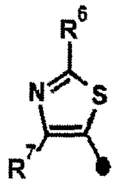 (1 i) 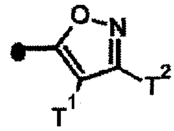 (1 j)

wherein each of $T^1$, $T^2$ and $T^3$ in formula (1a), (1b), (1c), (1d) or (1j) is a hydrogen atom, a halogen atom, a C1-C6 alkyl group, or a C1-C6 haloalkyl group wherein $T^1$, $T^2$ and $T^3$ may be the same or different,
$R^6$ in formula (1i) is a hydrogen atom or a C1-C6 alkyl group, and
$R^7$ in formula (1i) is a hydrogen atom, a C1-C6 alkyl group, or a C1-C6 haloalkyl group,
Q is formula (1p) shown below:

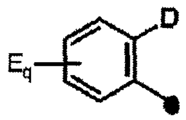 (1 p)

wherein D in formula (1p) is a halogen atom, a C1-C6 alkyl group, or a C1-C6 haloalkyl group,
E in formula (1p) is a hydrogen atom, a halogen atom, or a C1-C6 alkyl group, and
q is an integer of 1 to 4, and when q is 2 to 4, E may be the same or different, and
A is formula (1ka) shown below:

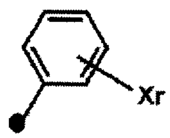 (1 k a)

wherein X in formula (1ka) is a hydrogen atom, a halogen atom, a C1-C8 alkyl group, a C1-C8 haloalkyl group, a C2-C8 alkenyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkoxy C1-C6 alkyl group, a C1-C6 alkylthio C1-C6 alkyl group, a C1-C6 haloalkylthio C1-C6 alkyl group, a C1-C6 alkoxy group, a C1-C6 haloalkoxy group, a C1-C6 alkylthio group, a C1-C6 haloalkylthio group, a C3-C6 cycloalkyloxy group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkylamino group, a di C1-C6 alkylamino group (the groups may be the same or different and may also combine with each other through an alkylene group to form a 3-membered ring, a 4-membered ring, a 5-membered ring, or a 6-membered ring), a phenyl group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), or a phenoxy group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), and
r is an integer of 1 to 5 and when r is an integer of 2 to 5, X may be the same or different.

[12] The imide derivative according to [1], wherein
$R^1$ is formula (1a), (1b), (1c), (1d), (1i) or (1j) shown below:

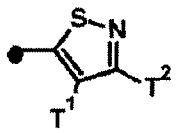 (1 a) 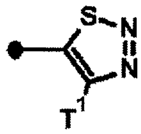 (1 b) 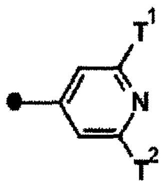 (1 c)

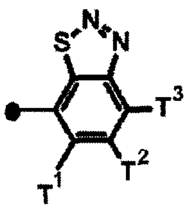 (1 d) 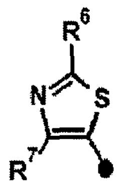 (1 i) 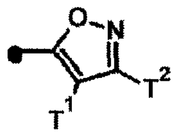 (1 j)

wherein each of $T^1$, $T^2$ and $T^3$ in formula (1a), (1b), (1c), (1d) or (1j) is a hydrogen atom, a halogen atom, a C1-C6 alkyl group, or a C1-C6 haloalkyl group wherein $T^1$, $T^2$ and $T^3$ may be the same or different,
$R^6$ in formula (1i) is a hydrogen atom or a C1-C6 alkyl group, and
$R^7$ in formula (1i) is a hydrogen atom, a C1-C6 alkyl group, or a C1-C6 haloalkyl group,
Q is formula (1p) shown below:

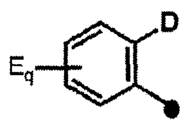 (1 p)

wherein D in formula (1p) is a halogen atom, a C1-C6 alkyl group, or a C1-C6 haloalkyl group,
E in formula (1p) is a hydrogen atom, a halogen atom, or a C1-C6 alkyl group, and
q is an integer of 1 to 4, and when q is 2 to 4, E may be the same or different, and
A is formula (11) shown below:

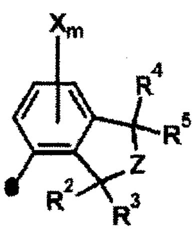 (1 l)

wherein X in formula (11) is a hydrogen atom, a halogen atom, or a C1-C6 alkyl group,
m is an integer of 1 to 3, and when m is an integer of 2 to 3, X may be the same or different,
each of $R^2$, $R^3$, $R^4$ and $R^5$ in formula (11) is a hydrogen atom or a C1-C6 alkyl group, and
Z is a methylene group or an oxygen atom.

[13] The imide derivative according to [1], wherein
$R^1$ is formula (1a), (1b), (1c), (1d), (1i) or (1j) shown below:

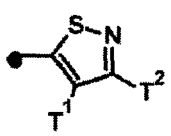 (1 a) 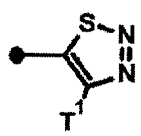 (1 b) 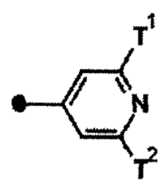 (1 c)

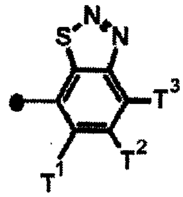 (1d) 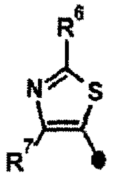 (1i) 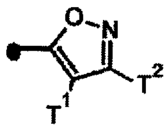 (1j)

wherein each of $T^1$, $T^2$ and $T^3$ in formula (1a), (1b), (1c), (1d) or (1j) is a hydrogen atom, a halogen atom, a C1-C6 alkyl group, or a C1-C6 haloalkyl group wherein $T^1$, $T^2$ and $T^3$ may be the same or different,
$R^6$ in formula (1i) is a hydrogen atom or a C1-C6 alkyl group, and
$R^7$ in formula (1i) is a hydrogen atom, a C1-C6 alkyl group, or a C1-C6 haloalkyl group,
Q is formula (1p) shown below:

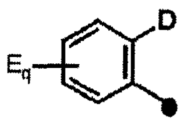 (1p)

wherein D in formula (1p) is a halogen atom, a C1-C6 alkyl group, or a C1-C6 haloalkyl group,
E in formula (1p) is a hydrogen atom, a halogen atom, or a C1-C6 alkyl group, and
q is an integer of 1 to 4, and when q is 2 to 4, E may be the same or different, and
A is formula (1m) shown below:

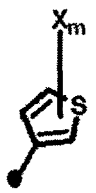 (1m)

X in formula (1m) is a hydrogen atom, a halogen atom, a C1-C8 alkyl group, a C1-C8 haloalkyl group, a C2-C8 alkenyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), or a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), and
m is an integer of 1 to 3, and when m is an integer of 2 to 3, X may be the same or different.

[14] The imide derivative according to [1], wherein
$R^1$ is formula (1e), (1f), (1g) or (1h) shown below:

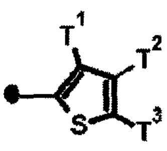 (1e) 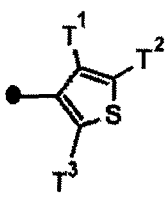 (1f) 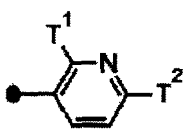 (1g)

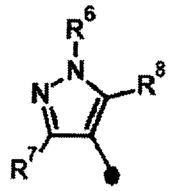

(1h)

wherein each of $T^1$, $T^2$ and $T^3$ in formula (1e), (1f) or (1g) is a hydrogen atom, a halogen atom, a C1-C6 alkyl group, or a C1-C6 haloalkyl group wherein $T^1$, $T^2$ and $T^3$ may be the same or different,
$R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group,
Q is formula (1h) shown below:

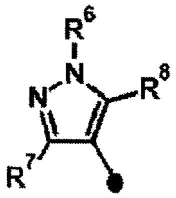

(1h)

wherein $R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group, and
A is formula (1ka) shown below:

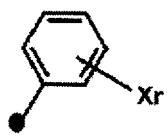

(1ka)

X in formula (1ka) is a hydrogen atom, a halogen atom, a C1-C8 alkyl group, a C1-C8 haloalkyl group, a C2-C8 alkenyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkoxy C1-C6 alkyl group, a C1-C6 alkylthio C1-C6 alkyl group, a C1-C6 haloalkylthio C1-C6 alkyl group, a C1-C6 alkoxy group, a C1-C6 haloalkoxy group, a C1-C6 alkylthio group, a C1-C6 haloalkylthio group, a C3-C6 cycloalkyloxy group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkylamino group, a di C1-C6 alkylamino group (the groups may be the same or different and may also combine with each other through an alkylene group to form a 3-membered ring, a 4-membered ring, a 5-membered ring, or a 6-membered ring), a phenyl group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), or a phenoxy group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), and
r is an integer of 1 to 5 and when r is an integer of 2 to 5, X may be the same or different.

[15] The imide derivative according to [1], wherein
$R^1$ is formula (1e), (If), (1g) or (1h) shown below:

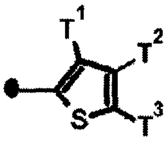 (1e) 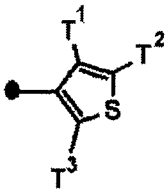 (1f) 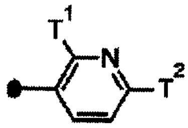 (1g)

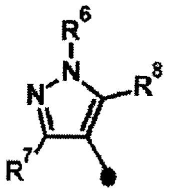 (1h)

wherein each of $T^1$, $T^2$ and $T^3$ in formula (1e), (1f) or (1g) is a hydrogen atom, a halogen atom, a C1-C6 alkyl group, or a C1-C6 haloalkyl group wherein $T^1$, $T^2$ and $T^3$ may be the same or different,
$R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group,
Q is formula (1h) shown below:

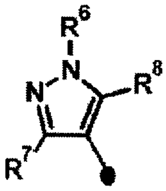 (1h)

wherein $R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group, and
A is formula (11) shown below:

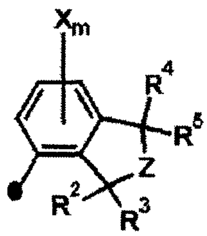 (1l)

wherein X in formula (11) is a hydrogen atom, a halogen atom, or a C1-C6 alkyl group,
m is an integer of 1 to 3, and when m is an integer of 2 to 3, X may be the same or different,
each of $R^2$, $R^3$, $R^4$ and $R^5$ in formula (11) is a hydrogen atom or a C1-C6 alkyl group, and
Z is a methylene group or an oxygen atom.

[16] The imide derivative according to [1], wherein
$R^1$ is formula (1e), (If), (1g) or (1h) shown below:

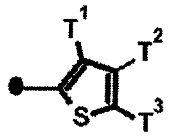 (1e) 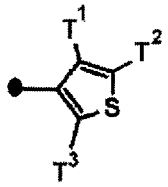 (1f) 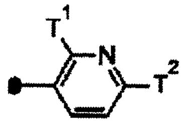 (1g)

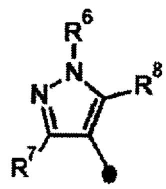 (1h)

wherein each of $T^1$, $T^2$ and $T^3$ in formula (1e), (1f) or (1g) is a hydrogen atom, a halogen atom, a C1-C6 alkyl group, or a C1-C6 haloalkyl group wherein $T^1$, $T^2$ and $T^3$ may be the same or different,
$R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group,
Q is formula (1h) shown below:

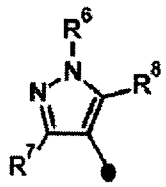 (1h)

wherein $R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group, and
A is formula (1m) shown below:

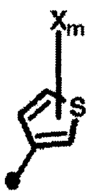 (1m)

wherein X in formula (1m) is a hydrogen atom, a halogen atom, a C1-C8 alkyl group, a C1-C8 haloalkyl group, a C2-C8 alkenyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), or a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), and
m is an integer of 1 to 3, and when m is an integer of 2 to 3, X may be the same or different.

[17] The imide derivative according to [1], wherein
$R^1$ is formula (1e), (1f), (1g) or (1h) shown below:

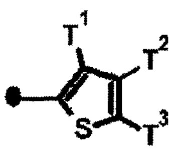 (1e) 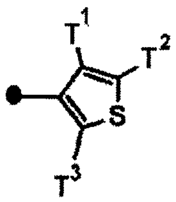 (1f) 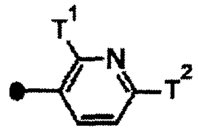 (1g)

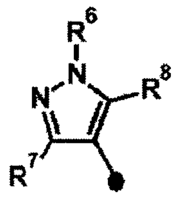 (1h)

wherein each of $T^1$, $T^2$ and $T^3$ in formula (1e), (1f) or (1g) is a hydrogen atom, a halogen atom, a C1-C6 alkyl group, or a C1-C6 haloalkyl group wherein $T^1$, $T^2$ and $T^3$ may be the same or different,
$R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group,
Q is formula (1i) shown below:

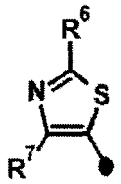 (1i)

$R^6$ in formula (1i) is a hydrogen atom or a C1-C6 alkyl group, and
$R^7$ in formula (1i) is a hydrogen atom, a C1-C6 alkyl group, or a C1-C6 haloalkyl group, and
A is formula (1ka) shown below:

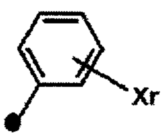 (1ka)

wherein X in formula (1ka) is a hydrogen atom, a halogen atom, a C1-C8 alkyl group, a C1-C8 haloalkyl group, a C2-C8 alkenyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkoxy C1-C6 alkyl group, a C1-C6 alkylthio C1-C6 alkyl group, a C1-C6 haloalkylthio C1-C6 alkyl group, a C1-C6 alkoxy group, a C1-C6 haloalkoxy group, a C1-C6 alkylthio group, a C1-C6 haloalkylthio group, a C3-C6 cycloalkyloxy group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkylamino group, a di C1-C6 alkylamino group (the groups may be the same or different and may also combine with each other through an alkylene group to form a 3-membered ring, a 4-membered ring, a 5-membered ring, or a 6-membered ring), a phenyl group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group, wherein the case where Q and $R^1$

in formula (1) are formula (1i), $R^6$ in formula (1i) is a methyl group, and $R^7$ is a difluoromethyl group is excluded), or a phenoxy group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), and
r is an integer of 1 to 5 and when r is an integer of 2 to 5, X may be the same or different.

[18] The imide derivative according to [1], wherein
$R^1$ is formula (1e), (1f), (1g) or (1h) shown below:

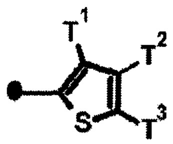
(1e)
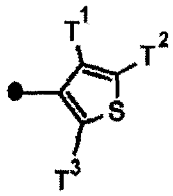
(1f)
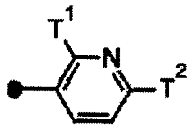
(1g)

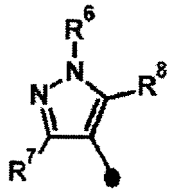
(1h)

wherein each of $T^1$, $T^2$ and $T^3$ in formula (1e), (1f) or (1g) is a hydrogen atom, a halogen atom, a C1-C6 alkyl group, or a C1-C6 haloalkyl group wherein $T^1$, $T^2$ and $T^3$ may be the same or different,
$R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group,
Q is formula (1i) shown below:

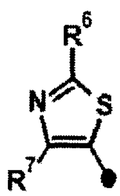
(1i)

wherein $R^6$ in formula (1i) is a hydrogen atom or a C1-C6 alkyl group, and
$R^7$ in formula (1i) is a hydrogen atom, a C1-C6 alkyl group, or a C1-C6 haloalkyl group, and
A is formula (11) shown below:

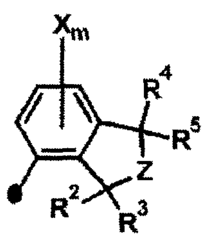
(1l)

wherein X in formula (11) is a hydrogen atom, a halogen atom, or a C1-C6 alkyl group,
m is an integer of 1 to 3, and when m is an integer of 2 to 3, X may be the same or different,
each of $R^2$, $R^3$, $R^4$ and $R^5$ in formula (11) is a hydrogen atom or a C1-C6 alkyl group, and
Z is a methylene group or an oxygen atom.

[19] The imide derivative according to [1], wherein
$R^1$ is formula (1e), (If), (1g) or (1h) shown below:

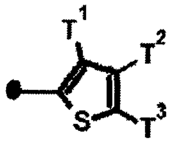 (1e) 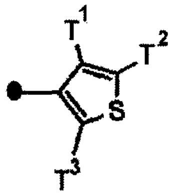 (1f) 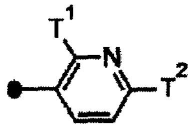 (1g)

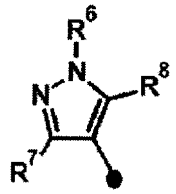 (1h)

wherein each of $T^1$, $T^2$ and $T^3$ in formula (1e), (1f) or (1g) is a hydrogen atom, a halogen atom, a C1-C6 alkyl group, or a C1-C6 haloalkyl group wherein $T^1$, $T^2$ and $T^3$ may be the same or different,
$R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group,
Q is formula (1i) shown below:

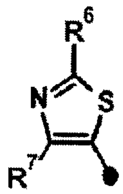 (1i)

wherein $R^6$ in formula (1i) is a hydrogen atom or a C1-C6 alkyl group, and
$R^7$ in formula (1i) is a hydrogen atom, a C1-C6 alkyl group, or a C1-C6 haloalkyl group, and
A is formula (1m) shown below:

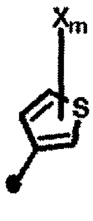 (1m)

wherein X in formula (1m) is a hydrogen atom, a halogen atom, a C1-C8 alkyl group, a C1-C8 haloalkyl group, a C2-C8 alkenyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), or a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), and
m is an integer of 1 to 3, and when m is an integer of 2 to 3, X may be the same or different.

[20] The imide derivative according to [1], wherein
$R^1$ is formula (1e), (If), (1g) or (1h) shown below:

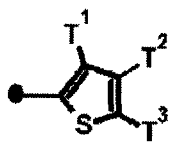 (1e) 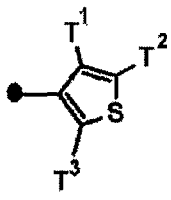 (1f) 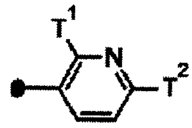 (1g)

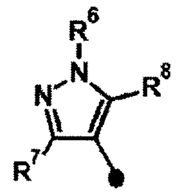 (1h)

wherein each of $T^1$, $T^2$ and $T^3$ in formula (1e), (1f) or (1g) is a hydrogen atom, a halogen atom, a C1-C6 alkyl group, or a C1-C6 haloalkyl group wherein $T^1$, $T^2$ and $T^3$ may be the same or different,
$R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group,
Q is formula (1o) shown below:

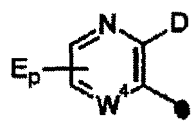 (1o)

D in formula (1o) is a halogen atom, a C1-C6 alkyl group, or a C1-C6 haloalkyl group,
E in formula (1o) is a hydrogen atom, a halogen atom, or a C1-C6 alkyl group,
$W^4$ in formula (1o) is a carbon atom or a nitrogen atom, and
p is an integer of 1 to 3 wherein when $W^4$ is a nitrogen atom, p cannot be 3, and when p is 2 to 3, E may be the same or different, and
A is formula (1ka) shown below:

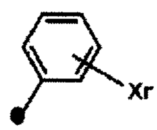 (1ka)

X in formula (1ka) is a hydrogen atom, a halogen atom, a C1-C8 alkyl group, a C1-C8 haloalkyl group, a C2-C8 alkenyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkoxy C1-C6 alkyl group, a C1-C6 alkylthio C1-C6 alkyl group, a C1-C6 haloalkylthio C1-C6 alkyl group, a C1-C6 alkoxy group, a C1-C6 haloalkoxy group, a C1-C6 alkylthio group, a C1-C6 haloalkylthio group, a C3-C6 cycloalkyloxy group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkylamino group, a di C1-C6 alkylamino group (the groups may be the same or different and may also combine with each other through an alkylene group to form a 3-membered ring, a 4-membered ring, a 5-membered ring, or a 6-membered ring), a phenyl group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), or a phenoxy group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), and
r is an integer of 1 to 5 and when r is an integer of 2 to 5, X may be the same or different.

[21] The imide derivative according to [1], wherein
$R^1$ is formula (1e), (If), (1g) or (1h) shown below:

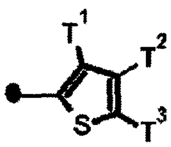
(1e)
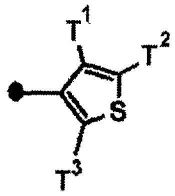
(1f)
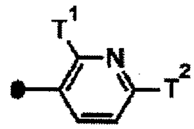
(1g)

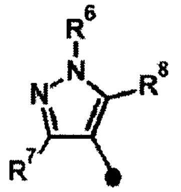
(1h)

wherein each of $T^1$, $T^2$ and $T^3$ in formula (1e), (1f) or (1g) is a hydrogen atom, a halogen atom, a C1-C6 alkyl group, or a C1-C6 haloalkyl group wherein $T^1$, $T^2$ and $T^3$ may be the same or different,
$R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group,
Q is formula (1o) shown below:

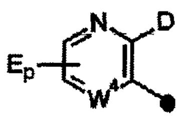
(1o)

wherein D in formula (1o) is a halogen atom, a C1-C6 alkyl group, or a C1-C6 haloalkyl group,
E in formula (1o) is a hydrogen atom, a halogen atom, or a C1-C6 alkyl group,
$W^4$ in formula (1o) is a carbon atom or a nitrogen atom, and
p is an integer of 1 to 3 wherein when $W^4$ is a nitrogen atom, p cannot be 3, and when p is 2 to 3, E may be the same or different, and
A is formula (11) shown below:

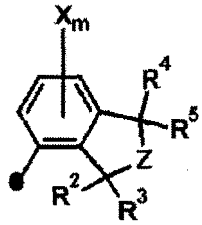
(1l)

wherein X in formula (11) is a hydrogen atom, a halogen atom, or a C1-C6 alkyl group,
m is an integer of 1 to 3, and when m is an integer of 2 to 3, X may be the same or different,
each of $R^2$, $R^3$, $R^4$ and $R^5$ in formula (11) is a hydrogen atom or a C1-C6 alkyl group, and
Z is a methylene group or an oxygen atom.

[22] The imide derivative according to [1], wherein
$R^1$ is formula (1e), (If), (1g) or (1h) shown below:

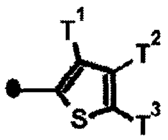 (1e) 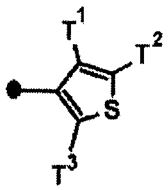 (1f) 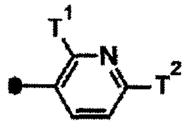 (1g)

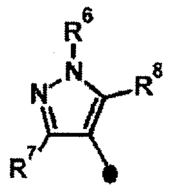 (1h)

wherein each of $T^1$, $T^2$ and $T^3$ in formula (1e), (1f) or (1g) is a hydrogen atom, a halogen atom, a C1-C6 alkyl group, or a C1-C6 haloalkyl group wherein $T^1$, $T^2$ and $T^3$ may be the same or different,
$R^6$ in formula (1h) is a C1-C6 alkyl group,
$R^7$ in formula (1h) is a hydrogen atom, a C1-C6 alkyl group, or a C1-C6 haloalkyl group,
$R^8$ in formula (1h) is a hydrogen atom, a halogen atom, a C1-C6 alkyl group, or a C1-C6 haloalkyl group,
Q is formula (1o) shown below:

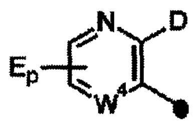 (1o)

wherein D in formula (1o) is a halogen atom, a C1-C6 alkyl group, or a C1-C6 haloalkyl group,
E in formula (1o) is a hydrogen atom, a halogen atom, or a C1-C6 alkyl group,
$W^4$ in formula (1o) is a carbon atom or a nitrogen atom, and
p is an integer of 1 to 3 wherein when $W^4$ is a nitrogen atom, p cannot be 3, and when p is 2 to 3, E may be the same or different, and
A is formula (1m) shown below:

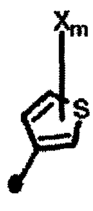 (1m)

wherein X in formula (1m) is a hydrogen atom, a halogen atom, a C1-C8 alkyl group, a C1-C8 haloalkyl group, a C2-C8 alkenyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), or a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), and
m is an integer of 1 to 3, and when m is an integer of 2 to 3, X may be the same or different.

[23] The imide derivative according to [1], wherein
$R^1$ is formula (1e), (If), (1g) or (1h) shown below:

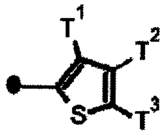 (1e) 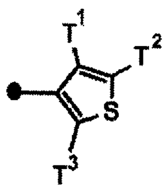 (1f) 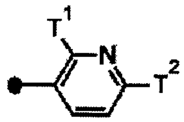 (1g)

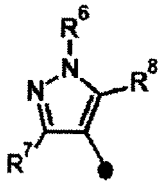 (1h)

wherein each of $T^1$, $T^2$ and $T^3$ in formula (1e), (1f) or (1g) is a hydrogen atom, a halogen atom, a C1-C6 alkyl group, or a C1-C6 haloalkyl group wherein $T^1$, $T^2$ and $T^3$ may be the same or different,
$R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group,
Q is formula (1p) shown below:

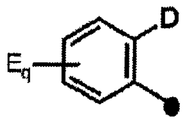 (1p)

wherein D in formula (1p) is a halogen atom, a C1-C6 alkyl group, or a C1-C6 haloalkyl group,
E in formula (1p) is a hydrogen atom, a halogen atom, or a C1-C6 alkyl group, and
q is an integer of 1 to 4, and when q is 2 to 4, E may be the same or different, and
A is formula (1ka) shown below:

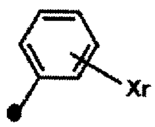 (1ka)

wherein X in formula (1ka) is a hydrogen atom, a halogen atom, a C1-C8 alkyl group, a C1-C8 haloalkyl group, a C2-C8 alkenyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkoxy C1-C6 alkyl group, a C1-C6 alkylthio C1-C6 alkyl group, a C1-C6 haloalkylthio C1-C6 alkyl group, a C1-C6 alkoxy group, a C1-C6 haloalkoxy group, a C1-C6 alkylthio group, a C1-C6 haloalkylthio group, a C3-C6 cycloalkyloxy group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkylamino group, a di C1-C6 alkylamino group (the groups may be the same or different and may also combine with each other through an alkylene group to form a 3-membered ring, a 4-membered ring, a 5-membered ring, or a 6-membered ring), a phenyl group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), or a phenoxy group which may be substituted (the group may be monosubstituted or polysubstituted by the same or different groups selected

from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), and
r is an integer of 1 to 5 and when r is an integer of 2 to 5, X may be the same or different.

[24] The imide derivative according to [1], wherein
$R^1$ is formula (1e), (If), (1g) or (1h) shown below:

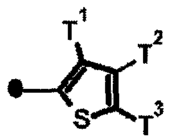
(1e)
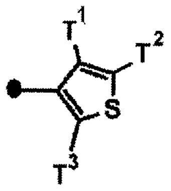
(1f)
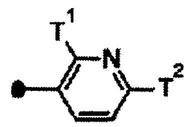
(1g)

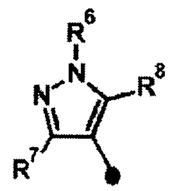
(1h)

wherein each of $T^1$, $T^2$ and $T^3$ in formula (1e), (1f) or (1g) is a hydrogen atom, a halogen atom, a C1-C6 alkyl group, or a C1-C6 haloalkyl group wherein $T^1$, $T^2$ and $T^3$ may be the same or different,
$R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group,
Q is formula (1p) shown below:

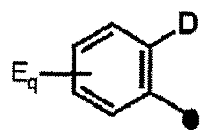
(1p)

wherein D in formula (1p) is a halogen atom, a C1-C6 alkyl group, or a C1-C6 haloalkyl group,
E in formula (1p) is a hydrogen atom, a halogen atom, or a C1-C6 alkyl group, and
q is an integer of 1 to 4, and when q is 2 to 4, E may be the same or different, and
A is formula (11) shown below:

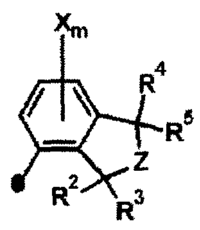
(1l)

wherein X in formula (11) is a hydrogen atom, a halogen atom, or a C1-C6 alkyl group,
m is an integer of 1 to 3, and when m is an integer of 2 to 3, X may be the same or different,
each of $R^2$, $R^3$, $R^4$ and $R^5$ in formula (11) is a hydrogen atom or a C1-C6 alkyl group, and
Z is a methylene group or an oxygen atom.

[25] The imide derivative according to [1], wherein
$R^1$ is formula (1e), (If), (1g) or (1h) shown below:

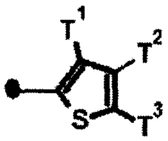 (1e) 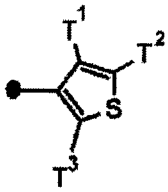 (1f) 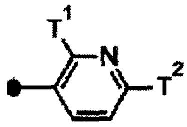 (1g)

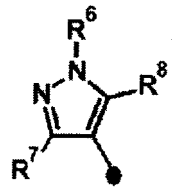 (1h)

wherein each of $T^1$, $T^2$ and $T^3$ in formula (1e), (1f) or (1g) is a hydrogen atom, a halogen atom, a C1-C6 alkyl group, or a C1-C6 haloalkyl group wherein $T^1$, $T^2$ and $T^3$ may be the same or different,
$R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group,
Q is formula (1p) shown below:

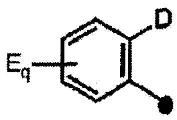 (1p)

wherein D in formula (1p) is a halogen atom, a C1-C6 alkyl group, or a C1-C6 haloalkyl group,
E in formula (1p) is a hydrogen atom, a halogen atom, or a C1-C6 alkyl group, and
q is an integer of 1 to 4, and when q is 2 to 4, E may be the same or different, and
A is formula (1m) shown below:

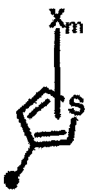 (1m)

X in formula (1m) is a hydrogen atom, a halogen atom, a C1-C8 alkyl group, a C1-C8 haloalkyl group, a C2-C8 alkenyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), or a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), and
m is an integer of 1 to 3, and when m is an integer of 2 to 3, X may be the same or different.

[26] An agricultural and horticultural fungicide containing, as an active ingredient, the imide derivative according to any one of [1] to [25].

EFFECTS OF INVENTION

The novel imide derivative of the present invention represented by formula (1) provides an excellent fungicidal effect in agricultural and horticultural situations.

DESCRIPTION OF EMBODIMENTS

The imide derivative according to the compound of the present invention, the production method thereof, and a fungicide in agricultural and horticultural situations containing the derivative as an active ingredient are specifically described.

In the imide derivative represented by formula (1) of the present invention (sometimes referred to as the imide derivative (1) of the present invention), examples of the halogen atom or the halogen atom as a substituent include fluorine, chlorine, bromine and iodine elements. The number of halogen atoms as a substituent may be 1 or may be 2 or more and in the case of 2 or more, respective halogen atoms may be the same or different. In addition, the substitution position of the halogen atom may be any position.

Examples of the halogen atom represented by $T^1$, $T^2$, $T^3$, X, $R^8$, D or E include a fluorine atom, a chlorine atom, a bromine atom, an iodine atom, *etc.,* and a fluorine atom, a chlorine atom and a bromine atom are preferred.

The C1-C6 alkyl group represented by $R^2$, $R^3$, $R^4$, $R^5$, $R^6$, $R^7$, $R^8$, $R^{11}$, $T^1$, $T^2$, $T^3$, D or E, which may be substituted, or the C1-C6 alkyl group as a substituent may be either linear or branched, and examples thereof include a methyl group, an ethyl group, a n-propyl group, an isopropyl group, a 1,1-dimethylpropyl group, a 1,2-dimethylpropyl group, a 1-ethyl-n-propyl group, a n-butyl group, an isobutyl group, a sec-butyl group, a tert-butyl group, a 1-methyl-n-butyl group, a 2-methyl-n-butyl group, a 3-methyl-n-butyl group, a 1-ethyl-n-butyl group, a 1,1-dimethyl-n-butyl group, a 1,2-dimethyl-n-butyl group, a 2,3-dimethyl-n-butyl group, a n-pentyl group, a neopentyl group, a 2-pentyl group, a tert-pentyl group, a 1-methyl-n-pentyl group, a 2-methyl-n-pentyl group, a 3-methyl-n-pentyl group, a n-hexyl group, an isohexyl group, a 2-hexyl group, a 3-hexyl group, *etc.,* and a methyl group and an ethyl group are preferred.

The C1-C16 alkyl group represented by X, which may be substituted, may be either linear or branched, and examples thereof include a methyl group, an ethyl group, a n-propyl group, an isopropyl group, a 1,1-dimethylpropyl group, a 1,2-dimethylpropyl group, a 1-ethyl-n-propyl group, a n-butyl group, an isobutyl group, a sec-butyl group, a tert-butyl group, a 1-methyl-n-butyl group, a 2-methyl-n-butyl group, a 3-methyl-n-butyl group, a 1-ethyl-n-butyl group, a 1,1-dimethyl-n-butyl group, a 1,2-dimethyl-n-butyl group, a 1,3-dimethyl-n-butyl group, a 2,3-dimethyl-n-butyl group, a 3,3-dimethyl-n-butyl group, a 1,3,3-trimethyl-n-butyl group, a 2-ethyl-2-methyl-n-butyl group, a 1-ethyl-3-methyl-n-butyl group, a 1-ethyl-3,3-dimethyl-n-butyl group, a n-pentyl group, a neopentyl group, a 2-pentyl group, a tert-pentyl group, a 1-methyl-n-pentyl group, a 2-methyl-n-pentyl group, a 3-methyl-n-pentyl group, a 4-methyl-n-pentyl group, a 1-ethyl-n-pentyl group, a 2-ethyl-n-pentyl group, a 1,3-dimethyl-n-pentyl group, a 1,4-dimethyl-n-pentyl group, a n-hexyl group, an isohexyl group, a 2-hexyl group, a 3-hexyl group, a 1-methyl-n-hexyl group, a 2-methyl-n-hexyl group, a 1-ethyl-n-hexyl group, a 2-ethyl-n-hexyl group, a 1,3-dimethyl-n-hexyl group, a n-heptyl group, a 1-methyl-n-heptyl group, a 1,3-dimethyl-n-heptyl group, a n-octyl group, a 1,3-dimethyl-n-octyl group, a n-nonyl group, a n-decyl group, a n-undecyl group, a n-dodecyl group, a n-tridecyl group, a n-tetradecyl group, a n-pentadecyl group, a n-hexadecyl group, a cyanomethyl group, a 1-cyanoethyl group, a 2-cyanoethyl group, a 3-cyano-n-propyl group, a 4-cyano-n-butyl group, a 4-cyano-n-pentyl group, a trimethylsilylmethyl group, a 2-trimethylsilylethyl group, a 1-methyl-2-trimethylsilylethyl group, a 2-cyano-1-methylethyl group, a 1,3-dimethyl-2-cyano-n-butyl group, a 1-methyl-2-cyano-n-butyl group, a 2,2-dicyano-1-methylethyl group, a 3-cyano-1-methyl-n-butyl group, a 1-cyano-3-methyl-n-butyl group, a 2-cyano-3-methyl-n-butyl group, *etc.,* and a n-butyl group, a 3-methyl-n-butyl group, a 1,3-dimethyl-n-butyl group, a 3,3-dimethyl-n-butyl group and a 1,3,3-trimethyl-n-butyl group are preferred.

The C1-C6 haloalkyl group represented by $R^6$, $R^7$, $R^8$, $T^1$, $T^2$, $T^3$ or D or the C1-C6 haloalkyl group as a substituent may be either linear or branched, and examples thereof include a monofluoromethyl group, a difluoromethyl group, a trifluoromethyl group, a 2,2,2-trifluoroethyl group, a 2-chloroethyl group, a trichloromethyl group, a 1-fluoroethyl group, a 2-fluoroethyl group, a 3,3,3-trifluoro-n-propyl group, a 4,4,4-trifluoro-n-butyl group, a 5,5,5-trifluoro-n-pentyl group, a 6-fluoro-n-hexyl group, a 6,6,6-trifluoro-n-hexyl group, *etc.,* and a difluoromethyl group and a trifluoromethyl group are preferred.

The C1-C6 haloalkyl group represented by X may be either linear or branched, and examples thereof include a monofluoromethyl group, a difluoromethyl group, a trifluoromethyl group, a 2,2,2-trifluoroethyl group, a 2-chloroethyl group, a trichloromethyl group, a 1-fluoroethyl group, a 2-fluoroethyl group, a 3,3,3-trifluoro-n-propyl group, a 4,4,4-trifluoro-n-butyl group, a 5,5,5-trifluoro-n-pentyl group, a 6-fluoro-n-hexyl group, a 6,6,6-trifluoro-n-hexyl group, a 7,7,7-trifluoro-n-heptyl group, a 2-trifluoromethyl-1-methylethyl group, a 2-fluoro-1,3-dimethyl-n-butyl group, a 2-chloro-1,3-dimethyl-n-butyl group, a 3-fluoro-1,3-dimethyl-n-butyl group, a 3-chloro-1,3-dimethyl-n-butyl group, a 2,2-difluoro-1,3-dimethyl-n-butyl group, a 2,2-dichloro-1,3-dimethyl-n-butyl group, a 3,3-difluoro-1-methyl-n-butyl group, a 3,3-dichloro-1-methyl-n-butyl group, a 2-fluoro-1-methyl-n-pentyl group, a 2-chloro-1-methyl-n-pentyl group, a 3-fluoro-1-methyl-n-pentyl group, a 3-chloro-1-methyl-n-pentyl group, a 3-trifluoromethyl-n-butyl group, a 3-trifluoromethyl-1-methyl-n-butyl group, a 3-chloro-1-methyl-n-octyl group, a 3-chloro-1-methyl-n-nonyl group, a 3-chloro-1-methyl-n-decanyl group, a 3-chloro-1-methyl-n-undecanyl group, a 3-chloro-1-methyl-n-tridecanyl group, a 3-chloro-1-methyl-n-tetradecanyl group, a 3-chloro-1-methyl-n-pentadecanyl group, a 3-chloro-1-methyl-n-hexadecanyl group, *etc.,* and a 3,3,3-trifluoro-n-propyl group, a 4,4,4-trifluoro-n-butyl group, a 2-fluoro-1,3-dimethyl-n-butyl group, a 3-fluoro-1,3-dimethyl-n-butyl group, a 3-chloro-1,3-dimethyl-n-butyl group, a 2,2-difluoro-1,3-dimethyl-n-butyl group, a 3,3-difluoro-1-methyl-n-butyl group and a 3,3-dichloro-1-methyl-n-butyl group are preferred.

The C2-C16 alkenyl group represented by X, which may be substituted, may be either linear or branched, and examples thereof include a vinyl group, a 1-propenyl group, a 2-propenyl group, a 1-methyl-1-propenyl group, a 2-methyl-1-propenyl group, a 1-methyl-2-propenyl group, a 2-methyl-2-propenyl group, a 1-ethyl-1-propenyl group, a 1-butenyl group, a 2-butenyl group, a 3-butenyl group, a 3-methyl-1-butenyl group, a 3,3-dimethyl-1-butenyl group, a 1,3-dimethyl-1-butenyl group, a 1,3-dimethyl-2-butenyl group, a 1,3,3-trimethyl-1-butenyl group, a 1-ethyl-3-methyl-1-butenyl group, a 1-ethyl-3-methyl-2-butenyl group, a 1-methyl-2-butenyl group, a 2-methyl-2-butenyl group, a 1-pentenyl group, a 2-pentenyl group, a 3-pentenyl group, a 4-pentenyl group, a 1,3-dimethyl-1-pentenyl group, a 1,3-dimethyl-2-pentenyl group, a 1,3-dimethyl-3-pentenyl group, a 1-hexenyl group, a 2-hexenyl group, a 3-hexenyl group, a 4-hexenyl group, a 5-hexenyl group, a 1-octenyl group, a 1-nonenyl group, a 1-decenyl group, a 1-undecenyl group, a 1-dodecenyl group, a 1-tridecenyl group, a 1-tetradecenyl group, a 1-pentadecenyl group, a 1-hexadecenyl group, a 4,4-dichloro-1,3-dimethyl-3-butenyl group, a 4,4-dichloro-1-methyl-3-butenyl group, *etc.,* and a 1-butenyl group, a 2-butenyl group, a 3-butenyl group, a 3-methyl-1-butenyl group, a 3,3-dimethyl-1-butenyl group, a 1,3-dimethyl-1-butenyl group, a 1,3-dimethyl-2-butenyl group, a 1,3,3-trimethyl-1-butenyl group, a 1-ethyl-3-methyl-1-butenyl group, a 1-ethyl-3-methyl-2-butenyl group, a 1-methyl-2-butenyl group and a 2-methyl-2-butenyl group are preferred.

The C2-C16 haloalkenyl group represented by X may be either linear or branched, and examples thereof include a 2,2-difluorovinyl group, a 2,2-dichlorovinyl group, a 3,3-fluoro-2-propenyl group, a 3,3-dichloro-2-propenyl group, a 3,3,3-trifluoro-1-propenyl group, a 3,3-dichloro-2-propenyl group, a 3,3-dichloro-1-methyl-3-propenyl group, a 4,4-difluoro-3-butenyl group, a 4,4-dichloro-3-butenyl group, a 4,4-dichloro-1-methyl-3-butenyl group, a 3,4,4-trifluoro-3-butenyl group, a 5,5-dichloro-4-pentenyl group, a 5,5-dichloro-1-methyl-4-pentenyl group, a 5,5-dichloro-1-methyl-4-pentenyl group, a 6,6-dichloro-1-methyl-5-hexenyl group, a 7,7-dichloro-1-methyl-6-heptenyl group, a 8,8-dichloro-1-methyl-7-octenyl group, a 9,9-dichloro-1-methyl-8-nonenyl group, a 10,10-dichloro-1-methyl-9-decenyl group, a 11,11-dichloro-1-methyl-10-undecenyl group, a 12,12-dichloro-1-methyl-11-dodecenyl group, a 13,13-dichloro-1-methyl-12-tridecenyl group, a 14,14-dichloro-1-methyl-13-tetradecenyl group, a 15,15-dichloro-1-methyl-14-pentadecenyl group, a 16,16-dichloro-1-methyl-15-hexadecenyl group, *etc.,* and a 4,4-difluoro-3-butenyl group, a 4,4-dichloro-3-butenyl group, a 4,4-dichloro-1-methyl-3-butenyl group and a 3,4,4-trifluoro-3-butenyl group are preferred.

The C2-C16 alkynyl group represented by X may be either linear or branched, and examples thereof include an ethynyl group, a 1-propynyl group, a propargyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group, a 1,1-dimethyl-2-butynyl group, a 1-pentynyl group, a 1-hexynyl group, a 1-heptynyl group, a 1-octynyl group, a 1-nonynyl group, a 1-decinyl group, a 1-undecinyl group, a 1-dodecinyl group, a 1-tridecinyl group, a 1-tetradecinyl group, a 1-pentadecinyl group, a 1-hexadecinyl group, *etc.,* and a 1-butynyl group, a 2-butynyl group, a 3-butynyl group, a 1-methyl-2-propynyl group and a 2-methyl-3-butynyl group are preferred.

The C2-C16 haloalkynyl group represented by X may be either linear or branched, and examples thereof include a 2-chloro-ethynyl group, a 3-chloro-1-propynyl group, a 3-bromo-1-propynyl group, a 3,3-difluoro-1-propynyl group, a 3,3,3-trifluoro-1-propynyl group, a 3,3,3-trichloro-1-propynyl group, a 4,4,4-trifluoro-1-butynyl group, a 4,4,4-trifluoro-2-butynyl, a 4,4,4-trichloro-1-butynyl group, a 5,5,5-trifluoro-1-pentynyl group, a 5-chloro-4-pentynyl group, a 6,6,6-trifluoro-1-hexenyl group, a 7,7,7-trifluoro-1-heptynyl group, a 8,8,8-trifluoro-1-octynyl group, a 9,9,9-trifluoro-1-nonenyl group, a 10,10,10-trifluoro-1-decinyl group, a 11,11,11-trifluoro-1-undecinyl group, a 12,12,12-trifluoro-1-dodecinyl group, a 13,13,13-trifluoro-1-tridecinyl group, a 14,14,14-trifluoro-1-tetradecinyl group, a 15,15,15-trifluoro-1-pentadecinyl group, a 16,16,16-trifluoro-1-hexadecinyl group, *etc.*

Examples of the C3-C6 cycloalkyl group represented by X, which may be substituted, include a cyclopropyl group, a 1-methylcyclopropyl group, a 2-methylcyclopropyl group, a 2,2-dimethylcyclopropyl group, a 2,2-difluorocyclopropyl group, a 2,2-dichlorocyclopropyl group, a 2-ethylcyclopropyl group, a 2-n-propylcyclopropyl group, a 2-isopropylcyclopropyl group, a 2-cyclopropylcyclopropyl group, a 2-n-butylcyclopropyl group, a 2-sec-butylcyclopropyl group, a 2-isobutylcyclopropyl group, a 2-(2,2-dimethylcyclopropyl)cyclopropyl group, a 2-(2,2-difluorocyclopropyl)cyclopropyl group, a 2-(2,2-dichlorocyclopropyl)cyclopropyl group, a cyclobutyl group, a cyclopentyl group, a cyclohexyl group, *etc.,* and a 2-ethylcyclopropyl group, a 2-n-propylcyclopropyl group, a 2-isopropylcyclopropyl group, a 2-cyclopropylcyclopropyl group, a cyclobutyl group, a cyclopentyl group and a cyclohexyl group are preferred.

The C3-C6 cycloalkyl C1-C6 alkyl group represented by X, which may be substituted, may be either linear or branched, and examples thereof include a cyclopropylmethyl group, a 1-methylcyclopropylmethyl group, a 2-methylcyclopropylmethyl group, a 1,2-dimethylcyclopropylmethyl group, a (2,2-dimethylcyclopropyl)methyl group, a (2,2-difluorocyclopropyl)methyl group, a (2,2-dichlorocyclopropyl)methyl group, a 2-cyclopropylethyl group, a 1-methyl-2-cyclopropylethyl group, a 2-cyclobutyl-1-methylethyl group, a 1-methyl-2-cyclopentylethyl group, a 2-cyclohexyl-1-methylethyl group, a 1-methyl-2-(1-methylcyclopropyl)ethyl group, a 1-methyl-2-(1-methylcyclobutyl)ethyl group, a 1-methyl-2-(1-methylcyclopentyl)ethyl group, a 1-methyl-2-(1-methylcyclohexyl)ethyl group, a cyclobutylmethyl group, a cyclopentylmethyl group, a cyclohexylmethyl group, a 3-cyclopropyl-n-propyl group, a 3-cyclobutyl-n-propyl group, a 3-cyclopentyl-n-propyl group, a 3-cyclohexyl-n-propyl group, a 1-methyl-3-cyclopropyl-n-propyl group, a 1-methyl-3-cyclobutyl-n-propyl group, a 1-methyl-3-cyclopentyl-n-propyl group, a 1-methyl-3-cyclohexyl-n-propyl group, a 1-methyl-3-(1-methyl-cyclopropyl)-n-propyl group, a 1-methyl-3-(1-methyl-cyclobutyl)-n-propyl group, a 1-methyl-3-(1-methyl-cyclopentyl)-n-propyl group, a 1-methyl-3-(1-methyl-cyclohexyl)-n-propyl group, *etc.,* and a 2-cyclopropylethyl group and a 1-methyl-2-cyclopropylethyl group are preferred.

The C1-C6 alkoxy C1-C6 alkyl group represented by X may be either linear or branched, and examples thereof include a methoxymethyl group, an ethoxymethyl group, a n-propoxymethyl group, an isopropoxymethyl group, a n-butoxymethyl group, a sec-butoxymethyl group, a tert-butoxymethyl group, a 1-pentyloxymethyl group, a 1-hexyloxymethyl group, a 2-methoxyethyl group, a 2-ethoxyethyl group, a 2-isopropoxyethyl group, a 2-isobutoxyethyl group, a 3-methoxypropyl group, a 2-methoxypropyl group, a 2-methoxy-1-methylethyl group, a 1-ethoxyethyl group, a 1-isopropoxyethyl group, *etc.*

The C1-C6 haloalkoxy C1-C6 alkyl group represented by X may be either linear or branched, and examples thereof include a trifluoromethoxymethyl group, a 2,2,2-trifluoroethoxymethyl group, a 2-(2,2,2-trifluoroethoxy)ethyl group, *etc.*

The C1-C6 alkoxy C1-C6 alkoxy C1-C6 alkyl group represented by X may be either linear or branched, and examples thereof include a methoxyethoxymethyl group, an ethoxyethoxymethyl group, a methoxyethoxyethyl group, an ethoxyethoxyethyl group, *etc.*

The C1-C6 alkylthio C1-C6 alkyl group represented by X may be either linear or branched, and examples thereof include a methylthiomethyl group, an ethylthiomethyl group, a n-propylthiomethyl group, an isopropylthiomethyl group, a 1-isopropylthioethyl group, a n-butylthiomethyl group, a sec-butylthiomethyl group, a 1-sec-butylthioethyl group, a tert-butylthiomethyl group, a n-pentylthiomethyl group, a n-hexylthiomethyl group, a 2-methylthioethyl group, a 2-ethylthioethyl group, a 2-isopropylthioethyl group, a 2-isobutylthioethyl group, a 3-methylthiopropyl group, *etc.*

The C1-C6 haloalkylthio C1-C6 alkyl group represented by X may be either linear or branched, and examples thereof include a monofluoromethylthiomethyl group, a difluoromethylthiomethyl group, a trifluoromethylthiomethyl group, a 2,2,2-trifluoroethylthiomethyl group, a 2-chloroethylthiomethyl group, a trichloromethylthiomethyl group, a 1-fluoroethylthiomethyl group, a 2-fluoroethylthiomethyl group, a 6-fluorohexylthiomethyl group, *etc.*

Examples of the C3-C6 cycloalkylthio C1-C6 alkyl group represented by X include a 1-(cyclopropylthio)ethyl group, a 1-(cyclobutylthio)ethyl group, a 1-(cyclopentylthio)ethyl group, a 1-(cyclohexylthio)ethyl group, *etc.*

The C1-C6 alkylsulfinyl C1-C6 alkyl group represented by X may be either linear or branched, and examples thereof include a methylsulfinylmethyl group, a ethylsulfinylmethyl group, a n-propylsulfinylmethyl group, a isopropylsulfinylmethyl group, a 1-isopropylsulfinylethyl group, a n-butylsulfinylmethyl group, a 1-sec-butylsulfinylethyl group, a tert-butylsulfinylmethyl group, a n-pentylsulfinylmethyl group, a n-hexylsulfinylmethyl group, a 2-methylsulfinylethyl group, a 2-ethylsulfinylethyl group, a 2-isopropylsulfinylethyl group, a 2-isobutylsulfinylethyl group, a 3-methylsulfinylpropyl group, *etc.*

The C1-C6 alkylsulfonyl C1-C6 alkyl group represented by X may be either linear or branched, and examples thereof include a methylsulfonylmethyl group, an ethylsulfonylmethyl group, a n-propylsulfonylmethyl group, an isopropylsulfonylmethyl group, a 1-isopropylsulfonylethyl group, a n-butylsulfonylmethyl group, a 1-sec-butylsulfonylethyl group, a tert-butylsulfonylmethyl group, a n-pentylsulfonylmethyl group, a n-hexylsulfonylmethyl group, a 2-methylsulfonylethyl group, a 2-ethylsulfonylethyl group, a 2-isopropylsulfonylethyl group, a 2-isobutylsulfonylethyl group, a 3-methylsulfonylpropyl group, *etc.*

The C1-C6 alkylamino C1-C6 alkyl group represented by X may be either linear or branched, and examples thereof include a methylaminomethyl group, a 1-methylaminoethyl group, a 2-methylaminoethyl group, a 1-ethylaminoethyl group, a 2-ethylaminoethyl group, a 1-n-propylaminoethyl group, a 1-isopropylaminoethyl group, a 1-n-butylaminoethyl group, , a 1-n-butylaminoethyl group, a 2-n-butylaminoethyl group, a 1-isobutylaminoethyl group, a 2-isobutylaminoethyl group, a 1-sec-butylaminoethyl group, a 2-sec-butylaminoethyl group, a 1-tert-butylaminoethyl group, a tert-butylaminoethyl group, *etc.*

The C1-C6 alkoxycarbonyl C1-C6 alkyl group represented by X may be either linear or branched, and examples thereof include a methoxycarbonylmethyl group, an ethoxycarbonylmethyl group, a n-propoxycarbonylmethyl group, an isopropoxycarbonylmethyl group, a n-butoxycarbonylmethyl group, a t-butoxycarbonylmethyl group, a n-pentyloxycarbonylmethyl group, a n-hexyloxycarbonylmethyl group, a 1-methoxycarbonylethyl group, a 1-ethoxycarbonylethyl group, a 2-methoxycarbonylethyl group, a 2-ethoxycarbonylethyl group, *etc.*

The C1-C6 alkylcarbonyl group represented by X may be either linear or branched, and examples thereof include an acetyl group, an ethylcarbonyl group, a n-propylcarbonyl group, an isopropylcarbonyl group, a n-butylcarbonyl group, a sec-butylcarbonyl group, an isopropylcarbonyl group, a tert-butylcarbonyl group, a n-pentylcarbonyl group, a n-hexylcarbonyl group, *etc.*

The C1-C6 haloalkylcarbonyl group represented by X may be either linear or branched, and examples thereof include a monofluoroacetyl group, a monochloroacetyl group, a monobromoacetyl group, a difluoroacetyl group, a trifluoroacetyl group, a trichloroacetyl group, a pentafluoroethylcarbonyl group, a heptafluoropropylcarbonyl group, a heptafluoroisopropylcarbonyl group, a 3-chloropropionylcarbonyl group, *etc.*

The C3-C6 cycloalkylcarbonyl group represented by X may be either linear or branched, and examples thereof include a cyclopropylcarbonyl group, a 1-methylcyclopropylcarbonyl group, a 1-methyl-2,2-difluorocyclopropylcarbonyl group, a 1-methyl-2,2-dichlorocyclopropylcarbonyl group, a 2,2-difluorocyclopropylcarbonyl group, a 2,2-dichlorocyclopropylcarbonyl group, a 2-methylcyclopropylcarbonyl group, a 1-trifluoromethylcyclopropylcarbonyl group, a cyclobutylcarbonyl group, a cyclopentylcarbonyl group, a 1-methylcyclopentylcarbonyl group, a cyclohexylcarbonyl group, a 1 -methylcyclohexylcarbonyl group, *etc.*

Examples of the benzenecarbonyl group represented by X, which may be substituted, include a benzoyl group, a 2-fluorophenylcarbonyl group, a 3-fluorophenylcarbonyl group, a 4-fluorophenylcarbonyl group, a 2-chlorophenylcarbonyl group, a 3-chlorophenylcarbonyl group, a 4-chlorophenylcarbonyl group, a 2,3-difluorophenylcarbonyl group, a 2,3-dichlorophenylcarbonyl group, a 2-chloro-3-fluorophenylcarbonyl group, a 3-chloro-2-fluorophenylcarbonyl group, a 2-chloro-3-methylphenylcarbonyl group, a 3-chloro-2-methylphenylcarbonyl group, a 3-fluoro-2-methylphenylcarbonyl group, a 3-chloro-2-methylphenylcarbonyl group, a 2-methylphenylcarbonyl group, a 3-methylphenylcarbonyl group, a 4-methylphenylcarbonyl group, a 2-methoxyphenylcarbonyl group, a 3-methoxyphenylcarbonyl group, a 4-methoxyphenylcarbonyl group, a 2-trifluoromethylphenylcarbonyl group, a 3-trifluoromethylphenylcarbonyl group, a 4-trifluoromethylphenylcarbonyl group, a 2-trifluoromethoxyphenylcarbonyl group, a 3-trifluoromethoxyphenylcarbonyl group, a 4-trifluoromethoxyphenylcarbonyl group, a 2-methylthiophenylcarbonyl group, a 3-methylthiophenylcarbonyl group, a 4-methylthiophenylcarbonyl group, a 2-trifluoromethylthiophenylcarbonyl group, a 3-trifluoromethylthiophenylcarbonyl group, a 4-trifluoromethylthiophenylcarbonyl group, a 2-cyanophenylcarbonyl group, a 3-cyanophenylcarbonyl group, a 4-cyanophenylcarbonyl group, a 2-nitrophenylcarbonyl group, a 3-nitrophenylcarbonyl group, a 4-nitrophenylcarbonyl group, etc.

The C1-C6 alkylaminocarbonyl group represented by X may be either linear or branched, and examples thereof include a methylaminocarbonyl group, an ethylaminocarbonyl group, a n-propylaminocarbonyl group, an isopropylaminocarbonyl group, a 1,1-dimethylpropylaminocarbonyl group, a 1,2-dimethylpropylaminocarbonyl group, a 1-ethyl-n-propylaminocarbonyl group, a n-butylaminocarbonyl group, an isobutylaminocarbonyl group, a sec-butylaminocarbonyl group, a tert-butylaminocarbonyl group, a n-pentylaminocarbonyl group, a n-hexylaminocarbonyl group, *etc.*

The di C1-C6 alkylaminocarbonyl group represented by X may be either linear or branched, and examples thereof include a dimethylaminocarbonyl group, a diethylaminocarbonyl group, a methylethylaminocarbonyl group, a methyl-n-propylaminocarbonyl group, a di-n-propylaminocarbonyl group, a di-n-butylaminocarbonyl group, a di-n-pentylaminocarbonyl group, a di-n-hexylaminocarbonyl group, a pyrrolidinocarbonyl group, a piperidinocarbonyl group, *etc.*

The C1-C6 alkylsulfonyl group represented by X may be either linear or branched, and examples thereof include a methylsulfonyl group, an ethylsulfonyl group, a n-propylsulfonyl group, an isopropylsulfonyl group, a 1,1-dimethylpropylsulfonyl group, a 1,2-dimethylpropylsulfonyl group, a 1-ethyl-n-propylsulfonyl group, a n-butylsulfonyl group, an isobutylsulfonyl group, a sec-butylsulfonyl group, a tert-butylsulfonyl group, a 1-methyl-n-butylsulfonyl group, a 2-methyl-n-butylsulfonyl group, a n-pentylsulfonyl group, a n-hexylsulfonyl group, *etc.*

The C1-C6 haloalkylsulfonyl group represented by X may be either linear or branched, and examples thereof include a trifluoromethylsulfonyl group, a pentafluoroethylsulfonyl group, a n-heptafluoropropylsulfonyl group, a n-nonafluorobutylsulfonyl group, *etc.*

The C1-C6 alkylaminosulfonyl group represented by X may be either linear or branched, and examples thereof include a methylaminosulfonyl group, an ethylaminosulfonyl group, a n-propylaminosulfonyl group, an isopropylaminosulfonyl group, a 1,1-dimethylpropylaminosulfonyl group, a 1,2-dimethylpropylaminosulfonyl group, a 1-ethyl-n-propylaminosulfonyl group, a n-butylaminosulfonyl group, an isobutylaminosulfonyl group, a sec-butylaminosulfonyl group, a tert-butylaminosulfonyl group, a n-pentylaminosulfonyl group, a n-hexylaminosulfonyl group, *etc.*

The di C1-C6 alkylaminosulfonyl group represented by X may be either linear or branched, and examples thereof include a dimethylaminosulfonyl group, a diethylaminosulfonyl group, a methylethylaminosulfonyl group, a methyl-n-propylaminosulfonyl group, a di-n-propylaminosulfonyl group, a di-n-butylaminosulfonyl group, a di-n-pentylaminosulfonyl group, a di-n-hexylaminosulfonyl group, a pyrrolidinosulfonyl group, a piperidinosulfonyl group, *etc.*

The C1-C6 alkoxycarbonyl group represented by X may be either linear or branched, and examples thereof include a methoxycarbonyl group, an ethoxycarbonyl group, a n-propoxycarbonyl group, an isopropoxycarbonyl group, a n-butoxycarbonyl group, a tert-butoxycarbonyl group, a n-pentyloxycarbonyl group, a n-hexyloxycarbonyl group, *etc.*

The C7-C12 aralkyl group represented by X, which may be substituted, may be either linear or branched, and examples thereof include a benzyl group, a 4-fluorobenzyl group, a 4-chlorobenzyl group, a 3,4-difluorobenzyl group, a 3,4,5-trifluorobenzyl group, a 1-phenethyl group, a 2-phenethyl group, a 1-phenylpropyl group, a 2-phenylpropyl group, a 3-phenylpropyl group, a 1-phenylbutyl group, a 1-phenylpentyl group, a 1-phenylhexyl group, *etc.*

The heterocyclic C1-C6 alkyl group represented by X may be either linear or branched, and examples thereof include a 3-(2-tetrahydrofuranyl)-n-propyl group, a 3-(3-tetrahydrofuranyl)-n-propyl group, a 1-methyl-3-(2-tetrahydrofuranyl)-n-propyl group, a 1-methyl-3-(3-tetrahydrofuranyl)-n-propyl group, *etc.*

The C1-C6 alkoxy group represented by X or as a substituent may be either linear or branched, and examples thereof include a methoxy group, an ethoxy group, a n-propoxy group, an isopropoxy group, a n-butoxy group, an isobutoxy group, a sec-butoxy group, a tert-butoxy group, a n-pentyloxy group, a n-hexyloxy group, *etc.,* and a n-propoxy group, an isopropoxy group, a n-butoxy group, an isobutoxy group and a sec-butoxy group are preferred.

The C1-C6 haloalkoxy group represented by X or as a substituent may be either linear or branched, and examples thereof include a monofluoromethoxy group, a difluoromethoxy group, a trifluoromethoxy group, a 2,2,2-trifluoroethoxy group, a 2-chloroethoxy group, a trichloromethoxy group, a 1-fluoroethoxy group, a 2-fluoroethoxy group, *etc.,* and a trifluoromethoxy group is preferred.

The C1-C6 alkylthio group represented by X or as a substituent may be either linear or branched, and examples thereof include a methylthio group, an ethylthio group, a n-propylthio group, an isopropylthio group, a n-butylthio group, an isobutylthio group, a sec-butylthio group, a tert-butylthio group, *etc.*

The C1-C6 haloalkylthio group represented by X or as a substituent may be either linear or branched, and examples thereof include a trifluoromethylthio group, a 2,2,2-trifluoroethylthio group, *etc.*

The C3-C6 cycloalkyloxy group represented by X, which may be substituted, may be either linear or branched, and examples thereof include a cyclopropyloxy group, a (1-methylcyclopropyl)oxy group, a (1-trifluoromethylcyclopropyl)oxy group, a (2-methylcyclopropyl)oxy group, a (2,2-dimethylcyclopropyl)oxy group, a (2,2-difluorocyclopropyl)oxy group, a (2,2-dichlorocyclopropyl)oxy group, a cyclobutyloxy group, a cyclopentyloxy group, a cyclohexyloxy group, *etc.*

The C3-C6 cycloalkyl C1-C6 alkoxy group represented by X, which may be substituted, may be either linear or branched, and examples thereof include a cyclopropylmethoxy group, a cyclobutylmethoxy group, a cyclopentylmethoxy group, a cyclohexylmethoxy group, *etc.*

The C1-C6 alkylamino group represented by X may be either linear or branched, and examples thereof include a methylamino group, an ethylamino group, an n-propylamino group, an isopropylamino group, an n-butylamino group, a sec-butylamino group, an isobutylamino group, a tert-butylamino group, an n-pentylamino group, an n-hexylamino group, etc.

Examples of the di C1-C6 alkylamino group represented by X, which may be the same or different, include a dimethylamino group, a diethylamino group, a methylethylamino group, a di n-propylamino group, a methyl n-propylamino group, a methyl n-butylamino group, a methyl sec-butylamino group, a methylisobutylamino group, a di n-butylamino group, an ethyl n-propylamino group, a di n-pentylamino group, a di n-hexylamino group, a pyrrolidino group, a piperidino group, *etc.* and a methylisobutylamino group is preferred.

The C1-C6 alkylthio C1-C6 alkoxy group represented by X may be either linear or branched, and examples thereof include a methylthiomethoxy group, an ethylthiomethoxy group, an isopropylthiomethoxy group, a 1-methylthioethoxy group, a 2-methylthioethoxy group, a 1-methylthio n-propoxy group, a 1-methylthio n-butoxy group, a 1-methylthio n-pentyloxy group, a 1-methylthio n-hexyloxy group, *etc.*

The C1-C6 alkoxy C1-C6 alkoxy group represented by X may be either linear or branched, and examples thereof include a methoxymethoxy group, a 1-methoxyethoxy group, a 2-methoxyethoxy group, a 3-methoxy n-propoxy group, a 4-methoxy n-butoxy group, a 5-methoxy n-pentyloxy group, a 6-methoxy n-hexyloxy group, a 2-ethoxy n-propoxy group, *etc.*

Examples of the phenyl group represented by X, which may be substituted, include a phenyl group, a 2-fluorophenyl group, a 3-fluorophenyl group, a 4-fluorophenyl group, a 3,4-difluorophenyl group, a 3,4,5-trifluorophenyl group, a 2-chlorophenyl group, a 3-chlorophenyl group, a 4-chlorophenyl group, a 3,4-dichlorophenyl group, a 2-methylphenyl group, a 3-methylphenyl group, a 4-methylphenyl group, a 2-methoxyphenyl group, a 3-methoxyphenyl group, a 4-methoxyphenyl group, a 2-trifluoromethylphenyl group, a 3-trifluoromethylphenyl group, a 4-trifluoromethylphenyl group, a 2-trifluoromethoxyphenyl group, a 3-trifluoromethoxyphenyl group, a 4-trifluoromethoxyphenyl group, a 2-methylthiophenyl group, a 3-methylthiophenyl group, a 4-methylthiophenyl group, a 2-trifluoromethylthiophenyl group, a 3-trifluoromethylthiophenyl group, a 4-trifluoromethylthiophenyl group, a 2-cyanophenyl group, a 3-cyanophenyl group, a 4-cyanophenyl group, a 2-nitrophenyl group, a 3-nitrophenyl group, a 4-nitrophenyl group, *etc.,* and a phenyl group, a 3,4-difluorophenyl group, a 3,4,5-trifluorophenyl group and a 4-chlorophenyl group are preferred.

Examples of the heterocyclic moiety of the heterocyclic ring represented by X, which may be substituted, include a 2-pyridyl group, a 3-pyridyl group, a 4-pyridyl group, a 2-thienyl group, a 3-thienyl group, a 2-pyrimidinyl group, a 5-pyrimidinyl group, a 2-pyrazinyl group, a 3-pyridazinyl group, a 2-thiazolyl group, a 2-oxazolyl group, a 1-pyrazolyl group, a 1-imidazolyl group, a 1,3,4-thiadiazolyl-2-yl group, a 1,3,4-oxazolyl-2-yl group, a 1,2,4-thiadiazolyl-5-yl group, a 1,2,4-oxazolyl-5-yl group, *etc.*

Examples of the phenoxy group represented by X, which may be substituted, include a phenoxy group, a 2-fluorophenoxy group, a 3-fluorophenoxy group, a 4-fluorophenoxy group, a 3,4-difluorophenoxy group, a 3,4,5-trifluorophenoxy group, a 2-chlorophenoxy group, a 3-chlorophenoxy group, a 4-chlorophenoxy group, a 2-methylphenoxy group, a 3-methylphenoxy group, a 4-methylphenoxy group, a 2-methoxyphenoxy group, a 3-methoxyphenoxy group, a 4-methoxyphenoxy group, a 2-trifluoromethylphenoxy group, a 3-trifluoromethylphenoxy group, a 4-trifluoromethylphenoxy group, a 2-trifluoromethoxyphenoxy group, a 3-trifluoromethoxyphenoxy group, a 4-trifluoromethoxyphenoxy group, a 2-methylthiophenoxy group, a 3-methylthiophenoxy group, a 4-methylthiophenoxy group, a 2-trifluoromethylthiophenoxy group, a 3-trifluoromethylthiophenoxy group, a 4-trifluoromethylthiophenoxy group, a 2-cyanophenoxy group, a 3-cyanophenoxy group, a 4-cyanophenoxy group, a 2-nitrophenoxy group, a 3-nitrophenoxy group, a 4-nitrophenoxy group, *etc.*

Examples of the C7-C12 aralkyloxy group represented by X, which may be substituted, include a benzyloxy group, a 1-phenethyloxy group, a 2-phenethyloxy group, a 1-phenylpropionyloxy group, a 2-phenylpropionyloxy group, a 3-phenylpropionyloxy group, a 1-phenylbutoxy group, a 1-phenylpentyloxy group, a 1-phenylhexyloxy group, *etc.*

Examples of the phenylthio group represented by X, which may be substituted, include a phenylthio group, a 2-fluorophenylthio group, a 3-fluorophenylthio group, a 4-fluorophenylthio group, a 3,4-difluorophenylthio group, a 3,4,5-trifluorophenylthio group, a 2-chlorophenylthio group, a 3-chlorophenylthio group, a 4-chlorophenylthio group, a 2-methylphenylthio group, a 3-methylphenylthio group, a 4-methylphenylthio group, a 2-methoxyphenylthio group, a 3-methoxyphenylthio group, a 4-methoxyphenylthio group, a 2-trifluoromethylphenylthio group, a 3-trifluoromethylphenylthio group, a 4-trifluoromethylphenylthio group, a 2-trifluoromethoxyphenylthio group, a 3-trifluoromethoxyphenylthio group, a 4-trifluoromethoxyphenylthio group, a 2-methylthiophenylthio group, a 3-methylthiophenylthio group, a 4-methylthiophenylthio group, a 2-trifluoromethylthiophenylthio group, a 3-trifluoromethylthiophenylthio group, a 4-trifluoromethylthiophenylthio group, a 2-cyanophenylthio group, a 3-cyanophenylthio group, a 4-cyanophenylthio group, a 2-nitrophenylthio group, a 3-nitrophenylthio group, a 4-nitrophenylthio group, *etc.*

In formula (1), $R^1$ represents formula (1a) to (1j).

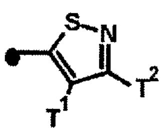 (1 a)

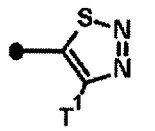 (1 b)

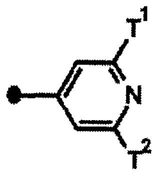 (1 c)

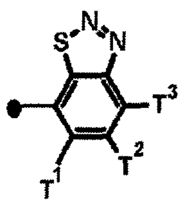 (1 d)

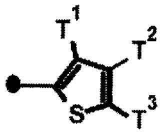 (1 e)

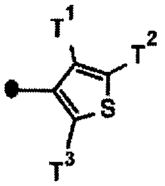 (1 f)

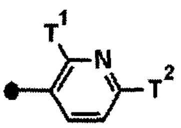 (1 g)

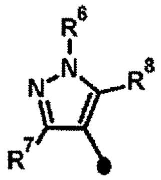 (1 h)

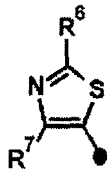 (1 i)

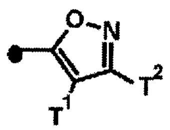 (1 j)

In formulae (1a) to (lj), each of $T^1$, $T^2$ and $T^3$ represents a hydrogen atom, a halogen atom, a C1-C6 alkyl group, or a C1-C6 haloalkyl group; $T^1$, $T^2$ and $T^3$ may be the same or different; each of $R^6$ and $R^7$ represents a hydrogen atom, a C1-C6 alkyl group, or a C1-C6 haloalkyl group; and $R^8$ represents a hydrogen atom, a halogen atom, a C1-C6 alkyl group, or a C1-C6 haloalkyl group.

In formula (1h), it is preferred that $R^6$ represents a C1-C6 alkyl group, $R^7$ represents a hydrogen atom, a C1-C6 alkyl group, or a C1-C6 haloalkyl group, and $R^8$ represents a hydrogen atom, a halogen atom, a C1-C6 alkyl group, or a C1-C6 haloalkyl group, and it is more preferred that $R^6$ represents a C1-C6 alkyl group, $R^7$ represents a C1-C6 haloalkyl group, and $R^8$ represents a halogen atom.

In formula (1i), it is preferred that $R^6$ represents a hydrogen atom or a C1-C6 alkyl group and $R^7$ represents a

hydrogen atom, a C1-C6 alkyl group, or a C1-C6 haloalkyl group, and it is more preferred that $R^6$ represents a hydrogen atom or a C1-C6 alkyl group and $R^7$ represents a C1-C6 haloalkyl group.

In formula (1), Q represents formula (1h), (1i), (1o), or (1p).

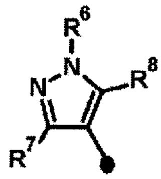 (1h) 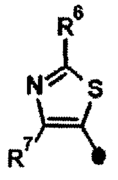 (1i) 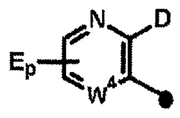 (1o)

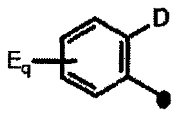 (1p)

Each of $R^6$ and $R^7$ in formula (1h) or (1i) represents a hydrogen atom, a C1-C6 alkyl group, or a C1-C6 haloalkyl group, and $R^8$ in formula (1h) represents a hydrogen atom, a halogen atom, a C1-C6 alkyl group, or a C1-C6 haloalkyl group.

D in formula (1o) or (1p) represents a halogen atom, a C1-C6 alkyl group, or a C1-C6 haloalkyl group; E in formula (1o) or (1p) represents a hydrogen atom, a halogen atom, or a C1-C6 alkyl group; $W^4$ in formula (1o) represents a carbon atom or a nitrogen atom; p represents an integer of 1 to 3 (with the proviso that when $W^4$ is a nitrogen atom, p cannot be 3), and when p is 2 to 3, E may be the same or different; and q represents an integer of 1 to 4, and when q is 2 to 4, E may be the same or different.

In formula (1h), it is preferred that $R^6$ represents a C1-C6 alkyl group or a C1-C6 haloalkyl group, $R^7$ represents a hydrogen atom, a C1-C6 alkyl group, or a C1-C6 haloalkyl group, and $R^8$ represents a hydrogen atom, a halogen atom, a C1-C6 alkyl group, or a C1-C6 haloalkyl group, and it is more preferred that $R^6$ represents a C1-C6 alkyl group, $R^7$ represents a C1-C6 alkyl group or a C1-C6 haloalkyl group, and $R^8$ represents a hydrogen atom, a halogen atom, or a C1-C6 alkyl group.

In formula (1i), it is preferred that $R^6$ represents a hydrogen atom, a C1-C6 alkyl group, or a C1-C6 haloalkyl group and $R^7$ represents a hydrogen atom, a C1-C6 alkyl group, or a C1-C6 haloalkyl group, and it is more preferred that $R^6$ represents a hydrogen atom or a C1-C6 alkyl group and $R^7$ represents a hydrogen atom or a C1-C6 haloalkyl group.

In formulae (1o) and (1p), it is preferred that D represents a halogen atom, a C1-C6 alkyl group, or a C1-C6 haloalkyl group and E represents a hydrogen atom, a halogen atom, or a C1-C6 alkyl group, and it is more preferred that D represents a halogen atom, a C1-C6 alkyl group, or a C1-C6 haloalkyl group and E represents a hydrogen atom.

In formula (1), A represents formula (1k) to (In).

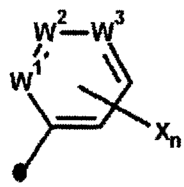 (1k) 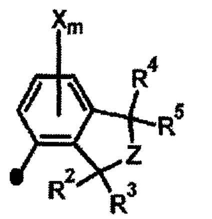 (1l) 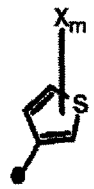 (1m)

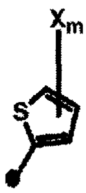 (1n)

X in formula (1k), (11), (1m), or (In) represents a hydrogen atom, a halogen atom, a C1-C16 alkyl group which may be substituted (the group may be monosubstituted or polysubstituted by the same or different groups selected from the group consisting of a C1-C6 trialkylsilyl group wherein the alkyl groups may be the same or different, and a cyano group), a C1-C16 haloalkyl group, a C2-C16 alkenyl group which may be substituted (the group may be

monosubstituted or polysubstituted by the same or different halogen atoms), a C2-C16 haloalkenyl group, a C2-C16 alkynyl group, a C2-C16 haloalkynyll group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkoxy C1-C6 alkyl group, a C1-C6 haloalkoxy C1-C6 alkyl group, a C1-C6 alkoxy C1-C6 alkoxy C1-C6 alkyl group, a C1-C6 alkylthio C1-C6 alkyl group, a C1-C6 haloalkylthio C1-C6 alkyl group, a C3-C6 cycloalkylthio C1-C6 alkyl group, a C1-C6 alkylsulfinyl C1-C6 alkyl group, a C1-C6 alkylsulfonyl C1-C6 alkyl group, a C1-C6 alkylamino C1-C6 alkyl group, a C1-C6 alkoxycarbonyl C1-C6 alkyl group, a C1-C6 alkylcarbonyl group, a C1-C6 haloalkylcarbonyl group, a C3-C6 cycloalkylcarbonyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a benzenecarbonyl group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), a C1-C6 alkylaminocarbonyl group, a di C1-C6 alkylaminocarbonyl group (the alkyl moieties of the group may be the same or different and may also combine with each other through an alkylene group to form a 3-membered ring, a 4-membered ring, a 5-membered ring, or a 6-membered ring), a C1-C6 alkylsulfonyl group, a C1-C6 haloalkylsulfonyl group, a C1-C6 alkylaminosulfonyl group, a di C1-C6 alkylaminosulfonyl group (the alkyl moieties of the group may be the same or different and may also combine with each other through an alkylene group to form a 3-membered ring, a 4-membered ring, a 5-membered ring, or a 6-membered ring), a C1-C6 alkoxycarbonyl group, a C7-C12 aralkyl group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), a heterocyclic C1-C6 alkyl group, a C1-C6 alkoxy group, a C1-C6 haloalkoxy group, a C1-C6 alkylthio group, C1-C6 haloalkylthio group, a C3-C6 cycloalkyloxy group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C3-C6 cycloalkyl C1-C6 alkoxy group, a C1-C6 alkylamino group, a di C1-C6 alkylamino group (the alkyl moieties of the group may be the same or different and may also combine with each other through an alkylene group to form a 3-membered ring, a 4-membered ring, a 5-membered ring, or a 6-membered ring), a C1-C6 alkylthio C1-C6 alkoxy group, a C1-C6 alkoxy C1-C6 alkoxy group, a phenyl group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), a heterocyclic ring which may be substituted (on the group, the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group may be monosubstituted or polysubstituted), a phenoxy group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), a C7-C12 aralkyloxy group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), or a phenylthio group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group);

n in formula (1k) represents an integer of 1 to 5 and when n is an integer of 2 to 5, X may be the same or different;

$W^1$, $W^2$ and $W^3$ in formula (1k) all represent a carbon atom or represent two carbon atoms and one nitrogen atom (when any of $W^1$, $W^2$ and $W^3$ is a nitrogen atom, n cannot be 5);

m in formulae (11), (1m), and (In) represents an integer of 1 to 3, and when m is an integer of 2 to 3, X may be the same or different;

each of $R^2$, $R^3$, $R^4$ and $R^5$ in formula (11) represents a hydrogen atom or a C1-C6 alkyl group; and

Z in formula (11) represents a methylene group or an oxygen atom.

In the case where $W^1$, $W^2$ and $W^3$ in formula (1k) all represent a carbon atom, formula (1k) is preferably represented by the following formula (1ka).

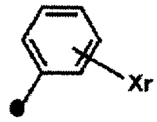
(1ka)

In formula (1ka), X represents a hydrogen atom, a halogen atom, a C1-C8 alkyl group, a C1-C8 haloalkyl group, a C2-C8 alkenyl group which may be substituted (the group may be monosubstituted or polysubstituted by the same or different halogen atoms), a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkoxy C1-C6 alkyl group, a C1-C6 alkylthio C1-C6 alkyl group, a C1-C6 haloalkylthio C1-C6 alkyl group, a C1-C6 alkoxy group, a C1-C6 haloalkoxy group, a C1-C6 alkylthio group, a C1-C6 haloalkylthio group, a C3-C6 cycloalkyloxy group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkylamino group, a di C1-C6 alkylamino group (the alkyl moieties of the group may be the same or different and may also combine with each other through an alkylene group to form a 3-membered ring, a 4-membered ring, a 5-membered ring, or a 6-membered ring), a phenyl group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), or a phenoxy group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group); and
r represents an integer of 1 to 5 and when r is an integer of 2 to 5, X may be the same or different.

X is preferably a halogen atom, a C1-C8 alkyl group, a C1-C8 haloalkyl group, a C2-C8 alkenyl group which may be substituted, a C3-C6 cycloalkyl group which may be substituted, a C3-C6 cycloalkyl C1-C6 alkyl group which may be substituted, a C1-C6 alkoxy C1-C6 alkyl group, a C1-C6 alkylthio C1-C6 alkyl group, a C3-C6 cycloalkylthio C1-C6 alkyl group, a C7-C12 aralkyl group which may be substituted, a heterocyclic C1-C6 alkyl group, a C1-C6 alkoxy group, a C1-C6 haloalkoxy group, a C3-C6 cycloalkyloxy group which may be substituted, a C3-C6 cycloalkyl C1-C6 alkoxy group, a di C1-C6 alkylamino group, a phenyl group which may be substituted, a phenoxy group which may be substituted, or a phenylthio group which may be substituted, more preferably a halogen atom, a C1-C8 alkyl group, a C3-C6 cycloalkyl group which may be substituted, a C1-C6 alkoxy group, a C1-C6 haloalkoxy group, or a phenyl group which may be substituted, and X is still more preferably a C1-C8 alkyl group, a C3-C6 cycloalkyl group which may be substituted, or a phenyl group which may be substituted.

In the case where $W^1$, $W^2$ and $W^3$ in formula (1k) represent two carbon atoms and one nitrogen atom, X in formula (1k) preferably represents a C1-C8 alkyl group or a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group).

In formula (11), X is preferably a hydrogen atom, a halogen atom, or a C1-C6 alkyl group, more preferably a hydrogen atom or a halogen atom, and still more preferably a hydrogen atom.

In formula (1m), X is preferably a hydrogen atom, a halogen atom, a C1-C8 alkyl group, a C1-C8 haloalkyl group, a C2-C8 alkenyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), or a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), more preferably a C1-C8 alkyl group or a C3-C6 cycloalkyl group which may be substituted, and still more preferably a C1-C8 alkyl group.

In formula (In), X is preferably a C1-C8 alkyl group.

Each of n in formula (1k), r in formula (1ka), and m in formulae (11), (1m) and (1n) preferably represents 1 or 2.

Although representative examples of the imide derivative represented by formula (1) are shown together in Tables 1 to 28 below, the present invention is not limited to these compounds. These compounds include compounds containing optical isomers, E forms, Z forms, and axially chiral compounds. The compound number is referred to in the later description.

In the Tables, respective notations below denote corresponding groups as follows. In addition, "H" stands for a hydrogen atom, "Me" stands for a methyl group, "Et" stands for an ethyl group, "nPr" stands for an normal-propyl group, "iPr" stands for an isopropyl group, "cPr" stands for a cyclopropyl group, "nBu" stands for an normal-butyl group, "sBu" stands for a sec-butyl group, "iBu" stands for an isobutyl group, "tBu" stands for a tert-butyl group, "cBu" stands for a cyclobutyl group, "cHex" stands for a cyclohexyl group, "nPen" stands for an normal-pentyl group, "nHex" stands for an normal-hexyl group, "nHep" stands for an normal-heptyl group, "cPen" stands for a cyclopentyl group, "Ph" stands for a phenyl group, and "Bn" stands for a benzyl group.

[Table 1-1]

Table 1.

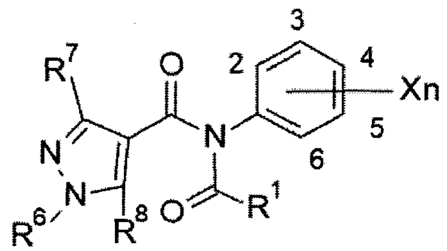

| No. | $R^1$ | $R^6$ | $R^7$ | $R^8$ | Xn |
|---|---|---|---|---|---|
| 1-1 | 3,4-$Cl_2$-isothiazol-5-yl | Me | $CF_3$ | H | 2-nBu |
| 1-2 | 3,4-$Cl_2$-isothiazol-5-yl | Me | $CF_3$ | H | 2-(3-Me-nBu) |
| 1-3 | 3,4-$Cl_2$-isothiazol-5-yl | Me | $CF_3$ | H | 2-(1,3-$Me_2$-nBu) |
| 1-4 | 3,4-$Cl_2$-isothiazol-5-yl | Me | $CF_3$ | H | 2-(3,3-$Me_2$-nBu) |
| 1-5 | 3,4-$Cl_2$-isothiazol-5-yl | Me | $CF_3$ | H | 2-(1,3,3-$Me_3$-nBu) |
| 1-6 | 3,4-$Cl_2$-isothiazol-5-yl | Me | $CHF_2$ | H | 2-Et |
| 1-7 | 3,4-$Cl_2$-isothiazol-5-yl | Me | $CHF_2$ | H | 2-nPr |
| 1-8 | 3,4-$Cl_2$-isothiazol-5-yl | Me | $CHF_2$ | H | 2-iPr |
| 1-9 | 3,4-$Cl_2$-isothiazol-5-yl | Me | $CHF_2$ | H | 2-(1-Et-nPr) |
| 1-10 | 3,4-$Cl_2$-isothiazol-5-yl | Me | $CHF_2$ | H | 2-(3-cPr-nPr) |
| 1-11 | 3,4-$Cl_2$-isothiazol-5-yl | Me | $CHF_2$ | H | 2-(3-cBu-nPr) |
| 1-12 | 3,4-$Cl_2$-isothiazol-5-yl | Me | $CHF_2$ | H | 2-(3-cPen-nPr) |
| 1-13 | 3,4-$Cl_2$-isothiazol-5-yl | Me | $CHF_2$ | H | 2-(3-cHex-nPr) |
| 1-14 | 3,4-$Cl_2$-isothiazol-5-yl | Me | $CHF_2$ | H | 2-(3 -(2-tetrahydrofuranyl)-nPr) |
| 1-15 | 3,4-$Cl_2$-isothiazol-5-yl | Me | $CHF_2$ | H | 2-(3 -(3 -tetrahvdrofuranyl)-nPr) |
| 1-16 | 3,4-$Cl_2$-isothiazol-5-yl | Me | $CHF_2$ | H | 2-(1-Me-3-cPr-nPr) |
| 1-17 | 3,4-$Cl_2$-isothiazol-5-yl | Me | $CHF_2$ | H | 2-(3-cBu-1-Me-nPr) |
| 1-18 | 3,4-$Cl_2$-isothiazol-5-yl | Me | $CHF_2$ | H | 2-(1-Me-3-cPen-nPr) |
| 1-19 | 3,4-$Cl_2$-isothiazol-5-yl | Me | $CHF_2$ | H | 2-(3-cHex-1-Me-nPr) |
| 1-20 | 3,4-$Cl_2$-isothiazol-5-yl | Me | $CHF_2$ | H | 2-(1-Me-3-(2-tetrahydrofuranyl)-nPr) |
| 1-21 | 3,4-$Cl_2$-isothiazol-5-yl | Me | $CHF_2$ | H | 2-(1-Me-3-(3-tetrahydrofuranyl)-nPr) |
| 1-22 | 3,4-$Cl_2$-isothiazol-5-yl | Me | $CHF_2$ | H | 2-[1-Me-3-(1-Me-cPr)-nPr)] |
| 1-23 | 3,4-$Cl_2$-isothiazol-5-yl | Me | $CHF_2$ | H | 2-[1-Me-3-(1-Me-cBu)-nPr)] |
| 1-24 | 3,4-$Cl_2$-isothiazol-5-yl | Me | $CHF_2$ | H | 2-[1-Me-3-(1-Me-cPen)-nPr)] |

[Table 1-2]

| No. | $R^1$ | $R^6$ | $R^7$ | $R^8$ | Xn |
|---|---|---|---|---|---|
| 1-25 | 3,4-$Cl_2$-isothiazol-5-yl | Me | $CHF_2$ | H | 2-[1-Me-3-(1-Me-cHex)-nPr)] |
| 1-26 | 3,4-$Cl_2$-isothiazol-5-yl | Me | $CHF_2$ | H | 2-(1-Me-cPr) |
| 1-27 | 3,4-$Cl_2$-isothiazol-5-yl | Me | $CHF_2$ | H | 2-(2-Me-cPr) |
| 1-28 | 3,4-$Cl_2$-isothiazol-5-yl | Me | $CHF_2$ | H | 2-(2-iPr-cPr) |
| 1-29 | 3,4-$Cl_2$-isothiazol-5-yl | Me | $CHF_2$ | H | 2-(2-cPr-cPr) |
| 1-30 | 3,4-$Cl_2$-isothiazol-5-yl | Me | $CHF_2$ | H | 2-nBu |
| 1-31 | 3,4-$Cl_2$-isothiazol-5-yl | Me | $CHF_2$ | H | 2-sBu |
| 1-32 | 3,4-$Cl_2$-isothiazol-5-yl | Me | $CHF_2$ | H | 2-(1-Me-nBu) |
| 1-33 | 3,4-$Cl_2$-isothiazol-5-yl | Me | $CHF_2$ | H | 2-(2-Me-nBu) |
| 1-34 | 3,4-$Cl_2$-isothiazol-5-yl | Me | $CHF_2$ | H | 2-(3-Me-nBu) |
| 1-35 | 3,4-$Cl_2$-isothiazol-5-yl | Me | $CHF_2$ | H | 2-(1,3-$Me_2$-nBu) |
| 1-36 | 3,4-$Cl_2$-isothiazol-5-yl | Me | $CHF_2$ | H | 3-(1,3-$Me_2$-nBu) |
| 1-37 | 3,4-$Cl_2$-isothiazol-5-yl | Me | $CHF_2$ | H | 2-(3,3-$Me_2$-nBu) |
| 1-38 | 3,4-$Cl_2$-isothiazol-5-yl | Me | $CHF_2$ | H | 2-(1,3,3-$Me_3$-nBu) |
| 1-39 | 3,4-$Cl_2$-isothiazol-5-yl | Me | $CHF_2$ | H | 2-(1-Et-3-Me-nBu) |
| 1-40 | 3,4-$Cl_2$-isothiazol-5-yl | Me | $CHF_2$ | H | 2-(1-Et-3,3-$Me_2$-nBu) |
| 1-41 | 3,4-$Cl_2$-isothiazol-5-yl | Me | $CHF_2$ | H | 2-nPen |
| 1-42 | 3,4-$Cl_2$-isothiazol-5-yl | Me | $CHF_2$ | H | 2-(1-Me-nPen) |
| 1-43 | 3,4-$Cl_2$-isothiazol-5-yl | Me | $CHF_2$ | H | 2-(1,3-$Me_2$-nPen) |
| 1-44 | 3,4-$Cl_2$-isothiazol-5-yl | Me | $CHF_2$ | H | 2-(1,4-$Me_2$-nPen) |
| 1-45 | 3,4-$Cl_2$-isothiazol-5-yl | Me | $CHF_2$ | H | 2-nHex |
| 1-46 | 3,4-$Cl_2$-isothiazol-5-yl | Me | $CHF_2$ | H | 2-(1-Me-nHex) |
| 1-47 | 3,4-$Cl_2$-isothiazol-5-yl | Me | $CHF_2$ | H | 2-(1-Me-nHep) |
| 1-48 | 3,4-$Cl_2$-isothiazol-5-yl | Me | $CHF_2$ | H | 2-(1-OEt-Et) |
| 1-49 | 3,4-$Cl_2$-isothiazol-5-yl | Me | $CHF_2$ | H | 2-(1-Me-2-OMe-Et) |
| 1-50 | 3,4-$Cl_2$-isothiazol-5-yl | Me | $CHF_2$ | H | 2-(1-OiPr-Et) |
| 1-51 | 3,4-$Cl_2$-isothiazol-5-yl | Me | $CHF_2$ | H | 2-(4,4-$Cl_2$-1,3-$Me_2$-3-butenyl) |
| 1-52 | 3,4-$Cl_2$-isothiazol-5-yl | Me | $CHF_2$ | H | 2-(4,4-$Cl_2$-1-Me-3 -butenyl) |
| 1-53 | 3,4-$Cl_2$-isothiazol-5-yl | Me | $CHF_2$ | H | 2-(1-Me-2-CN-Et) |
| 1-54 | 3,4-$Cl_2$-isothiazol-5-yl | Me | $CHF_2$ | H | 2-(2-CN-1,3-$Me_2$-nBu) |

[Table 1-3]

| No. | $R^1$ | $R^6$ | $R^7$ | $R^8$ | Xn |
|---|---|---|---|---|---|
| 1-55 | 3,4-$Cl_2$-isothiazol-5-yl | Me | $CHF_2$ | H | 2-(2-CN-1-Me-nBu) |
| 1-56 | 3,4-$Cl_2$-isothiazol-5-yl | Me | $CHF_2$ | H | 2-(2,2-$CN_2$-1-Me-Et) |
| 1-57 | 3,4-$Cl_2$-isothiazol-5-yl | Me | $CHF_2$ | H | 2-(3-CN-1-Me-nBu) |
| 1-58 | 3,4-$Cl_2$-isothiazol-5-yl | Me | $CHF_2$ | H | 2-(1-CN-3-Me-nBu) |

(continued)

| No. | $R^1$ | $R^6$ | $R^7$ | $R^8$ | Xn |
|---|---|---|---|---|---|
| 1-59 | 3,4-$Cl_2$-isothiazol-5-yl | Me | $CHF_2$ | H | 2-(1-Me-3-CN-nBu) |
| 1-60 | 3,4-$Cl_2$-isothiazol-5-yl | Me | $CHF_2$ | H | 2-(2-$CF_3$-1-Me-Et) |
| 1-61 | 3,4-$Cl_2$-isothiazol-5-yl | Me | $CHF_2$ | H | 2-Ph |
| 1-62 | 3,4-$Cl_2$-isothiazol-5-yl | Me | $CHF_2$ | H | 2-(4-F-Ph) |
| 1-63 | 3,4-$Cl_2$-isothiazol-5-yl | Me | $CHF_2$ | H | 2-(4-Cl-Ph) |
| 1-64 | 3,4-$Cl_2$-isothiazol-5-yl | Me | $CHF_2$ | H | 2-(3,4-$F_2$-Ph) |
| 1-65 | 3,4-$Cl_2$-isothiazol-5-yl | Me | $CHF_2$ | H | 2-(3,4,5-$F_3$-Ph) |
| 1-66 | 3,4-$Cl_2$-isothiazol-5-yl | Me | $CHF_2$ | H | 2-Bn |
| 1-67 | 3,4-$Cl_2$-isothiazol-5-yl | Me | $CHF_2$ | H | 2-(4-F-Bn) |
| 1-68 | 3,4-$Cl_2$-isothiazol-5-yl | Me | $CHF_2$ | H | 2-(4-Cl-Bn) |
| 1-69 | 3,4-$Cl_2$-isothiazol-5-yl | Me | $CHF_2$ | H | 2-(3,4-$F_2$-Bn) |
| 1-70 | 3,4-$Cl_2$-isothiazol-5-yl | Me | $CHF_2$ | H | 2-(3,4,5-$F_3$-Bn) |
| 1-71 | 3,4-$Cl_2$-isothiazol-5-yl | Me | $CHF_2$ | H | 2-$OCH_2$cPr |
| 1-72 | 3,4-$Cl_2$-isothiazot-5-yl | Me | $CHF_2$ | H | 2-sBuO |
| 1-73 | 3,4-$Cl_2$-isothiazol-5-yl | Me | $CHF_2$ | H | 2-iBuO |
| 1-74 | 3,4-$Cl_2$-isothiazol-5-yl | Me | $CHF_2$ | H | 2-tBuO |
| 1-75 | 3,4-$Cl_2$-isothiazol-5-yl | Me | $CHF_2$ | H | 2-cPenO |
| 1-76 | 3,4-$Cl_2$-isothiazol-5-yl | Me | $CHF_2$ | H | 2-cHexO |
| 1-77 | 3,4-$Cl_2$-isothiazol-5-yl | Me | $CHF_2$ | H | 2-PhO |
| 1-78 | 3,4-$Cl_2$-isothiazol-5-yl | Me | $CHF_2$ | H | 2-(2-Cl-PhO) |
| 1-79 | 3,4-$Cl_2$-isothiazol-5-yl | Me | $CHF_2$ | H | 2-(4-Cl-PhO) |
| 1-80 | 3,4-$Cl_2$-isothiazol-5-yl | Me | $CHF_2$ | H | 2-(3,4-$F_2$-PhO) |
| 1-81 | 3,4-$Cl_2$-isothiazol-5-yl | Me | $CHF_2$ | H | 2-(3,4,5-$F_3$-PhO) |
| 1-82 | 3,4-$Cl_2$-isothiazol-5-yl | Me | $CHF_2$ | H | 2-PhS |
| 1-83 | 3,4-$Cl_2$-isothiazol-5-yl | Me | $CHF_2$ | H | 2-(4-F-PhS) |
| 1-84 | 3,4-$Cl_2$-isothiazol-5-yl | Me | $CHF_2$ | H | 2-(4-Cl-PhS) |

[Table 1-4]

| No. | $R^1$ | $R^6$ | $R^7$ | $R^8$ | Xn |
|---|---|---|---|---|---|
| 1-85 | 3,4-$Cl_2$-isothiazol-5-yl | Me | $CHF_2$ | H | 2-(3,4-$F_2$-PhS) |
| 1-86 | 3,4-$Cl_2$-isothiazol-5-yl | Me | $CHF_2$ | H | 2-(3,4,5-$F_3$-PhS) |
| 1-87 | 3,4-$Cl_2$-isothiazol-5-yl | Me | $CHF_2$ | H | 2-$NEt_2$ |
| 1-88 | 3,4-$Cl_2$-isothiazol-5-yl | Me | $CHF_2$ | H | 2-N(Me)nBu |
| 1-89 | 3,4-$Cl_2$-isothiazol-5-yl | Me | $CHF_2$ | H | 2-N(Me)iBu |
| 1-90 | 3,4-$Cl_2$-isothiazol-5-yl | Me | $CHF_2$ | H | 2-$CH_2$S-sBu |
| 1-91 | 3,4-$Cl_2$-isothiazol-5-yl | Me | $CHF_2$ | H | 2-$CH_2$S-tBu |
| 1-92 | 3,4-$Cl_2$-isothiazol-5-yl | Me | $CHF_2$ | H | 2-CH(Me)S-iPr |

(continued)

| No. | $R^1$ | $R^6$ | $R^7$ | $R^8$ | Xn |
|---|---|---|---|---|---|
| 1-93 | 3,4-$Cl_2$-isothiazol-5-yl | Me | $CHF_2$ | H | 2-CH(Me)S-sBu |
| 1-94 | 3,4-$Cl_2$-isothiazol-5-yl | Me | $CHF_2$ | H | 2-CH(Me)S-cPen |
| 1-95 | 3,4-$Cl_2$-isothiazol-5-yl | Me | $CHF_2$ | H | 2-Cl |
| 1-96 | 3,4-$Cl_2$-isothiazol-5-yl | Me | $CHF_2$ | H | 2-Br |
| 1-97 | 3,4-$Cl_2$-isothiazol-5-yl | Me | $CHF_2$ | H | 2,3-$Me_2$ |
| 1-98 | 3,4-$Cl_2$-isothiazol-5-yl | Me | $CHF_2$ | H | 2-iPr-3-Me |
| 1-99 | 3,4-$Cl_2$-isothiazol-5-yl | Me | $CHF_2$ | H | 2-Et-3-iPr |
| 1-100 | 3,4-$Cl_2$-isothiazol-5-yl | Me | $CHF_2$ | H | 2-Et-3-cPr |
| 1-101 | 3,4-$Cl_2$-isothiazol-5-yl | Me | $CHF_2$ | H | 2-cPr-3-Et |
| 1-102 | 3,4-$Cl_2$-isothiazol-5-yl | Me | $CHF_2$ | H | 2-Me-3-cPr |
| 1-103 | 3,4-$Cl_2$-isothiazol-5-yl | Me | $CHF_2$ | H | 2-iPr-3-iPr |
| 1-104 | 3,4-$Cl_2$-isothiazol-5-yl | Me | $CHF_2$ | H | 2-nBu-3-Me |
| 1-105 | 3,4-$Cl_2$-isothiazol-5-yl | Me | $CHF_2$ | H | 3-Me-2-(1-Me-nBu) |
| 1-106 | 3,4-$Cl_2$-isothiazol-5-yl | Me | $CHF_2$ | H | 2-Me-3-(1-Me-nBu) |
| 1-107 | 3,4-$Cl_2$-isothiazol-5-yl | Me | $CHF_2$ | H | 2-(1,3-$Me_2$-nBu)-4-Me |
| 1-108 | 3,4-$Cl_2$-isothiazol-5-yl | Me | $CHF_2$ | H | 2-Et-3-tBu |
| 1-109 | 3,4-$Cl_2$-isothiazol-5-yl | Me | $CHF_2$ | H | 2-vinyl-3-tBu |
| 1-110 | 3,4-$Cl_2$-isothiazol-5-yl | Me | $CHF_2$ | H | 2-nPr-4-F |
| 1-111 | 3,4-$Cl_2$-isothiazol-5-yl | Me | $CHF_2$ | H | 2-iPr-4-F |
| 1-112 | 3,4-$Cl_2$-isothiazol-5-yl | Me | $CHF_2$ | H | 2-(2-iPr-cPr)-4-F |
| 1-113 | 3,4-$Cl_2$-isothiazol-5-yl | Me | $CHF_2$ | H | 2-(2-cPr-cPr)-4-F |
| 1-114 | 3,4-$Cl_2$-isothiazol-5-yl | Me | $CHF_2$ | H | 2-nBu-4-F |

[Table 1-5]

| No. | $R^1$ | $R^6$ | $R^7$ | $R^8$ | Xn |
|---|---|---|---|---|---|
| 1-115 | 3,4-$Cl_2$-isothiazol-5-yl | Me | $CHF_2$ | H | 2-(1,3-$Me_2$-nBu)-4-F |
| 1-116 | 3,4-$Cl_2$-isothiazol-5-yl | Me | $CHF_2$ | H | 2-sBu-4-F |
| 1-117 | 3,4-$Cl_2$-isothiazol-5-yl | Me | $CHF_2$ | H | 2-nPen-4-F |
| 1-118 | 3,4-$Cl_2$-isothiazol-5-yl | Me | $CHF_2$ | H | 2-cPen-4-F |
| 1-119 | 3,4-$Cl_2$-isothiazol-5-yl | Me | $CHF_2$ | H | 2-(3,4-$Cl_2$-Ph)-4-F |
| 1-120 | 3,4-$Cl_2$-isothiazol-5-yl | Me | $CHF_2$ | H | 3-Me-2-nPrO |
| 1-121 | 3,4-$Cl_2$-isothiazol-5-yl | Me | $CHF_2$ | H | 2-Me-3-OiPr |
| 1-122 | 3,4-$Cl_2$-isothiazol-5-yl | Me | $CHF_2$ | H | 2-Me-3-OsBu |
| 1-123 | 3,4-$Cl_2$-isothiazol-5-yl | Me | $CHF_2$ | H | 2-nBu-4-MeO |
| 1-124 | 3,4-$Cl_2$-isothiazol-5-yl | Me | $CHF_2$ | H | 2-nPrO-4-F |
| 1-125 | 3,4-$Cl_2$-isothiazol-5-yl | Me | $CHF_2$ | H | 2-nBuO-3-Cl |
| 1-126 | 3,4-$Cl_2$-isothiazol-5-yl | Me | $CHF_2$ | H | 2,6-$Br_2$-4-$OCF_3$ |

(continued)

| No. | $R^1$ | $R^6$ | $R^7$ | $R^8$ | Xn |
|---|---|---|---|---|---|
| 1-127 | 3,4-$Cl_2$-isothiazol-5-yl | Me | $CF_3$ | F | 2-(1,3-$Me_2$-nBu) |
| 1-128 | 3,4-$Cl_2$-isothiazol-5-yl | Me | $CHF_2$ | F | 2-(1,3-$Me_2$-nBu) |
| 1-129 | 3,4-$Cl_2$-isothiazol-5-yl | Me | Me | F | 2-nBu |
| 1-130 | 3,4-$Cl_2$-isothiazol-5-yl | Me | Me | F | 2-(3-Me-nBu) |
| 1-131 | 3,4-$Cl_2$-isothiazol-5-yl | Me | Me | F | 2-(1,3-$Me_2$-nBu) |
| 1-132 | 3,4-$Cl_2$-isothiazol-5-yl | Me | Me | F | 2-(3,3-$Me_2$-nBu) |
| 1-133 | 3,4-$Cl_2$-isothiazol-5-yl | Me | Me | F | 2-(1,3,3-$Me_3$-nBu) |
| 1-134 | 3,4-$Cl_2$-isothiazol-5-yl | $CH_2CF_3$ | Me | H | 2-(1,3-$Me_2$-nBu) |
| 1-135 | 3-Me-isothiazol-5-yl | Me | $CF_3$ | H | 2-(1,3-$Me_2$-nBu) |
| 1-136 | 3-Me-isothiazol-5-yl | Me | $CHF_2$ | H | 2-(1,3-$Me_2$-nBu) |
| 1-137 | 3-Me-isothiazol-5-yl | Me | $CHF_2$ | F | 2-(1,3-$Me_2$-nBu) |
| 1-138 | 3-Me-isothiazol-5-yl | Me | Me | F | 2-(1,3-$Me_2$-nBu) |
| 1-139 | 3-Me-4-Cl-isothiazol-5-yl | Me | $CF_3$ | H | 2-(1,3-$Me_2$-nBu) |
| 1-140 | 3-Me-4-Cl-isothiazol-5-yl | Me | $CHF_2$ | H | 2-(1,3-$Me_2$-nBu) |
| 1-141 | 3-Me-4-Cl-isothiazol-5-yl | Me | $CHF_2$ | F | 2-(1,3-$Me_2$-nBu) |
| 1-142 | 3-Me-4-Cl-isothiazol-5-vl | Me | Me | F | 2-(1,3-$Me_2$-nBu) |
| 1-143 | 4-Me-1,2,3-thiadiazol-5-yl | Me | $CF_3$ | H | 2-(1,3-$Me_2$-nBu) |
| 1-144 | 4-Me-1,2,3-thiadiazol-5-yl | Me | $CHF_2$ | H | 2-nBu |

[Table 1-6]

| No. | $R^1$ | $R^6$ | $R^7$ | $R^8$ | Xn |
|---|---|---|---|---|---|
| 1-145 | 4-Me-1,2,3-thiadiazol-5-yl | Me | $CHF_2$ | H | 2-(3-Me-nBu) |
| 1-146 | 4-Me-1,2,3-thiadiazol-5-yl | Me | $CHF_2$ | H | 2-(1,3-$Me_2$-nBu) |
| 1-147 | 4-Me-1,2,3-thiadiazol-5-yl | Me | $CHF_2$ | H | 3-(1,3-$Me_2$-nBu) |
| 1-148 | 4-Me-1,2,3-thiadiazol-5-yl | Me | $CHF_2$ | H | 2-(3,3-$Me_2$-nBu) |
| 1-149 | 4-Me-1,2,3-thiadiazol-5-yl | Me | $CHF_2$ | H | 2-(1,3,3-$Me_3$-nBu) |
| 1-150 | 4-Me-1,2,3-thiadiazol-5-yl | Me | $CF_3$ | F | 2-(1,3-$Me_2$-nBu) |
| 1-151 | 4-Me-1,2,3-thiadiazol-5-yl | Me | $CHF_2$ | F | 2-nBu |
| 1-152 | 4-Me-1,2,3-thiadiazol-5-yl | Me | $CHF_2$ | F | 2-(3-Me-nBu) |
| 1-153 | 4-Me-1,2,3-thiadiazol-5-yl | Me | $CHF_2$ | F | 2-(1,3-$Me_2$-nBu) |
| 1-154 | 4-Me-1,2,3-thiadiazol-5-yl | Me | $CHF_2$ | F | 2-(3,3-$Me_2$-nBu) |
| 1-155 | 4-Me-1,2,3-thiadiazol-5-yl | Me | $CHF_2$ | F | 2-(1,3,3-$Me_3$-nBu) |
| 1-156 | 4-Me-1,2,3-thiadiazol-5-yl | Me | Me | F | 2-nBu |
| 1-157 | 4-Me-1,2,3-thiadiazol-5-yl | Me | Me | F | 2-(3-Me-nBu) |
| 1-158 | 4-Me-1,2,3-thiadiazol-5-yl | Me | Me | F | 2-(1,3-$Me_2$-nBu) |
| 1-159 | 4-Me-1,2,3-thiadiazol-5 -yl | Me | Me | F | 2-(3,3-$Me_2$-nBu) |
| 1-160 | 4-Me-1,2,3-thiadiazol-5-yl | Me | Me | F | 2-(1,3,3-$Me_3$-nBu) |

(continued)

| No. | $R^1$ | $R^6$ | $R^7$ | $R^8$ | Xn |
|---|---|---|---|---|---|
| 1-161 | 4-Me-1,2,3-thiadiazol-5-yl | $CH_2CF_3$ | Me | H | 2-(1,3-$Me_2$-nBu) |
| 1-162 | 4-$CF_3$-1,2,3-thiadiazol-5-yl | Me | $CF_3$ | H | 2-nBu |
| 1-163 | 4-$CF_3$-1,2,3-thiadiazol-5-yl | Me | $CF_3$ | H | 2-(3-Me-nBu) |
| 1-164 | 4-$CF_3$-1,2,3-thiadiazol-5-yl | Me | $CF_3$ | H | 2-(1,3-$Me_2$-nBu) |
| 1-165 | 4-$CF_3$-1,2,3-thiadiazol-5-yl | Me | $CF_3$ | H | 2-(3,3-$Me_2$-nBu) |
| 1-166 | 4-$CF_3$-1,2,3-thiadiazol-5-yl | Me | $CF_3$ | H | 2-(1,3,3-$Me_3$-nBu) |
| 1-167 | 4-$CF_3$-1,2,3-thiadiazol-5-yl | Me | $CHF_2$ | H | 2-(3-cPr-nPr) |
| 1-168 | 4-$CF_3$-1,2,3-thiadiazol-5-yl | Me | $CHF_2$ | H | 2-(3-cBu-nPr) |
| 1-169 | 4-$CF_3$-1,2,3-thiadiazol-5-yl | Me | $CHF_2$ | H | 2-(3-cPen-nPr) |
| 1-170 | 4-$CF_3$-1,2,3-thiadiazol-5-yl | Me | $CHF_2$ | H | 2-(3 -(2-tetrahydrofuranyl)-nPr) |
| 1-171 | 4-$CF_3$-1,2,3-thiadiazol-5-yl | Me | $CHF_2$ | H | 2-(3-(3-tetrahydrofuranyl)-nPr) |
| 1-172 | 4-$CF_3$-1,2,3-thiadiazol-5-yl | Me | $CHF_2$ | H | 2-(1-Me-3-cPr-nPr) |
| 1-173 | 4-$CF_3$-1,2,3-thiadiazol-5-yl | Me | $CHF_2$ | H | 2-(3-cBu-1-Me-nPr) |
| 1-174 | 4-$CF_3$-1,2,3-thiadiazol-5-yl | Me | $CHF_2$ | H | 2-(1-Me-3-cPen-nPr) |

[Table 1-7]

| No. | $R^1$ | $R^6$ | $R^7$ | $R^8$ | Xn |
|---|---|---|---|---|---|
| 1-175 | 4-$CF_3$-1,2,3-thiadiazol-5-yl | Me | $CHF_2$ | H | 2-(3-cHex-1-Me-nPr) |
| 1-176 | 4-$CF_3$-1,2,3-thiadiazol-5-yl | Me | $CHF_2$ | H | 2-(1-Me-3-(2-tetrahydrofuranyl)-nPr) |
| 1-177 | 4-$CF_3$-1,2,3-thiadiazol-5-yl | Me | $CHF_2$ | H | 2-(1-Me-3-(3-tetrahydrofuranyl)-nPr) |
| 1-178 | 4-$CF_3$-1,2,3-thiadiazol-5-yl | Me | $CHF_2$ | H | 2-[1-Me-3-(1-Me-cPr)-nPr)] |
| 1-179 | 4-$CF_3$-1,2,3-thiadiazol-5-yl | Me | $CHF_2$ | H | 2-[1-Me-3-(1-Me-cBu)-nPr)] |
| 1-180 | 4-$CF_3$-1,2,3-thiadiazol-5-yl | Me | $CHF_2$ | H | 2-[1-Me-3-(1-Me-cPen)-nPr)] |
| 1-181 | 4-$CF_3$-1,2,3-thiadiazol-5-yl | Me | $CHF_2$ | H | 2-[1-Me-3-(1-Me-cHex)-nPr)] |
| 1-182 | 4-$CF_3$-1,2,3-thiadiazol-5-yl | Me | $CHF_2$ | H | 2-(2-iPr-cPr) |
| 1-183 | 4-$CF_3$-1,2,3-thiadiazol-5-yl | Me | $CHF_2$ | H | 2-(2-cPr-cPr) |
| 1-184 | 4-$CF_3$-1,2,3-thiadiazol-5-yl | Me | $CHF_2$ | H | 2-nBu |
| 1-185 | 4-$CF_3$-1,2,3-thiadiazol-5-yl | Me | $CHF_2$ | H | 2-(1-Me-nBu) |
| 1-186 | 4-$CF_3$-1,2,3-thiadiazol-5-yl | Me | $CHF_2$ | H | 2-(3-Me-nBu) |
| 1-187 | 4-$CF_3$-1,2,3-thiadiazol-5-yl | Me | $CHF_2$ | H | 2-(1,3-$Me_2$-nBu) |
| 1-188 | 4-$CF_3$-1,2,3-thiadiazol-5-yl | Me | $CHF_2$ | H | 3-(1,3-$Me_2$-nBu) |
| 1-189 | 4-$CF_3$-1,2,3-thiadiazol-5-yl | Me | $CHF_2$ | H | 2-(3,3-$Me_2$-nBu) |
| 1-190 | 4-$CF_3$-1,2,3-thiadiazol-5-yl | Me | $CHF_2$ | H | 2-(1,3,3-$Me_3$-nBu) |
| 1-191 | 4-$CF_3$-1,2,3-thiadiazol-5-yl | Me | $CHF_2$ | H | 2-(1-Et-3-Me-nBu) |
| 1-192 | 4-$CF_3$-1,2,3-thiadiazol-5-yl | Me | $CHF_2$ | H | 2-nPen |
| 1-193 | 4-$CF_3$-1,2,3-thiadiazol-5-yl | Me | $CHF_2$ | H | 2-(1-Me-nPen) |
| 1-194 | 4-$CF_3$-1,2,3-thiadiazol-5-yl | Me | $CHF_2$ | H | 2-(1,3-$Me_2$-nPen) |

(continued)

| No. | $R^1$ | $R^6$ | $R^7$ | $R^8$ | Xn |
|---|---|---|---|---|---|
| 1-195 | 4-$CF_3$-1,2,3-thiadiazol-5-yl | Me | $CHF_2$ | H | 2-(1,4-$Me_2$-nPen) |
| 1-196 | 4-$CF_3$-1,2,3-thiadiazol-5-yl | Me | $CHF_2$ | H | 2-nHex |
| 1-197 | 4-$CF_3$-1,2,3-thiadiazol-5-yl | Me | $CHF_2$ | H | 2-(3,4,5-$F_3$-Ph) |
| 1-198 | 4-$CF_3$-1,2,3-thiadiazol-5-yl | Me | $CHF_2$ | H | 2-Me-3-OiPr |
| 1-199 | 4-$CF_3$-1,2,3-thiadiazol-5-yl | Me | $CHF_2$ | H | 2-Me-3-OsecBu |
| 1-200 | 4-$CF_3$-1,2,3-thiadiazol-5-yl | Me | $CF_3$ | F | 2-(1,3-$Me_2$-nBu) |
| 1-201 | 4-$CF_3$-1,2,3-thiadiazol-5-yl | Me | $CHF_2$ | F | 2-nBu |
| 1-202 | 4-$CF_3$-1,2,3-thiadiazol-5-yl | Me | $CHF_2$ | F | 2-(3-Me-nBu) |
| 1-203 | 4-$CF_3$-1,2,3-thiadiazol-5-yl | Me | $CHF_2$ | F | 2-(1,3-$Me_2$-nBu) |
| 1-204 | 4-$CF_3$-1,2,3-thiadiazol-5-yl | Me | $CHF_2$ | F | 2-(3,3-$Me_2$-nBu) |

[Table 1-8]

| No. | $R^1$ | $R^6$ | $R^7$ | $R^8$ | Xn |
|---|---|---|---|---|---|
| 1-205 | 4-$CF_3$-1,2,3-thiadiazol-5-yl | Me | $CHF_2$ | F | 2-(1,3,3-$Me_3$-nBu) |
| 1-206 | 4-$CF_3$-1,2,3-thiadiazol-5-yl | Me | Me | F | 2-nBu |
| 1-207 | 4-$CF_3$-1,2,3-thiadiazol-5-yl | Me | Me | F | 2-(3-Me-nBu) |
| 1-208 | 4-$CF_3$-1,2,3-thiadiazol-5-yl | Me | Me | F | 2-(1,3-$Me_2$-nBu) |
| 1-209 | 4-$CF_3$-1,2,3-thiadiazol-5-yl | Me | Me | F | 2-(3,3-$Me_2$-nBu) |
| 1-210 | 4-$CF_3$-1,2,3-thiadiazol-5-yl | Me | Me | F | 2-(1,3,3-$Me_3$-nBu) |
| 1-211 | 4-$CF_3$-1,2,3-thiadiazol-5-yl | $CH_2CF_3$ | Me | H | 2-(1,3-$Me_2$-nBu) |
| 1-212 | 4-$CHF_2$-1,2,3-thiadiazol-5-yl | Me | $CF_3$ | H | 2-nBu |
| 1-213 | 4-$CHF_2$-1,2,3-thiadiazol-5-yl | Me | $CF_3$ | H | 2-(3-Me-nBu) |
| 1-214 | 4-$CHF_2$-1,2,3-thiadiazol-5-yl | Me | $CF_3$ | H | 2-(1,3-$Me_2$-nBu) |
| 1-215 | 4-$CHF_2$-1,2,3-thiadiazol-5-yl | Me | $CF_3$ | H | 2-(3,3-$Me_2$-nBu) |
| 1-216 | 4-$CHF_2$-1,2,3-thiadiazol-5-yl | Me | $CF_3$ | H | 2-(1,3,3-$Me_3$-nBu) |
| 1-217 | 4-$CHF_2$-1,2,3-thiadiazol-5-yl | Me | $CHF_2$ | H | 2-(3-cPr-nPr) |
| 1-218 | 4-$CHF_2$-1,2,3-thiadiazol-5-yl | Me | $CHF_2$ | H | 2-(3-cBu-nPr) |
| 1-219 | 4-$CHF_2$-1,2,3-thiadiazol-5-yl | Me | $CHF_2$ | H | 2-(3-cPen-nPr) |
| 1-220 | 4-$CHF_2$-1,2,3-thiadiazol-5-yl | Me | $CHF_2$ | H | 2-(3 -(2-tetrahydrofuranyl)-nPr) |
| 1-221 | 4-$CHF_2$-1,2,3-thiadiazol-5-yl | Me | $CHF_2$ | H | 2-(3 -(3 -tetrahydrofuranyl)-nPr) |
| 1-222 | 4-$CHF_2$-1,2,3-thiadiazol-5-yl | Me | $CHF_2$ | H | 2-(1-Me-3-cPr-nPr) |
| 1-223 | 4-$CHF_2$-1,2,3-thiadiazol-5-yl | Me | $CHF_2$ | H | 2-(3-cBu-1-Me-nPr) |
| 1-224 | 4-$CHF_2$-1,2,3-thiadiazol-5-yl | Me | $CHF_2$ | H | 2-(1-Me-3-cPen-nPr) |
| 1-225 | 4-$CHF_2$-1,2,3-thiadiazol-5-yl | Me | $CHF_2$ | H | 2-(3-cHex-1-Me-nPr) |
| 1-226 | 4-CHF2-1,2,3-thiadiazol-5-yl | Me | $CHF_2$ | H | 2-(1-Me-3-(2-tetrahydrofuranyl)-nPr) |
| 1-227 | 4-CH-$F_2$-1,2,3-thiadiazol-5-yl | Me | $CHF_2$ | H | 2-(1-Me-3-(3-tetrahydrofuranyl)-nPr) |
| 1-228 | 4-$CHF_2$-1,2,3-thiadiazol-5-yl | Me | $CHF_2$ | H | 2-[1-Me-3-(1-Me-cPr)-nPr)] |

(continued)

| No. | $R^1$ | $R^6$ | $R^7$ | $R^8$ | Xn |
|---|---|---|---|---|---|
| 1-229 | 4-$CHF_2$-1,2,3-thiadiazol-5-yl | Me | $CHF_2$ | H | 2-[1-Me-3-(1-Me-eBu)-nPr)] |
| 1-230 | 4-$CHF_2$-1,2,3-thiadiazol-5-yl | Me | $CHF_2$ | H | 2-[1-Me-3-(1-Me-cPen)-nPr)] |
| 1-231 | 4-$CHF_2$-1,2,3-thiadiazol-5-yl | Me | $CHF_2$ | H | 2-[1-Me-3-(1-Me-cHex)-nPr)] |
| 1-232 | 4-$CHF_2$-1,2,3-thiadiazol-5-yl | Me | $CHF_2$ | H | 2-(2-iPr-cPr) |
| 1-233 | 4-$CHF_2$-1,2,3-thiadiazol-5-yl | Me | $CHF_2$ | H | 2-(2-cPr-cPr) |
| 1-234 | 4-$CHF_2$-1,2,3-thiadiazol-5-yl | Me | $CHF_2$ | H | 2-nBu |

[Table 1-9]

| No. | $R^1$ | $R^6$ | $R^7$ | $R^8$ | Xn |
|---|---|---|---|---|---|
| 1-235 | 4-$CHF_2$-1,2,3-thiadiazol-5-yl | Me | $CHF_2$ | H | 2-(1-Me-nBu) |
| 1-236 | 4-$CHF_2$-1,2,3-thiadiazol-5-yl | Me | $CHF_2$ | H | 2-(3-Me-nBu) |
| 1-237 | 4-$CHF_2$-1,2,3-thiadiazol-5-yl | Me | $CHF_2$ | H | 2-(1,3-$Me_2$-nBu) |
| 1-238 | 4-$CHF_2$-1,2,3-thiadiazol-5-yl | Me | $CHF_2$ | H | 3-(1,3-$Me_2$-nBu) |
| 1-239 | 4-$CHF_2$-1,2,3-thiadiazol-5-yl | Me | $CHF_2$ | H | 2-(3,3-$Me_2$-nBu) |
| 1-240 | 4-$CHF_2$-1,2,3-thiadiazol-5-yl | Me | $CHF_2$ | H | 2-(1,3,3-$Me_3$-nBu) |
| 1-241 | 4-$CHF_2$-1,2,3-thiadiazol-5-yl | Me | $CHF_2$ | H | 2-(1-Et-3-Me-nBu) |
| 1-242 | 4-$CHF_2$-1,2,3-thiadiazol-5-yl | Me | $CHF_2$ | H | 2-nPen |
| 1-243 | 4-$CHF_2$-1,2,3-thiadiazol-5-yl | Me | $CHF_2$ | H | 2-(1-Me-nPen) |
| 1-244 | 4-$CHF_2$-1,2,3-thiadiazol-5-yl | Me | $CHF_2$ | H | 2-(1,3-$Me_2$-nPen) |
| 1-245 | 4-$CHF_2$-1,2,3-thiadiazol-5-yl | Me | $CHF_2$ | H | 2-(1,4-$Me_2$-nPen) |
| 1-246 | 4-$CHF_2$-1,2,3-thiadiazol-5-yl | Me | $CHF_2$ | H | 2-nHex |
| 1-247 | 4-$CHF_2$-1,2,3-thiadiazol-5-yl | Me | $CHF_2$ | H | 2-(3,4,5-$F_3$-Ph) |
| 1-248 | 4-$CHF_2$-1,2,3-thiadiazol-5-yl | Me | $CHF_2$ | H | 2-Me-3-OiPr |
| 1-249 | 4-$CHF_2$-1,2,3-thiadiazol-5-yl | Me | $CHF_2$ | H | 2-Me-3-OsBu |
| 1-250 | 4-$CHF_2$-1,2,3-thiadiazol-5-yl | Me | $CF_3$ | F | 2-(1,3-$Me_2$-nBu) |
| 1-251 | 4-$CHF_2$-1,2,3-thiadiazol-5-yl | Me | $CHF_2$ | F | 2-nBu |
| 1-252 | 4-$CHF_2$-1,2,3-thiadiazol-5-yl | Me | $CHF_2$ | F | 2-(3-Me-nBu) |
| 1-253 | 4-$CHF_2$-1,2,3-thiadiazol-5-yl | Me | $CHF_2$ | F | 2-(1,3-$Me_2$-nBu) |
| 1-254 | 4-$CHF_2$-1,2,3-thiadiazol-5-yl | Me | $CHF_2$ | F | 2-(3,3-$Me_2$-nBu) |
| 1-255 | 4-$CHF_2$-1,2,3-thiadiazol-5-yl | Me | $CHF_2$ | F | 2-(1,3,3-$Me_3$-nBu) |
| 1-256 | 4-$CHF_2$-1,2,3-thiadiazol-5-yl | Me | Me | F | 2-nBu |
| 1-257 | 4-$CHF_2$-1,2,3-thiadiazol-5-yl | Me | Me | F | 2-(3-Me-nBu) |
| 1-258 | 4-$CHF_2$-1,2,3-thiadiazol-5-yl | Me | Me | F | 2-(1,3-$Me_2$-nBu) |
| 1-259 | 4-$CHF_2$-1,2,3-thiadiazol-5-yl | Me | Me | F | 2-(3,3-$Me_2$-nBu) |
| 1-260 | 4-$CHF_2$-1,2,3-thiadiazol-5-yl | Me | Me | F | 2-(1,3,3-$Me_3$-nBu) |
| 1-261 | 4-$CHF_2$-1,2,3-thiadiazol-5-yl | $CH_2CF_3$ | Me | H | 2-(1,3-$Me_2$-nBu) |
| 1-262 | 2,6-$Cl_2$-pyridin-4-yl | Me | $CF_3$ | H | 2-(1,3-$Me_2$-nBu) |

(continued)

| No. | $R^1$ | $R^6$ | $R^7$ | $R^8$ | Xn |
|---|---|---|---|---|---|
| 1-263 | 2,6-$Cl_2$-pridin-4-yl | Me | $CHF_2$ | H | 2-(1,3-$Me_2$-nBu) |
| 1-264 | 2,6-$Cl_2$-pyridin-4-yl | Me | $CF_3$ | F | 2-(1,3-$Me_2$-nBu) |

[Table 1-10]

| No. | $R^1$ | $R^6$ | $R^7$ | $R^8$ | Xn |
|---|---|---|---|---|---|
| 1-265 | 2,6-$Cl_2$-pyridin-4-yl | Me | $CHF_2$ | F | 2-(1,3-$Me_2$-nBu) |
| 1-266 | 2,6-$Cl_2$-pyridin-4-yl | Me | Me | F | 2-(1,3-$Me_2$-nBu) |
| 1-267 | 2,6-$Cl_2$-pyridin-4-yl | $CH_2CF_3$ | Me | H | 2-(1,3-$Me_2$-nBu) |
| 1-268 | 1,2,3-benzothiadiazol-7-yl | Me | $CF_3$ | H | 2-(1,3-$Me_2$-nBu) |
| 1-269 | 1,2,3-benzothiadiazol-7-yl | Me | $CHF_2$ | H | 2-nBu |
| 1-270 | 1,2,3-benzothiadiazol-7-yl | Me | $CHF_2$ | H | 2-(3-Me-nBu) |
| 1-271 | 1,2,3-benzothiadiazol-7-yl | Me | $CHF_2$ | H | 2-(1,3-$Me_2$-nBu) |
| 1-272 | 1,2,3-benzothiadiazol-7-yl | Me | $CHF_2$ | H | 3-(1,3-$Me_2$-nBu) |
| 1-273 | 1,2,3-benzothiadiazol-7-yl | Me | $CHF_2$ | H | 2-(3,3 -$Me_2$-nBu) |
| 1-274 | 1,2,3-benzothiadiazol-7-yl | Me | $CHF_2$ | H | 2-(1,3,3-$Me_3$-nBu) |
| 1-275 | 1,2,3-benzothiadiazol-7-yl | Me | $CF_3$ | F | 2-(1,3-$Me_2$-nBu) |
| 1-276 | 1,2,3-benzothiadiazol-7-yl | Me | $CHF_2$ | F | 2-(1,3-$Me_2$-nBu) |
| 1-277 | 1,2,3-benzothiadiazol-7-yl | Me | Me | F | 2-nBu |
| 1-278 | 1,2,3-benzothiadiazol-7-yl | Me | Me | F | 2-(3-Me-nBu) |
| 1-279 | 1,2,3-benzothiadiazol-7-yl | Me | Me | F | 2-(1,3-$Me_2$-nBu) |
| 1-280 | 1,2,3-benzothiadiazol-7-yl | Me | Me | F | 2-(3,3-$Me_2$-nBu) |
| 1-281 | 1,2,3-benzothiadiazol-7-yl | Me | Me | F | 2-(1,3,3-$Me_3$-nBu) |
| 1-282 | 1,2,3-benzothiadiazol-7-yl | $CH_2CF_3$ | Me | H | 2-(1,3-$Me_2$-nBu) |
| 1-283 | 4-$CF_3$-2-Et-thiazole-5-yl | Me | $CF_3$ | H | 2-(1,3-$Me_2$-nBu) |
| 1-284 | 4-$CF_3$-2-Et-thiazole-5-yl | Me | $CHF_2$ | H | 2-(1,3-$Me_2$-nBu) |
| 1-285 | 4-$CF_3$-2-Et-thiazole-5-yl | Me | $CF_3$ | F | 2-(1,3-$Me_2$-nBu) |
| 1-286 | 4-$CF_3$-2-Et-thiazole-5-yl | Me | $CHF_2$ | F | 2-(1,3-$Me_2$-nBu) |
| 1-287 | 4-$CF_3$-2-Et-thiazole-5-yl | Me | Me | F | 2-(1,3-$Me_2$-nBu) |
| 1-288 | 4-$CHF_2$-2-Et-thiazole-5-yl | Me | $CF_3$ | H | 2-(1,3-$Me_2$-nBu) |
| 1-289 | 4-$CHF_2$-2-Et-thiazole-5-yl | Me | $CHF_2$ | H | 2-(1,3-$Me_2$-nBu) |
| 1-290 | 4-$CHF_2$-2-Et-thiazole-5-yl | Me | $CF_3$ | F | 2-(1,3-$Me_2$-nBu) |
| 1-291 | 4-$CHF_2$-2-Et-thiazole-5-yl | Me | $CHF_2$ | F | 2-(1,3-$Me_2$-nBu) |
| 1-292 | 4-$CHF_2$-2-Et-thiazole-5-yl | Me | Me | F | 2-(1,3-$Me_2$-nBu) |
| 1-293 | 4-$CF_3$-2-Me-thiazole-5-yl | Me | $CF_3$ | H | 2-nBu |
| 1-294 | 4-$CF_3$-2-Me-thiazole-5-yl | Me | $CF_3$ | H | 2-(3-Me-nBu) |

[Table 1-11]

| No. | $R^1$ | $R^6$ | $R^7$ | $R^8$ | Xn |
|---|---|---|---|---|---|
| 1-295 | 4-$CF_3$-2-Me-thiazole-5-yl | Me | $CF_3$ | H | 2-(1,3-$Me_2$-nBu) |
| 1-296 | 4-$CF_3$-2-Me-thiazole-5-yl | Me | $CF_3$ | H | 2-(3,3-$Me_2$-nBu) |
| 1-297 | 4-$CF_3$-2-Me-thiazole-5-yl | Me | $CF_3$ | H | 2-(1,3,3-$Me_3$-nBu) |
| 1-298 | 4-$CF_3$-2-Me-thiazole-5-yl | Me | $CHF_2$ | H | 2-nBu |
| 1-299 | 4-$CF_3$-2-Me-thiazole-5-yl | Me | $CHF_2$ | H | 2-(3-Me-nBu |
| 1-300 | 4-$CF_3$-2-Me-thiazole-5-yl | Me | $CHF_2$ | H | 2-(1,3-$Me_2$-nBu) |
| 1-301 | 4-$CF_3$-2-Me-thiazole-5-yl | Me | $CHF_2$ | H | 2-(3,3-$Me_2$-nBu) |
| 1-302 | 4-$CF_3$-2-Me-thiazole-5-yl | Me | $CHF_2$ | H | 2-(1,3,3-$Me_3$-nBu) |
| 1-303 | 4-$CF_3$-2-Me-thiazole-5-yl | Me | $CF_3$ | F | 2-(1,3-$Me_2$-nBu) |
| 1-304 | 4-$CF_3$-2-Me-thiazole-5-yl | Me | $CHF_2$ | F | 2-(1,3-$Me_2$-nBu) |
| 1-305 | 4-$CF_3$-2-Me-thiazole-5-yl | Me | Me | F | 2-nBu |
| 1-306 | 4-$CF_3$-2-Me-thiazole-5-yl | Me | Me | F | 2-(3-Me-nBu) |
| 1-307 | 4-$CF_3$-2-Me-thiazole-5-yl | Me | Me | F | 2-(1,3-$Me_2$-nBu) |
| 1-308 | 4-$CF_3$-2-Me-thiazole-5-yl | Me | Me | F | 2-(3,3-$Me_2$-nBu) |
| 1-309 | 4-$CF_3$-2-Me-thiazole-5-yl | Me | Me | F | 2-(1,3,3-$Me_3$-nBu) |
| 1-310 | 4-$CF_3$-2-Me-thiazole-5-yl | $CH_2CF_3$ | Me | H | 2-(1,3-$Me_2$-nBu) |
| 1-311 | 4-$CHF_2$-2-Me-thiazole-5-yl | Me | $CF_3$ | H | 2-(1,3-$Me_2$-nBu) |
| 1-312 | 4-$CHF_2$-2-Me-thiazole-5-yl | Me | $CHF_2$ | H | 2-nBu |
| 1-313 | 4-$CHF_2$-2-Me-thiazole-5-yl | Me | $CHF_2$ | H | 2-(3-Me-nBu) |
| 1-314 | 4-$CHF_2$-2-Me-thiazole-5-yl | Me | $CHF_2$ | H | 2-(1,3-$Me_2$-nBu) |
| 1-315 | 4-$CHF_2$-2-Me-thiazole-5-yl | Me | $CHF_2$ | H | 2-(3,3-$Me_2$-nBu) |
| 1-316 | 4-$CHF_2$-2-Me-thiazole-5-yl | Me | $CHF_2$ | H | 2-(1,3,3-$Me_3$-nBu) |
| 1-317 | 4-$CHF_2$-2-Me-thiazole-5-yl | Me | $CF_3$ | F | 2-(1,3-$Me_2$-nBu) |
| 1-318 | 4-$CHF_2$-2-Me-thiazole-5-yl | Me | $CHF_2$ | F | 2-nBu |
| 1-319 | 4-$CHF_2$-2-Me-thiazole-5-yl | Me | $CHF_2$ | F | 2-(3-Me-nBu) |
| 1-320 | 4-$CHF_2$-2-Me-thiazole-5-yl | Me | $CHF_2$ | F | 2-(1,3-$Me_2$-nBu) |
| 1-321 | 4-$CHF_2$-2-Me-thiazole-5-yl | Me | $CHF_2$ | F | 2-(3,3-$Me_2$-nBu) |
| 1-322 | 4-$CHF_2$-2-Me-thiazole-5-yl | Me | $CHF_2$ | F | 2-(1,3,3-$Me_3$-nBu) |
| 1-323 | 4-$CHF_2$-2-Me-thiazole-5-yl | Me | Me | F | 2-nBu |
| 1-324 | 4-$CHF_2$-2-Me-thiazole-5-yl | Me | Me | F | 2-(3-Me-nBu) |

[Table 1-12]

| No. | $R^1$ | $R^6$ | $R^7$ | $R^8$ | Xn |
|---|---|---|---|---|---|
| 1-325 | 4-$CHF_2$-2-Me-thiazole-5-yl | Me | Me | F | 2-(1,3-$Me_2$-nBu) |
| 1-326 | 4-$CHF_2$-2-Me-thiazole-5-yl | Me | Me | F | 2-(3,3-$Me_2$-nBu) |
| 1-327 | 4-$CHF_2$-2-Me-thiazole-5-yl | Me | Me | F | 2-(1,3,3-$Me_3$-nBu) |
| 1-328 | 4-$CHF_2$-2-Me-thiazole-5-yl | $CH_2CF_3$ | Me | H | 2-(1,3-$Me_2$-nBu) |
| 1-329 | 4-$CF_3$-thiazole-5-yl | Me | $CF_3$ | H | 2-(1,3-$Me_2$-nBu) |

(continued)

| No. | $R^1$ | $R^6$ | $R^7$ | $R^8$ | Xn |
|---|---|---|---|---|---|
| 1-330 | 4-$CF_3$-thiazole-5-yl | Me | $CHF_2$ | H | 2-nBu |
| 1-331 | 4-$CF_3$-thiazole-5-yl | Me | $CHF_2$ | H | 2-(3-Me-nBu) |
| 1-332 | 4-$CF_3$-thiazole-5-yl | Me | $CHF_2$ | H | 2-(1,3-$Me_2$-nBu) |
| 1-333 | 4-$CF_3$-thiazole-5-yl | Me | $CHF_2$ | H | 2-(1,3-$Me_2$-nBu) |
| 1-334 | 4-$CF_3$-thiazole-5-yl | Me | $CF_3$ | F | 2-(1,3-$Me_2$-nBu) |
| 1-335 | 4-$CF_3$-thiazole-5-yl | Me | $CHF_2$ | F | 2-(1,3-$Me_2$-nBu) |
| 1-336 | 4-$CF_3$-thiazole-5-yl | Me | Me | F | 2-(1,3-$Me_2$-nBu) |
| 1-337 | 4-$CF_3$-thiazole-5-yl | $CH_2CF_3$ | Me | H | 2-(1,3-$Me_2$-nBu) |
| 1-338 | 4-$CHF_2$-thiazole-5-yl | Me | $CF_3$ | H | 2-(1,3-$Me_2$-nBu) |
| 1-339 | 4-$CHF_2$-thiazole-5-yl | Me | $CHF_2$ | H | 2-nBu |
| 1-340 | 4-$CHF_2$-thiazole-5-yl | Me | $CHF_2$ | H | 2-(3-Me-nBu) |
| 1-341 | 4-$CHF_2$-thiazole-5-yl | Me | $CHF_2$ | H | 2-(1,3-$Me_2$-nBu) |
| 1-342 | 4-$CHF_2$-thiazole-5-yl | Me | $CHF_2$ | H | 2-(3,3-$Me_2$-nBu) |
| 1-343 | 4-$CHF_2$-thiazole-5-yl | Me | $CHF_2$ | H | 2-(1,3,3-$Me_3$-nBu) |
| 1-344 | 4-$CHF_2$-thiazole-5-yl | Me | $CF_3$ | F | 2-(1,3-$Me_2$-nBu) |
| 1-345 | 4-$CHF_2$-thiazole-5-yl | Me | $CHF_2$ | F | 2-(1,3-$Me_2$-nBu) |
| 1-346 | 4-$CHF_2$-thiazole-5-yl | Me | Me | F | 2-(1,3-$Me_2$-nBu) |
| 1-347 | 4-$CHF_2$-thiazole-5-yl | $CH_2CF_3$ | Me | H | 2-(1,3-$Me_2$-nBu) |
| 1-348 | 2-$CHF_2$-pyridine-3-yl | Me | $CF_3$ | H | 2-(1,3-$Me_2$-nBu) |
| 1-349 | 2-$CHF_2$-pyridine-3-yl | Me | $CHF_2$ | H | 2-(1,3-$Me_2$-nBu) |
| 1-350 | 2-$CHF_2$-pyridine-3-yl | Me | $CF_3$ | F | 2-(1,3-$Me_2$-nBu) |
| 1-351 | 2-$CHF_2$-pyridine-3-yl | Me | $CHF_2$ | F | 2-(1,3-$Me_2$-nBu) |
| 1-352 | 2-$CHF_2$-pyridine-3-yl | Me | Me | F | 2-(1,3-$Me_2$-nBu) |
| 1-353 | 5-Cl-3-$CHF_2$-1-Me-pyrazole-4-yl | Me | $CF_3$ | H | 2-(1,3-$Me_2$-nBu) |
| 1-354 | 5-Cl-3-$CHF_2$-1-Me-pyrazole-4-yl | Me | $CHF_2$ | H | 2-(1,3-$Me_2$-nBu) |

[Table 1-13]

| No. | $R^1$ | $R^6$ | $R^7$ | $R^8$ | Xn |
|---|---|---|---|---|---|
| 1-355 | 5-Cl-3-$CHF_2$-1-Me-pyrazole-4-yl | Me | $CF_3$ | F | 2-(1,3-$Me_2$-nBu) |
| 1-356 | 5-Cl-3-$CHF_2$-1-Me-pyrazole-4-yl | Me | $CHF_2$ | F | 2-(1,3-$Me_2$-nBu) |
| 1-357 | 5-Cl-3-$CHF_2$-1-Me-pyrazole-4-yl | Me | Me | F | 2-(1,3-$Me_2$-nBu) |
| 1-358 | 2,3-$Cl_2$-thiophen-5-yl | Me | $CF_3$ | H | 2-(1,3-$Me_2$-nBu) |
| 1-359 | 2,3-$Cl_2$-thiophen-5-yl | Me | $CHF_2$ | H | 2-(1,3-$Me_2$-nBu) |
| 1-360 | 2,3-$Cl_2$-thiophen-5-yl | Me | $CF_3$ | F | 2-(1,3-$Me_2$-nBu) |
| 1-361 | 2,3-$Cl_2$-thiophen-5-yl | Me | $CHF_2$ | F | 2-(1,3-$Me_2$-nBu) |
| 1-362 | 2,3-$Cl_2$-thiophen-5-yl | Me | Me | F | 2-(1,3-$Me_2$-nBu) |
| 1-363 | 2,3-$Cl_2$-thiophen-4-yl | Me | $CF_3$ | H | 2-(1,3-$Me_2$-nBu) |

(continued)

| No. | $R^1$ | $R^6$ | $R^7$ | $R^8$ | Xn |
|---|---|---|---|---|---|
| 1-364 | 2,3-$Cl_2$-thiophen-4-yl | Me | $CHF_2$ | H | 2-(1,3-$Me_2$-nBu) |
| 1-365 | 2,3-$Cl_2$-thiophen-4-yl | Me | $CF_3$ | F | 2-(1,3-$Me_2$-nBu) |
| 1-366 | 2,3-$Cl_2$-thiophen-4-yl | Me | $CHF_2$ | F | 2-(1,3-$Me_2$-nBu) |
| 1-367 | 2,3-$Cl_2$-thiophen-4-yl | Me | Me | F | 2-(1,3-$Me_2$-nBu) |
| 1-368 | 4-Me-1,2,3-thiadiazol-5-yl | Me | $CHF_2$ | H | 2-(3,4,5-$F_3$-Ph) |
| 1-369 | 1,2,3-benzothiadiazol-7-yl | Me | $CHF_2$ | H | 2-(3,4,5-$F_3$-Ph) |
| 1-370 | 4-$CHF_2$-thiazole-5-yl | Me | $CHF_2$ | H | 2-(3,4,5-$F_3$-Ph) |
| 1-371 | 4-$CF_3$-thiazole-5-yl | Me | $CHF_2$ | H | 2-(3,4,5-$F_3$-Ph) |
| 1-372 | 4-$CHF_2$-2-Me-thiazole-5-yl | Me | $CHF_2$ | H | 2-(3,4,5-$F_3$-Ph) |
| 1-373 | 2,3-$Cl_2$-thiophen-5-yl | Me | $CHF_2$ | H | 2-(3,4,5-$F_3$-Ph) |
| 1-374 | 3,4-$Cl_2$-isoxazole-5-yl | Me | $CHF_2$ | H | 2-(3,4,5-$F_3$-Ph) |
| 1-375 | 2,6-$Cl_2$-pyridin-4-yl | Me | $CHF_2$ | H | 2-(3,4,5-$F_3$-Ph) |
| 1-376 | 3-Cl-isoxazole-5-vl | Me | Me | F | 2-(1,3-$Me_2$-nBu) |
| 1-377 | 3,4-$Cl_2$-isothiazol- 5-yl | Me | Me | Me | 2-(1,3-$Me_2$-nBu) |
| 1-378 | 3,4-$Cl_2$-isothiazol-5-yl | Me | Me | Cl | 2-(1,3-$Me_2$-nBu) |
| 1-379 | 3,4-$Cl_2$-isothiazol-5-yl | Me | $CHF_2$ | Cl | 2-(1,3-$Me_2$-nBu) |
| 1-380 | 2,6-$F_2$-pyridin-4-yl | Me | $CHF_2$ | H | 2-(1,3-$Me_2$-nBu) |
| 1-381 | 2,6-$F_2$-pyridin-4-yl | Me | Me | F | 2-(1,3-$Me_2$-nBu) |
| 1-382 | 2,6-$F_2$-pyridin-4-yl | Me | Me | Me | 2-(1,3-$Me_2$-nBu) |
| 1-383 | 2,6-$F_2$-pyridin-4-yl | Me | Me | Cl | 2-(1,3-$Me_2$-nBu) |
| 1-384 | 2,6-$F_2$-pyridin-4-yl | Me | $CHF_2$ | Cl | 2-(1,3-$Me_2$-nBu) |

[Table 2-1]

Table 2

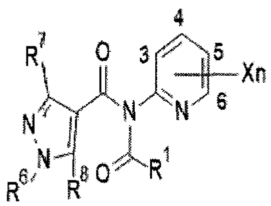

| No. | $R^1$ | $R^6$ | $R^7$ | $R^8$ | Xn |
|---|---|---|---|---|---|
| 2-1 | 3,4-$Cl_2$-isothiazol-5-yl | Me | $CF_3$ | H | 3-(1,3-$Me_2$-nBu) |
| 2-2 | 3,4-$Cl_2$-isothiazol-5-yl | Me | $CHF_2$ | H | 3-(1,3-$Me_2$-nBu) |
| 2-3 | 3,4-$Cl_2$-isothiazol-5-yl | Me | $CHF_2$ | H | 3-(1,3-$Me_2$-nBu) |
| 2-4 | 3,4-$Cl_2$-isothiazol-5-yl | Me | Me | H | 3-(1,3-$Me_2$-nBu) |
| 2-5 | 3-Me-isothiazol-5-yl | Me | $CF_3$ | H | 3-(1,3-$Me_2$-nBu) |
| 2-6 | 3-Me-isothiazol-5-yl | Me | $CHF_2$ | H | 3-(1,3-$Me_2$-nBu) |
| 2-7 | 3-Me-isothiazol-5-yl | Me | $CHF_2$ | F | 3-(1,3-$Me_2$-nB) |
| 2-8 | 3-Me-isothiazol-5-yl | Me | Me | F | 3-(1,3-$Me_2$-nB) |

(continued)

| No. | $R^1$ | $R^6$ | $R^7$ | $R^8$ | Xn |
|---|---|---|---|---|---|
| 2-9 | 3-Me-4-Cl-isothiazol-5-yl | Me | $CF_3$ | H | 3-(1,3-$Me_2$-nB) |
| 2-10 | 3-Me-4-Cl-isothiazol-5-yl | Me | $CHF_2$ | H | 3-(1,3-$Me_2$-nB) |
| 2-11 | 3-Me-4-Cl-isothiazol-5-yl | Me | $CHF_2$ | F | 3-(1,3-$Me_2$-nBu) |
| 2-12 | 3-Me-4-Cl-isothiazol-5-yl | Me | Me | F | 3-(1,3-$Me_2$-nB) |
| 2-13 | 4-Me-1,2,3-thiadiazol-5-yl | Me | $CF_3$ | H | 3-(1,3-$Me_2$-nBu) |
| 2-14 | 4-Me-1,2,3-thiadiazol-5-yl | Me | $CHF_2$ | H | 3-(1,3-$Me_2$-nB) |
| 2-15 | 4-Me-1,2,3-thiadiazol-5-yl | Me | $CHF_2$ | F | 3-(1,3-$Me_2$-nBu) |
| 2-16 | 4-Me-1,2,3-thiadiazol-5-yl | Me | Me | F | 3-(1,3-$Me_2$-nBu) |
| 2-17 | 1,2,3-benzothiadiazol-7-yl | Me | $CF_3$ | H | 3-(1,3-$Me_2$-nBu) |
| 2-18 | 1,2,3-benzothiadiazol-7-yl | Me | $CHF_2$ | H | 3-(1,3-$Me_2$-nB) |
| 2-19 | 1,2,3-benzothiadiazol-7-yl | Me | $CHF_2$ | F | 3-(1,3-$Me_2$-nBu) |
| 2-20 | 1,2,3-benzothiadiazol-7-yl | Me | Me | F | 3-(1,3-$Me_2$-nBu) |
| 2-21 | 4-$CF_3$-2-Me-thiazole-5-yl | Me | $CF_3$ | H | 3-(1,3-$Me_2$-nBu) |
| 2-22 | 4-$CF_3$-2-Me-thiazole-5-yl | Me | $CHF_2$ | H | 3-(1,3-$Me_2$-nBu) |
| 2-23 | 4-$CF_3$-2-Me-thiazole-5-yl | Me | $CHF_2$ | F | 3-(1,3-$Me_2$-nBu) |

[Table 2-2]

| No. | $R^1$ | $R^6$ | $R^7$ | $R^8$ | Xn |
|---|---|---|---|---|---|
| 2-24 | 4-$CF_3$-2-Me-thiazole-5-yl | Me | Me | F | 3-(1,3-$Me_2$-nB) |
| 2-25 | 4-$CHF_2$-2-Me-thiazole-5-yl | Me | $CF_3$ | H | 3-(1,3-$Me_2$-nB) |
| 2-26 | 4-$CHF_2$-2-Me-thiazole-5-yl | Me | $CHF_2$ | H | 3-(1,3-$Me_2$-nBu) |
| 2-27 | 4-$CHF_2$-2-Me-thiazole-5-yl | Me | $CHF_2$ | F | 3-(1,3-$Me_2$-nB) |
| 2-28 | 4-$CHF_2$-2-Me-thiazole-5-yl | Me | Me | F | 3-(1,3-$Me_2$-nBu) |
| 2-29 | 4-$CF_3$-thiazole-5-yl | Me | $CF_3$ | H | 3-(1,3-$Me_2$-nB) |
| 2-30 | 4-$CF_3$-thiazole-5-yl | Me | $CHF_2$ | H | 3-(1,3-$Me_2$-nBu) |
| 2-31 | 4-$CF_3$-thiazole-5-yl | Me | $CHF_2$ | F | 3-(1,3-$Me_2$-nB) |
| 2-32 | 4-$CF_3$-thiazole-5-yl | Me | Me | F | 3-(1,3-$Me_2$-nBu) |
| 2-33 | 4-$CHF_2$-thiazole-5-yl | Me | $CF_3$ | H | 3-(1,3-$Me_2$-nBu) |
| 2-34 | 4-$CHF_2$-thiazole-5-yl | Me | $CHF_2$ | H | 3-(1,3-$Me_2$-nBu) |
| 2-35 | 4-$CHF_2$-thiazole-5-yl | Me | $CHF_2$ | F | 3-(1,3-$Me_2$-nBu) |
| 2-36 | 4-$CHF_2$-thiazole-5-yl | Me | Me | F | 3-(1,3-$Me_2$-nB) |

[Table 3-1]

Table 3.

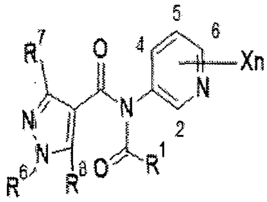

| No. | $R^1$ | $R^6$ | $R^7$ | $R^8$ | Xn |
|---|---|---|---|---|---|
| 3-1 | 3,4-$Cl_2$-isothiazol-5-yl | Me | $CF_3$ | H | 2-(2-cPr-cPr) |
| 3-2 | 3,4-$Cl_2$-isothiazol-5-yl | Me | $CF_3$ | H | 2-nBu |
| 3-3 | 3,4-$Cl_2$-isothiazol-5-yl | Me | $CF_3$ | H | 2-(3-Me-nBu) |
| 3-4 | 3,4-$Cl_2$-isothiazol-5-yl | Me | $CF_3$ | H | 2-(1,3-$Me_2$-nBu) |
| 3-5 | 3,4-$Cl_2$-isothiazol-5-yl | Me | $CF_3$ | H | 2-(1,3,3-$Me_3$-nBu) |
| 3-6 | 3,4-$Cl_2$-isothiazol-5-yl | Me | $CF_3$ | H | 2-(1,3-$Me_2$-nPen) |
| 3-7 | 3,4-$Cl_2$-isothiazol-5-yl | Me | $CF_3$ | H | 2-(1,4-$Me_2$-nPen) |
| 3-8 | 3,4-$Cl_2$-isothiazol-5-yl | Me | $CHF_2$ | H | 2-(2-cPr-cPr) |
| 3-9 | 3,4-$Cl_2$-isothiazol-5-yl | Me | $CHF_2$ | H | 2-nBu |
| 3-10 | 3,4-$Cl_2$-isothiazol-5-yl | Me | $CHF_2$ | H | 2-(3-Me-nBu) |
| 3-11 | 3,4-$Cl_2$-isothiazol-5-yl | Me | $CHF_2$ | H | 2-(1,3-$Me_2$-nBu) |
| 3-12 | 3,4-$Cl_2$-isothiazol-5-yl | Me | $CHF_2$ | H | 2-(1,3,3-$Me_3$-nBu) |
| 3-13 | 3,4-$Cl_2$-isothiazol-5-yl | Me | $CHF_2$ | H | 2-(1,3-$Me_2$-nPen) |
| 3-14 | 3,4-$Cl_2$-isothiazol-5-yl | Me | $CHF_2$ | H | 2-(1,4-$Me_2$-nPen) |
| 3-15 | 3,4-$Cl_2$-isothiazol-5-yl | Me | $CF_3$ | F | 2-(1,3-$Me_2$-nBu) |
| 3-16 | 3,4-$Cl_2$-isothiazol-5-yl | Me | $CHF_2$ | F | 2-(1,3-$Me_2$-nBu) |
| 3-17 | 3,4-$Cl_2$-isothiazol-5-yl | Me | Me | F | 2-nBu |
| 3-18 | 3,4-$Cl_2$-isothiazol-5-yl | Me | Me | F | 2-(3-Me-nBu) |
| 3-19 | 3,4-$Cl_2$-isothiazol-5-yl | Me | Me | F | 2-(1,3-$Me_2$-nBu) |
| 3-20 | 3,4-$Cl_2$-isothiazol-5-yl | Me | Me | F | 2-(3,3-$Me_2$-nBu) |
| 3-21 | 3,4-$Cl_2$-isothiazol-5-yl | Me | Me | F | 2-(1,3,3-$Me_3$-nBu) |
| 3-22 | 3,4-$Cl_2$-isothiazol-5-yl | $CH_2CF_3$ | Me | H | 2-(1,3-$Me_2$-nBu) |
| 3-23 | 3-Me-isothiazol-5-yl | Me | $CF_3$ | H | 2-(1,3-$Me_2$-nBu) |
| 3-24 | 3-Me-isothiazol-5-yl | Me | $CHF_2$ | H | 2-(1,3-$Me_2$-nBu) |
| 3-25 | 3-Me-isothiazol-5-yl | Me | $CHF_2$ | F | 2-(1,3-$Me_2$-nBu) |

[Table 3-2]

| No. | $R^1$ | $R^6$ | $R^7$ | $R^8$ | Xn |
|---|---|---|---|---|---|
| 3-26 | 3-Me-isothiazol-5-yl | Me | Me | F | 2-(1,3-$Me_2$-nBu) |
| 3-27 | 3-Me-4-Cl-isothiazol-5-yl | Me | $CF_3$ | H | 2-(1,3-$Me_2$-nBu) |
| 3-28 | 3-Me-4-Cl-isothiazol-5-yl | Me | $CHF_2$ | H | 2-(1,3-$Me_2$-nBu) |
| 3-29 | 3-Me-4-Cl-isothiazol-5-yl | Me | $CHF_2$ | F | 2-(1,3-$Me_2$-nBu) |

(continued)

| No. | $R^1$ | $R^6$ | $R^7$ | $R^8$ | Xn |
|---|---|---|---|---|---|
| 3-30 | 3-Me-4-Cl-isothiazol-5-yl | Me | Me | F | 2-(1,3-$Me_2$-nBu) |
| 3-31 | 4-Me-1,2,3-thiadiazol-5-yl | Me | $CF_3$ | H | 2-(1,3-$Me_2$-nBu) |
| 3-32 | 4-Me-1,2,3-thiadiazol-5-yl | Me | $CHF_2$ | H | 2-(2-cPr-cPr) |
| 3-33 | 4-Me-1,2,3-thiadiazol-5-yl | Me | $CHF_2$ | H | 2-nBu |
| 3-34 | 4-Me-1,2,3-thiadiazol-5-yl | Me | $CHF_2$ | H | 2-(3-Me-nBu) |
| 3-35 | 4-Me-1,2,3-thiadiazol-5-yl | Me | $CHF_2$ | H | 2-(1,3-$Me_2$-nBu) |
| 3-36 | 4-Me-1,2,3-thiadiazol-5-yl | Me | $CHF_2$ | H | 2-(1,3,3-$Me_3$-nBu) |
| 3-37 | 4-Me-1,2,3-thiadiazol-5-yl | Me | $CHF_2$ | H | 2-(1,3-$Me_2$-nPen) |
| 3-38 | 4-Me-1,2,3-thiadiazol-5-yl | Me | $CHF_2$ | H | 2-(1,4-$Me_2$-nPen) |
| 3-39 | 4-Me-1,2,3-thiadiazol-5-yl | Me | $CHF_2$ | F | 2-(1,3-$Me_2$-nBu) |
| 3-40 | 4-Me-1,2,3-thiadiazol-5-yl | Me | Me | F | 2-(1,3-$Me_2$-nBu) |
| 3-41 | 4-$CF_3$-1,2,3-thiadiazol-5-yl | Me | $CF_3$ | H | 2-(1,3-$Me_2$-nBu) |
| 3-42 | 4-$CF_3$-1,2,3-thiadiazol-5-yl | Me | $CHF_2$ | H | 2-(2-cPr-cPr) |
| 3-43 | 4-$CF_3$-1,2,3-thiadiazol-5-yl | Me | $CHF_2$ | H | 2-nBu |
| 3-44 | 4-$CF_3$-1,2,3-thiadiazol-5-yl | Me | $CHF_2$ | H | 2-(3-Me-nBu) |
| 3-45 | 4-$CF_3$-1,2,3-thiadiazol-5-yl | Me | $CHF_2$ | H | 2-(1,3-$Me_2$-nBu) |
| 3-46 | 4-$CF_3$-1,2,3-thiadiazol-5-yl | Me | $CHF_2$ | H | 2-(1,3,3-$Me_3$-nBu) |
| 3-47 | 4-$CF_3$-1,2,3-thiadiazol-5-yl | Me | $CHF_2$ | H | 2-(1,3-$Me_2$-nPen) |
| 3-48 | 4-$CF_3$-1,2,3-thiadiazol-5-yl | Me | $CHF_2$ | H | 2-(1,4-$Me_2$-nPen) |
| 3-49 | 4-$CF_3$-1,2,3-thiadiazol-5-yl | Me | $CHF_2$ | F | 2-(1,3-$Me_2$-nBu) |
| 3-50 | 4-$CF_3$-1,2,3-thiadiazol-5-yl | Me | Me | F | 2-(1,3-$Me_2$-nBu) |
| 3-51 | 4-$CHF_2$-1,2,3-thiadiazol-5-yl | Me | $CF_3$ | H | 2-(1,3-$Me_2$-nBu) |
| 3-52 | 4-$CHF_2$-1,2,3-thiadiazol-5-yl | Me | $CHF_2$ | H | 2-(2-cPr-cPr) |
| 3-53 | 4-$CHF_2$-1,2,3-thiadiazol-5-yl | Me | $CHF_2$ | H | 2-nBu |
| 3-54 | 4-$CHF_2$-1,2,3-thiadiazol-5-yl | Me | $CHF_2$ | H | 2-(3-Me-nBu) |
| 3-55 | 4-$CHF_2$-1,2,3-thiadiazol-5-yl | Me | $CHF_2$ | H | 2-(1,3-$Me_2$-nBu) |

[Table 3-3]

| No. | $R^1$ | $R^6$ | $R^7$ | $R^8$ | Xn |
|---|---|---|---|---|---|
| 3-56 | 4-$CHF_2$-1,2,3-thiadiazol-5-yl | Me | $CHF_2$ | H | 2-(1,3,3-$Me_3$-nBu) . |
| 3-57 | 4-$CHF_2$-1,2,3-thiadiazol-5-yl | Me | $CHF_2$ | H | 2-(1,3-$Me_2$-nPen) |
| 3-58 | 4-$CHF_2$-1,2,3-thiadiazol-5-yl | Me | $CHF_2$ | H | 2-(1,4-$Me_2$-nPen) |
| 3-59 | 4-$CHF_2$-1,2,3-thiadiazol-5-yl | Me | $CHF_2$ | F | 2-(1,3-$Me_2$-nBu) |
| 3-60 | 4-$CHF_2$-1,2,3-thiadiazol-5-yl | Me | Me | F | 2-(1,3-$Me_2$-nBu) |
| 3-61 | 1,2,3-benzothiadiazol-7-yl | Me | $CF_3$ | H | 2-(1,3-$Me_2$-nBu) |
| 3-62 | 1,2,3-benzothiadiazol-7-yl | Me | $CHF_2$ | H | 2-(2-cPr-cPr) |
| 3-63 | 1,2,3-benzothiadiazol-7-yl | Me | $CHF_2$ | H | 2-nBu |

(continued)

| No. | $R^1$ | $R^6$ | $R^7$ | $R^8$ | Xn |
|---|---|---|---|---|---|
| 3-64 | 1,2,3-benzothiadiazol-7-yl | Me | $CHF_2$ | H | 2-(3-Me-nBu) |
| 3-65 | 1,2,3-benzothiadiazol-7-yl | Me | $CHF_2$ | H | 2-(1,3-$Me_2$-nBu) |
| 3-66 | 1,2,3-benzothiadiazol-7-yl | Me | $CHF_2$ | H | 2-(1,3,3-$Me_3$-nBu) |
| 3-67 | 1,2,3-benzothiadiazol-7-yl | Me | $CHF_2$ | H | 2-(1,3-$Me_2$-nPen) |
| 3-68 | 1,2,3-benzothiadiazol-7-yl | Me | $CHF_2$ | H | 2-(1,4-$Me_2$-nPen) |
| 3-69 | 1,2,3-benzothiadiazol-7-yl | Me | $CHF_2$ | F | 2-(1,3-$Me_2$-nBu) |
| 3-70 | 1,2,3-benzothiadiazol-7-yl | Me | Me | F | 2-(1,3-$Me_2$-nBu) |
| 3-71 | 4-$CF_3$-2-Me-thiazole-5-yl | Me | $CF_3$ | H | 2-(1,3-$Me_2$-nBu) |
| 3-72 | 4-$CF_3$-2-Me-thiazole-5-yl | Me | $CHF_2$ | H | 2-(2-cPr-cPr) |
| 3-73 | 4-$CF_3$-2-Me-thiazole-5-yl | Me | $CHF_2$ | H | 2-nBu |
| 3-74 | 4-$CF_3$-2-Me-thiazole-5-yl | Me | $CHF_2$ | H | 2-(3-Me-nBu) |
| 3-75 | 4-$CF_3$-2-Me-thiazole-5-yl | Me | $CHF_2$ | H | 2-(1,3-$Me_2$-nBu) |
| 3-76 | 4-$CF_3$-2-Me-thiazole-5-yl | Me | $CHF_2$ | H | 2-(1,3,3-$Me_3$-nBu) |
| 3-77 | 4-$CF_3$-2-Me-thiazole-5-yl | Me | $CHF_2$ | H | 2-(1,3-$Me_2$-nPen) |
| 3-78 | 4-$CF_3$-2-Me-thiazole-5-yl | Me | $CHF_2$ | H | 2-(1,4-$Me_2$-nPen) |
| 3-79 | 4-$CF_3$-2-Me-thiazole-5-yl | Me | $CHF_2$ | F | 2-(1,3-$Me_2$-nBu) |
| 3-80 | 4-$CF_3$-2-Me-thiazole-5-yl | Me | Me | F | 2-(1,3-$Me_2$-nBu) |
| 3-81 | 4-$CHF_2$-2-Me-thiazole-5-yl | Me | $CF_3$ | H | 2-(1,3-$Me_2$-nBu) |
| 3-82 | 4-$CHF_2$-2-Me-thiazole-5-yl | Me | $CHF_2$ | H | 2-(2-cPr-cPr) |
| 3-83 | 4-$CHF_2$-2-Me-thiazole-5-yl | Me | $CHF_2$ | H | 2-nBu |
| 3-84 | 4-$CHF_2$-2-Me-thiazole-5-yl | Me | $CHF_2$ | H | 2-(3-Me-nBu) |
| 3-85 | 4-$CHF_2$-2-Me-thiazole-5-yl | Me | $CHF_2$ | H | 2-(1,3-$Me_2$-nBu) |

[Table 3-4]

| No. | $R^1$ | $R^6$ | $R^7$ | $R^8$ | Xn |
|---|---|---|---|---|---|
| 3-86 | 4-$CHF_2$-2-Me-thiazole-5-yl | Me | $CHF_2$ | H | 2-(1,3,3-$Me_3$-nBu) |
| 3-87 | 4-$CHF_2$-2-Me-thiazole-5-yl | Me | $CHF_2$ | H | 2-(1,3-$Me_2$-nPen) |
| 3-88 | 4-$CHF_2$-2-Me-thiazole-5-yl | Me | $CHF_2$ | H | 2-(1,4-$Me_2$-nPen) |
| 3-89 | 4-$CHF_2$-2-Me-thiazole-5-yl | Me | $CHF_2$ | F | 2-(1,3-$Me_2$-nBu) |
| 3-90 | 4-$CHF_2$-2-Me-thiazole-5-yl | Me | Me | F | 2-(1,3-$Me_2$-nBu) |
| 3-91 | 4-$CF_3$-thiazole-5-yl | Me | $CF_3$ | H | 2-(1,3-$Me_2$-nBu) |
| 3-92 | 4-$CF_3$-thiazole-5-yl | Me | $CHF_2$ | H | 2-(2-cPr-cPr) |
| 3-93 | 4-$CF_3$-thiazole-5-yl | Me | $CHF_2$ | H | 2-nBu |
| 3-94 | 4-$CF_3$-thiazole-5-yl | Me | $CHF_2$ | H | 2-(3-Me-nBu) |
| 3-95 | 4-$CF_3$-thiazole-5-yl | Me | $CHF_2$ | H | 2-(1,3-$Me_2$-nBu) |
| 3-96 | 4-$CF_3$-thiazole-5-yl | Me | $CHF_2$ | H | 2-(1,3,3-$Me_3$-nBu) |
| 3-97 | 4-$CF_3$-thiazole-5-yl | Me | $CHF_2$ | H | 2-(1,3-$Me_2$-nPen) |

(continued)

| No. | $R^1$ | $R^6$ | $R^7$ | $R^8$ | Xn |
|---|---|---|---|---|---|
| 3-98 | 4-$CF_3$-thiazole-5-yl | Me | $CHF_2$ | H | 2-(1,4-$Me_2$-nPen) |
| 3-99 | 4-$CF_3$-thiazole-5-yl | Me | $CHF_2$ | F | 2-(1,3-$Me_2$-nBu) |
| 3-100 | 4-$CF_3$-thiazole-5-yl | Me | Me | F | 2-(1,3-$Me_2$-nBu) |
| 3-101 | 4-$CHF_2$-thiazole-5-yl | Me | $CF_3$ | H | 2-(1,3-$Me_2$-nBu) |
| 3-102 | 4-$CHF_2$-thiazole-5-yl | Me | $CHF_2$ | H | 2-(2-cPr-cPr) |
| 3-103 | 4-$CHF_2$-thiazole-5-yl | Me | $CHF_2$ | H | 2-nBu |
| 3-104 | 4-$CHF_2$-thiazole-5-yl | Me | $CHF_2$ | H | 2-(3-Me-nBu) |
| 3-105 | 4-$CHF_2$-thiazole-5-yl | Me | $CHF_2$ | H | 2-(1,3-$Me_2$-nBu) |
| 3-106 | 4-$CHF_2$-thiazole-5-yl | Me | $CHF_2$ | H | 2-(1,3,3-$Me_3$-nBu) |
| 3-107 | 4-$CHF_2$-thiazole-5-yl | Me | $CHF_2$ | H | 2-(1,3-$Me_2$-nPen) |
| 3-108 | 4-$CHF_2$-thiazole-5-yl | Me | $CHF_2$ | H | 2-(1,4-$Me_2$-nPen) |
| 3-109 | 4-$CHF_2$-thiazole-5-yl | Me | $CHF_2$ | F | 2-(1,3-$Me_2$-nBu) |
| 3-110 | 4-$CHF_2$-thiazole-5-yl | Me | Me | F | 2-(1,3-$Me_2$-nBu) |
| 3-111 | 2-$CHF_2$-pyridin-3-yl | Me | $CHF_2$ | H | 2-(1,3-$Me_2$-nBu) |
| 3-112 | 5-Cl-3-$CHF_2$-1-Me-pyrazol-4-yl | Me | $CHF_2$ | H | 2-(1,3-$Me_2$-nBu) |
| 3-113 | 2,3-$Cl_2$-thiophen-5-yl | Me | $CHF_2$ | H | 2-(1,3-$Me_2$-nBu) |
| 3-114 | 2,3-$Cl_2$-thiophen-4-yl | Me | $CHF_2$ | H | 2-(1,3-$Me_2$-nBu) |

[Table 4-1]

Table 4.

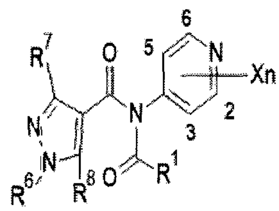

| No. | $R^1$ | $R^6$ | $R^7$ | $R^8$ | Xn |
|---|---|---|---|---|---|
| 4-1 | 3,4-$Cl_2$-isothiazol-5-yl | Me | $CF_3$ | H | 3-(1,3-$Me_2$-nBu) |
| 4-2 | 3,4-$Cl_2$-isothiazol-5-yl | Me | $CHF_2$ | H | 3-(1,3-$Me_2$-nBu) |
| 4-3 | 3,4-$Cl_2$-isothiazol-5-yl | Me | $CHF_2$ | F | 3-(1,3-$Me_2$-nBu) |
| 4-4 | 3,4-$Cl_2$-isothiazol-5-yl | Me | Me | F | 3-(1,3-$Me_2$-nBu) |
| 4-5 | 3-Me-isothiazol-5-yl | Me | $CF_3$ | H | 3-(1,3-$Me_2$-nBu) |
| 4-6 | 3-Me-isothiazol-5-yl | Me | $CHF_2$ | H | 3-(1,3-$Me_2$-nBu) |
| 4-7 | 3-Me-isothiazol-5-yl | Me | $CHF_2$ | F | 3-(1,3-$Me_2$-nBu) |
| 4-8 | 3-Me-isothiazol-5-yl | Me | Me | F | 3-(1,3-$Me_2$-nBu) |
| 4-9 | 3-Me-4-Cl-isothiazol-5-yl | Me | $CF_3$ | H | 3-(1,3-$Me_2$-nBu) |
| 4-10 | 3-Me-4-Cl-isothiazol-5-yl | Me | $CHF_2$ | H | 3-(1,3-$Me_2$-nBu) |
| 4-11 | 3-Me-4-Cl-isothiazol-5-yl | Me | $CHF_2$ | F | 3-(1,3-$Me_2$-nBu) |
| 4-12 | 3-Me-4-Cl-isothiazol-5-yl | Me | Me | F | 3-(1,3-$Me_2$-nBu) |

(continued)

| No. | $R^1$ | $R^6$ | $R^7$ | $R^8$ | Xn |
|---|---|---|---|---|---|
| 4-13 | 4-Me-1,2,3-thiadiazol-5-yl | Me | $CF_3$ | H | 3-(1,3-$Me_2$-nBu) |
| 4-14 | 4-Me-1,2,3-thiadiazol-5-vl | Me | $CHF_2$ | H | 3-(1,3-$Me_2$-nBu) |
| 4-15 | 4-Me-1,2,3-thiadiazol-5-yl | Me | $CHF_2$ | F | 3-(1,3-$Me_2$-nBu) |
| 4-16 | 4-Me-1,2,3-thiadiazol-5-yl | Me | Me | F | 3-(1,3-$Me_2$-nBu) |
| 4-17 | 4-$CF_3$-1,2,3-thiadiazol-5-yl | Me | $CF_3$ | H | 3-(1,3-$Me_2$-nBu) |
| 4-18 | 4-$CF_3$-1,2,3-thiadiazol-5-yl | Me | $CHF_2$ | H | 3-(1,3-$Me_2$-nBu) |
| 4-19 | 4-$CF_3$-1,2,3-thiadiazol-5-yl | Me | $CHF_2$ | F | 3-(1,3-$Me_2$-nBu) |
| 4-20 | 4-$CF_3$-1,2,3-thiadiazol-5-yl | Me | Me | F | 3-(1,3-$Me_2$-nBu) |
| 4-21 | 4-$CHF_2$-1,2,3-thiadiazol-5-yl | Me | $CF_3$ | H | 3-(1,3-$Me_2$-nBu) |
| 4-22 | 4-$CHF_2$-1,2,3-thiadiazol-5-yl | Me | $CHF_2$ | H | 3-(1,3-$Me_2$-nBu) |
| 4-23 | 4-$CHF_2$-1,2,3-thiadiazol-5-yl | Me | $CHF_2$ | F | 3-(1,3-$Me_2$-nBu) |

[Table 4-2]

| No. | $R^1$ | $R^6$ | $R^7$ | $R^8$ | Xn |
|---|---|---|---|---|---|
| 4-24 | 4-$CHF_2$-1,2,3-thiadiazol-5-yl | Me | Me | F | 3-(1,3-$Me_2$-nBu) |
| 4-25 | 1,2,3-benzothiadiazol-7-yl | Me | $CF_3$ | H | 3-(1,3-$Me_2$-nBu) |
| 4-26 | 1,2,3-benzothiadiazol-7-yl | Me | $CHF_2$ | H | 3-(1,3-$Me_2$-nBu) |
| 4-27 | 1,2,3-benzothiadiazol-7-yl | Me | $CHF_2$ | F | 3-(1,3-$Me_2$-nBu) |
| 4-28 | 1,2,3-benzothiadiazol-7-yl | Me | Me | F | 3-(1,3-$Me_2$-nBu) |
| 4-29 | 4-$CF_3$-2-Me-thiazole-5-yl | Me | $CF_3$ | H | 3-(1,3-$Me_2$-nBu) |
| 4-30 | 4-$CF_3$-2-Me-thiazole-5-yl | Me | $CHF_2$ | H | 3-(1,3-$Me_2$-nBu) |
| 4-31 | 4-$CF_3$-2-Me-thiazole-5-yl | Me | $CHF_2$ | F | 3-(1,3-$Me_2$-nBu) |
| 4-32 | 4-$CF_3$-2-Me-thiazole-5-yl | Me | Me | F | 3-(1,3-$Me_2$-nBu) |
| 4-33 | 4-$CHF_2$-2-Me-thiazole-5-yl | Me | $CF_3$ | H | 3-(1,3-$Me_2$-nBu) |
| 4-34 | 4-$CHF_2$-2-Me-thiazole-5-yl | Me | $CHF_2$ | H | 3-(1,3-$Me_2$-nBu) |
| 4-35 | 4-$CHF_2$-2-Me-thiazole-5-yl | Me | $CHF_2$ | F | 3-(1,3-$Me_2$-nBu) |
| 4-36 | 4-$CHF_2$-2-Me-thiazole-5-yl | Me | Me | F | 3-(1,3-$Me_2$-nBu) |
| 4-37 | 4-$CF_3$-thiazole-5-yl | Me | $CF_3$ | H | 3-(1,3-$Me_2$-nBu) |
| 4-38 | 4-$CF_3$-thiazole-5-yl | Me | $CHF_2$ | H | 3-(1,3-$Me_2$-nBu) |
| 4-39 | 4-$CF_3$-thiazole-5-yl | Me | $CHF_2$ | F | 3-(1,3-$Me_2$-nBu) |
| 4-40 | 4-$CF_3$-thiazole-5-yl | Me | Me | F | 3-(1,3-$Me_2$-nBu) |
| 4-41 | 4-$CHF_2$-thiazole-5-yl | Me | $CF_3$ | H | 3-(1,3-$Me_2$-nBu) |
| 4-42 | 4-$CHF_2$-thiazole-5-yl | Me | $CHF_2$ | H | 3-(1,3-$Me_2$-nBu) |
| 4-43 | 4-$CHF_2$-thiazole-5-yl | Me | $CHF_2$ | F | 3-(1,3-$Me_2$-nBu) |
| 4-44 | 4-$CHF_2$-thiazole-5-yl | Me | Me | F | 3-(1,3-$Me_2$-nBu) |

[Table 5-1]

Table 5

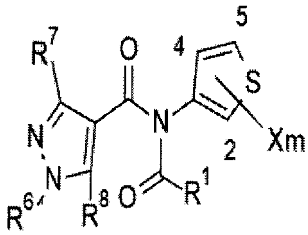

| No. | $R^1$ | $R^6$ | $R^7$ | $R^8$ | Xm |
|---|---|---|---|---|---|
| 5-1 | 3,4-$Cl_2$-isothiazol-5-yl | Me | $CF_3$ | H | 2-(1,3-$Me_2$-nBu) |
| 5-2 | 3,4-$Cl_2$-isothiazol-5-yl | Me | $CHF_2$ | H | 2-(2-cPr-cPr |
| 5-3 | 3,4-$Cl_2$-isothiazol-5-yl | Me | $CHF_2$ | H | 2-nBu |
| 5-4 | 3,4-$Cl_2$-isothiazol-5-yl | Me | $CHF_2$ | H | 2-(3-Me-nBu) |
| 5-5 | 3,4-$Cl_2$-isothiazol-5-yl | Me | $CHF_2$ | H | 2-(1,3-$Me_2$-nBu) |
| 5-6 | 3,4-$Cl_2$-isothiazol-5-yl | Me | $CHF_2$ | H | 2-(1,3,3-$Me_3$-nBu) |
| 5-7 | 3,4-$Cl_2$-isothiazol-5-yl | Me | $CHF_2$ | H | 2-(1,3-$Me_2$-nPen) |
| 5-8 | 3,4-$Cl_2$-isothiazol-5-yl | Me | $CHF_2$ | H | 2-(1,4-$Me_2$-nPen) |
| 5-9 | 3,4-$Cl_2$-isothiazol-5-yl | Me | $CHF_2$ | F | 2-(1,3-$Me_2$-nBu) |
| 5-10 | 3,4-$Cl_2$-isothiazol-5-yl | Me | Me | F | 2-(1,3-$Me_2$-nBu) |
| 5-11 | 3-Me-isothiazol-5-yl | Me | $CF_3$ | H | 2-(1,3-$Me_2$-nBu) |
| 5-12 | 3-Me-isothiazol-5-yl | Me | $CHF_2$ | H | 2-(1,3-$Me_2$-nBu) |
| 5-13 | 3-Me-isothiazol-5-yl | Me | $CHF_2$ | F | 2-(1,3-$Me_2$-nBu) |
| 5-14 | 3-Me-isothiazol-5-yl | Me | Me | F | 2-(1,3-$Me_2$-nBu) |
| 5-15 | 3-Me-4-Cl-isothiazol-5-yl | Me | $CF_3$ | H | 2-(1,3-$Me_2$-nBu) |
| 5-16 | 3-Me-4-Cl-isothiazol-5-yl | Me | $CHF_2$ | H | 2-(1,3-$Me_2$-nBu) |
| 5-17 | 3-Me-4-Cl-isothiazol-5-yl | Me | $CHF_2$ | F | 2-(1,3-$Me_2$-nBu) |
| 5-18 | 3-Me-4-Cl-isothiazol-5-yl | Me | Me | F | 2-(1,3-$Me_2$-nBu) |
| 5-19 | 4-Me-1,2,3-thiadiazol-5-yl | Me | $CF_3$ | H | 2-(1,3-$Me_2$-nBu) |
| 5-20 | 4-Me-1,2,3-thiadiazol-5-yl | Me | $CHF_2$ | H | 2-(2-cPr-cPr) |
| 5-21 | 4-Me-1,2,3-thiadiazol-5-yl | Me | $CHF_2$ | H | 2-nBu |
| 5-22 | 4-Me-1,2,3-thiadiazol-5-yl | Me | $CHF_2$ | H | 2-(3-Me-nBu) |
| 5-23 | 4-Me-1,2,3-thiadiazol-5-yl | Me | $CHF_2$ | H | 2-(1,3-$Me_2$-nBu) |

[Table 5-2]

| No. | $R^1$ | $R^6$ | $R^7$ | $R^8$ | Xm |
|---|---|---|---|---|---|
| 5-24 | 4-Me-1,2,3-thiadiazol-5-yl | Me | $CHF_2$ | H | 2-(1,3,3-$Me_3$-nBu) |
| 5-25 | 4-Me-1,2,3-thiadiazol-5-yl | Me | $CHF_2$ | H | 2-(1,3-$Me_2$-nPen) |
| 5-26 | 4-Me-1,2,3-thiadiazol-5-yl | Me | $CHF_2$ | H | 2-(1,4-$Me_2$-nPen) |
| 5-27 | 4-Me-1,2,3-thiadiazol-5-yl | Me | $CHF_2$ | F | 2-(1,3-$Me_2$-nBu) |
| 5-28 | 4-Me-1,2,3-thiadiazol-5-yl | Me | Me | F | 2-(1,3-$Me_2$-nBu) |

(continued)

| No. | $R^1$ | $R^6$ | $R^7$ | $R^8$ | Xm |
|---|---|---|---|---|---|
| 5-29 | 4-$CF_3$-1,2,3-thiadiazol-5-yl | Me | $CF_3$ | H | 2-(1,3-$Me_2$-nBu) |
| 5-30 | 4-$CF_3$-1,2,3-thiadiazol-5-yl | Me | $CHF_2$ | H | 2-(2-cPr-cPr) |
| 5-31 | 4-$CF_3$-1,2,3-thiadiazol-5-yl | Me | $CHF_2$ | H | 2-nBu |
| 5-32 | 4-$CF_3$-1,2,3-thiadiazol-5-yl | Me | $CHF_2$ | H | 2-(3-Me-nBu) |
| 5-33 | 4-$CF_3$-1,2,3-thiadiazol-5-yl | Me | $CHF_2$ | H | 2-(1,3-$Me_2$-nBu) |
| 5-34 | 4-$CF_3$-1,2,3-thiadiazol-5-yl | Me | $CHF_2$ | H | 2-(1,3,3-$Me_3$-nBu) |
| 5-35 | 4-$CF_3$-1,2,3-thiadiazol-5-yl | Me | $CHF_2$ | H | 2-(1,3-$Me_2$-nPen) |
| 5-36 | 4-$CF_3$-1,2,3-thiadiazol-5-yl | Me | $CHF_2$ | H | 2-(1,4-$Me_2$-nPen) |
| 5-37 | 4-$CF_3$-1,2,3-thiadiazol-5-yl | Me | $CHF_2$ | F | 2-(1,3-$Me_2$-nBu) |
| 5-38 | 4-$CF_3$-1,2,3-thiadiazol-5-yl | Me | Me | F | 2-(1,3-$Me_2$-nBu) |
| 5-39 | 4-$CHF_2$-1,2,3-thiadiazol-5-yl | Me | $CF_3$ | H | 2-(1,3-$Me_2$-nBu) |
| 5-40 | 4-$CHF_2$-1,2,3-thiadiazol-5-yl | Me | $CHF_2$ | H | 2-(2-cPr-cPr) |
| 5-41 | 4-$CHF_2$-1,2,3-thiadiazol-5-yl | Me | $CHF_2$ | H | 2-nBu |
| 5-42 | 4-$CHF_2$-1,2,3-thiadiazol-5-yl | Me | $CHF_2$ | H | 2-(3-Me-nBu) |
| 5-43 | 4-$CHF_2$-1,2,3-thiadiazol-5-yl | Me | $CHF_2$ | H | 2-(1,3-$Me_2$-nBu) |
| 5-44 | 4-$CHF_2$-1,2,3-thiadiazol-5-yl | Me | $CHF_2$ | H | 2-(1,3,3-$Me_3$-nBu) |
| 5-45 | 4-$CHF_2$-1,2,3-thiadiazol-5-yl | Me | $CHF_2$ | H | 2-(1,3-$Me_2$-nPen) |
| 5-46 | 4-$CHF_2$-1,2,3-thiadiazol-5-yl | Me | $CHF_2$ | H | 2-(1,4-$Me_2$-nPen) |
| 5-47 | 4-$CHF_2$-1,2,3-thiadiazol-5-yl | Me | $CHF_2$ | F | 2-(1,3-$Me_2$-nBu) |
| 5-48 | 4-$CHF_2$-1,2,3-thiadiazol-5-yl | Me | Me | F | 2-(1,3-$Me_2$-nBu) |
| 5-49 | 1,2,3-benzothiadiazol-7-yl | Me | $CF_3$ | H | 2-(1,3-$Me_2$-nBu) |
| 5-50 | 1,2,3-benzothiadiazol-7-yl | Me | $CHF_2$ | H | 2-(2-cPr-cPr) |
| 5-51 | 1,2,3-benzothiadiazol-7-yl | Me | $CHF_2$ | H | 2-nBu |
| 5-52 | 1,2,3-benzothiadiazol-7-yl | Me | $CHF_2$ | H | 2-(3-Me-nBu) |

[Table 5-3]

| No. | $R^1$ | $R^6$ | $R^7$ | $R^8$ | Xm |
|---|---|---|---|---|---|
| 5-53 | 1,2,3-benzothiadiazol-7-yl | Me | $CHF_2$ | H | 2-(1,3-$Me_2$-nBu) |
| 5-54 | 1,2,3-benzothiadiazol-7-yl | Me | $CHF_2$ | H | 2-(1,3,3-$Me_3$-nBu) |
| 5-55 | 1,2,3-benzothiadiazol-7-yl | Me | $CHF_2$ | H | 2-(1,3-$Me_2$-nPen) |
| 5-56 | 1,2,3-benzothiadiazol-7-yl | Me | $CHF_2$ | H | 2-(1,4-$Me_2$-nPen) |
| 5-57 | 1,2,3-benzothiadiazol-7-yl | Me | $CHF_2$ | F | 2-(1,3-$Me_2$-nBu) |
| 5-58 | 1,2,3-benzothiadiazol-7-yl | Me | Me | F | 2-(1,3-$Me_2$-nBu) |
| 5-59 | 4-$CF_3$-2-Me-thiazole-5-yl | Me | $CF_3$ | H | 2-(1,3-$Me_2$-nBu) |
| 5-60 | 4-$CF_3$-2-Me-thiazole-5-yl | Me | $CHF_2$ | H | 2-(2-cPr-cPr) |
| 5-61 | 4-$CF_3$-2-Me-thiazole-5-yl | Me | $CHF_2$ | H | 2-nBu |
| 5-62 | 4-$CF_3$-2-Me-thiazole-5-yl | Me | $CHF_2$ | H | 2-(3-Me-nBu) |

(continued)

| No. | $R^1$ | $R^6$ | $R^7$ | $R^8$ | Xm |
|---|---|---|---|---|---|
| 5-63 | 4-$CF_3$-2-Me-thiazole-5-yl | Me | $CHF_2$ | H | 2-(1,3-$Me_2$-nBu) |
| 5-64 | 4-$CF_3$-2-Me-thiazole-5-yl | Me | $CHF_2$ | H | 2-(1,3,3-$Me_3$-nBu) |
| 5-65 | 4-$CF_3$-2-Me-thiazole-5-yl | Me | $CHF_2$ | H | 2-(1,3-$Me_2$-nPen) |
| 5-66 | 4-$CF_3$-2-Me-thiazole-5-yl | Me | $CHF_2$ | H | 2-(1,4-$Me_2$-nPen) |
| 5-67 | 4-$CF_3$-2-Me-thiazole-5-yl | Me | $CHF_2$ | F | 2-(1,3-$Me_2$-nBu) |
| 5-68 | 4-$CF_3$-2-Me-thiazole-5-yl | Me | Me | F | 2-(1,3-$Me_2$-nBu) |
| 5-69 | 4-$CHF_2$-2-Me-thiazole-5-yl | Me | $CF_3$ | H | 2-(1,3-$Me_2$-nBu) |
| 5-70 | 4-$CHF_2$-2-Me-thiazole-5-yl | Me | $CHF_2$ | H | 2-(2-cPr-cPr) |
| 5-71 | 4-$CHF_2$-2-Me-thiazole-5-yl | Me | $CHF_2$ | H | 2-nBu |
| 5-72 | 4-$CHF_2$-2-Me-thiazole-5-yl | Me | $CHF_2$ | H | 2-(3-Me-nBu) |
| 5-73 | 4-$CHF_2$-2-Me-thiazole-5-yl | Me | $CHF_2$ | H | 2-(1,3-$Me_2$-nBu) |
| 5-74 | 4-$CHF_2$-2-Me-thiazole-5-yl | Me | $CHF_2$ | H | 2-(1,3,3-$Me_3$-nBu) |
| 5-75 | 4-$CHF_2$-2-Me-thiazole-5-yl | Me | $CHF_2$ | H | 2-(1,3-$Me_2$-nPen) |
| 5-76 | 4-$CHF_2$-2-Me-thiazole-5-yl | Me | $CHF_2$ | H | 2-(1,4-$Me_2$-nPen) |
| 5-77 | 4-$CHF_2$-2-Me-thiazole-5-yl | Me | $CHF_2$ | F | 2-(1,3-$Me_2$-nBu) |
| 5-78 | 4-$CHF_2$-2-Me-thiazole-5-yl | Me | Me | F | 2-(1,3-$Me_2$-nBu) |
| 5-79 | 4-$CF_3$-thiazole-5-yl | Me | $CF_3$ | H | 2-(1,3-$Me_2$-nBu) |
| 5-80 | 4-$CF_3$-thiazole-5-yl | Me | $CHF_2$ | H | 2-(2-cPr-cPr) |
| 5-81 | 4-$CF_3$-thiazole-5-yl | Me | $CHF_2$ | H | 2-nBu |

[Table 5-4]

| No. | $R^1$ | $R^6$ | $R^7$ | $R^8$ | Xm |
|---|---|---|---|---|---|
| 5-82 | 4-$CF_3$-thiazole-5-yl | Me | $CHF_2$ | H | 2-(3-Me-nBu) |
| 5-83 | 4-$CF_3$-thiazole-5-yl | Me | $CHF_2$ | H | 2-(1,3-$Me_2$-nBu) |
| 5-84 | 4-$CF_3$-thiazole-5-yl | Me | $CHF_2$ | H | 2-(1,3,3-$Me_3$-nBu) |
| 5-85 | 4-$CF_3$-thiazole-5-yl | Me | $CHF_2$ | H | 2-(1,3-$Me_2$-nPen) |
| 5-86 | 4-$CF_3$-thiazole-5-yl | Me | $CHF_2$ | H | 2-(1,4-$Me_2$-nPen) |
| 5-87 | 4-$CF_3$-thiazole-5-yl | Me | $CHF_2$ | F | 2-(1,3-$Me_2$-nBu) |
| 5-88 | 4-$CF_3$-thiazole-5-yl | Me | Me | F | 2-(1,3-$Me_2$-nBu) |
| 5-89 | 4-$CHF_2$-thiazole-5-yl | Me | $CF_3$ | H | 2-(1,3-$Me_2$-nBu) |
| 5-90 | 4-$CHF_2$-thiazole-5-yl | Me | $CHF_2$ | H | 2-(2-cPr-cPr) |
| 5-91 | 4-$CHF_2$-thiazole-5-yl | Me | $CHF_2$ | H | 2-nBu |
| 5-92 | 4-$CHF_2$-thiazole-5-yl | Me | $CHF_2$ | H | 2-(3-Me-nBu) |
| 5-93 | 4-$CHF_2$-thiazole-5-yl | Me | $CHF_2$ | H | 2-(1,3-$Me_2$-nBu) |
| 5-94 | 4-$CHF_2$-thiazole-5-yl | Me | $CHF_2$ | H | 2-(1,3,3-$Me_3$-nBu) |
| 5-95 | 4-$CHF_2$-thiazole-5-yl | Me | $CHF_2$ | H | 2-(1,3-$Me_2$-nPen) |
| 5-96 | 4-$CHF_2$-thiazole-5-yl | Me | $CHF_2$ | H | 2-(1,4-$Me_2$-nPen) |

(continued)

| No. | $R^1$ | $R^6$ | $R^7$ | $R^8$ | Xm |
|---|---|---|---|---|---|
| 5-97 | 4-$CHF_2$-thiazole-5-yl | Me | $CHF_2$ | F | 2-(1,3-$Me_2$-nBu) |
| 5-98 | 4-$CHF_2$-thiazole-5-yl | Me | Me | F | 2-(1,3-$Me_2$-nBu) |

[Table 6-1]

Table 6

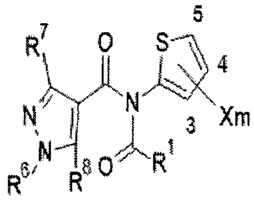

| No. | $R^1$ | $R^6$ | $R^7$ | $R^8$ | Xm |
|---|---|---|---|---|---|
| 6-1 | 3,4-$Cl_2$-isothiazol-5-yl | Me | $CF_3$ | H | 3-(1,3-$Me_2$-nBu) |
| 6-2 | 3,4-$Cl_2$-isothiazol-5-yl | Me | $CHF_2$ | H | 3-(1,3-$Me_2$-nBu) |
| 6-3 | 3,4-$Cl_2$-isothiazol-5-yl | Me | $CHF_2$ | F | 3-(1,3-$Me_2$-nBu) |
| 6-4 | 3,4-$Cl_2$-isothiazol-5-vl | Me | Me | F | 3-(1,3-$Me_2$-nBu) |
| 6-5 | 3-Me-isothiazol-5-yl | Me | $CF_3$ | H | 3-(1,3-$Me_2$-nBu) |
| 6-6 | 3-Me-isothiazol-5-yl | Me | $CHF_2$ | H | 3-(1,3-$Me_2$-nBu) |
| 6-7 | 3-Me-isothiazol-5-yl | Me | $CHF_2$ | F | 3-(1,3-$Me_2$-nBu) |
| 6-8 | 3-Me-isothiazol-5-yl | Me | Me | F | 3-(1,3-$Me_2$-nBu) |
| 6-9 | 3-Me-4-Cl-isothiazol-5-yl | Me | $CF_3$ | H | 3-(1,3-$Me_2$-nBu) |
| 6-10 | 3-Me-4-Cl-isothiazol-5-yl | Me | $CHF_2$ | H | 3-(1,3-$Me_2$-nBu) |
| 6-11 | 3-Me-4-Cl-isothiazol-5-yl | Me | $CHF_2$ | F | 3-(1,3-$Me_2$-nBu) |
| 6-12 | 3-Me-4-Cl-isothiazol-5-yl | Me | Me | F | 3-(1,3-$Me_2$-nBu) |
| 6-13 | 4-Me-1,2,3-thiadiazol-5-yl | Me | $CF_3$ | H | 3-(1,3-$Me_2$-nBu) |
| 6-14 | 4-Me-1,2,3-thiadiazol-5-yl | Me | $CHF_2$ | H | 3-(1,3-$Me_2$-nBu) |
| 6-15 | 4-Me-1,2,3-thiadiazol-5-yl | Me | $CHF_2$ | F | 3-(1,3-$Me_2$-nBu) |
| 6-16 | 4-Me-1,2,3-thiadiazol-5-yl | Me | Me | F | 3-(1,3-$Me_2$-nBu) |
| 6-17 | 4-$CF_3$-1,2,3-thiadiazol-5-yl | Me | $CF_3$ | H | 3-(1,3-$Me_2$-nBu) |
| 6-18 | 4-$CF_3$-1,2,3-thiadiazol-5-yl | Me | $CHF_2$ | H | 3-(1,3-$Me_2$-nBu) |
| 6-19 | 4-$CF_3$-1,2,3-thiadiazol-5-yl | Me | $CHF_2$ | F | 3-(1,3-$Me_2$-nBu) |
| 6-20 | 4-$CF_3$-1,2,3-thiadiazol-5-yl | Me | Me | F | 3-(1,3-$Me_2$-nBu) |
| 6-21 | 4-$CHF_2$-1,2,3-thiadiazol-5-yl | Me | $CF_3$ | H | 3-(1,3-$Me_2$-nBu) |
| 6-22 | 4-$CHF_2$-1,2,3-thiadiazol-5-yl | Me | $CHF_2$ | H | 3-(1,3-$Me_2$-nBu) |
| 6-23 | 4-$CHF_2$-1,2,3-thiadiazol-5-yl | Me | $CHF_2$ | F | 3-(1,3-$Me_2$-nBu) |

[Table 6-2]

| No. | $R^1$ | $R^6$ | $R^7$ | $R^8$ | Xm |
|---|---|---|---|---|---|
| 6-24 | 4-$CHF_2$-1,2,3-thiadiazol-5-yl | Me | Me | F | 3-(1,3-$Me_2$-nBu) |

(continued)

| No. | $R^1$ | $R^6$ | $R^7$ | $R^8$ | Xm |
|---|---|---|---|---|---|
| 6-25 | 1,2,3-benzothiadiazol-7-yl | Me | $CF_3$ | H | 3-(1,3-$Me_2$-nBu) |
| 6-26 | 1,2,3-benzothiadiazol-7-yl | Me | $CHF_2$ | H | 3-(1,3-$Me_2$-nBu) |
| 6-27 | 1,2,3-benzothiadiazol-7-yl | Me | $CHF_2$ | F | 3-(1,3-$Me_2$-nBu) |
| 6-28 | 1,2,3-benzothiadiazol-7-yl | Me | Me | F | 3-(1,3-$Me_2$-nBu) |
| 6-29 | 4-$CF_3$-2-Me-thiazole-5-yl | Me | $CF_3$ | H | 3-(1,3-$Me_2$-nBu) |
| 6-30 | 4-$CF_3$-2-Me-thiazole-5-yl | Me | $CHF_2$ | H | 3-(1,3-$Me_2$-nBu) |
| 6-31 | 4-$CF_3$-2-Me-thiazole-5-yl | Me | $CHF_2$ | F | 3-(1,3-$Me_2$-nBu) |
| 6-32 | 4-$CF_3$-2-Me-thiazole-5-yl | Me | Me | F | 3-(1,3-$Me_2$-nBu) |
| 6-33 | 4-$CHF_2$-2-Me-thiazole-5-yl | Me | $CF_3$ | H | 3-(1,3-$Me_2$-nBu) |
| 6-34 | 4-$CHF_2$-2-Me-thiazole-5-yl | Me | $CHF_2$ | H | 3-(1,3-$Me_2$-nBu) |
| 6-35 | 4-$CHF_2$-2-Me-thiazole-5-yl | Me | $CHF_2$ | F | 3-(1,3-$Me_2$-nBu) |
| 6-36 | 4-$CHF_2$-2-Me-thiazole-5-yl | Me | Me | F | 3-(1,3-$Me_2$-nBu) |
| 6-37 | 4-$CF_3$-thiazole-5-yl | Me | $CF_3$ | H | 3-(1,3-$Me_2$-nBu) |
| 6-38 | 4-$CF_3$-thiazole-5-yl | Me | $CHF_2$ | H | 3-(1,3-$Me_2$-nBu) |
| 6-39 | 4-$CF_3$-thiazole-5-yl | Me | $CHF_2$ | F | 3-(1,3-$Me_2$-nBu) |
| 6-40 | 4-$CF_3$-thiazole-5-yl | Me | Me | F | 3-(1,3-$Me_2$-nBu) |
| 6-41 | 4-$CHF_2$-thiazole-5-yl | Me | $CF_3$ | H | 3-(1,3-$Me_2$-nBu) |
| 6-42 | 4-$CHF_2$-thiazole-5-yl | Me | $CHF_2$ | H | 3-(1,3-$Me_2$-nBu) |
| 6-43 | 4-$CHF_2$-thiazole-5-yl | Me | $CHF_2$ | F | 3-(1,3-$Me_2$-nBu) |
| 6-44 | 4-$CHF_2$-thiazole-5-yl | Me | Me . | F | 3-(1,3-$Me_2$-nBu) |

[Table 7-1]

Table 7

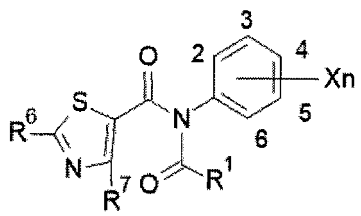

| No. | $R^1$ | $R^6$ | $R^7$ | Xn |
|---|---|---|---|---|
| 7-1 | 3,4-$Cl_2$-isothiazol-5-vl | H | $CF_3$ | 2-(1,3-$Me_2$-nBu) |
| 7-2 | 3,4-$Cl_2$-isothiazol-5-yl | H | $CHF_2$ | 2-(1,3-$Me_2$-nBu) |
| 7-3 | 3,4-$Cl_2$-isothiazol-5-yl | Me | $CF_3$ | 2-nBu |
| 7-4 | 3,4-$Cl_2$-isothiazol-5-yl | Me | $CF_3$ | 2-(3-Me-nBu) |
| 7-5 | 3,4-$Cl_2$-isothiazol-5-yl | Me | $CF_3$ | 2-(1,3-$Me_2$-nBu) |
| 7-6 | 3,4-$Cl_2$-isothiazol-5-yl | Me | $CF_3$ | 3-(1,3-$Me_2$-nBu) |
| 7-7 | 3,4-$Cl_2$-isothiazol-5-vl | Me | $CF_3$ | 2-(3,3-$Me_2$-nBu) |
| 7-8 | 3,4-$Cl_2$-isothiazol-5-yl | Me | $CF_3$ | 2-(1,3,3-$Me_3$-nBu) |
| 7-9 | 3,4-$Cl_2$-isothiazol-5-yl | Me | $CHF_2$ | 2-Et |

(continued)

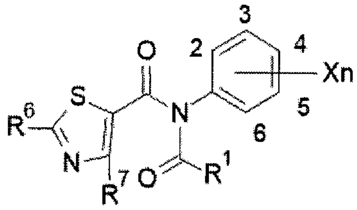

Table 7

| No. | $R^1$ | $R^6$ | $R^7$ | Xn |
|---|---|---|---|---|
| 7-10 | 3,4-$Cl_2$-isothiazol-5-vl | Me | $CHF_2$ | 2-nPr |
| 7-11 | 3,4-$Cl_2$-isothiazol-5-yl | Me | $CHF_2$ | 2-iPr |
| 7-12 | 3,4-$Cl_2$-isothiazol-5-yl | Me | $CHF_2$ | 2-(1-Et-nPr) |
| 7-13 | 3,4-$Cl_2$-isothiazol-5-yl | Me | $CHF_2$ | 2-(3-cPr-nPr) |
| 7-14 | 3,4-$Cl_2$-isothiazol-5-yl | Me | $CHF_2$ | 2-(3-cBu-nPr) |
| 7-15 | 3,4-$Cl_2$-isothiazol-5-yl | Me | $CHF_2$ | 2-(3-cPen-nPr) |
| 7-16 | 3,4-$Cl_2$-isothiazol-5-yl | Me | $CHF_2$ | 2-(3-cHex-nPr) |
| 7-17 | 3,4-$Cl_2$-isothiazol-5-yl | Me | $CHF_2$ | 2-(3-(2-tetrahydrofuranyl)-nPr) |
| 7-18 | 3,4-$Cl_2$-isothiazol-5-yl | Me | $CHF_2$ | 2-(3 -(3 -tetrahydrofuranyl)-nPr) |
| 7-19 | 3,4-$Cl_2$-isothiazol-5-yl | Me | $CHF_2$ | 2-(1-Me-3-cPr-nPr) |
| 7-20 | 3,4-$Cl_2$-isothiazol-5-yl | Me | $CHF_2$ | 2-(3-cBu-1-Me-nPr) |
| 7-21 | 3,4-$Cl_2$-isothiazol-5-yl | Me | $CHF_2$ | 2-(1-Me-3-cPen-nPr) |
| 7-22 | 3,4-$Cl_2$-isothiazol-5-yl | Me | $CHF_2$ | 2-(3-cHex-1-Me-nPr) |
| 7-23 | 3,4-$Cl_2$-isothiazol-5-yl | Me | $CHF_2$ | 2-(1-Me-3-(2-tetrahydrofuranyl)-nPr) |
| 7-24 | 3,4-$Cl_2$-isothiazol-5-yl | Me | $CHF_2$ | 2-(1-Me-3-(3-tetrahydrofuranyl)-nPr) |
| 7-25 | 3,4-$Cl_2$-isothiazol-5-yl | Me | $CHF_2$ | 2-[1-Me-3-(1-Me-cPr)-nPr)] |

[Table 7-2]

| No. | $R^1$ | $R^6$ | $R^7$ | Xn |
|---|---|---|---|---|
| 7-26 | 3,4-$Cl_2$-isothiazol-5-yl | Me | $CHF_2$ | 2-[1-Me-3-(1-Me-cBu)-nPr)] |
| 7-27 | 3,4-$Cl_2$-isothiazol-5-yl | Me | $CHF_2$ | 2-[1-Me-3-(1-Me-cPen)-nPr)] |
| 7-28 | 3,4-$Cl_2$-isothiazol-5-yl | Me | $CHF_2$ | 2-[1-Me-3-(1-Me-cHex)-nPr)] |
| 7-29 | 3,4-$Cl_2$-isothiazol-5-yl | Me | $CHF_2$ | 2-(1-Me-cPr) |
| 7-30 | 3,4-$Cl_2$-isothiazol-5-yl | Me | $CHF_2$ | 2-(2-Me-cPr) |
| 7-31 | 3,4-$Cl_2$-isothiazol-5-yl | Me | $CHF_2$ | 2-(2-iPr-cPr) |
| 7-32 | 3,4-$Cl_2$-isothiazol-5-yl | Me | $CHF_2$ | 2-(2-cPr-cPr) |
| 7-33 | 3,4-$Cl_2$-isothiazol-5-yl | Me | $CHF_2$ | 2-nBu |
| 7-34 | 3,4-$Cl_2$-isothiazol-5-yl | Me | $CHF_2$ | 2-sBu |
| 7-35 | 3,4-$Cl_2$-isothiazol-5-yl | Me | $CHF_2$ | 2-(1-Me-nBu) |
| 7-36 | 3,4-$Cl_2$-isothiazol-5-yl | Me | $CHF_2$ | 2-(2-Me-nBu) |
| 7-37 | 3,4-$Cl_2$-isothiazol-5-yl | Me | $CHF_2$ | 2-(3-Me-nBu) |
| 7-38 | 3,4-$Cl_2$-isothiazol-5-yl | Me | $CHF_2$ | 2-(1,3-$Me_2$-nBu) |

(continued)

| No. | $R^1$ | $R^6$ | $R^7$ | Xn |
|---|---|---|---|---|
| 7-39 | 3,4-$Cl_2$-isothiazol-5-yl | Me | $CHF_2$ | 3-(1,3-$Me_2$-nBu) |
| 7-40 | 3,4-$Cl_2$-isothiazol-5-yl | Me | $CHF_2$ | 2-(3,3-$Me_2$-nBu) |
| 7-41 | 3,4-$Cl_2$-isothiazol-5-vl | Me | $CHF_2$ | 2-(1,3,3-$Me_3$-nBu) |
| 7-42 | 3,4-$Cl_2$-isothiazol-5-yl | Me | $CHF_2$ | 2-(1-Et-3-Me-nBu) |
| 7-43 | 3,4-$Cl_2$-isothiazol-5-yl | Me | $CHF_2$ | 2-(1-Et-3,3-$Me_2$-nBu) |
| 7-44 | 3,4-$Cl_2$-isothiazol-5-yl | Me | $CHF_2$ | 2-nPen |
| 7-45 | 3,4-$Cl_2$-isothiazol-5-yl | Me | $CHF_2$ | 2-(1-Me-nPen) |
| 7-46 | 3,4-$Cl_2$-isothiazol-5-yl | Me | $CHF_2$ | 2-(1,3-$Me_2$-nPen) |
| 7-47 | 3,4-$Cl_2$-isothiazol-5-yl | Me | $CHF_2$ | 2-(1,4-$Me_2$-nPen) |
| 7-48 | 3,4-$Cl_2$-isothiazol-5-yl | Me | $CHF_2$ | 2-nHex |
| 7-49 | 3,4-$Cl_2$-isothiazol-5-yl | Me | $CHF_2$ | 2-(1-Me-nHex) |
| 7-50 | 3,4-$Cl_2$-isothiazol-5-yl | Me | $CHF_2$ | 2-(1-Me-nHep) |
| 7-51 | 3,4-$Cl_2$-isothiazol-5-yl | Me | $CHF_2$ | 2-(1-OEt-Et) |
| 7-52 | 3,4-$Cl_2$-isothiazol-5-yl | Me | $CHF_2$ | 2-(1-Me-2-OMe-Et) |
| 7-53 | 3,4-$Cl_2$-isothiazol-5-yl | Me | $CHF_2$ | 2-(1-OiPr-Et) |
| 7-54 | 3,4-$Cl_2$-isothiazol-5-yl | Me | $CHF_2$ | 2-(4,4-$Cl_2$-1,3-$Me_2$-3-butenyl) |
| 7-55 | 3,4-$Cl_2$-isothiazol-5-yl | Me | $CHF_2$ | 2-(4,4-$Cl_2$-1-Me-3-butenyl) |

[Table 7-3]

| No. | $R^1$ | $R^6$ | $R^7$ | Xn |
|---|---|---|---|---|
| 7-56 | 3,4-$Cl_2$-isothiazol-5-yl | Me | $CHF_2$ | 2-(1-Me-2-CN-Et) |
| 7-57 | 3,4-$Cl_2$-isothiazol-5-yl | Me | $CHF_2$ | 2-(2-CN-1,3-$Me_2$-nBu) |
| 7-58 | 3,4-$Cl_2$-isothiazol-5-yl | Me | $CHF_2$ | 2-(2-CN-1-Me-nBu) |
| 7-59 | 3,4-$Cl_2$-isothiazol-5-yl | Me | $CHF_2$ | 2-(2,2-$CN_2$-1-Me-Et) |
| 7-60 | 3,4-$Cl_2$-isothiazol-5-yl | Me | $CHF_2$ | 2-(3-CN-1-Me-nBu) |
| 7-61 | 3,4-$Cl_2$-isothiazol-5-yl | Me | $CHF_2$ | 2-(1-CN-3-Me-nBu) |
| 7-62 | 3,4-$Cl_2$-isothiazol-5-yl | Me | $CHF_2$ | 2-(1-Me-3-CN-nBu) |
| 7-63 | 3,4-$Cl_2$-isothiazol-5-yl | Me | $CHF_2$ | 2-(2-$CF_3$-1-Me-Et) |
| 7-64 | 3,4-$Cl_2$-isothiazol-5-yl | Me | $CHF_2$ | 2-Ph |
| 7-65 | 3,4-$Cl_2$-isothiazol-5-yl | Me | $CHF_2$ | 2-(4-F-Ph) |
| 7-66 | 3,4-$Cl_2$-isothiazol-5-yl | Me | $CHF_2$ | 2-(4-Cl-Ph) |
| 7-67 | 3,4-$Cl_2$-isothiazol-5-yl | Me | $CHF_2$ | 2-(3,4-$F_2$-Ph) |
| 7-68 | 3,4-$Cl_2$-isothiazol-5-yl | Me | $CHF_2$ | 2-(3,4,5-$F_3$-Ph) |
| 7-69 | 3,4-$Cl_2$-isothiazol-5-yl | Me | $CHF_2$ | 2-Bn |
| 7-70 | 3,4-$Cl_2$-isothiazol-5-yl | Me | $CHF_2$ | 2-(4-F-Bn) |
| 7-71 | 3,4-$Cl_2$-isothiazol-5-yl | Me | $CHF_2$ | 2-(4-Cl-Bn) |
| 7-72 | 3,4-$Cl_2$-isothiazol-5-yl | Me | $CHF_2$ | 2-(3,4-$F_2$-Bn) |

(continued)

| No. | $R^1$ | $R^6$ | $R^7$ | Xn |
|---|---|---|---|---|
| 7-73 | 3,4-$Cl_2$-isothiazol-5-yl | Me | $CHF_2$ | 2-(3,4,5-$F_3$-Bn) |
| 7-74 | 3,4-$Cl_2$-isothiazol-5-yl | Me | $CHF_2$ | 2-$OCH_2$cPr |
| 7-75 | 3,4-$Cl_2$-isothiazol-5-yl | Me | $CHF_2$ | 2-sBuO |
| 7-76 | 3,4-$Cl_2$-isothiazol-5-yl | Me | $CHF_2$ | 2-iBuO |
| 7-77 | 3,4-$Cl_2$-isothiazol-5-yl | Me | $CHF_2$ | 2-tBuO |
| 7-78 | 3,4-$Cl_2$-isothiazol-5-yl | Me | $CHF_2$ | 2-cPenO |
| 7-79 | 3,4-$Cl_2$-isothiazol-5-yl | Me | $CHF_2$ | 2-cHexO |
| 7-80 | 3,4-$Cl_2$-isothiazol-5-yl | Me | $CHF_2$ | 2-PhO |
| 7-81 | 3,4-$Cl_2$-isothiazol-5-yl | Me | $CHF_2$ | 2-(2-Cl-PhO) |
| 7-82 | 3,4-$Cl_2$-isothiazol-5-yl | Me | $CHF_2$ | 2-(4-Cl-PhO) |
| 7-83 | 3,4-$Cl_2$-isothiazol-5-yl | Me | $CHF_2$ | 2-(3,4-$F_2$-PhO) |
| 7-84 | 3,4-$Cl_2$-isothiazol- 5-yl | Me | $CHF_2$ | 2-(3,4,5-$F_3$-PhO) |
| 7-85 | 3,4-$Cl_2$-isothiazol-5-yl | Me | $CHF_2$ | 2-PhS |

[Table 7-4]

| No. | $R^1$ | $R^6$ | $R^7$ | Xn |
|---|---|---|---|---|
| 7-86 | 3,4-$Cl_2$-isothiazol-5-yl | Me | $CHF_2$ | 2-(4-F-PhS) |
| 7-87 | 3,4-$Cl_2$-isothiazol-5-yl | Me | $CHF_2$ | 2-(4-Cl-PhS) |
| 7-88 | 3,4-$Cl_2$-isothiazol-5-yl | Me | $CHF_2$ | 2-(3,4-$F_2$-PhS) |
| 7-89 | 3,4-$Cl_2$-isothiazol-5-yl | Me | $CHF_2$ | 2-(3,4,5-$F_3$-PhS) |
| 7-90 | 3,4-$Cl_2$-isothiazol-5-yl | Me | $CHF_2$ | 2-$NEt_2$ |
| 7-91 | 3,4-$Cl_2$-isothiazol-5-yl | Me | $CHF_2$ | 2-N(Me)nBu |
| 7-92 | 3,4-$Cl_2$-isothiazol-5-yl | Me | $CHF_2$ | 2-N(Me)iBu |
| 7-93 | 3,4-$Cl_2$-isothiazol-5-yl | Me | $CHF_2$ | 2-$CH_2$S-sBu |
| 7-94 | 3,4-$Cl_2$-isothiazol-5-yl | Me | $CHF_2$ | 2-$CH_2$S-tBu |
| 7-95 | 3,4-$Cl_2$-isothiazol-5-yl | Me | $CHF_2$ | 2-CH(Me )S-iPr |
| 7-96 | 3,4-$Cl_2$-isothiazol-5-yl | Me | $CHF_2$ | 2-CH(Me)S-sBu |
| 7-97 | 3,4-$Cl_2$-isothiazol1-5-yl | Me | $CHF_2$ | 2-CH(Me)S-cPen |
| 7-98 | 3,4-$Cl_2$-isothiazol-5-yl | Me | $CHF_2$ | 2-Cl |
| 7-99 | 3,4-$Cl_2$-isothiazol-5-yl | Me | $CHF_2$ | 2-Br |
| 7-100 | 3,4-$Cl_2$-isothiazol-5-yl | Me | $CHF_2$ | 2,3-$Me_2$ |
| 7-101 | 3,4-$Cl_2$-isothiazol-5-yl | Me | $CHF_2$ | 2-iPr-3-Me |
| 7-102 | 3,4-$Cl_2$-isothiazol-5-yl | Me | $CHF_2$ | 2-Et-3-iPr |
| 7-103 | 3,4-$Cl_2$-isothiazol-5-yl | Me | $CHF_2$ | 2-Et-3-cPr |
| 7-104 | 3,4-$Cl_2$-isothiazol-5-yl | Me | $CHF_2$ | 2-cPr-3-Et |
| 7-105 | 3,4-$Cl_2$-isothiazol-5-yl | Me | $CHF_2$ | 2-Me-3-cPr |
| 7-106 | 3,4-$Cl_2$-isothiazol-5-yl | Me | $CHF_2$ | 2-iPr-3-iPr |

(continued)

| No. | $R^1$ | $R^6$ | $R^7$ | Xn |
|---|---|---|---|---|
| 7-107 | 3,4-$Cl_2$-isothiazol-5-yl | Me | $CHF_2$ | 2-nBu-3-Me |
| 7-108 | 34-$Cl_2$-isothiazol-5-yl | Me | $CHF_2$ | 3-Me-2-(1-Me-nBu) |
| 7-109 | 3,4-$Cl_2$-isothiazol-5-yl | Me | $CHF_2$ | 2-Me-3-(1-Me-nBu) |
| 7-110 | 3,4-$Cl_2$-isothiazol-5-yl | Me | $CHF_2$ | 2-(1,3-$Me_2$-nBu)-4-Me |
| 7-111 | 3,4-$Cl_2$-isothiazol-5-yl | Me | $CHF_2$ | 2-Et-3-tBu |
| 7-112 | 3,4-$Cl_2$-isothiazol-5-yl | Me | $CHF_2$ | 2-vinyl-3-tBu |
| 7-113 | 3,4-$Cl_2$-isothiazol-5-yl | Me | $CHF_2$ | 2-nPr-4-F |
| 7-114 | 3,4-$Cl_2$-isothiazol-5-yl | Me | $CHF_2$ | 2-iPr-4-F |
| 7-115 | 3,4-$Cl_2$-isothiazol-5-yl | Me | $CHF_2$ | 2-(2-iPr-cPr)-4-F |

[Table 7-5]

| No. | $R^1$ | $R^6$ | $R^7$ | Xn |
|---|---|---|---|---|
| 7-116 | 3,4-$Cl_2$-isothiazol-5-yl | Me | $CHF_2$ | 2-(2-cPr-cPr)-4-F |
| 7-117 | 3,4-$Cl_2$-isothiazol-5-yl | Me | $CHF_2$ | 2-nBu-4-F |
| 7-118 | 3,4-$Cl_2$-isothiazol-5-yl | Me | $CHF_2$ | 2-(1,3-$Me_2$-nBu)-4-F |
| 7-119 | 3,4-Ch-isothiazol-5-yl | Me | $CHF_2$ | 2-sBu-4-F |
| 7-120 | 3,4-$Cl_2$-isothiazol-5-yl | Me | $CHF_2$ | 2-nPen-4-F |
| 7-121 | 3,4-$Cl_2$-isothiazol-5-yl | Me | $CHF_2$ | 2-cPen-4-F |
| 7-122 | 3,4-$Cl_2$-isothiazol-5-yl | Me | $CHF_2$ | 2-(3,4-$Cl_2$-Ph)-4-F |
| 7-123 | 3,4-$Cl_2$-isothiazol-5-yl | Me | $CHF_2$ | 3-Me-2-nPrO |
| 7-124 | 3,4-$Cl_2$-isothiazol-5-yl | Me | $CHF_2$ | 2-Me-3-OiPr |
| 7-125 | 3,4-$Cl_2$-isothiazol-5-yl | Me | $CHF_2$ | 2-Me-3-OsBu |
| 7-126 | 3,4-$Cl_2$-isothiazol-5-yl | Me | $CHF_2$ | 2-nBu-4-MeO |
| 7-127 | 3,4-$Cl_2$-isothiazol-5-yl | Me | $CHF_2$ | 2-nPrO-4-F |
| 7-128 | 3,4-$Cl_2$-isothiazol-5-yl | Me | $CHF_2$ | 2-nBuO-3-Cl |
| 7-129 | 3,4-$Cl_2$-isothiazol-5-yl | Me | $CHF_2$ | 2,6-$Br_2$-4-$OCF_3$ |
| 7-130 | 3,4-$Cl_2$-isothiazol-5-yl | Me | $CHF_2$ | 2-(1,3-$Me_2$-nBu) |
| 7-131 | 3,4-$Cl_2$-isothiazol-5-yl | Et | $CF_3$ | 2-(1,3-$Me_2$-nBu) |
| 7-132 | 3,4-$Cl_2$-isothiazol-5-yl | Et | $CHF_2$ | 2-(1,3-$Me_2$-nBu) |
| 7-133 | 3-Me-isothiazol-5-yl | H | $CF_3$ | 2-(1,3-$Me_2$-nBu) |
| 7-134 | 3-Me-isothiazol-5-yl | H | $CHF_2$ | 2-(1,3-$Me_2$-nBu) |
| 7-135 | 3-Me-isothiazol-5-yl | Me | $CF_3$ | 2-(1,3-$Me_2$-nBu) |
| 7-136 | 3-Me-isothiazol-5-yl | Me | $CHF_2$ | 2-(1,3-$Me_2$-nBu) |
| 7-137 | 3-Me-isothiazol-5-yl | Me | Me | 2-(1,3-$Me_2$-nBu) |
| 7-138 | 3-Me-isothiazol-5-yl | Et | $CF_3$ | 2-(1,3-$Me_2$-nBu) |
| 7-139 | 3-Me-isothiazol-5-yl | Et | $CHF_2$ | 2-(1,3-$Me_2$-nBu) |
| 7-140 | 3-Me-4-Cl-isothiazol-5-yl | H | $CF_3$ | 2-(1,3-$Me_2$-nBu) |

(continued)

| No. | $R^1$ | $R^6$ | $R^7$ | Xn |
|---|---|---|---|---|
| 7-141 | 3-Me-4-Cl-isothiazol-5-yl | H | $CHF_2$ | 2-(1,3-$Me_2$-nBu) |
| 7-142 | 3-Me-4-Cl-isothiazol-5-l | Me | $CF_3$ | 2-(1,3-$Me_2$-nBu) |
| 7-143 | 3-Me-4-Cl-isothiazol-5-yl | Me | $CHF_2$ | 2-(1,3-$Me_2$-nBu) |
| 7-144 | 3-Me-4-Cl-isothiazol-5-yl | Me | Me | 2-(1,3-$Me_2$-nBu) |
| 7-145 | 3-Me-4-Cl-isothiazol-5-yl | Et | $CF_3$ | 2-(1,3-$Me_2$-nBu) |

[Table 7-6]

| No. | $R^1$ | $R^6$ | $R^7$ | Xn |
|---|---|---|---|---|
| 7-146 | 3-Me-4-Cl-isothiazol-5-yl | Et | $CHF_2$ | 2-(1,3-$Me_2$-nBu) |
| 7-147 | 4-Me-1,2,3-thiadiazol-5-yl | H | $CF_3$ | 2-(1,3-$Me_2$-nBu) |
| 7-148 | 4-Me-1,2,3-thiadiazol-5-yl | H | $CHF_2$ | 2-(1,3-$Me_2$-nBu) |
| 7-149 | 4-Me-1,2,3-thiadiazol-5-yl | Me | $CF_3$ | 2-(1,3-$Me_2$-nBu) |
| 7-150 | 4-Me-1,2,3-thiadiazol-5-yl | Me | $CHF_2$ | 2-nBu |
| 7-151 | 4-Me-1,2,3-thiadiazol-5-yl | Me | $CHF_2$ | 2-(3-Me-nBu) |
| 7-152 | 4-Me-1,2,3-thiadiazol-5-yl | Me | $CHF_2$ | 2-(1,3-$Me_2$-nBu) |
| 7-153 | 4-Me-1,2,3-thiadiazol-5-yl | Me | $CHF_2$ | 3-(1,3-$Me_2$-nBu) |
| 7-154 | 4-Me-1,2,3-thiadiazol-5-yl | Me | $CHF_2$ | 2-(3,3-$Me_2$-nBu) |
| 7-155 | 4-Me-1,2,3-thiadiazol-5-yl | Me | $CHF_2$ | 2-(1,3,3-$Me_3$-nBu) |
| 7-156 | 4-Me-1,2,3-thiadiazol-5-yl | Me | Me | 2-(1,3-$Me_2$-nBu) |
| 7-157 | 4-Me-1,2,3-thiadiazol-5-yl | Et | $CF_3$ | 2-(1,3-$Me_2$-nBu) |
| 7-158 | 4-Me-1,2,3-thiadiazol-5-yl | Et | $CHF_2$ | 2-(1,3-$Me_2$-nBu) |
| 7-159 | 4-$CF_3$-1,2,3-thiadiazol-5-yl | H | $CF_3$ | 2-(1,3-$Me_2$-nBu) |
| 7-160 | 4-$CF_3$-1,2,3-thiadiazol-5-yl | H | $CHF_2$ | 2-(1,3-$Me_2$-nBu) |
| 7-161 | 4-$CF_3$-1,2,3-thiadiazol-5-yl | Me | $CF_3$ | 2-(1,3-$Me_2$-nBu) |
| 7-162 | 4-$CF_3$-1,2,3-thiadiazol-5-yl | Me | $CHF_2$ | 2-nBu |
| 7-163 | 4-$CF_3$-1,2,3-thiadiazol-5-yl | Me | $CHF_2$ | 2-(3-Me-nBu) |
| 7-164 | 4-$CF_3$-1,2,3-thiadiazol-5-yl | Me | $CHF_2$ | 2-(1,3-$Me_2$-nBu) |
| 7-165 | 4-$CF_3$-1,2,3-thiadiazol-5-yl | Me | $CHF_2$ | 3-(1,3-$Me_2$-nBu) |
| 7-166 | 4-$CF_3$-1,2,3-thiadiazol-5-yl | Me | $CHF_2$ | 2-(3,3-$Me_2$-nBu) |
| 7-167 | 4-$CF_3$-1,2,3-thiadiazol-5-yl | Me | $CHF_2$ | 2-(1,3,3-$Me_3$-nBu) |
| 7-168 | 4-$CF_3$-1,2,3-thiadiazol-5-yl | Me | Me | 2-(1,3-$Me_2$-nBu) |
| 7-169 | 4-$CF_3$-1,2,3-thiadiazol-5-yl | Et | $CF_3$ | 2-(1,3-$Me_2$-nBu) |
| 7-170 | 4-$CF_3$-1,2,3-thiadiazol-5-yl | Et | $CHF_2$ | 2-(1,3-$Me_2$-nBu) |
| 7-171 | 4-$CHF_2$-1,2,3-thiadiazol-5-yl | H | $CF_3$ | 2-(1,3-$Me_2$-nBu) |
| 7-172 | 4-$CHF_2$-1,2,3-thiadiazol-5-yl | H | $CHF_2$ | 2-(1,3-$Me_2$-nBu) |
| 7-173 | 4-$CHF_2$-1,2,3-thiadiazol-5-yl | Me | $CF_3$ | 2-(1,3-$Me_2$-nBu) |
| 7-174 | 4-$CHF_2$-1,2,3-thiadiazol-5-yl | Me | $CHF_2$ | 2-nBu |

(continued)

| No. | $R^1$ | $R^6$ | $R^7$ | Xn |
|---|---|---|---|---|
| 7-175 | 4-$CHF_2$-1,2,3-thiadiazol-5-yl | Me | $CHF_2$ | 2-(3-Me-nBu) |

[Table 7-7]

| No. | $R^1$ | $R^6$ | $R^7$ | Xn |
|---|---|---|---|---|
| 7-176 | 4-$CRF_2$-1,2,3-thiadiazol-5-yl | Me | $CHF_2$ | 2-(1,3-$Me_2$-nBu) |
| 7-177 | 4-$CHF_2$-1,2,3-thiadiazol-5-yl | Me | $CHF_2$ | 3-(1,3-$Me_2$-nBu) |
| 7-178 | 4-$CHF_2$-1,2,3-thiadiazol-5-yl | Me | $CHF_2$ | 2-(3,3-$Me_2$-nBu) |
| 7-179 | 4-$CHF_2$-1,2,3-thiadiazol-5-yl | Me | $CHF_2$ | 2-(1,3,3-$Me_3$-nBu) |
| 7-180 | 4-$CHF_2$-1,2,3-thiadiazol-5-yl | Me | Me | 2-(1,3-$Me_2$-nBu) |
| 7-181 | 4-$CHF_2$-1,2,3-thiadiazol-5-yl | Et | $CF_3$ | 2-(1,3-$Me_2$-nBu) |
| 7-182 | 4-$CHF_2$-1,2,3-thiadiazol-5-yl | Et | $CHF_2$ | 2-(1,3-$Me_2$-nBu) |
| 7-183 | 2,6-$Cl_2$-pyridin-4-yl | H | $CF_3$ | 2-(1,3-$Me_2$-nBu) |
| 7-184 | 2,6-$Cl_2$-pyridin-4-yl | H | $CHF_2$ | 2-(1,3-$Me_2$-nBu) |
| 7-185 | 2,6-$Cl_2$-pyridin-4-yl | Me | $CF_3$ | 2-(1,3-$Me_2$-nBu) |
| 7-186 | 2,6-$Cl_2$-pyridin-4-yl | Me | $CHF_2$ | 2-nBu |
| 7-187 | 2,6-$Cl_2$-pyridin-4-yl | Me | $CHF_2$ | 2-(3-Me-nBu) |
| 7-188 | 2,6-$Cl_2$-pyridin-4-yl | Me | $CHF_2$ | 2-(1,3-$Me_2$-nBu) |
| 7-189 | 2,6-$Cl_2$-pyridin-4-yl | Me | $CHF_2$ | 3-(1,3-$Me_2$-nBu) |
| 7-190 | 2,6-$Cl_2$-pyridin-4-yl | Me | $CHF_2$ | 2-(3,3-$Me_2$-nBu) |
| 7-191 | 2,6-$Cl_2$-pyridin-4-yl | Me | $CHF_2$ | 2-(1,3,3-$Me_3$-nBu) |
| 7-192 | 2,6-$Cl_2$-pyridin-4-yl | Me | Me | 2-(1,3-$Me_2$-nBu) |
| 7-193 | 2,6-$Cl_2$-pyridin-4-yl | Et | $CF_3$ | 2-(1,3-$Me_2$-nBu) |
| 7-194 | 2,6-$Cl_2$-pyridin-4-yl | Et | $CHF_2$ | 2-(1,3-$Me_2$-nBu) |
| 7-195 | 1,2,3-benzothiadiazol-7-yl | H | $CF_3$ | 2-(1,3-$Me_2$-nBu) |
| 7-196 | 1,2,3-benzothiadiazol-7-yl | H | $CHF_2$ | 2-(1,3-$Me_2$-nBu) |
| 7-197 | 1,2,3-benzothiadiazol-7-yl | Me | $CF_3$ | 2-(1,3-$Me_2$-nBu) |
| 7-198 | 1,2,3-benzothiadiazol-7-yl | Me | $CHF_2$ | 2-nBu |
| 7-199 | 1,2,3-benzothiadiazol-7-yl | Me | $CHF_2$ | 2-(3-Me-nBu) |
| 7-200 | 1,2,3-benzothiadiazol-7-yl | Me | $CHF_2$ | 2-(1,3-$Me_2$-nBu) |
| 7-201 | 1,2,3-benzothiadiazol-7-yl | Me | $CHF_2$ | 3-(1,3-$Me_2$-nBu) |
| 7-202 | 1,2,3-benzothiadiazol-7-yl | Me | $CHF_2$ | 2-(3,3-$Me_2$-nBu) |
| 7-203 | 1,2,3-benzothiadiazol-7-yl | Me | $CHF_2$ | 2-(1,3,3-$Me_3$-nBu) |
| 7-204 | 1,2,3-benzothiadiazol-7-yl | Me | Me | 2-(1,3-$Me_2$-nBu) |
| 7-205 | 1,2,3-benzothiadiazol-7-yl | Et | $CF_3$ | 2-(1,3-$Me_2$-nBu) |

[Table 7-8]

| No. | $R^1$ | $R^6$ | $R^7$ | Xn |
|---|---|---|---|---|
| 7-206 | 1,2,3-benzothiadiazol-7-yl | Et | $CHF_2$ | 2-(1,3-$Me_2$-nBu) |
| 7-207 | 4-$CF_3$-2-Et-thiazole-5-yl | H | $CF_3$ | 2-(1,3-$Me_2$-nBu) |
| 7-208 | 4-$CF_3$-2-Et-thiazole-5-yl | H | $CHF_2$ | 2-(1,3-$Me_2$-nBu) |
| 7-209 | 4-$CF_3$-2-Et-thiazole-5-yl | Me | $CF_3$ | 2-(1,3-$Me_2$-nBu) |
| 7-210 | 4-$CF_3$-2-Et-thiazole-5-yl | Me | $CHF_2$ | 2-(1,3-$Me_2$-nBu) |
| 7-211 | 4-$CF_3$-2-Et-thiazole-5-yl | Me | Me | 2-(1,3-$Me_2$-nBu) |
| 7-212 | 4-$CF_3$-2-Et-thiazole-5-yl | Et | $CF_3$ | 2-(1,3-$Me_2$-nBu) |
| 7-213 | 4-$CF_3$-2-Et-thiazole-5-yl | Et | $CHF_2$ | 2-(1,3-$Me_2$-nBu) |
| 7-214 | 4-$CHF_2$-2-Et-thiazole-5-yl | H | $CHF_2$ | 2-(1,3-$Me_2$-nBu) |
| 7-215 | 4-$CHF_2$-2-Et-thiazole-5-yl | Me | $CF_3$ | 2-(1,3-$Me_2$-nBu) |
| 7-216 | 4-$CHF_2$-2-Et-thiazole-5-yl | Me | $CHF_2$ | 2-(1,3-$Me_2$-nBu) |
| 7-217 | 4-$CHF_2$-2-Et-thiazole-5-yl | Me | Me | 2-(1,3-$Me_2$-nBu) |
| 7-218 | 4-$CHF_2$-2-Et-thiazole-5-yl | Et | $CF_3$ | 2-1,3-$Me_2$-nBu) |
| 7-219 | 4-$CHF_2$-2-Et-thiazole-5-vl | Et | $CHF_2$ | 2-(1,3-$Me_2$-nBu) |
| 7-220 | 4-$CHF_2$-2-Et-thiazole-5-yl | Me | Me | 2-(1,3-$Me_2$-nBu) |
| 7-221 | 4-$CF_3$-2-Me-thiazole-5-yl | H | $CF_3$ | 2-(1,3-$Me_2$-nBu) |
| 7-222 | 4-$CF_3$-2-Me-thiazole-5-yl | H | $CHF_2$ | 2-(1,3-$Me_2$-nBu) |
| 7-223 | 4-$CF_3$-2-Me-thiazole-5-yl | Me | $CF_3$ | 2-(1,3-$Me_2$-nBu) |
| 7-224 | 4-$CF_3$-2-Me-thiazo le-5-yl | Me | $CHF_2$ | 2-nBu |
| 7-225 | 4-$CF_3$-2-Me-thiazole-5-yl | Me | $CHF_2$ | 2-(3-Me-nBu) |
| 7-226 | 4-$CF_3$-2-Me-thiazole-5-yl | Me | $CHF_2$ | 2-(1,3-$Me_2$-nBu |
| 7-227 | 4-$CF_3$-2-Me-thiazole-5-yl | Me | $CHF_2$ | 3-(1,3-$Me_2$-nBu) |
| 7-228 | 4-$CF_3$-2-Me-thiazole-5-yl | Me | $CHF_2$ | 2-(3,3-$Me_2$-nBu) |
| 7-229 | 4-$CF_3$-2-Me-thiazole-5-yl | Me | $CHF_2$ | 2-(1,3,3-$Me_3$-nBu) |
| 7-230 | 4-$CF_3$-2-Me-thiazole-5-yl | Me | Me | 2-(1,3-$Me_2$-nBu) |
| 7-231 | 4-$CF_3$-2-Me-thiazole-5-yl | Et | $CF_3$ | 2-(1,3-$Me_2$-nBu) |
| 7-232 | 4-$CF_3$-2-Me-thiazole-5-yl | Et | $CHF_2$ | 2-(1,3-$Me_2$-nBu) |
| 7-233 | 4-$CHF_2$-2-Me-thiazole-5-yl | H | $CF_3$ | 2-(1,3-$Me_2$-nBu) |
| 7-234 | 4-$CHF_2$-2-Me-thiazole-5-yl | H | $CHF_2$ | 2-(1,3-$Me_2$-nBu) |
| 7-235 | 4-$CRF_2$-2-Me-thiazole-5-yl | Me | $CF_3$ | 2-(1,3-$Me_2$-nBu) |

[Table 7-9]

| No. | $R^1$ | $R^6$ | $R^7$ | Xn |
|---|---|---|---|---|
| 7-236 | 4-$CHF_2$-2-Me-thiazole-5-yl | Me | $CHF_2$ | 2-nBu |
| 7-237 | 4-$CHF_2$-2-Me-thiazole-5-yl | Me | $CHF_2$ | 2-(3-Me-nBu) |
| 7-238 | 4-$CHF_2$-2-Me-thiazole-5-yl | Me | $CHF_2$ | 2-(1,3-$Me_2$-nBu) |
| 7-239 | 4-$CHF_2$-2-Me-thiazole-5-yl | Me | $CHF_2$ | 3-(1,3-$Me_2$-nBu) |
| 7-240 | 4-$CHF_2$-2-Me-thiazole-5-yl | Me | $CHF_2$ | 2-(3,3-$Me_2$-nBu) |

(continued)

| No. | $R^1$ | $R^6$ | $R^7$ | Xn |
|---|---|---|---|---|
| 7-241 | 4-$CHF_2$-2-Me-thiazole-5-yl | Me | $CHF_2$ | 2-(1,3,3-$Me_3$-nBu) |
| 7-242 | 4-$CHF_2$-2-Me-thiazole-5-yl | Me | Me | 2-(1,3-$Me_2$-nBu) |
| 7-243 | 4-$CHF_2$-2-Me-thiazole-5-yl | Et | $CF_3$ | 2-(1,3-$Me_2$-nBu) |
| 7-244 | 4-$CHF_2$-2-Me-thiazole-5-yl | Et | $CHF_2$ | 2-(1,3-$Me_2$-nBu) |
| 7-245 | 4-$CF_3$-thiazole-5-yl | H | $CF_3$ | 2-(1,3-$Me_2$-nBu) |
| 7-246 | 4-$CF_3$-thiazole-5-yl | H | $CHF_2$ | 2-(1,3-$Me_2$-nBu) |
| 7-247 | 4-$CF_3$-thiazole-5-yl | Me | $CF_3$ | 2-(1,3-$Me_2$-nBu) |
| 7-248 | 4-$CF_3$-thiazole-5-yl | Me | $CHF_2$ | 2-nBu |
| 7-249 | 4-$CF_3$-thiazole-5-yl | Me | $CHF_2$ | 2-(3-Me-nBu) |
| 7-250 | 4-$CF_3$-thiazole-5-yl | Me | $CHF_2$ | 2-(1,3-$Me_2$-nBu) |
| 7-251 | 4-$CF_3$-thiazole-5-yl | Me | $CHF_2$ | 3-(1,3-$Me_2$-nBu) |
| 7-252 | 4-$CF_3$-thiazole-5-yl | Me | $CHF_2$ | 2-(3,3-$Me_2$-nBu) |
| 7-253 | 4-$CF_3$-thiazole-5-yl | Me | $CHF_2$ | 2-(1,3,3-$Me_3$-nBu) |
| 7-254 | 4-$CF_3$-thiazole-5-yl | Me | Me | 2-(1,3-$Me_2$-nBu) |
| 7-255 | 4-$CF_3$-thiazole-5-yl | Et | $CF_3$ | 2-(1,3-$Me_2$-nBu) |
| 7-256 | 4-$CF_3$-thiazole-5-yl | Et | $CHF_2$ | 2-(1,3-$Me_2$-nBu) |
| 7-257 | 4-$CHF_2$-thiazole-5-yl | H | $CF_3$ | 2-(1,3-$Me_2$-nBu) |
| 7-258 | 4-$CHF_2$-thiazole-5-yl | H | $CHF_2$ | 2-(1,3-$Me_2$-nBu) |
| 7-259 | 4-$CHF_2$-thiazole-5-yl | Me | $CF_3$ | 2-(1,3-$Me_2$-nBu) |
| 7-260 | 4-$CHF_2$-thiazole-5-yl | Me | $CHF_2$ | 2-nBu |
| 7-261 | 4-$CHF_2$-thiazole-5-yl | Me | $CHF_2$ | 2-(3-Me-nBu) |
| 7-262 | 4-$CHF_2$-thiazole-5-yl | Me | $CHF_2$ | 2-(1,3-$Me_2$-nBu) |
| 7-263 | 4-$CHF_2$-thiazole-5-yl | Me | $CHF_2$ | 3-(1,3-$Me_2$-nBu) |
| 7-264 | 4-$CHF_2$-thiazole-5-yl | Me | $CHF_2$ | 2-(3,3-$Me_2$-nBu) |
| 7-265 | 4-$CHF_2$-thiazole-5-yl | Me | $CHF_2$ | 2-(1,3,3-$Me_3$-nBu) |

[Table 7-10]

| No. | $R^1$ | $R^6$ | $R^7$ | Xn |
|---|---|---|---|---|
| 7-266 | 4-$CHF_2$-thiazole-5-yl | Me | Me | 2-(1,3-$Me_2$-nBu) |
| 7-267 | 4-$CHF_2$-thiazole-5-yl | Et | $CF_3$ | 2-(1,3-$Me_2$-nBu) |
| 7-268 | 4-$CHF_2$-thiazole-5-yl | Et | $CHF_2$ | 2-(1,3-$Me_2$-nBu) |
| 7-269 | 2-$CHF_2$-pyridin-3-yl | Me | $CF_3$ | 2-(1,3-$Me_2$-nBu) |
| 7-270 | 2-$CHF_2$-pyridin-3-yl | Me | $CHF_2$ | 2-(1,3-$Me_2$-nBu) |
| 7-271 | 5-Cl-3-$CRF_2$-1-Me-pyrazol-4-yl | Me | $CF_3$ | 2-(1,3-$Me_2$-nBu) |
| 7-272 | 5-Cl-3-$CHF_2$-1-Me-pyrazol-4-yl | Me | $CHF_2$ | 2-(1,3-$Me_2$-nBu) |
| 7-273 | 2,3-$Cl_2$-thiophen-5-yl | Me | $CF_3$ | 2-(1,3-$Me_2$-nBu) |
| 7-274 | 2,3-$Cl_2$-thiophen-5-yl | Me | $CHF_2$ | 2-(1,3-$Me_2$-nBu) |

(continued)

| No. | $R^1$ | $R^6$ | $R^7$ | Xn |
|---|---|---|---|---|
| 7-275 | 2,3-$Cl_2$-thiophen-4-yl | Me | $CF_3$ | 2-(1,3-$Me_2$-nBu) |
| 7-276 | 2,3-$Cl_2$-thiophen-4-yl | Me | $CHF_2$ | 2-(1,3-$Me_2$-nBu) |
| 7-277 | 3,4-$Cl_2$-isoxazole-5-yl | Me | $CHF_2$ | 2-(1,3-$Me_2$-nBu) |
| 7-278 | 3-Cl-isoxazole-5-yl | Me | H | 2-(1,3-$Me_2$-nBu) |
| 7-279 | 3,4-$Cl_2$-isothiazol-5-yl | Me | $CF_3$ | 2,6-$Br_2$-4-$OCF_3$ |
| 7-280 | 4-Me-1,2,3-thiadiazol-5-yl | Me | $CF_3$ | 2,6-$Br_2$-4-$OCF_3$ |
| 7-281 | 4-$CHF_2$-1,2,3-thiadiazol-5-yl | Me | $CF_3$ | 2,6-$Br_2$-4-$OCF_3$ |
| 7-282 | 1,2,3-benzothiadiazol-7-yl | Me | $CF_3$ | 2,6-$Br_2$-4-$OCF_3$ |
| 7-283 | 4-$CHF_2$-thiazole-5-yl | Me | $CF_3$ | 2,6-$Br_2$-4-$OCF_3$ |
| 7-284 | 4-$CF_3$-thiazole-5-yl | Me | $CF_3$ | 2,6-$Br_2$-4-$OCF_3$ |
| 7-285 | 4-$CHF_2$-2-Me-thiazole-5-yl | Me | $CF_3$ | 2,6-$Br_2$-4-$OCF_3$ |
| 7-286 | 4-$CF_3$-2-Me-thiazole-5-yl | Me | $CF_3$ | 2,6-$Br_2$-4-$OCF_3$ |
| 7-287 | 4-$CHF_2$-2-Me-thiazole-5-yl | Me | $CHF_2$ | 2,6-$Br_2$-4-$OCF_3$ |
| 7-288 | 2,6-$F_2$-pyridin-4-yl | Me | $CHF_2$ | 2-(1,3-$Me_2$-nBu) |

[Table 8-1]

Table 8

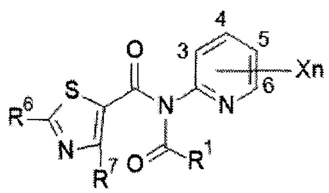

| No. | $R^1$ | $R^6$ | $R^7$ | $X_n$ |
|---|---|---|---|---|
| 8-1 | 3,4-$Cl_2$-isothiazol-5-yl | H | $CF_3$ | 3-(1,3-$Me_2$-nBu) |
| 8-2 | 3,4-$Cl_2$-isothiazol-5-yl | H | $CHF_2$ | 3-(1,3-$Me_2$-nBu) |
| 8-3 | 3,4-$Cl_2$-isothiazol-5-yl | Me | $CF_3$ | 3-(1,3-$Me_2$-nBu) |
| 8-4 | 3,4-$Cl_2$-isothiazol-5-yl | Me | $CHF_2$ | 3-(1,3-$Me_2$-nBu) |
| 8-5 | 3,4-$Cl_2$-isothiazol-5-yl | Et | $CF_3$ | 3-(1,3-$Me_2$-nBu) |
| 8-6 | 3,4-$Cl_2$-isothiazol-5-yl | Et | $CHF_2$ | 3-(1,3-$Me_2$-nBu) |
| 8-7 | 3-Me-isothiazol-5-yl | H | $CF_3$ | 3-(1,3-$Me_2$-nBu) |
| 8-8 | 3-Me-isothiazol-5-vl | H | $CHF_2$ | 3-(1,3-$Me_2$-nBu) |
| 8-9 | 3-Me-isothiazol-5-yl | Me | $CF_3$ | 3-(1,3-$Me_2$-nBu) |
| 8-10 | 3-Me-isothiazol-5-yl | Me | $CHF_2$ | 3-(1,3-$Me_2$-nBu) |
| 8-11 | 3-Me-isothiazol-5-yl | Et | $CF_3$ | 3-(1,3-$Me_2$-nBu) |
| 8-12 | 3-Me-isothiazol-5-vl | Et | $CHF_2$ | 3-(1,3-$Me_2$-nBu) |
| 8-13 | 3-Me-4-Cl-isothiazol-5-yl | H | $CF_3$ | 3-(1,3-$Me_2$-nBu) |
| 8-14 | 3-Me-4-Cl-isothiazol-5-yl | H | $CHF_2$ | 3-(1,3-$Me_2$-nBu) |
| 8-15 | 3-Me-4-Cl-isothiazol-5-yl | Me | $CF_3$ | 3-(1,3-$Me_2$-nBu) |
| 8-16 | 3-Me-4-Cl-isothiazol-5-yl | Me | $CHF_2$ | 3-(1,3-$Me_2$-nBu) |

(continued)

| No. | $R^1$ | $R^6$ | $R^7$ | $X_n$ |
|---|---|---|---|---|
| 8-17 | 3-Me-4-Cl-isothiazol-5-yl | Et | $CF_3$ | 3-(1,3-$Me_2$-nBu) |
| 8-18 | 3-Me-4-Cl-isothiazol-5-yl | Et | $CHF_2$ | 3-(1,3-$Me_2$-nBu) |
| 8-19 | 4-Me-1,2,3-thiadiazol-5-yl | Me | $CF_3$ | 3-(1,3-$Me_2$-nBu) |
| 8-20 | 4-Me-1,2,3-thiadiazol-5-yl | Me | $CHF_2$ | 3-(1,3-$Me_2$-nBu) |
| 8-21 | 4-$CF_3$-1,2,3-thiadiazol-5-yl | Me | $CF_3$ | 3-(1,3-$Me_2$-nBu) |
| 8-22 | 4-$CF_3$-1,2,3-thiadiazol-5-yl | Me | $CHF_2$ | 3-(1,3-$Me_2$-nBu) |
| 8-23 | 4-$CHF_2$-1,2,3-thiadiazol-5-yl | Me | $CF_3$ | 3-(1,3-$Me_2$-nBu) |

[Table 8-2]

| No. | $R^1$ | $R^6$ | $R^7$ | Xn |
|---|---|---|---|---|
| 8-24 | 4-$CHF_2$-1,2,3-thiadiazol-5-yl | Me | $CHF_2$ | 3-(1,3-$Me_2$-nBu) |
| 8-25 | 1,2,3-benzothiadiazol-7-yl | Me | $CF_3$ | 3-(1,3-$Me_2$-nBu) |
| 8-26 | 1,2,3-benzothiadiazol-7-yl | Me | $CHF_2$ | 3-(1,3-$Me_2$-nBu) |
| 8-27 | 1,2,3-benzothiadiazol-7-yl | Me | $CHF_2$ | 3-(1,3-$Me_2$-nBu) |
| 8-28 | 1,2,3-benzothiadiazol-7-yl | Me | $CHF_2$ | 3-(1,3-$Me_2$-nBu) |
| 8-29 | 4-$CF_3$-2-Me-thiazole-5-yl | Me | $CF_3$ | 3-(1,3-$Me_2$-nBu) |
| 8-30 | 4-$CF_3$-2-Me-thiazole-5-yl | Me | $CHF_2$ | 3-(1,3-$Me_2$-nBu) |
| 8-31 | 4-$CHF_2$-2-Me-thiazole-5-yl | Me | $CF_3$ | 3-(1,3-$Me_2$-nBu) |
| 8-32 | 4-$CHF_2$-2-Me-thiazole-5-yl | Me | $CHF_2$ | 3-(1,3-$Me_2$-nBu) |
| 8-33 | 4-$CF_3$-thiazole-5-yl | Me | $CF_3$ | 3-(1,3-$Me_2$-nBu) |
| 8-34 | 4-$CF_3$-thiazole-5-yl | Me | $CHF_2$ | 3-(1,3-$Me_2$-nBu) |
| 8-35 | 4-$CHF_2$-thiazole-5-yl | Me | $CF_3$ | 3-(1,3-$Me_2$-nBu) |
| 8-36 | 4-$CHF_2$-thiazole-5-yl | Me | $CHF_2$ | 3-(1,3-$Me_2$-nBu) |

[Table 9-1]

Table 9

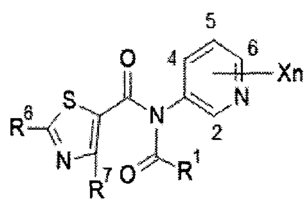

| No. | $R^1$ | $R^6$ | $R^7$ | Xn |
|---|---|---|---|---|
| 9-1 | 3,4-$Cl_2$-isothiazol-5-yl | H | $CF_3$ | 2-(1,3-$Me_2$-nBu) |
| 9-2 | 3,4-$Cl_2$-isothiazol-5-yl | H | $CHF_2$ | 2-(1,3-$Me_2$-nBu) |
| 9-3 | 3,4-$Cl_2$-isothiazol-5-yl | Me | $CF_3$ | 2-(2-cPr-cPr) |
| 9-4 | 3,4-$Cl_2$-isothiazol-5-yl | Me | $CF_3$ | 2-nBu |
| 9-5 | 3,4-$Cl_2$-isothiazol-5-yl | Me | $CF_3$ | 2-(3-Me-nBu) |
| 9-6 | 3,4-$Cl_2$-isothiazol-5-yl | Me | $CF_3$ | 2-(1,3-$Me_2$-nBu) |

(continued)

| No. | $R^1$ | $R^6$ | $R^7$ | Xn |
|---|---|---|---|---|
| 9-7 | 3,4-$Cl_2$-isothiazol-5-yl | Me | $CF_3$ | 2-(1,3,3-$Me_3$-nBu) |
| 9-8 | 3,4-$C1_2$-isothiazol-5-yl | Me | $CF_3$ | 2-(1,3-$Me_2$-nPen) |
| 9-9 | 3,4-$Cl_2$-isothiazol-5-yl | Me | $CF_3$ | 2-(1,4-$Me_2$-nPen) |
| 9-10 | 3,4-$Cl_2$-isothiazol-5-yl | Me | $CHF_2$ | 2-(2-cPr-cPr) |
| 9-11 | 3,4-$Cl_2$-isothiazol-5-yl | Me | $CHF_2$ | 2-nBu |
| 9-12 | 3,4-$Cl_2$-isothiazol-5-yl | Me | $CHF_2$ | 2-(3-Me-nBu) |
| 9-13 | 3,4-$Cl_2$-isothiazol-5-yl | Me | $CHF_2$ | 2-(1,3-$Me_2$-nBu) |
| 9-14 | 3,4-$Cl_2$-isothiazol-5-yl | Me | $CHF_2$ | 2-(1,3,3-$Me_3$-nBu) |
| 9-15 | 3,4-$Cl_2$-isothiazol-5-yl | Me | $CHF_2$ | 2-(1,3-$Me_2$-nPen) |
| 9-16 | 3,4-$Cl_2$-isothiazol-5-yl | Me | $CHF_2$ | 2-(1,4-$Me_2$-nPen) |
| 9-17 | 3,4-$Cl_2$-isothiazol-5-yl | Me | Me | 2-(1,3-$Me_2$-nBu) |
| 9-18 | 3,4-$Cl_2$-isothiazol-5-yl | Et | $CF_3$ | 2-(1,3-$Me_2$-nBu) |
| 9-19 | 3,4-$Cl_2$-isothiazol-5-yl | Et | $CHF_2$ | 2-(1,3-$Me_2$-nBu) |
| 9-20 | 3-Me-isothiazol-5-yl | Me | $CF_3$ | 2-(1,3-$Me_2$-nBu) |
| 9-21 | 3-Me-isothiazol-5-yl | Me | $CHF_2$ | 2-(1,3-$Me_2$-nBu) |
| 9-22 | 3-Me-4-Cl-isothiazol-5-yl | Me | $CF_3$ | 2-(1,3-$Me_2$-nBu) |
| 9-23 | 3-Me-4-Cl-isothiazol-5-yl | Me | $CHF_2$ | 2-(1,3-$Me_2$-nBu) |

[Table 9-2]

| No. | $R^1$ | $R^6$ | $R^7$ | Xn |
|---|---|---|---|---|
| 9-24 | 4-Me-1,2,3-thiadiazol-5-yl | Me | $CF_3$ | 2-(1,3-$Me_2$-nBu) |
| 9-25 | 4-Me-1,2,3-thiadiazol-5-yl | Me | $CHF_2$ | 2-(2-cPr-cPr) |
| 9-26 | 4-Me-1,2,3-thiadiazol-5-yl | Me | $CHF_2$ | 2-nBu |
| 9-27 | 4-Me-1,2,3-thiadiazol-5-yl | Me | $CHF_2$ | 2-(3-Me-nBu) |
| 9-28 | 4-Me-1,2,3-thiadiazol-5-yl | Me | $CHF_2$ | 2-(1,3-$Me_2$-nBu) |
| 9-29 | 4-Me-1,2,3-thiadiazol-5-yl | Me | $CHF_2$ | 2-(1,3,3-$Me_3$-nBu) |
| 9-30 | 4-Me-1,2,3-thiadiazol-5-yl | Me | $CHF_2$ | 2-(1,3-$Me_2$-nPen) |
| 9-31 | 4-Me-1,2,3-thiadiazol-5-yl | Me | $CHF_2$ | 2-(1,4-$Me_2$-nPen) |
| 9-32 | 4-$CF_3$-1,2,3-thiadiazol-5-yl | Me | $CF_3$ | 2-(1,3-$Me_2$-nBu) |
| 9-33 | 4-$CF_3$-1,2,3-thiadiazol-5-yl | Me | $CHF_2$ | 2-(2-cPr-cPr) |
| 9-34 | 4-$CF_3$-1,2,3-thiadiazol-5-yl | Me | $CHF_2$ | 2-nBu |
| 9-35 | 4-$CF_3$-1,2,3-thiadiazol-5-yl | Me | $CHF_2$ | 2-(3-Me-nBu) |
| 9-36 | 4-$CF_3$-1,2,3-thiadiazol-5-yl | Me | $CHF_2$ | 2-(1,3-$Me_2$-nBu) |
| 9-37 | 4-$CF_3$-1,2,3-thiadiazol-5-yl | Me | $CHF_2$ | 2-(1,3,3-$Me_3$-nBu) |
| 9-38 | 4-$CF_3$-1,2,3-thiadiazol-5-yl | Me | $CHF_2$ | 2-(1,3-$Me_2$-nPen) |
| 9-39 | 4-$CF_3$-1,2,3-thiadiazol-5-yl | Me | $CHF_2$ | 2-(1,4-$Me_2$-nPen) |
| 9-40 | 4-$CHF_2$-1,2,3-thiadiazol-5-yl | Me | $CF_3$ | 2-(1,3-$Me_2$-nBu) |

(continued)

| No. | $R^1$ | $R^6$ | $R^7$ | Xn |
|---|---|---|---|---|
| 9-41 | 4-$CHF_2$-1,2,3-thiadiazol-5-yl | Me | $CHF_2$ | 2-(2-cPr-cPr) |
| 9-42 | 4-$CHF_2$-1,2,3-thiadiazol-5-yl | Me | $CHF_2$ | 2-nBu |
| 9-43 | 4-$CHF_2$-1,2,3-thiadiazol-5-yl | Me | $CHF_2$ | 2-(3-Me-nBu) |
| 9-44 | 4-$CHF_2$-1,2,3-thiadiazol-5-yl | Me | $CHF_2$ | 2-(1,3-$Me_2$-nBu) |
| 9-45 | 4-$CHF_2$-1,2,3-thiadiazol-5-yl | Me | $CHF_2$ | 2-(1,3,3-$Me_3$-nBu) |
| 9-46 | 4-$CHF_2$-1,2,3-thiadiazol-5-yl | Me | $CHF_2$ | 2-(1,3-$Me_2$-nPen) |
| 9-47 | 4-$CHF_2$-1,2,3-thiadiazol-5-yl | Me | $CHF_2$ | 2-(1,4-$Me_2$-nPen) |
| 9-48 | 1,2,3 -benzothiadiazol-7-yl | Me | $CF_3$ | 2-(1,3-$Me_2$-nBu) |
| 9-49 | 1,2,3-benzothiadiazol-7-yl | Me | $CHF_2$ | 2-(2-cPr-cPr) |
| 9-50 | 1,2,3-benzothiadiazol-7-yl | Me | $CHF_2$ | 2-nBu |
| 9-51 | 1,2,3-benzothiadiazol-7-yl | Me | $CHF_2$ | 2-(3-Me-nBu) |

[Table 9-3]

| No. | $R^1$ | $R^6$ | $R^7$ | Xn |
|---|---|---|---|---|
| 9-52 | 1,2,3-benzothiadiazol-7-yl | Me | $CHF_2$ | 2-(1,3-$Me_2$-nBu) |
| 9-53 | 1,2,3-benzothiadiazol-7-yl | Me | $CHF_2$ | 2-(1,3,3-$Me_3$-nBu) |
| 9-54 | 1,2,3-benzothiadiazol-7-yl | Me | $CHF_2$ | 2-(1,3-$Me_2$-nPen) |
| 9-55 | 1,2,3-benzothiadiazol-7-yl | Me | $CHF_2$ | 2-(1,4-$Me_2$-nPen) |
| 9-56 | 4-$CF_3$-2-Me-thiazole-5-yl | Me | $CF_3$ | 2-(1,3-$Me_2$-nBu |
| 9-57 | 4-$CF_3$-2-Me-thiazole-5-yl | Me | $CHF_2$ | 2-(2-cPr-cPr) |
| 9-58 | 4-$CF_3$-2-Me-thiazole-5-yl | Me | $CHF_2$ | 2-nBu |
| 9-59 | 4-$CF_3$-2-Me-thiazole-5-yl | Me | $CHF_2$ | 2-(3-Me-nBu) |
| 9-60 | 4-$CF_3$-2-Me-thiazole-5-yl | Me | $CHF_2$ | 2-(1,3-$Me_2$-nBu) |
| 9-61 | 4-$CF_3$-2-Me-thiazole-5-yl | Me | $CHF_2$ | 2-(1,3,3-$Me_3$-nBu) |
| 9-62 | 4-$CF_3$-2-Me-thiazole-5-yl | Me | $CHF_2$ | 2-(1,3-$Me_2$-nPen |
| 9-63 | 4-$CF_3$-2-Me-thiazole-5-yl | Me | $CHF_2$ | 2-(1,4-$Me_2$-nPen) |
| 9-64 | 4-$CHF_2$-2-Me-thiazole-5-yl | Me | $CF_3$ | 2-(1,3-$Me_2$-nBu) |
| 9-65 | 4-$CHF_2$-2-Me-thiazole-5-yl | Me | $CHF_2$ | 2-(2-cPr-cPr) |
| 9-66 | 4-$CHF_2$-2-Me-thiazole-5-yl | Me | $CHF_2$ | 2-nBu |
| 9-67 | 4-$CHF_2$-2-Me-thiazole-5-yl | Me | $CHF_2$ | 2-(3-Me-nBu) |
| 9-68 | 4-$CHF_2$-2-Me-thiazole-5-yl | Me | $CHF_2$ | 2-(1,3-$Me_2$-nBu) |
| 9-69 | 4-$CHF_2$-2-Me-thiazole-5-yl | Me | $CHF_2$ | 2-(1,3,3-$Me_3$-nBu |
| 9-70 | 4-$CHF_2$-2-Me-thiazole-5-yl | Me | $CHF_2$ | 2-(1,3-$Me_2$-nPen) |
| 9-71 | 4-$CHF_2$-2-Me-thiazole-5-yl | Me | $CHF_2$ | 2-(1,4-$Me_2$-nPen) |
| 9-72 | 4-$CF_3$-thiazole-5-yl | Me | $CF_3$ | 2-(1,3-$Me_2$-nBu) |
| 9-73 | 4-$CF_3$-thiazole-5-yl | Me | $CHF_2$ | 2-(2-cPr-cPr) |
| 9-74 | 4-$CF_3$-thiazole-5-yl | Me | $CHF_2$ | 2-nBu |

(continued)

| No. | $R^1$ | $R^6$ | $R^7$ | Xn |
|---|---|---|---|---|
| 9-75 | 4-$CF_3$-thiazole-5-yl | Me | $CHF_2$ | 2-(3-Me-nBu) |
| 9-76 | 4-$CF_3$-thiazole-5-yl | Me | $CHF_2$ | 2-(1,3-$Me_2$-nBu) |
| 9-77 | 4-$CF_3$-thiazole-5-yl | Me | $CHF_2$ | 2-(1,3,3-$Me_3$-nBu) |
| 9-78 | 4-$CF_3$-thiazole-5-yl | Me | $CHF_2$ | 2-(1,3-$Me_2$-nPen) |
| 9-79 | 4-$CF_3$-thiazole-5-yl | Me | $CHF_2$ | 2-(1,4-$Me_2$-nPen) |

[Table 9-4]

| No. | $R^1$ | $R^6$ | $R^7$ | Xn |
|---|---|---|---|---|
| 9-80 | 4-$CHF_2$-thiazole-5-yl | Me | $CF_3$ | 2-(1,3-$Me_2$-nBu) |
| 9-81 | 4-$CHF_2$-thiazole-5-yl | Me | $CHF_2$ | 2-(2-cPr-cPr) |
| 9-82 | 4-$CHF_2$-thiazole-5-yl | Me | $CHF_2$ | 2-nBu |
| 9-83 | 4-$CHF_2$-thiazole-5-yl | Me | $CHF_2$ | 2-(3-Me-nBu) |
| 9-84 | 4-$CHF_2$-thiazole-5-yl | Me | $CHF_2$ | 2-(1,3-$Me_2$-nBu) |
| 9-85 | 4-$CHF_2$-thiazole-5-yl | Me | $CHF_2$ | 2-(1,3,3-$Me_3$-nBu |
| 9-86 | 4-$CHF_2$-thiazole-5-yl | Me | $CHF_2$ | 2-(1,3-$Me_2$-nPen) |
| 9-87 | 4-$CHF_2$-thiazole-5-yl | Me | $CHF_2$ | 2-(1,4-$Me_2$-nPen) |
| 9-88 | 2-$CHF_2$-pyridin-3-yl | Me | $CHF_2$ | 2-(1,3-$Me_2$-nBu) |
| 9-89 | 5-Cl-3-$CHF_2$-1-Me-pyrazol-4-yl | Me | $CHF_2$ | 2-(1,3-$Me_2$-nBu) |
| 9-90 | 2,3-$Cl_2$-thiophen-5-yl | Me | $CHF_2$ | 2-(1,3-$Me_2$-nBu) |
| 9-91 | 2,3-$Cl_2$-thiophen-4-yl | Me | $CHF_2$ | 2-(1,3-$Me_2$-nBu) |

[Table 10-1]

Table 10

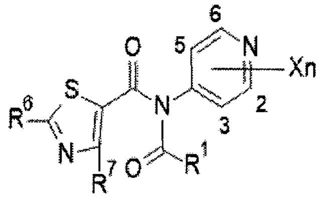

| No. | $R^1$ | $R^6$ | $R^7$ | Xn |
|---|---|---|---|---|
| 10-1 | 3,4-$Cl_2$-isothiazol-5-yl | H | $CF_3$ | 3-(1,3-$Me_2$-nBu) |
| 10-2 | 3,4-$Cl_2$-isothiazol-5-yl | H | $CHF_2$ | 3-(1,3-$Me_2$-nBu) |
| 10-3 | 3-Me-isothiazol-5-yl | H | $CF_3$ | 3-(1,3-$Me_2$-nBu) |
| 10-4 | 3-Me-isothiazol-5-yl | H | $CHF_2$ | 3-(1,3-$Me_2$-nBu) |
| 10-5 | 3-Me-4-Cl-isothiazol-5-yl | H | $CF_3$ | 3-(1,3-$Me_2$-nBu) |
| 10-6 | 3-Me-4-Cl-isothiazol-5-yl | H | $CHF_2$ | 3-(1,3-$Me_2$-nBu) |
| 10-7 | 4-Me-1,2,3-thiadiazol-5-yl | Me | $CF_3$ | 3-(1,3-$Me_2$-nBu) |
| 10-8 | 4-Me-1,2,3-thiadiazol-5-yl | Me | $CHF_2$ | 3-(1,3-$Me_2$-nBu) |
| 10-9 | 4-$CF_3$-1,2,3-thiadiazol-5-yl | Me | $CF_3$ | 3-(1,3-$Me_2$-nBu) |

(continued)

| No. | $R^1$ | $R^6$ | $R^7$ | Xn |
|---|---|---|---|---|
| 10-10 | 4-$CF_3$-1,2,3-thiadiazol-5-yl | Me | $CHF_2$ | 3-(1,3-$Me_2$-nBu) |
| 10-11 | 4-$CHF_2$-1,2,3-thiadiazol-5-yl | Me | $CF_3$ | 3-(1,3-$Me_2$-nBu) |
| 10-12 | 4-$CHF_2$-1,2,3-thiadiazol-5-yl | Me | $CHF_2$ | 3-(1,3-$Me_2$-nBu) |
| 10-21 | 1,2,3-benzothiadiazol-7-yl | Me | $CF_3$ | 3-(1,3-$Me_2$-nBu) |
| 10-22 | 1,2,3-benzothiadiazol-7-yl | Me | $CHF_2$ | 3-(1,3-$Me_2$-nBu) |
| 10-13 | 4-$CF_3$-2-Me-thiazole-5-yl | Me | $CF_3$ | 3-(1,3-$Me_2$-nBu) |
| 10-14 | 4-$CF_3$-2-Me-thiazole-5-yl | Me | $CHF_2$ | 3-(1,3-$Me_2$-nBu) |
| 10-15 | 4-$CHF_2$-2-Me-thiazole-5-yl | Me | $CF_3$ | 3-(1,3-$Me_2$-nBu) |
| 10-16 | 4-$CHF_2$-2-Me-thiazole-5-yl | Me | $CHF_2$ | 3-(1,3-$Me_2$-nBu) |
| 10-17 | 4-$CF_3$-thiazole-5-yl | Me | $CF_3$ | 3-(1,3-$Me_2$-nBu) |
| 10-18 | 4-$CF_3$-thiazole-5-yl | Me | $CHF_2$ | 3-(1,3-$Me_2$-nBu) |
| 10-19 | 4-$CHF_2$-thiazole-5-yl | Me | $CF_3$ | 3-(1,3-$Me_2$-nBu) |
| 10-20 | 4-$CHF_2$-thiazole-5-yl | Me | $CHF_2$ | 3-(1,3-$Me_2$-nBu) |

[Table 11-1]

Table 11

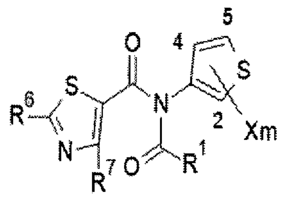

| No. | $R^1$ | $R^6$ | $R^7$ | Xm |
|---|---|---|---|---|
| 11-1 | 3,4-$Cl_2$-isothiazol-5-yl | H | $CF_3$ | 2-(1,3-$Me_2$-nBu) |
| 11-2 | 3,4-$Cl_2$-isothiazol-5-yl | H | $CHF_2$ | 2-(1,3-$Me_2$-nBu) |
| 11-3 | 3,4-$Cl_2$-isothiazol-5-yl | Me | $CF_3$ | 2-(1,3-$Me_2$-nBu) |
| 11-4 | 3,4-$Cl_2$-isothiazol-5-yl | Me | $CHF_2$ | 2-(2-iPr-cPr) |
| 11-5 | 3,4-$Cl_2$-isothiazol-5-yl | Me | $CHF_2$ | 2-nBu |
| 11-6 | 3,4-$Cl_2$-isothiazol-5-yl | Me | $CHF_2$ | 2-(3-Me-nBu) |
| 11-7 | 3,4-$Cl_2$-isothiazol-5-yl | Me | $CHF_2$ | 2-(2-cPr-cPr) |
| 11-8 | 3,4-$Cl_2$-isothiazol-5-yl | Me | $CHF_2$ | 2-(1,3-$Me_2$-nBu) |
| 11-9 | 3,4-$Cl_2$-isothiazol-5-yl | Me | $CHF_2$ | 2-(1,3,3-$Me_3$-nBu) |
| 11-10 | 3,4-$Cl_2$-isothiazol-5-yl | Me | $CHF_2$ | 2-(1,3-$Me_2$-nPen) |
| 11-11 | 3,4-$Cl_2$-isothiazol-5-yl | Me | $CHF_2$ | 2-(1,4-$Me_2$-nPen) |
| 11-12 | 3,4-$Cl_2$-isothiazol-5-yl | Me | Me | 2-(1,3-$Me_2$-nBu) |
| 11-13 | 3,4-$Cl_2$-isothiazol-5-yl | Et | $CF_3$ | 2-(1,3-$Me_2$-nBu) |
| 11-14 | 3,4-$Cl_2$-isothiazol-5-yl | Et | $CHF_2$ | 2-(1,3-$Me_2$-nBu) |
| 11-15 | 3-Me-isothiazol-5-yl | Me | $CF_3$ | 2-(1,3-$Me_2$-nBu) |
| 11-16 | 3-Me-isothiazol-5-yl | Me | $CHF_2$ | 2-(1,3-$Me_2$-nBu) |
| 11-17 | 3-Me-4-Cl-isothiazol-5-yl | Me | $CF_3$ | 2-(1,3-$Me_2$-nBu) |

(continued)

| No. | $R^1$ | $R^6$ | $R^7$ | Xm |
|---|---|---|---|---|
| 11-18 | 3-Me-4-Cl-isothiazol-5-yl | Me | $CHF_2$ | 2-(1,3-$Me_2$-nBu) |
| 11-19 | 4-Me-1,2,3-thiadiazol-5-yl | Me | $CF_3$ | 2-(1,3-$Me_2$-nBu) |
| 11-20 | 4-Me-1,2,3-thiadiazol-5-yl | Me | $CHF_2$ | 2-(1,3-$Me_2$-nBu) |
| 11-21 | 4-$CF_3$-1,2,3-thiadiazol-5-yl | Me | $CF_3$ | 2-(1,3-$Me_2$-nBu) |
| 11-22 | 4-$CF_3$-1,2,3-thiadiazol-5-yl | Me | $CHF_2$ | 2-(1,3-$Me_2$-nBu) |
| 11-23 | 4-$CHF_2$-1,2,3-thiadiazol-5-yl | Me | $CF_3$ | 2-(1,3-$Me_2$-nBu) |

[Table 11-2]

| No. | $R^1$ | $R^6$ | $R^7$ | Xm |
|---|---|---|---|---|
| 11-24 | 4-$CHF_2$-1,2,3-thiadiazol-5-yl | Me | $CHF_2$ | 2-(1,3-$Me_2$-nBu) |
| 11-25 | 1,2,3-benzothiadiazol-7-yl | Me | $CF_3$ | 2-(1,3-$Me_2$-nBu) |
| 11-26 | 1,2,3-benzothiadiazol-7-yl | Me | $CHF_2$ | 2-(1,3-$Me_2$-nBu) |
| 11-27 | 4-$CF_3$-2-Et-thiazole-5-yl | Me | $CHF_2$ | 2-(1,3-$Me_2$-nBu) |
| 11-28 | 4-$CF_3$-2-Et-thiazole-5-yl | Me | $CHF_2$ | 2-(1,3-$Me_2$-nBu) |
| 11-29 | 4-$CHF_2$-2-Et-thiazole-5-yl | Me | $CF_3$ | 2-(1,3-$Me_2$-nBu) |
| 11-30 | 4-$CHF_2$-2-Et-thiazole-5-yl | Me | $CHF_2$ | 2-(1,3-$Me_2$-nBu) |
| 11-31 | 4-$CF_3$-2-Me-thiazole-5-yl | Me | $CHF_2$ | 2-(1,3-$Me_2$-nBu) |
| 11-32 | 4-$CF_3$-2-Me-thiazole-5-yl | Me | $CHF_2$ | 2-(1,3-$Me_2$-nBu) |
| 11-33 | 4-$CHF_2$-2-Me-thiazole-5-yl | Me | $CF_3$ | 2-(1,3-$Me_2$-nBu) |
| 11-34 | 4-$CHF_2$-2-Me-thiazole-5-yl | Me | $CHF_2$ | 2-(1,3-$Me_2$-nBu) |
| 11-35 | 4-$CF_3$-thiazole-5-yl | Me | $CF_3$ | 2-(1,3-$Me_2$-nBu) |
| 11-36 | 4-$CF_3$-thiazole-5-yl | Me | $CHF_2$ | 2-(1,3-$Me_2$-nBu) |
| 11-37 | 4-$CHF_2$-thiazole-5-yl | Me | $CF_3$ | 2-(1,3-$Me_2$-nBu) |
| 11-38 | 4-$CHF_2$-thiazole-5-yl | Me | $CHF_2$ | 2-(1,3-$Me_2$-nBu) |

[Table 12-1]

Table 12

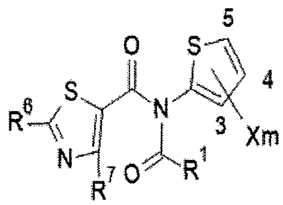

| No. | $R^1$ | $R^6$ | $R^7$ | Xm |
|---|---|---|---|---|
| 12-1 | 3,4-$Cl_2$-isothiazol-5-yl | H | $CF_3$ | 3-(1,3-$Me_2$-nBu) |
| 12-2 | 3,4-$Cl_2$-isothiazol-5-yl | H | $CHF_2$ | 3-(1,3-$Me_2$-nBu) |
| 12-3 | 3,4-$Cl_2$-isothiazol-5-yl | Me | $CF_3$ | 3-(1,3-$Me_2$-nBu) |
| 12-4 | 3,4-$Cl_2$-isothiazol-5-yl | Me | $CHF_2$ | 3-(1,3-$Me_2$-nBu) |
| 12-5 | 3,4-$Cl_2$-isothiazol-5-yl | Me | Me | 3-(1,3-$Me_2$-nBu) |

(continued)

| No. | $R^1$ | $R^6$ | $R^7$ | Xm |
|---|---|---|---|---|
| 12-6 | 3,4-$Cl_2$-isothiazol-5-yl | Et | $CF_3$ | 3-(1,3-$Me_2$-nBu) |
| 12-7 | 3,4-$Cl_2$-isothiazol-5-yl | Et | $CHF_2$ | 3-(1,3-$Me_2$-nBu) |
| 12-8 | 3-Me-isothiazol-5-yl | Me | $CF_3$ | 3-(1,3-$Me_2$-nBu) |
| 12-9 | 3-Me-isothiazol-5-yl | Me | $CHF_2$ | 3-(1,3-$Me_2$-nBu) |
| 12-10 | 3-Me-4-Cl-isothiazol-5-yl | Me | $CF_3$ | 3-(1,3-$Me_2$-nBu) |
| 12-11 | 3-Me-4-Cl-isothiazol-5-yl | Me | $CHF_2$ | 3-(1,3-$Me_2$-nBu) |
| 12-12 | 4-Me-1,2,3-thiadiazol-5-yl | Me | $CF_3$ | 3-(1,3-$Me_2$-nBu) |
| 12-13 | 4-Me-1,2,3-thiadiazol-5-yl | Me | $CHF_2$ | 3-(1,3-$Me_2$-nBu) |
| 12-14 | 4-$CF_3$-1,2,3-thiadiazol-5-yl | Me | $CF_3$ | 3-(1,3-$Me_2$-nBu) |
| 12-15 | 4-$CF_3$-1,2,3-thiadiazol-5-yl | Me | $CHF_2$ | 3-(1,3-$Me_2$-nBu) |
| 12-16 | 4-$CHF_2$-1,2,3-thiadiazol-5-yl | Me | $CF_3$ | 3-(1,3-$Me_2$-nBu) |
| 12-17 | 4-$CHF_2$-1,2,3-thiadiazol-5-yl | Me | $CHF_2$ | 3-(1,3-$Me_2$-nBu) |
| 12-18 | 1,2,3-benzothiadiazol-7-yl | Me | $CF_3$ | 3-(1,3-$Me_2$-nBu) |
| 12-19 | 1,2,3-benzothiadiazol-7-yl | Me | $CHF_2$ | 3-(1,3-$Me_2$-nBu) |
| 12-20 | 4-$CF_3$-2-Me-thiazole-5-yl | Me | $CF_3$ | 3-(1,3-$Me_2$-nBu) |
| 12-21 | 4-$CF_3$-2-Me-thiazole-5-yl | Me | $CHF_2$ | 3-(1,3-$Me_2$-nBu) |
| 12-22 | 4-$CHF_2$-2-Me-thiazole-5-yl | Me | $CF_3$ | 3-(1,3-$Me_2$-nBu) |
| 12-23 | 4-$CHF_2$-2-Me-thiazole-5-yl | Me | $CHF_2$ | 3-(1,3-$Me_2$-nBu) |

[Table 12-2]

| No. | $R^1$ | $R^6$ | $R^7$ | Xm |
|---|---|---|---|---|
| 12-24 | 4-$CF_3$-thiazole-5-yl | Me | $CF_3$ | 3-(1,3-$Me_2$-nBu) |
| 12-25 | 4-$CF_3$-thiazole-5-yl | Me | $CHF_2$ | 3-(1,3-$Me_2$-nBu) |
| 12-26 | 4-$CHF_2$-thiazole-5-yl | Me | $CF_3$ | 3-(1,3-$Me_2$-nBu) |
| 12-27 | 4-$CHF_2$-thiazole-5-yl | Me | $CHF_2$ | 3-(1,3-$Me_2$-nBu) |

[Table 13-1]

Table 13

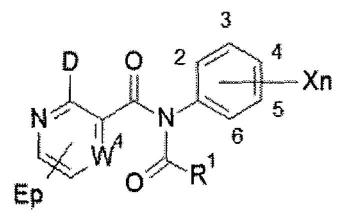

| No. | $R^1$ | $W^4$ | D | Ep | Xn |
|---|---|---|---|---|---|
| 13-1 | 3,4-$Cl_2$-isothiazol-5-yl | CH | $CF_3$ | H | 2-(1,3-$Me_2$-nBu) |
| 13-2 | 3,4-$Cl_2$-isothiazol-5-yl | CH | $CHF_2$ | H | 2-(1,3-$Me_2$-nBu) |
| 13-3 | 3,4-$Cl_2$-isothiazol-5-yl | CH | $CHF_2$ | H | 2-(1,3,3-$Me_3$-nBu) |
| 13-4 | 3,4-$Cl_2$-isothiazol-5-yl | CH | $CHF_2$ | H | 2-(1,3-$Me_2$-nPen) |

(continued)

| No. | $R^1$ | $W^4$ | D | Ep | Xn |
|---|---|---|---|---|---|
| 13-5 | 3-Me-isothiazol-5-yl | CH | $CF_3$ | H | 2-(1,3-$Me_2$-nBu) |
| 13-6 | 3-Me-isothiazol-5-yl | CH | $CHF_2$ | H | 2-(1,3-$Me_2$-nBu) |
| 13-7 | 3-Me-4-Cl-isothiazol-5-vl | CH | $CF_3$ | H | 2-(1,3-$Me_2$-nBu) |
| 13-8 | 3-Me-4-Cl-isothiazol-5-yl | CH | $CHF_2$ | H | 2-(1,3-$Me_2$-nBu) |
| 13-9 | 4-Me-1,2,3-thiadiazol-5-yl | CH | $CF_3$ | H | 2-(1,3-$Me_2$-nBu) |
| 13-10 | 4-Me-1,2,3-thiadiazol-5-yl | CH | $CHF_2$ | H | 2-(1,3-$Me_2$-nBu) |
| 13-11 | 4-$CF_3$-1,2,3-thiadiazol-5-yl | CH | $CF_3$ | H | 2-(1,3-$Me_2$-nBu) |
| 13-12 | 4-$CF_3$-1,2,3-thiadiazol-5-yl | CH | $CHF_2$ | H | 2-(1,3-$Me_2$-nBu) |
| 13-13 | 4-$CHF_2$-1,2,3-thiadiazol-5-yl | CH | $CF_3$ | H | 2-(1,3-$Me_2$-nBu) |
| 13-14 | 4-$CHF_2$-1,2,3-thiadiazol-5-yl | CH | $CHF_2$ | H | 2-(1,3-$Me_2$-nBu) |
| 13-15 | 1,2,3-benzothiadiazol-7-yl | CH | $CF_3$ | H | 2-(1,3-$Me_2$-nBu) |
| 13-16 | 1,2,3-benzothiadiazol-7-yl | CH | $CHF_2$ | H | 2-(1,3-$Me_2$-nBu) |
| 13-17 | 4-$CF_3$-2-Me-thiazole-5-yl | CH | $CF_3$ | H | 2-(1,3-$Me_2$-nBu) |
| 13-18 | 4-$CF_3$-2-Me-thiazole-5-yl | CH | $CHF_2$ | H | 2-(1,3-$Me_2$-nBu) |
| 13-19 | 4-$CHF_2$-2-Me-thiazole-5-yl | CH | $CF_3$ | H | 2-(1,3-$Me_2$-nBu) |
| 13-20 | 4-$CHF_2$-2-Me-thiazole-5-yl | CH | $CHF_2$ | H | 2-(1,3-$Me_2$-nBu) |
| 13-21 | 4-$CF_3$-thiazole-5-yl | CH | $CF_3$ | H | 2-(1,3-$Me_2$-nBu) |
| 13-22 | 4-$CF_3$-thiazole-5-yl | CH | $CHF_2$ | H | 2-(1,3-$Me_2$-nBu) |
| 13-23 | 4-$CHF_2$-thiazole-5-yl | CH | $CF_3$ | H | 2-(1,3-$Me_2$-nBu) |

[Table 13-2]

| No. | $R^1$ | $W^4$ | D | Ep | Xn |
|---|---|---|---|---|---|
| 13-24 | 4-$CHF_2$-thiazole-5-yl | CH | $CHF_2$ | H | 2-(1,3-$Me_2$-nBu) |
| 13-25 | 3,4-$Cl_2$-isothiazol-5-yl | N | $CF_3$ | H | 2-(1,3-$Me_2$-nBu) |
| 13-26 | 3,4-$Cl_2$-isothiazol-5-yl | N | $CHF_2$ | H | 2-(1,3-$Me_2$-nBu) |
| 13-27 | 3,4-$Cl_2$-isothiazol-5-yl | N | $CHF_2$ | H | 2-(1,3,3-$Me_3$-nBu) |
| 13-28 | 3,4-$Cl_2$-isothiazol-5-yl | N | $CHF_2$ | H | 2-(1,3-$Me_2$-nPen) |
| 13-29 | 3-Me-isothiazol-5-yl | N | $CF_3$ | H | 2-(1,3-$Me_2$-nBu) |
| 13-30 | 3-Me-isothiazol-5-yl | N | $CHF_2$ | H | 2-(1,3-$Me_2$-nBu) |
| 13-31 | 3-Me-4-Cl-isothiazol-5-yl | N | $CF_3$ | H | 2-(1,3-$Me_2$-nBu) |
| 13-32 | 3-Me-4-Cl-isothiazol-5-yl | N | $CHF_2$ | H | 2-(1,3-$Me_2$-nBu) |
| 13-33 | 4-Me-1,2,3-thiadiazol-5-yl | N | $CF_3$ | H | 2-(1,3-$Me_2$-nBu) |
| 13-34 | 4-Me-1,2,3-thiadiazol-5-yl | N | $CHF_2$ | H | 2-(1,3-$Me_2$-nBu) |
| 13-35 | 4-$CF_3$-1,2,3-thiadiazol-5-yl | N | $CF_3$ | H | 2-(1,3-$Me_2$-nBu) |
| 13-36 | 4-$CF_3$-1,2,3-thiadiazol-5-yl | N | $CHF_2$ | H | 2-(1,3-$Me_2$-nBu) |
| 13-37 | 4-$CHF_2$-1,2,3-thiadiazol-5-yl | N | $CF_3$ | H | 2-(1,3-$Me_2$-nBu) |
| 13-38 | 4-$CHF_2$-1,2,3-thiadiazol-5-yl | N | $CHF_2$ | H | 2-(1,3-$Me_2$-nBu) |

(continued)

| No. | $R^1$ | $W^4$ | D | Ep | Xn |
|---|---|---|---|---|---|
| 13-39 | 1,2,3-benzothiadiazol-7-yl | N | $CF_3$ | H | 2-(1,3-$Me_2$-nBu) |
| 13-40 | 1,2,3-benzothiadiazol-7-yl | N | $CHF_2$ | H | 2-(1,3-$Me_2$-nBu) |
| 13-41 | 4-$CF_3$-2-Me-thiazole-5-yl | N | $CF_3$ | H | 2-(1,3-$Me_2$-nBu) |
| 13-42 | 4-$CF_3$-2-Me-thiazole-5-yl | N | $CHF_2$ | H | 2-(1,3-$Me_2$-nBu) |
| 13-43 | 4-$CHF_2$-2-Me-thiazole-5-yl | N | $CF_3$ | H | 2-(1,3-$Me_2$-nBu) |
| 13-44 | 4-$CHF_2$-2-Me-thiazole-5-yl | N | $CHF_2$ | H | 2-(1,3-$Me_2$-nBu) |
| 13-45 | 4-$CF_3$-thiazole-5-yl | N | $CF_3$ | H | 2-(1,3-$Me_2$-nBu) |
| 13-46 | 4-$CF_3$-thiazole-5-yl | N | $CHF_2$ | H | 2-(1,3-$Me_2$-nBu) |
| 13-47 | 4-$CHF_2$-thiazole-5-yl | N | $CF_3$ | H | 2-(1,3-$Me_2$-nBu) |
| 13-48 | 4-$CHF_2$-thiazole-5-yl | N | $CHF_2$ | H | 2-(1,3-$Me_2$-nBu) |
| 13-49 | 3,4-$Cl_2$-isoxazole-5-yl | CH | $CHF_2$ | H | 2-(1,3-$Me_2$-nBu) |
| 13-50 | 2,3-$Cl_2$-thiophen-5-yl | CH | $CHF_2$ | H | 2-(1,3-$Me_2$-nBu) |
| 13-51 | 3,4-$Cl_2$-isothiazol-5-yl | CH | F | H | 2-(1,3-$Me_2$-nBu) |

[Table 13-3]

| No. | $R^1$ | $W^4$ | D | Ep | Xn |
|---|---|---|---|---|---|
| 13-52 | 3,4-$Cl_2$-isothiazol-5-yl | CH | Me | H | 2-(1,3-$Me_2$-nBu) |
| 13-53 | 3,4-$Cl_2$-isothiazol-5-yl | CH | Cl | H | 2-(1,3-$Me_2$-nBu) |
| 13-54 | 3,4-$Cl_2$-isothiazol-5-yl | CH | Br | H | 2-(1,3-$Me_2$-nBu) |
| 13-55 | 3,4-$Cl_2$-isothiazol-5-yl | N | $CF_3$ | H | 2-(3,4-$F_2$-Ph) |
| 13-56 | 3,4-$Cl_2$-isothiazol-5-yl | CH | $CH_2F$ | H | 2-(1,3-$Me_2$-nBu) |
| 13-57 | 3,4-$Cl_2$-isothiazol-5-yl | CH | $CHFCH_3$ | H | 2-(1,3-$Me_2$-nBu) |
| 13-58 | 2,6-$F_2$-pyridin-4-yl | CH | $CHF_2$ | H | 2-(1,3-$Me_2$-nBu) |

[Table 14-1]

Table 14

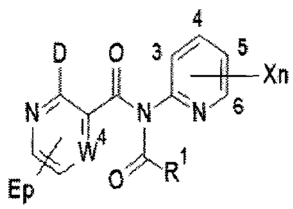

| No. | $R^1$ | $W^4$ | D | Ep | Xn |
|---|---|---|---|---|---|
| 14-1 | 3,4-$Cl_2$-isothiazol-5-yl | CH | $CF_3$ | H | 3-(1,3-$Me_2$-nBu) |
| 14-2 | 3,4-$Cl_2$-isothiazol-5-yl | CH | $CHF_2$ | H | 3-(1,3-$Me_2$-nBu) |
| 14-3 | 3-Me-isothiazol-5-yl | CH | $CF_3$ | H | 3-(1,3-$Me_2$-nBu) |
| 14-4 | 3-Me-isothiazol-5-yl | CH | $CHF_2$ | H | 3-(1,3-$Me_2$-nBu) |
| 14-5 | 3-Me-4-Cl-isothiazol-5-yl | CH | $CF_3$ | H | 3-(1,3-$Me_2$-nBu) |
| 14-6 | 3-Me-4-Cl-isothiazol-5-yl | CH | $CHF_2$ | H | 3-(1,3-$Me_2$-nBu) |

(continued)

| No. | $R^1$ | $W^4$ | D | Ep | Xn |
|---|---|---|---|---|---|
| 14-7 | 4-Me-1,2,3-thiadiazol-5-yl | CH | $CF_3$ | H | 3-(1,3-$Me_2$-nBu) |
| 14-8 | 4-Me-1,2,3-thiadiazol-5-yl | CH | $CHF_2$ | H | 3-(1,3-$Me_2$-nBu) |
| 14-9 | 4-$CF_3$-1,2,3-thiadiazol-5-yl | CH | $CF_3$ | H | 3-(1,3-$Me_2$-nBu) |
| 14-10 | 4-$CF_3$-1,2,3-thiadiazol-5-yl | CH | $CHF_2$ | H | 3-(1,3-$Me_2$-nBu) |
| 14-11 | 4-$CHF_2$-1,2,3-thiadiazol-5-yl | CH | $CF_3$ | H | 3-(1,3-$Me_2$-nBu) |
| 14-12 | 4-$CHF_2$-1,2,3-thiadiazol-5-yl | CH | $CHF_2$ | H | 3-(1,3-$Me_2$-nBu) |
| 14-13 | 1,2,3-benzothiadiazol-7-yl | CH | $CF_3$ | H | 3-(1,3-$Me_2$-nBu) |
| 14-14 | 1,2,3-benzothiadiazol-7-yl | CH | $CHF_2$ | H | 3-(1,3-$Me_2$-nBu) |
| 14-15 | 4-$CF_3$-2-Me-thiazole-5-yl | CH | $CF_3$ | H | 3-(1,3-$Me_2$-nBu) |
| 14-16 | 4-$CF_3$-2-Me-thiazole-5-yl | CH | $CHF_2$ | H | 3-(1,3-$Me_2$-nBu) |
| 14-17 | 4-$CHF_2$-2-Me-thiazole-5-yl | CH | $CF_3$ | H | 3-(1,3-$Me_2$-nBu) |
| 14-18 | 4-$CHF_2$-2-Me-thiazole-5-yl | CH | $CHF_2$ | H | 3-(1,3-$Me_2$-nBu) |
| 14-19 | 4-$CF_3$-thiazole-5-yl | CH | $CF_3$ | H | 3-(1,3-$Me_2$-nBu) |
| 14-20 | 4-$CF_3$-thiazole-5-yl | CH | $CHF_2$ | H | 3-(1,3-$Me_2$-nBu) |
| 14-21 | 4-$CHF_2$-thiazole-5-yl | CH | $CF_3$ | H | 3-(1,3-$Me_2$-nBu) |
| 14-22 | 4-$CHF_2$-thiazole-5-yl | CH | $CHF_2$ | H | 3-(1,3-$Me_2$-nBu) |
| 14-23 | 3,4-$Cl_2$-isothiazol-5-yl | N | $CF_3$ | H | 3-(1,3-$Me_2$-nBu) |

[Table 14-2]

| No. | $R^1$ | $W^4$ | D | Ep | Xn |
|---|---|---|---|---|---|
| 14-24 | 3,4-$Cl_2$-isothiazol-5-yl | N | $CHF_2$ | H | 3-(1,3-$Me_2$-nBu) |
| 14-25 | 3-Me-isothiazol-5-yl | N | $CF_3$ | H | 3-(1,3-$Me_2$-nBu) |
| 14-26 | 3-Me-isothiazol-5-yl | N | $CHF_2$ | H | 3-(1,3-$Me_2$-nBu) |
| 14-27 | 3-Me-4-Cl-isothiazol-5-yl | N | $CF_3$ | H | 3-(1,3-$Me_2$-nBu) |
| 14-28 | 3-Me-4-Cl-isothiazol-5-yl | N | $CHF_2$ | H | 3-(1,3-$Me_2$-nBu) |
| 14-29 | 4-Me-1,2,3-thiadiazol-5-yl | N | $CF_3$ | H | 3-(1,3-$Me_2$-nBu) |
| 14-30 | 4-Me-1,2,3-thiadiazol-5-yl | N | $CHF_2$ | H | 3-(1,3-$Me_2$-nBu) |
| 14-31 | 4-$CF_3$-1,2,3-thiadiazol-5-yl | N | $CF_3$ | H | 3-(1,3-$Me_2$-nBu) |
| 14-32 | 4-$CF_3$-1,2,3-thiadiazol-5-yl | N | $CHF_2$ | H | 3-(1,3-$Me_2$-nBu) |
| 14-33 | 4-$CHF_2$-1,2,3-thiadiazol-5-yl | N | $CF_3$ | H | 3-(1,3-$Me_2$-nBu) |
| 14-34 | 4-$CHF_2$-1,2,3-thiadiazol-5-yl | N | $CHF_2$ | H | 3-(1,3-$Me_2$-nBu) |
| 14-35 | 1,2,3-benzothiadiazol-7-yl | N | $CF_3$ | H | 3-(1,3-$Me_2$-nBu) |
| 14-36 | 1,2,3-benzothiadiazol-7-yl | N | $CHF_2$ | H | 3-(1,3-$Me_2$-nBu) |
| 14-37 | 4-$CF_3$-2-Me-thiazole-5-yl | N | $CF_3$ | H | 3-(1,3-$Me_2$-nBu) |
| 14-38 | 4-$CF_3$-2-Me-thiazole-5-yl | N | $CHF_2$ | H | 3-(1,3-$Me_2$-nBu) |
| 14-39 | 4-$CHF_2$-2-Me-thiazole-5-yl | N | $CF_3$ | H | 3-(1,3-$Me_2$-nBu) |
| 14-40 | 4-$CHF_2$-2-Me-thiazole-5-yl | N | $CHF_2$ | H | 3-(1,3-$Me_2$-nBu) |

(continued)

| No. | $R^1$ | $W^4$ | D | Ep | Xn |
|---|---|---|---|---|---|
| 14-41 | 4-$CF_3$-thiazole-5-yl | N | $CF_3$ | H | 3-(1,3-$Me_2$-nBu) |
| 14-42 | 4-$CF_3$-thiazole-5-yl | N | $CHF_2$ | H | 3-(1,3-$Me_2$-nBu) |
| 14-43 | 4-$CHF_2$-thiazole-5-yl | N | $CF_3$ | H | 3-(1,3-$Me_2$-nBu) |
| 14-44 | 4-$CHF_2$-thiazole-5-yl | N | $CHF_2$ | H | 3-(1,3-$Me_2$-nBu) |

[Table 15-1]

Table 15

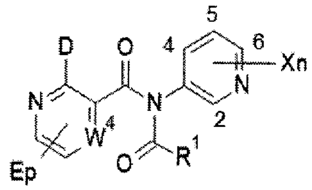

| No. | $R^1$ | $W^4$ | D | Ep | Xn |
|---|---|---|---|---|---|
| 15-1 | 3,4-$Cl_2$-isothiazol-5-yl | CH | $CF_3$ | H | 2-(1,3-$Me_2$-nBu) |
| 15-2 | 3,4-$Cl_2$-isothiazol-5-yl | CH | $CHF_2$ | H | 2-(2-cPr-cPr) |
| 15-3 | 3,4-$Cl_2$-isothiazol-5-yl | CH | $CHF_2$ | H | 2-nBu |
| 15-4 | 3,4-$Cl_2$-isothiazol-5-yl | CH | $CHF_2$ | H | 2-(3-Me-nBu |
| 15-5 | 3,4-$Cl_2$-isothiazol-5-yl | CH | $CHF_2$ | H | 2-(1,3-$Me_2$-nBu) |
| 15-6 | 3,4-$Cl_2$-isothiazol-5-yl | CH | $CHF_2$ | H | 2-(1,3,3-$Me_3$-nBu) |
| 15-7 | 3,4-$Cl_2$-isothiazol-5-yl | CH | $CHF_2$ | H | 2-(1,3-$Me_2$-nPen) |
| 15-8 | 3,4-$Cl_2$-isothiazol-5-yl | CH | $CHF_2$ | H | 2-(1,4-$Me_2$-nPen) |
| 15-9 | 3-Me-isothiazol-5-yl | CH | $CF_3$ | H | 2-(1,3-$Me_2$-nBu |
| 15-10 | 3-Me-isothiazol-5-yl | CH | $CHF_2$ | H | 2-(1,3-$Me_2$-nBu) |
| 15-11 | 3-Me-4-Cl-isothiazol-5-yl | CH | $CF_3$ | H | 2-(1,3-$Me_2$-nBu) |
| 15-12 | 3-Me-4-Cl-isothiazol-5-yl | CH | $CHF_2$ | H | 2-(1,3-$Me_2$-nBu) |
| 15-13 | 4-Me-1,2,3-thiadiazol-5-yl | CH | $CF_3$ | H | 2-(1,3-$Me_2$-nBu) |
| 15-14 | 4-Me-1,2,3-thiadiazol-5-yl | CH | $CHF_2$ | H | 2-(1,3-$Me_2$-nBu) |
| 15-15 | 4-$CF_3$-1,2,3-thiadiazol-5-yl | CH | $CF_3$ | H | 2-(1,3-$Me_2$-nBu |
| 15-16 | 4-$CF_3$-1,2,3-thiadiazol-5-yl | CH | $CHF_2$ | H | 2-(1,3-$Me_2$-nBu) |
| 15-17 | 4-$CHF_2$-1,2,3-thiadiazol-5-yl | CH | $CF_3$ | H | 2-(1,3-$Me_2$-nBu) |
| 15-18 | 4-$CHF_2$-1,2,3-thiadiazol-5-yl | CH | $CHF_2$ | H | 2-(1,3-$Me_2$-nBu) |
| 15-19 | 1,2,3-benzothiadiazol-7-yl | CH | $CF_3$ | H | 2-(1,3-$Me_2$-nBu) |
| 15-20 | 1,2,3-benzothiadiazol-7-yl | CH | $CHF_2$ | H | 2-(1,3-$Me_2$-nBu) |
| 15-21 | 4-$CF_3$-2-Me-thiazole-5-yl | CH | $CF_3$ | H | 2-(1,3-$Me_2$-nBu) |
| 15-22 | 4-$CF_3$-2-Me-thiazole-5-yl | CH | $CHF_2$ | H | 2-(1,3-$Me_2$-nBu) |
| 15-23 | 4-$CHF_2$-2-Me-thiazole-5-yl | CH | $CF_3$ | H | 2-(1,3-$Me_2$-nBu) |

[Table 15-2]

| No. | $R^1$ | $W^4$ | D | Ep | Xn |
|---|---|---|---|---|---|
| 15-24 | 4-$CHF_2$-2-Me-thiazole-5-yl | CH | $CHF_2$ | H | 2-(1,3-$Me_2$-nBu) |
| 15-25 | 3,4-$Cl_2$-isothiazol-5-yl | N | $CF_3$ | H | 2-(1,3-$Me_2$-nBu) |
| 15-26 | 3,4-$Cl_2$-isothiazol-5-yl | N | $CHF_2$ | H | 2-(2-cPr-cPr) |
| 15-27 | 3,4-$Cl_2$-isothiazol-5-yl | N | $CHF_2$ | H | 2-nBu |
| 15-28 | 3,4-$Cl_2$-isothiazol-5-yl | N | $CHF_2$ | H | 2-(3-Me-nBu) |
| 15-29 | 3,4-$Cl_2$-isothiazol-5-yl | N | $CHF_2$ | H | 2-(1,3-$Me_2$-nBu) |
| 15-30 | 3,4-$Cl_2$-isothiazol-5-yl | N | $CHF_2$ | H | 2-(1,3,3-$Me_3$-nBu) |
| 15-31 | 3,4-$Cl_2$-isothiazol-5-yl | N | $CHF_2$ | H | 2-(1,3-$Me_2$-nPen) |
| 15-32 | 3,4-$Cl_2$-isothiazol-5-yl | N | $CHF_2$ | H | 2-(1,4-$Me_2$-nPen) |
| 15-33 | 3-Me-isothiazol-5-yl | N | $CF_3$ | H | 2-(1,3-$Me_2$-nBu) |
| 15-34 | 3-Me-isothiazol-5-yl | N | $CHF_2$ | H | 2-(1,3-$Me_2$-nBu) |
| 15-35 | 3-Me-4-Cl-isothiazol-5-yl | N | $CF_3$ | H | 2-(1,3-$Me_2$-nBu) |
| 15-36 | 3-Me-4-Cl-isothiazol-5-yl | N | $CHF_2$ | H | 2-(1,3-$Me_2$-nBu) |
| 15-37 | 4-Me-1,2,3-thiadiazol-5-yl | N | $CF_3$ | H | 2-(1,3-$Me_2$-nBu) |
| 15-38 | 4-Me-1,2,3-thiadiazol-5-yl | N | $CHF_2$ | H | 2-(1,3-$Me_2$-nBu) |
| 15-39 | 4-$CF_3$-1,2,3-thiadiazol-5-yl | N | $CF_3$ | H | 2-(1,3-$Me_2$-nBu) |
| 15-40 | 4-$CF_3$-1,2,3-thiadiazol-5-yl | N | $CHF_2$ | H | 2-(1,3-$Me_2$-nBu) |
| 15-41 | 4-$CHF_2$-1,2,3-thiadiazol-5-yl | N | $CF_3$ | H | 2-(1,3-$Me_2$-nBu) |
| 15-42 | 4-$CHF_2$-1,2,3-thiadiazol-5-yl | N | $CHF_2$ | H | 2-(1,3-$Me_2$-nBu) |
| 15-43 | 1,2,3-benzothiadiazol-7-yl | N | $CF_3$ | H | 2-(1,3-$Me_2$-nBu) |
| 15-44 | 1,2,3-benzothiadiazol-7-yl | N | $CHF_2$ | H | 2-(1,3-$Me_2$-nBu) |
| 15-45 | 4-$CF_3$-2-Me-thiazole-5-yl | N | $CF_3$ | H | 2-(1,3-$Me_2$-nBu) |
| 15-46 | 4-$CF_3$-2-Me-thiazole-5-yl | N | $CHF_2$ | H | 2-(1,3-$Me_2$-nBu) |
| 15-47 | 4-$CHF_2$-2-Me-thiazole-5-yl | N | $CF_3$ | H | 2-(1,3-$Me_2$-nBu) |
| 15-48 | 4-$CHF_2$-2-Me-thiazole-5-yl | N | $CHF_2$ | H | 2-(1,3-$Me_2$-nBu) |

[Table 16-1]

Table 16

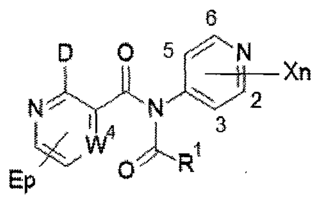

| No. | $R^1$ | $W^4$ | D | Ep | Xn |
|---|---|---|---|---|---|
| 16-1 | 3,4-$Cl_2$-isothiazol-5-yl | CH | $CF_3$ | H | 3-(1,3-$Me_2$-nBu) |
| 16-2 | 3,4-$Cl_2$-isothiazol-5-yl | CH | $CHF_2$ | H | 3-(1,3-$Me_2$-nBu) |
| 16-3 | 3-Me-isothiazol-5-l | CH | $CF_3$ | H | 3-(1,3-$Me_2$-nBu) |
| 16-4 | 3-Me-isothiazol-5-yl | CH | $CHF_2$ | H | 3-(1,3-$Me_2$-nBu) |
| 16-5 | 3-Me-4-Cl-isothiazol-5-yl | CH | $CF_3$ | H | 3-(1,3-$Me_2$-nBu) |

(continued)

| No. | $R^1$ | $W^4$ | D | Ep | Xn |
|---|---|---|---|---|---|
| 16-6 | 3-Me-4-Cl-isothiazol-5-yl | CH | $CHF_2$ | H | 3-(1,3-$Me_2$-nBu) |
| 16-7 | 4-Me-1,2,3-thiadiazol-5-yl | CH | $CF_3$ | H | 3-(1,3-$Me_2$-nBu) |
| 16-8 | 4-Me-1,2,3-thiadiazol-5-yl | CH | $CHF_2$ | H | 3-(1,3-$Me_2$-nBu) |
| 16-9 | 4-$CF_3$-1,2,3-thiadiazol-5-yl | CH | $CHF_2$ | H | 3-(1,3,3-$Me_3$-nBu) |
| 16-10 | 4-$CF_3$-1,2,3-thiadiazol-5-yl | CH | $CHF_2$ | H | 3-(1,3-$Me_2$-nPen) |
| 16-11 | 4-$CHF_2$-1,2,3-thiadiazol-5-yl | CH | $CF_3$ | H | 3-(1,3-$Me_2$-nBu) |
| 16-12 | 4-$CHF_2$-1,2,3-thiadiazol-5-yl | CH | $CF_3$ | H | 3-(1,3,3-$Me_3$-nBu) |
| 16-13 | 1,2,3-benzothiadiazol-7-yl | CH | $CF_3$ | H | 3-(1,3-$Me_2$-nPen) |
| 16-14 | 1,2,3-benzothiadiazol-7-yl | CH | $CHF_2$ | H | 3-(1,3-$Me_2$-nBu) |
| 16-15 | 4-$CF_3$-2-Me-thiazole-5-yl | CH | $CHF_2$ | H | 3-(1,3,3-$Me_3$-nBu) |
| 16-16 | 4-$CF_3$-2-Me-thiazole-5-yl | CH | $CHF_2$ | H | 3-(1,3-$Me_2$-nPen) |
| 16-17 | 4-$CHF_2$-2-Me-thiazole-5-yl | CH | $CF_3$ | H | 3-(1,3-$Me_2$-nBu) |
| 16-18 | 4-$CHF_2$-2-Me-thiazole-5-yl | CH | $CF_3$ | H | 3-(1,3,3-$Me_3$-nBu) |
| 16-19 | 4-$CF_3$-thiazole-5-yl | CH | $CF_3$ | H | 3-(1,3-$Me_2$-nPen) |
| 16-20 | 4-$CF_3$-thiazole-5-yl | CH | $CHF_2$ | H | 3-(1,3-$Me_2$-nBu) |
| 16-21 | 4-$CHF_2$-thiazole-5-yl | CH | $CHF_2$ | H | 3-(1,3,3-$Me_3$-nBu) |
| 16-22 | 4-$CHF_2$-thiazole-5-yl | CH | $CHF_2$ | H | 3-(1,3-$Me_2$-nPen) |
| 16-23 | 3,4-$Cl_2$-isothiazol-5-yl | N | $CF_3$ | H | 3-(1,3-$Me_2$-nBu) |

[Table 16-2]

| No. | $R^1$ | $W^4$ | D | Ep | Xn |
|---|---|---|---|---|---|
| 16-24 | 3,4-$Cl_2$-isothiazol-5-yl | N | $CHF_2$ | H | 3-(1,3-$Me_2$-nBu) |
| 16-25 | 3-Me-isothiazol-5-yl | N | $CF_3$ | H | 3-(1,3-$Me_2$-nBu) |
| 16-26 | 3-Me-isothiazol-5-yl | N | $CHF_2$ | H | 3-(1,3-$Me_2$-nBu) |
| 16-27 | 3-Me-4-Cl-isothiazol-5-yl | N | $CF_3$ | H | 3-(1,3-$Me_2$-nBu) |
| 16-28 | 3-Me-4-Cl-isothiazol-5-yl | N | $CHF_2$ | H | 3-(1,3-$Me_2$-nBu) |
| 16-29 | 4-Me-1,2,3-thiadiazol-5-yl | N | $CF_3$ | H | 3-(1,3-$Me_2$-nBu) |
| 16-30 | 4-Me-1,2,3-thiadiazol-5-yl | N | $CHF_2$ | H | 3-(1,3-$Me_2$-nBu) |
| 16-31 | 4-$CF_3$-1,2,3-thiadiazol-5-yl | N | $CHF_2$ | H | 3-(1,3,3-$Me_3$-nBu) |
| 16-32 | 4-$CF_3$-1,2,3-thiadiazol-5-yl | N | $CHF_2$ | H | 3-(1,3-$Me_2$-nPen) |
| 16-33 | 4-$CHF_2$-1,2,3-thiadiazol-5-yl | N | $CF_3$ | H | 3-(1,3-$Me_2$-nBu) |
| 16-34 | 4-$CHF_2$-1,2,3-thiadiazol-5-yl | N | $CF_3$ | H | 3-(1,3,3-$Me_3$-nBu) |
| 16-35 | 1,2,3-benzothiadiazol-7-yl | N | $CF_3$ | H | 3-(1,3-$Me_2$-nPen) |
| 16-36 | 1,2,3-benzothiadiazol-7-yl | N | $CHF_2$ | H | 3-(1,3-$Me_2$-nBu) |
| 16-37 | 4-$CF_3$-2-Me-thiazole-5-yl | N | $CHF_2$ | H | 3-(1,3,3-$Me_3$-nBu) |
| 16-38 | 4-$CF_3$-2-Me-thiazole-5-yl | N | $CHF_2$ | H | 3-(1,3-$Me_2$-nPen) |
| 16-39 | 4-$CHF_2$-2-Me-thiazole-5-yl | N | $CF_3$ | H | 3-(1,3-$Me_2$-nBu) |

(continued)

| No. | $R^1$ | $W^4$ | D | Ep | Xn |
|---|---|---|---|---|---|
| 16-40 | 4-$CHF_2$-2-Me-thiazole-5-yl | N | $CF_3$ | H | 3-(1,3,3-$Me_3$-nBu) |
| 16-41 | 4-$CF_3$-thiazole-5-yl | N | $CF_3$ | H | 3-(1,3-$Me_2$-nPen) |
| 16-42 | 4-$CF_3$-thiazole-5-yl | N | $CHF_2$ | H | 3-(1,3-$Me_2$-nBu) |
| 16-43 | 4-$CHF_2$-thiazole-5-yl | N | $CHF_2$ | H | 3-(1,3,3-$Me_3$-nBu) |
| 16-44 | 4-$CHF_2$-thiazole-5-yl | N | $CHF_2$ | H | 3-(1,3-$Me_2$-nPen) |

[Table 17-1]

Table 17

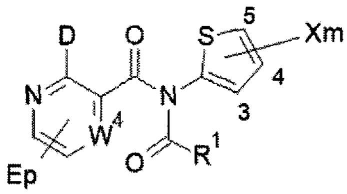

| No. | $R^1$ | $W^4$ | D | Ep | Xm |
|---|---|---|---|---|---|
| 17-1 | 3,4-$Cl_2$-isothiazol-5-yl | CH | $CF_3$ | H | 3-(1,3-$Me_2$-nBu) |
| 17-2 | 3,4-$Cl_2$-isothiazol-5-yl | CH | $CHF_2$ | H | 3-(1,3-$Me_2$-nBu) |
| 17-3 | 3-Me-isothiazol-5-yl | CH | $CF_3$ | H | 3-(1,3-$Me_2$-nBu) |
| 17-4 | 3-Me-isothiazol-5-yl | CH | $CHF_2$ | H | 3-(1,3-$Me_2$-nBu) |
| 17-5 | 3-Me-4-Cl-isothiazol-5-yl | CH | $CF_3$ | H | 3-(1,3-$Me_2$-nBu) |
| 17-6 | 3-Me-4-Cl-isothiazol-5-yl | CH | $CHF_2$ | H | 3-(1,3-$Me_2$-nBu) |
| 17-7 | 4-Me-1,2,3-thiadiazol-5-yl | CH | $CF_3$ | H | 3-(1,3-$Me_2$-nBu) |
| 17-8 | 4-Me-1,2,3-thiadiazol-5-yl | CH | $CHF_2$ | H | 3-(1,3-$Me_2$-nBu) |
| 17-9 | 4-$CF_3$-1,2,3-thiadiazol-5-yl | CH | $CF_3$ | H | 3-(1,3-$Me_2$-nBu) |
| 17-10 | 4-$CF_3$-1,2,3-thiadiazol-5-yl | CH | $CHF_2$ | H | 3-(1,3-$Me_2$-nBu) |
| 17-11 | 4-$CHF_2$-1,2,3-thiadiazol-5-yl | CH | $CF_3$ | H | 3-(1,3-$Me_2$-nBu) |
| 17-12 | 4-$CHF_2$-1,2,3-thiadiazol-5-yl | CH | $CHF_2$ | H | 3-(1,3-$Me_2$-nBu) |
| 17-13 | 1,2,3-benzothiadiazol-7-yl | CH | $CF_3$ | H | 3-(1,3-$Me_2$-nBu) |
| 17-14 | 1,2,3-benzothiadiazol-7-yl | CH | $CHF_2$ | H | 3-(1,3-$Me_2$-nBu) |
| 17-15 | 4-$CF_3$-2-Me-thiazole-5-yl | CH | $CF_3$ | H | 3-(1,3-$Me_2$-nBu) |
| 17-16 | 4-$CF_3$-2-Me-thiazole-5-yl | CH | $CHF_2$ | H | 3-(1,3-$Me_2$-nBu) |
| 17-17 | 4-$CHF_2$-2-Me-thiazole-5-yl | CH | $CF_3$ | H | 3-(1,3-$Me_2$-nBu) |
| 17-18 | 4-$CHF_2$-2-Me-thiazole-5-yl | CH | $CHF_2$ | H | 3-(1,3-$Me_2$-nBu) |
| 17-19 | 4-$CF_3$-thiazole-5-yl | CH | $CF_3$ | H | 3-(1,3-$Me_2$-nBu) |
| 17-20 | 4-$CF_3$-thiazole-5-yl | CH | $CHF_2$ | H | 3-(1,3-$Me_2$-nBu) |
| 17-21 | 4-$CHF_2$-thiazole-5-yl | CH | $CF_3$ | H | 3-(1,3-$Me_2$-nBu) |
| 17-22 | 4-$CHF_2$-thiazole-5-yl | CH | $CHF_2$ | H | 3-(1,3-$Me_2$-nBu) |
| 17-23 | 3,4-$Cl_2$-isothiazol-5-yl | N | $CF_3$ | H | 3-(1,3-$Me_2$-nBu) |

[Table 17-2]

| No. | $R^1$ | $W^4$ | D | Ep | Xm |
|---|---|---|---|---|---|
| 17-24 | 3,4-$Cl_2$-isothiazol-5-yl | N | $CHF_2$ | H | 3-(1,3-$Me_2$-nBu) |
| 17-25 | 3-Me-isothiazol-5-yl | N | $CF_3$ | H | 3-(1,3-$Me_2$-nBu) |
| 17-26 | 3-Me-isothiazol-5-yl | N | $CHF_2$ | H | 3-(1,3-$Me_2$-nBu) |
| 17-27 | 3-Me-4-Cl-isothiazol5-yl | N | $CF_3$ | H | 3-(1,3-$Me_2$-nBu) |
| 17-28 | 3-Me-4-Cl-isothiazol-5-yl | N | $CHF_2$ | H | 3-(1,3-$Me_2$-nBu) |
| 17-29 | 4-Me-1,2,3-thiadiazol-5-yl | N | $CF_3$ | H | 3-(1,3-$Me_2$-nBu) |
| 17-30 | 4-Me-1,2,3-thiadiazol-5-yl | N | $CHF_2$ | H | 3-(1,3-$Me_2$-nBu) |
| 17-31 | 4-$CF_3$-1,2,3-thiadiazol-5-yl | N | $CF_3$ | H | 3-(1,3-$Me_2$-nBu) |
| 17-32 | 4-$CF_3$-1,2,3-thiadiazol-5-yl | N | $CHF_2$ | H | 3-(1,3-$Me_2$-nBu) |
| 17-33 | 4-$CHF_2$-1,2,3-thiadiazol-5-yl | N | $CF_3$ | H | 3-(1,3-$Me_2$-nBu) |
| 17-34 | 4-$CHF_2$-1,2,3-thiadiazol-5-yl | N | $CHF_2$ | H | 3-(1,3-$Me_2$-nBu) |
| 17-35 | 1,2,3-benzothiadiazol-7-yl | N | $CF_3$ | H | 3-(1,3-$Me_2$-nBu) |
| 17-36 | 1,2,3-benzothiadiazol-7-yl | N | $CHF_2$ | H | 3-(1,3-$Me_2$-nBu) |
| 17-37 | 4-$CF_3$-2-Me-thiazole-5-yl | N | $CF_3$ | H | 3-(1,3-$Me_2$-nBu) |
| 17-38 | 4-$CF_3$-2-Me-thiazole-5-yl | N | $CHF_2$ | H | 3-(1,3-$Me_2$-nBu) |
| 17-39 | 4-$CHF_2$-2-Me-thiazole-5-yl | N | $CF_3$ | H | 3-(1,3-$Me_2$-nBu) |
| 17-40 | 4-$CHF_2$-2-Me-thiazole-5-yl | N | $CHF_2$ | H | 3-(1,3-$Me_2$-nBu) |
| 17-41 | 4-$CF_3$-thiazole-5-yl | N | $CF_3$ | H | 3-(1,3-$Me_2$-nBu) |
| 17-42 | 4-$CF_3$-thiazole-5-yl | N | $CHF_2$ | H | 3-(1,3-$Me_2$-nBu) |
| 17-43 | 4-$CHF_2$-thiazole-5-yl | N | $CF_3$ | H | 3-(1,3-$Me_2$-nBu) |
| 17-44 | 4-$CHF_2$-thiazole-5-yl | N | $CHF_2$ | H | 3-(1,3-$Me_2$-nBu) |

[Table 18-1]

Table 18

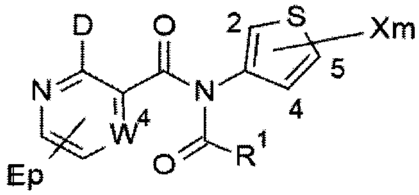

| No. | $R^1$ | $W^4$ | D | Ep | Xm |
|---|---|---|---|---|---|
| 18-1 | 3,4-$Cl_2$-isothiazol-5-yl | CH | $CF_3$ | H | 2-(1,3-$Me_2$-nBu) |
| 18-2 | 3,4-$Cl_2$-isothiazol-5-yl | CH | $CHF_2$ | H | 2-(1,3-$Me_2$-nBu) |
| 18-3 | 3-Me-isothiazol-5-yl | CH | $CF_3$ | H | 2-(1,3-$Me_2$-nBu) |
| 18-4 | 3-Me-isothiazol-5-yl | CH | $CHF_2$ | H | 2-(1,3-$Me_2$-nBu) |
| 18-5 | 3-Me-4-Cl-isothiazol-5-vl | CH | $CF_3$ | H | 2-(1,3-$Me_2$-nBu) |
| 18-6 | 3-Me-4-Cl-isothiazol-5-yl | CH | $CHF_2$ | H | 2-(1,3-$Me_2$-nBu) |
| 18-7 | 4-Me-1,2,3-thiadiazol-5-yl | CH | $CF_3$ | H | 2-(1,3-$Me_2$-nBu) |
| 18-8 | 4-Me-1,2,3-thiadiazol-5-yl | CH | $CHF_2$ | H | 2-(1,3-$Me_2$-nBu) |

(continued)

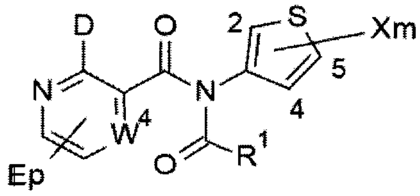

Table 18

| No. | $R^1$ | $W^4$ | D | Ep | Xm |
|---|---|---|---|---|---|
| 18-9 | 4-$CF_3$-1,2,3-thiadiazol-5-yl | CH | $CF_3$ | H | 2-(1,3-$Me_2$-nBu) |
| 18-10 | 4-$CF_3$-1,2,3-thiadiazol-5-yl | CH | $CHF_2$ | H | 2-(1,3-$Me_2$-nBu) |
| 18-11 | 4-$CHF_2$-1,2,3-thiadiazol-5-yl | CH | $CF_3$ | H | 2-(1,3-$Me_2$-nBu) |
| 18-12 | 4-$CHF_2$-1,2,3-thiadiazol-5-yl | CH | $CHF_2$ | H | 2-(1,3-$Me_2$-nBu) |
| 18-13 | 1,2,3-benzothiadiazol-7-yl | CH | $CF_3$ | H | 2-(1,3-$Me_2$-nBu) |
| 18-14 | 1,2,3-benzothiadiazol-7-yl | CH | $CHF_2$ | H | 2-(1,3-$Me_2$-nBu) |
| 18-15 | 4-$CF_3$-2-Me-thiazole-5-yl | CH | $CF_3$ | H | 2-(1,3-$Me_2$-nBu) |
| 18-16 | 4-$CF_3$-2-Me-thiazole-5-yl | CH | $CHF_2$ | H | 2-(1,3-$Me_2$-nBu) |
| 18-17 | 4-$CHF_2$-2-Me-thiazole-5-yl | CH | $CF_3$ | H | 2-(1,3-$Me_2$-nBu) |
| 18-18 | 4-$CHF_2$-2-Me-thiazole-5-yl | CH | $CHF_2$ | H | 2-(1,3-$Me_2$-nBu) |
| 18-19 | 4-$CF_3$-thiazole-5-yl | CH | $CF_3$ | H | 2-(1,3-$Me_2$-nBu) |
| 18-20 | 4-$CF_3$-thiazole-5-yl | CH | $CHF_2$ | H | 2-(1,3-$Me_2$-nBu) |
| 18-21 | 4-$CHF_2$-thiazole-5-yl | CH | $CF_3$ | H | 2-(1,3-$Me_2$-nBu) |
| 18-22 | 4-$CHF_2$-thiazole-5-yl | CH | $CHF_2$ | H | 2-(1,3-$Me_2$-nBu) |
| 18-23 | 3,4-$Cl_2$-isothiazol-5-yl | N | $CF_3$ | H | 2-(1,3-$Me_2$-nBu) |

[Table 18-2]

| No. | $R^1$ | $W^4$ | D | Ep | Xm |
|---|---|---|---|---|---|
| 18-24 | 3,4-$Cl_2$-isothiazol-5-yl | N | $CHF_2$ | H | 2-(1,3-$Me_2$-nBu) |
| 18-25 | 3-Me-isothiazol-5-yl | N | $CF_3$ | H | 2-(1,3-$Me_2$-nBu) |
| 18-26 | 3-Me-isothiazol-5-yl | N | $CHF_2$ | H | 2-(1,3-$Me_2$-nBu) |
| 18-27 | 3-Me-4-Cl-isothiazol-5-yl | N | $CF_3$ | H | 2-(1,3-$Me_2$-nBu) |
| 18-28 | 3-Me-4-Cl-isothiazol-5-yl | N | $CHF_2$ | H | 2-(1,3-$Me_2$-nBu) |
| 18-29 | 4-Me-1,2,3-thiadiazol-5-yl | N | $CF_3$ | H | 2-(1,3-$Me_2$-nBu) |
| 18-30 | 4-Me-1,2,3-thiadiazol-5-yl | N | $CHF_2$ | H | 2-(1,3-$Me_2$-nBu) |
| 18-31 | 4-$CF_3$-1,2,3-thiadiazol-5-yl | N | $CF_3$ | H | 2-(1,3-$Me_2$-nBu) |
| 18-32 | 4-$CF_3$-1,2,3-thiadiazol-5-yl | N | $CHF_2$ | H | 2-(1,3-$Me_2$-nBu) |
| 18-33 | 4-$CHF_2$-1,2,3-thiadiazol-5-yl | N | $CF_3$ | H | 2-(1,3-$Me_2$-nBu) |
| 18-34 | 4-$CHF_2$-1,2,3-thiadiazol-5-yl | N | $CHF_2$ | H | 2-(1,3-$Me_2$-nBu) |
| 18-35 | 1,2,3-benzothiadiazol-7-yl | N | $CF_3$ | H | 2-(1,3-$Me_2$-nBu) |
| 18-36 | 1,2,3-benzothiadiazol-7-yl | N | $CHF_2$ | H | 2-(1,3-$Me_2$-nBu) |
| 18-37 | 4-$CF_3$-2-Me-thiazole-5-yl | N | $CF_3$ | H | 2-(1,3-$Me_2$-nBu) |
| 18-38 | 4-$CF_3$-2-Me-thiazole-5-yl | N | $CHF_2$ | H | 2-(1,3-$Me_2$-nBu) |

(continued)

| No. | $R^1$ | $W^4$ | D | Ep | Xm |
|---|---|---|---|---|---|
| 18-39 | 4-$CHF_2$-2-Me-thiazole-5-yl | N | $CF_3$ | H | 2-(1,3-$Me_2$-nBu) |
| 18-40 | 4-$CHF_2$-2-Me-thiazole-5-yl | N | $CHF_2$ | H | 2-(1,3-$Me_2$-nBu) |
| 18-41 | 4-$CF_3$-thiazole-5-yl | N | $CF_3$ | H | 2-(1,3-$Me_2$-nBu) |
| 18-42 | 4-$CF_3$-thiazole-5-yl | N | $CHF_2$ | H | 2-(1,3-$Me_2$-nBu) |
| 18-43 | 4-$CHF_2$-thiazole-5-yl | N | $CF_3$ | H | 2-(1,3-$Me_2$-nBu) |
| 18-44 | 4-$CHF_2$-thiazole-5-yl | N | $CHF_2$ | H | 2-(1,3-$Me_2$-nBu) |

[Table 19-1]

Table 19

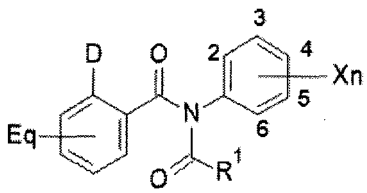

| No. | $R^1$ | D | Eq | Xn |
|---|---|---|---|---|
| 19-1 | 3,4-$Cl_2$-isothiazol-5-yl | $CF_3$ | H | 2-(1,3-$Me_2$-nBu) |
| 19-2 | 3,4-$Cl_2$-isothiazol-5-yl | $CHF_2$ | H | 2-(1,3-$Me_2$-nBu) |
| 19-3 | 3,4-$Cl_2$-isothiazol-5-yl | $CHF_2$ | H | 2-(1,3,3-$Me_3$-nBu) |
| 19-4 | 3,4-$Cl_2$-isothiazol-5-yl | $CHF_2$ | H | 2-(1,3-$Me_2$-nPen) |
| 19-5 | 3-Me-isothiazol-5-yl | $CF_3$ | H | 2-(1,3-$Me_2$-nBu) |
| 19-6 | 3-Me-isothiazol-5-yl | $CHF_2$ | H | 2-(1,3-$Me_2$-nBu) |
| 19-7 | 3-Me-4-Cl-isothiazol-5-yl | $CF_3$ | H | 2-(1,3-$Me_2$-nBu) |
| 19-8 | 3-Me-4-Cl-isothiazol-5-yl | $CHF_2$ | H | 2-(1,3-$Me_2$-nBu) |
| 19-9 | 4-Me-1,2,3-thiadiazol-5-yl | $CF_3$ | H | 2-(1,3-$Me_2$-nBu) |
| 19-10 | 4-Me-1,2,3-thiadiazol-5-yl | $CHF_2$ | H | 2-(1,3-$Me_2$-nBu) |
| 19-11 | 4-$CF_3$-1,2,3-thiadiazol-5-yl | $CF_3$ | H | 2-(1,3-$Me_2$-nBu) |
| 19-12 | 4-$CF_3$-1,2,3-thiadiazol-5-yl | $CHF_2$ | H | 2-(1,3-$Me_2$-nBu) |
| 19-13 | 4-$CHF_2$-1,2,3-thiadiazol-5-yl | $CF_3$ | H | 2-(1,3-$Me_2$-nBu) |
| 19-14 | 4-$CHF_2$-1,2,3-thiadiazol-5-yl | $CHF_2$ | H | 2-(1,3-$Me_2$-nBu) |
| 19-15 | 1,2,3-benzothiadiazol-7-yl | $CF_3$ | H | 2-(1,3-$Me_2$-nBu) |
| 19-16 | 1,2,3-benzothiadiazol-7-yl | $CRF_2$ | H | 2-(1,3-$Me_2$-nBu) |
| 19-17 | 4-$CF_3$-2-Me-thiazole-5-yl | $CF_3$ | H | 2-(1,3-$Me_2$-nBu) |
| 19-18 | 4-$CF_3$-2-Me-thiazole-5-yl | $CHF_2$ | H | 2-(1,3-$Me_2$-nBu) |
| 19-19 | 4-$CHF_2$-2-Me-thiazole-5-yl | $CF_3$ | H | 2-(1,3-$Me_2$-nBu) |
| 19-20 | 4-$CHF_2$-2-Me-thiazole-5-yl | $CHF_2$ | H | 2-(1,3-$Me_2$-nBu) |
| 19-21 | 4-$CF_3$-thiazole-5-yl | $CF_3$ | H | 2-(1,3-$Me_2$-nBu) |
| 19-22 | 4-$CF_3$-thiazole-5-yl | $CHF_2$ | H | 2-(1,3-$Me_2$-nBu) |
| 19-23 | 4-$CHF_2$-thiazole-5-yl | $CF_3$ | H | 2-(1,3-$Me_2$-nBu) |
| 19-24 | 4-$CHF_2$-thiazole-5-yl | $CHF_2$ | H | 2-(1,3-$Me_2$-nBu) |

[Table 20-1]

Table 20

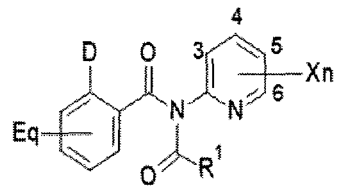

| No. | $R^1$ | D | Eq | Xn |
|---|---|---|---|---|
| 20-1 | 3,4-$Cl_2$-isothiazol-5-yl | $CF_3$ | H | 3-(1,3-$Me_2$-nBu) |
| 20-2 | 3,4-$Cl_2$-isothiazol-5-yl | $CHF_2$ | H | 3-(1,3-$Me_2$-nBu) |
| 20-3 | 3,4-$Cl_2$-isothiazol-5-yl | $CHF_2$ | H | 3-(1,3,3-$Me_3$-nBu) |
| 20-4 | 3,4-$Cl_2$-isothiazol-5-yl | $CHF_2$ | H | 3-(1,3-$Me_2$-nPen) |
| 20-5 | 3-Me-isothiazol-5-yl | $CF_3$ | H | 3-(1,3-$Me_2$-nBu) |
| 20-6 | 3-Me-isothiazol-5-yl | $CHF_2$ | H | 3-(1,3-$Me_2$-nBu) |
| 20-7 | 3-Me-4-Cl-isothiazol-5-yl | $CF_3$ | H | 3-(1,3-$Me_2$-nBu) |
| 20-8 | 3-Me-4-Cl-isothiazol-5-yl | $CHF_2$ | H | 3-(1,3-$Me_2$-nBu) |
| 20-9 | 4-Me-1,2,3-thiadiazol-5-yl | $CF_3$ | H | 3-(1,3-$Me_2$-nBu) |
| 20-10 | 4-Me-1,2,3-thiadiazol-5-yl | $CHF_2$ | H | 3-(1,3-$Me_2$-nBu) |
| 20-11 | 4-$CF_3$-1,2,3-thiadiazol-5-yl | $CF_3$ | H | 3-(1,3-$Me_2$-nBu) |
| 20-12 | 4-$CF_3$-1,2,3-thiadiazol-5-yl | $CHF_2$ | H | 3-(1,3-$Me_2$-nBu) |
| 20-13 | 4-$CHF_2$-1,2,3-thiadiazol-5-yl | $CF_3$ | H | 3-(1,3-$Me_2$-nBu) |
| 20-14 | 4-$CHF_2$-1,2,3-thiadiazol-5-yl | $CHF_2$ | H | 3-(1,3-$Me_2$-nBu) |
| 20-15 | 1,2,3-benzothiadiazol-7-yl | $CF_3$ | H | 3-(1,3-$Me_2$-nBu) |
| 20-16 | 1,2,3-benzothiadiazol-7-yl | $CHF_2$ | H | 3-(1,3-$Me_2$-nBu) |
| 20-17 | 4-$CF_3$-2-Me-thiazole-5-yl | $CF_3$ | H | 3-(1,3-$Me_2$-nBu) |
| 20-18 | 4-$CF_3$-2-Me-thiazole-5-yl | $CHF_2$ | H | 3-(1,3-$Me_2$-nBu) |
| 20-19 | 4-$CHF_2$-2-Me-thiazole-5-yl | $CF_3$ | H | 3-(1,3-$Me_2$-nBu) |
| 20-20 | 4-$CHF_2$-2-Me-thiazole-5-yl | $CHF_2$ | H | 3-(1,3-$Me_2$-nBu) |
| 20-21 | 4-$CF_3$-thiazole-5-yl | $CF_3$ | H | 3-(1,3-$Me_2$-nBu) |
| 20-22 | 4-$CF_3$-thiazole-5-yl | $CHF_2$ | H | 3-(1,3-$Me_2$-nBu) |
| 20-23 | 4-$CHF_2$-thiazole-5-yl | $CF_3$ | H | 3-(1,3-$Me_2$-nBu) |
| 20-24 | 4-$CHF_2$-thiazole-5-yl | $CHF_2$ | H | 3-(1,3-$Me_2$-nBu) |

[Table 21-1]

Table 21

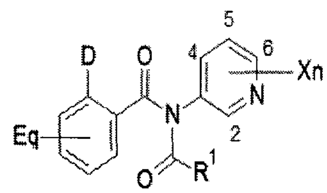

| No. | $R^1$ | D | Eq | Xn |
|---|---|---|---|---|
| 21-1 | 3,4-$Cl_2$-isothiazol-5-yl | $CF_3$ | H | 2-(1,3-$Me_2$-nBu) |

(continued)

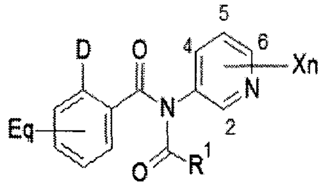

Table 21

| No. | $R^1$ | D | Eq | Xn |
|---|---|---|---|---|
| 21-2 | 3,4-$Cl_2$-isothiazol-5-yl | $CHF_2$ | H | 2-(1,3-$Me_2$-nBu) |
| 21-3 | 3,4-$Cl_2$-isothiazol-5-yl | $CHF_2$ | H | 2-(1,3,3-$Me_3$-nBu) |
| 21-4 | 3,4-$Cl_2$-isothiazol-5-yl | $CHF_2$ | H | 2-(1,3-$Me_2$-nPen) |
| 21-5 | 3-Me-isothiazol-5-yl | $CF_3$ | H | 2-(1,3-$Me_z$-nBu) |
| 21-6 | 3-Me-isothiazol-5-yl | $CHF_2$ | H | 2-(1,3-$Me_2$-nBu) |
| 21-7 | 3-Me-4-Cl-isothiazol-5-yl | $CF_3$ | H | 2-(1,3-$Me_2$-nBu) |
| 21-8 | 3-Me-4-Cl-isothiazol-5-yl | $CHF_2$ | H | 2-(1,3-$Me_2$-nBu) |
| 21-9 | 4-Me-1,2,3-thiadiazol-5-yl | $CF_3$ | H | 2-(1,3-$Me_2$-nBu) |
| 21-10 | 4-Me-1,2,3-thiadiazol-5-yl | $CHF_2$ | H | 2-(1,3-$Me_2$-nBu) |
| 21-11 | 4-$CF_3$-1,2,3-thiadiazol-5-yl | $CF_3$ | H | 2-(1,3-$Me_2$-nBu) |
| 21-12 | 4-$CF_3$-1,2,3-thiadiazol-5-yl | $CHF_2$ | H | 2-(1,3-$Me_2$-nBu) |
| 21-13 | 4-$CHF_2$-1,2,3-thiadiazol-5-yl | $CF_3$ | H | 2-(1,3-$Me_2$-nBu) |
| 21-14 | 4-$CHF_2$-1,2,3-thiadiazol-5-yl | $CHF_2$ | H | 2-(1,3-$Me_2$-nBu) |
| 21-15 | 1,2,3-benzothiadiazol-7-yl | $CF_3$ | H | 2-(1,3-$Me_2$-nBu) |
| 21-16 | 1,2,3-benzothiadiazol-7-yl | $CHF_2$ | H | 2-(1,3-$Me_2$-nBu) |
| 21-17 | 4-$CF_3$-2-Me-thiazole-5-yl | $CF_3$ | H | 2-(1,3-$Me_2$-nBu) |
| 21-18 | 4-$CF_3$-2-Me-thiazole-5-yl | $CHF_2$ | H | 2-(1,3-$Me_2$-nBu) |
| 21-19 | 4-$CHF_2$-2-Me-thiazole-5-yl | $CF_3$ | H | 2-(1,3-$Me_2$-nBu) |
| 21-20 | 4-$CHF_2$-2-Me-thiazole-5-yl | $CHF_2$ | H | 2-(1,3-$Me_2$-nBu) |
| 21-21 | 4-$CF_3$-thiazole-5-yl | $CF_3$ | H | 2-(1,3-$Me_2$-nBu) |
| 21-22 | 4-$CF_3$-thiazole-5-yl | $CHF_2$ | H | 2-(1,3-$Me_2$-nBu) |
| 21-23 | 4-$CHF_2$-thiazole-5-yl | $CF_3$ | H | 2-(1,3-$Me_2$-nBu) |
| 21-24 | 4-$CHF_2$-thiazole-5-yl | $CHF_2$ | H | 2-(1,3-$Me_2$-nBu) |

[Table 22-1]

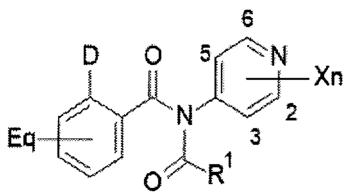

Table 22

| No. | $R^1$ | D | Eq | Xn |
|---|---|---|---|---|
| 22-1 | 3,4-$Cl_2$-isothiazol-5-yl | $CF_3$ | H | 3-(1,3-$Me_2$-nBu) |
| 22-2 | 3,4-$Cl_2$-isothiazol-5-yl | $CHF_2$ | H | 3-(1,3-$Me_2$-nBu) |

(continued)

Table 22

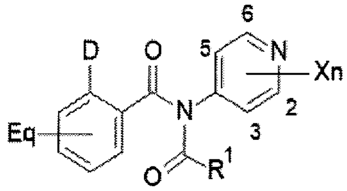

| No. | $R^1$ | D | Eq | Xn |
|---|---|---|---|---|
| 22-3 | 3,4-$Cl_2$-isothiazol-5-yl | $CHF_2$ | H | 3-(1,3,3-$Me_3$-nBu) |
| 22-4 | 3,4-$Cl_2$-isothiazol-5-yl | $CHF_2$ | H | 3-(1,3-$Me_2$-nPen) |
| 22-5 | 3-Me-isothiazol-5-yl | $CF_3$ | H | 3-(1,3-$Me_2$-nBu) |
| 22-6 | 3-Me-isothiazol-5-yl | $CHF_2$ | H | 3-(1,3-$Me_2$-nBu) |
| 22-7 | 3-Me-4-Cl-isothiazol-5-yl | $CF_3$ | H | 3-(1,3-$Me_2$-nBu) |
| 22-8 | 3-Me-4-Cl-isothiazol-5-yl | $CHF_2$ | H | 3-(1,3-$Me_2$-nBu) |
| 22-9 | 4-Me-1,2,3-thiadiazol-5-yl | $CF_3$ | H | 3-(1,3-$Me_2$-nBu) |
| 22-10 | 4-Me-1,2,3-thiadiazol-5-yl | $CHF_2$ | H | 3-(1,3-$Me_2$-nBu) |
| 22-11 | 4-$CF_3$-1,2,3-thiadiazol-5-yl | $CF_3$ | H | 3-(1,3-$Me_2$-nBu) |
| 22-12 | 4-$CF_3$-1,2,3-thiadiazol-5-yl | $CHF_2$ | H | 3-(1,3-$Me_2$-nBu) |
| 22-13 | 4-$CHF_2$-1,2,3-thiadiazol-5-yl | $CF_3$ | H | 3-(1,3-$Me_2$-nBu) |
| 22-14 | 4-$CHF_2$-1,2,3-thiadiazol-5-yl | $CHF_2$ | H | 3-(1,3-$Me_2$-nBu) |
| 22-15 | 1,2,3-benzothiadiazol-7-yl | $CF_3$ | H | 3-(1,3-$Me_2$-nBu) |
| 22-16 | 1,2,3-benzothiadiazol-7-yl | $CHF_2$ | H | 3-(1,3-$Me_2$-nBu) |
| 22-17 | 4-$CF_3$-2-Me-thiazole-5-yl | $CF_3$ | H | 3-(1,3-$Me_2$-nBu) |
| 22-18 | 4-$CF_3$-2-Me-thiazole-5-yl | $CHF_2$ | H | 3-(1,3-$Me_2$-nBu) |
| 22-19 | 4-$CHF_2$-2-Me-thiazole-5-yl | $CF_3$ | H | 3-(1,3-$Me_2$-nBu) |
| 22-20 | 4-$CHF_2$-2-Me-thiazole-5-yl | $CHF_2$ | H | 3-(1,3-$Me_2$-nBu) |
| 22-21 | 4-$CF_3$-thiazole-5-yl | $CF_3$ | H | 3-(1,3-$Me_2$-nBu) |
| 22-22 | 4-$CF_3$-thiazole-5-yl | $CHF_2$ | H | 3-(1,3-$Me_2$-nBu) |
| 22-23 | 4-$CHF_2$-thiazole-5-yl | $CF_3$ | H | 3-(1,3-$Me_2$-nBu) |
| 22-24 | 4-$CHF_2$-thiazole-5-yl | $CHF_2$ | H | 3-(1,3-$Me_2$-nBu) |

[Table 23-1]

Table 23

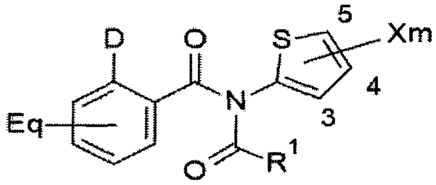

| No. | $R^1$ | D | Eq | Xm |
|---|---|---|---|---|
| 23-1 | 3,4-$Cl_2$-isothiazol-5-yl | $CF_3$ | H | 3-(1,3-$Me_2$-nBu) |
| 23-2 | 3,4-$Cl_2$-isothiazol-5-yl | $CHF_2$ | H | 3-(1,3-$Me_2$-nBu) |
| 23-3 | 3,4-$Cl_2$-isothiazol-5-yl | $CHF_2$ | H | 3-(1,3,3-$Me_3$-nBu) |

(continued)

| No. | $R^1$ | D | Eq | Xm |
|---|---|---|---|---|
| 23-4 | 3,4-$Cl_2$-isothiazol-5-yl | $CHF_2$ | H | 3-(1,3-$Me_2$-nPen) |
| 23-5 | 3-Me-isothiazol-5-yl | $CF_3$ | H | 3-(1,3-$Me_2$-nBu) |
| 23-6 | 3-Me-isothiazol-5-yl | $CHF_2$ | H | 3-(1,3-$Me_2$-nBu) |
| 23-7 | 3-Me-4-Cl-isothiazol-5-yl | $CF_3$ | H | 3-(1,3-$Me_2$-nBu) |
| 23-8 | 3-Me-4-Cl-isothiazol-5-yl | $CHF_2$ | H | 3-(1,3-$Me_2$-nBu) |
| 23-9 | 4-Me-1,2,3-thiadiazol-5-yl | $CF_3$ | H | 3-(1,3-$Me_2$-nBu) |
| 23-10 | 4-Me-1,2,3-thiadiazol-5-yl | $CHF_2$ | H | 3-(1,3-$Me_2$-nBu) |
| 23-11 | 4-$CF_3$-1,2,3-thiadiazol-5-yl | $CF_3$ | H | 3-(1,3-$Me_2$-nBu) |
| 23-12 | 4-$CF_3$-1,2,3-thiadiazol-5-yl | $CHF_2$ | H | 3-(1,3-$Me_2$-nBu) |
| 23-13 | 4-$CHF_2$-1,2,3-thiadiazol-5-yl | $CF_3$ | H | 3-(1,3-$Me_2$-nBu) |
| 23-14 | 4-$CHF_2$-1,2,3-thiadiazol-5-yl | $CHF_2$ | H | 3-(1,3-$Me_2$-nBu) |
| 23-15 | 1,2,3-benzothiadiazol-7-yl | $CF_3$ | H | 3-(1,3-$Me_2$-nBu) |
| 23-16 | 1,2,3-benzothiadiazol-7-yl | $CHF_2$ | H | 3-(1,3-$Me_2$-nBu) |
| 23-17 | 4-$CF_3$-2-Me-thiazole-5-yl | $CF_3$ | H | 3-(1,3-$Me_2$-nBu) |
| 23-18 | 4-$CF_3$-2-Me-thiazole-5-yl | $CHF_2$ | H | 3-(1,3-$Me_2$-nBu) |
| 23-19 | 4-$CHF_2$-2-Me-thiazole-5-yl | $CF_3$ | H | 3-(1,3-$Me_2$-nBu) |
| 23-20 | 4-$CHF_2$-2-Me-thiazole-5-yl | $CHF_2$ | H | 3-(1,3-$Me_2$-nBu) |
| 23-21 | 4-$CF_3$-thiazole-5-yl | $CF_3$ | H | 3-(1,3-$Me_2$-nBu) |
| 23-22 | 4-$CF_3$-thiazole-5-yl | $CHF_2$ | H | 3-(1,3-$Me_2$-nBu) |
| 23-23 | 4-$CHF_2$-thiazole-5-yl | $CF_3$ | H | 3-(1,3-$Me_2$-nBu) |
| 23-24 | 4-$CHF_2$-thiazole-5-yl | $CHF_2$ | H | 3-(1,3-$Me_2$-nBu) |

[Table 24-1]

Table 24

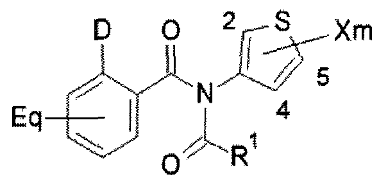

| No. | $R^1$ | D | Eq | Xm |
|---|---|---|---|---|
| 24-1 | 3,4-$Cl_2$-isothiazol-5-yl | $CF_3$ | H | 2-(1,3-$Me_2$-nBu) |
| 24-2 | 3,4-$Cl_2$-isothiazol-5-yl | $CHF_2$ | H | 2-(1,3-$Me_2$-nBu) |
| 24-3 | 3,4-$Cl_2$-isothiazol-5-yl | $CHF_2$ | H | 2-(1,3,3-$Me_3$-nBu) |
| 24-4 | 3,4-$Cl_2$-isothiazol-5-yl | $CHF_2$ | H | 2-(1,3-$Me_2$-nPen) |
| 24-5 | 3-Me-isothiazol-5-yl | $CF_3$ | H | 2-(1,3-$Me_2$-nBu) |
| 24-6 | 3-Me-isothiazol-5-yl | $CHF_2$ | H | 2-(1,3-$Me_2$-nBu) |
| 24-7 | 3-Me-4-Cl-isothiazol-5-yl | $CF_3$ | H | 2-(1,3-$Me_2$-nBu) |
| 24-8 | 3-Me-4-Cl-isothiazol-5-vl | $CHF_2$ | H | 2-(1,3-$Me_2$-nBu) |
| 24-9 | 4-Me-1,2,3-thiadiazol-5-yl | $CF_3$ | H | 2-(1,3-$Me_2$-nBu) |

(continued)

| No. | $R^1$ | D | Eq | Xm |
|---|---|---|---|---|
| 24-10 | 4-Me-1,2,3-thiadiazol-5-yl | $CHF_2$ | H | 2-(1,3-$Me_2$-nBu) |
| 24-11 | 4-$CF_3$-1,2,3-thiadiazol-5-yl | $CF_3$ | H | 2-(1,3-$Me_2$-nBu) |
| 24-12 | 4-$CF_3$-1,2,3-thiadiazol-5-yl | $CHF_2$ | H | 2-(1,3-$Me_2$-nBu) |
| 24-13 | 4-$CHF_2$-1,2,3-thiadiazol-5-yl | $CF_3$ | H | 2-(1,3-$Me_2$-nBu) |
| 24-14 | 4-$CHF_2$-1,2,3-thiadiazol-5-yl | $CHF_2$ | H | 2-(1,3-$Me_2$-nBu) |
| 24-15 | 1,2,3-benzothiadiazol-7-yl | $CF_3$ | H | 2-(1,3-$Me_2$-nBu) |
| 24-16 | 1,2,3-benzothiadiazol-7-yl | $CHF_2$ | H | 2-(1,3-$Me_2$-nBu) |
| 24-17 | 4-$CF_3$-2-Me-thiazole-5-yl | $CF_3$ | H | 2-(1,3-$Me_2$-nBu) |
| 24-18 | 4-$CF_3$-2-Me-thiazole-5-yl | $CHF_2$ | H | 2-(1,3-$Me_2$-nBu) |
| 24-19 | 4-$CHF_2$-2-Me-thiazole-5-yl | $CF_3$ | H | 2-(1,3-$Me_2$-nBu) |
| 24-20 | 4-$CHF_2$-2-Me-thiazole-5-yl | $CHF_2$ | H | 2-(1,3-$Me_2$-nBu) |
| 24-21 | 4-$CF_3$-thiazole-5-yl | $CF_3$ | H | 2-(1,3-$Me_2$-nBu) |
| 24-22 | 4-$CF_3$-thiazole-5-yl | $CHF_2$ | H | 2-(1,3-$Me_2$-nBu) |
| 24-23 | 4-$CHF_2$-thiazole-5-yl | $CF_3$ | H | 2-(1,3-$Me_2$-nBu) |
| 24-24 | 4-$CHF_2$-thiazole-5-yl | $CHF_2$ | H | 2-(1,3-$Me_2$-nBu) |

[Table 25-1]

Table 25

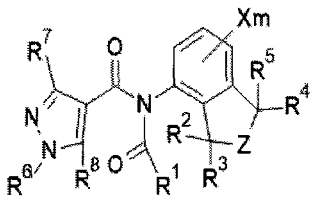

| No. | $R^1$ | $R^2$ | $R^3$ | $R^4$ | $R^5$ | $R^6$ | $R^7$ | $R^8$ | Z | Xm |
|---|---|---|---|---|---|---|---|---|---|---|
| 25-1 | 3,4-$Cl_2$-isothiazol-5-yl | Me | H | Me | Me | Me | $CF_3$ | H | $CH_2$ | H |
| 25-2 | 3,4-$Cl_2$-isothiazol-5-yl | Me | H | Me | Me | Me | $CF_3$ | H | O | H |
| 25-3 | 3,4-$Cl_2$-isothiazol-5-yl | Me | Me | Me | H | Me | $CHF_2$ | H | $CH_2$ | H |
| 25-4 | 3,4-$Cl_2$-isothiazol-5-yl | Me | H | Me | Me | Me | $CHF_2$ | H | $CH_2$ | H |
| 25-5 | 3,4-$Cl_2$-isothiazol-5-yl | Me | Me | Me | Me | Me | $CHF_2$ | H | $CH_2$ | H |
| 25-6 | 3,4-$Cl_2$-isothiazol-5-yl | Me | Et | Me | H | Me | $CHF_2$ | H | $CH_2$ | H |
| 25-7 | 3,4-$Cl_2$-isothiazol-5-yl | Me | H | Me | Et | Me | $CHF_2$ | H | $CH_2$ | H |
| 25-8 | 3,4-$Cl_2$-isothiazol-5-yl | H | H | Et | H | Me | $CHF_2$ | H | $CH_2$ | H |
| 25-9 | 3,4-$Cl_2$-isothiazol-5-yl | Me | Me | Me | H | Me | $CHF_2$ | H | O | H |
| 25-10 | 3,4-$Cl_2$-isothiazol-5-yl | Me | H | Me | Me | Me | $CHF_2$ | H | O | H |
| 25-11 | 3,4-$Cl_2$-isothiazol-5-yl | Me | Me | Me | Me | Me | $CHF_2$ | H | O | H |
| 25-12 | 3,4-$Cl_2$-isothiazol-5-yl | Me | Et | Me | H | Me | $CHF_2$ | H | O | H |
| 25-13 | 3,4-$Cl_2$-isothiazol-5-yl | Me | H | Me | Et | Me | $CHF_2$ | H | O | H |
| 25-14 | 3,4-$Cl_2$-isothiazol-5-yl | H | H | Et | H | Me | $CHF_2$ | H | O | H |

(continued)

| No. | $R^1$ | $R^2$ | $R^3$ | $R^4$ | $R^5$ | $R^6$ | $R^7$ | $R^8$ | Z | Xm |
|---|---|---|---|---|---|---|---|---|---|---|
| 25-15 | 3,4-$Cl_2$-isothiazol-5-yl | Me | Me | Me | H | Me | $CHF_2$ | H | $CH_2$ | 4-F |
| 25-16 | 3,4-$Cl_2$-isothiazol-5-yl | Me | H | Me | Me | Me | $CHF_2$ | H | $CH_2$ | 4-F |
| 25-17 | 3,4-$Cl_2$-isothiazol-5-yl | Me | Me | Me | Me | Me | $CHF_2$ | H | $CH_2$ | 4-F |
| 25-18 | 3,4-$Cl_2$-isothiazol-5-yl | Me | Et | Me | H | Me | $CHF_2$ | H | $CH_2$ | 4-F |
| 25-19 | 3,4-$Cl_2$-isothiazol-5-yl | Me | H | Me | Et | Me | $CHF_2$ | H | $CH_2$ | 4-F |
| 25-20 | 3,4-$Cl_2$-isothiazol-5-yl | H | H | Et | H | Me | $CHF_2$ | H | $CH_2$ | 4-F |
| 25-21 | 3,4-$Cl_2$-isothiazol-5-yl | Me | Me | Me | H | Me | $CHF_2$ | H | O | 4-F |
| 25-22 | 3,4-$Cl_2$-isothiazol-5-yl | Me | H | Me | Me | Me | $CHF_2$ | H | O | 4-F |
| 25-23 | 3,4-$Cl_2$-isothiazol-5-yl | Me | Me | Me | Me | Me | $CHF_2$ | H | O | 4-F |

[Table 25-2]

| No. | $R^1$ | $R^2$ | $R^3$ | $R^4$ | $R^5$ | $R^6$ | $R^7$ | $R^8$ | Z | Xm |
|---|---|---|---|---|---|---|---|---|---|---|
| 25-24 | 3,4-$Cl_2$-isothiazol-5-yl | Me | Et | Me | H | Me | $CHF_2$ | H | O | 4-F |
| 25-25 | 3,4-$Cl_2$-isothiazol-5-yl | Me | H | Me | Et | Me | $CHF_2$ | H | O | 4-F |
| 25-26 | 3,4-$Cl_2$-isothiazol-5-yl | H | H | Et | H | Me | $CHF_2$ | H | O | 4-F |
| 25-27 | 3,4-$Cl_2$-isothiazol-5-yl | Me | H | Me | Me | Me | $CF_3$ | F | $CH_2$ | H |
| 25-28 | 3,4-$Cl_2$-isothiazol-5-yl | Me | H | Me | Me | Me | $CF_3$ | F | O | H |
| 25-29 | 3,4-$Cl_2$-isothiazol-5-yl | Me | H | Me | Me | Me | $CHF_2$ | F | $CH_2$ | H |
| 25-30 | 3,4-$Cl_2$-isothiazol-5-yl | Me | H | Me | Me | Me | $CHF_2$ | F | O | H |
| 25-31 | 3,4-$Cl_2$-isothiazol-5-yl | Me | Me | Me | H | Me | Me | F | $CH_2$ | H |
| 25-32 | 3,4-$Cl_2$-isothiazol-5-yl | Me | H | Me | Me | Me | Me | F | $CH_2$ | H |
| 25-33 | 3,4-$Cl_2$-isothiazol-5-yl | Me | Me | Me | Me | Me | Me | F | $CH_2$ | H |
| 25-34 | 3,4-$Cl_2$-isothiazol-5-yl | Me | Et | Me | H | Me | Me | F | $CH_2$ | H |
| 25-35 | 3,4-$Cl_2$-isothiazol-5-yl | Me | H | Me | Et | Me | Me | F | $CH_2$ | H |
| 25-36 | 3,4-$Cl_2$-isothiazol-5-yl | H | H | Et | H | Me | Me | F | $CH_2$ | H |
| 25-37 | 3,4-$Cl_2$-isothiazol-5-yl | Me | Me | Me | H | Me | Me | F | O | H |
| 25-38 | 3,4-$Cl_2$-isothiazol-5-yl | Me | H | Me | Me | Me | Me | F | O | H |
| 25-39 | 3,4-$Cl_2$-isothiazol-5-yl | Me | Me | Me | Me | Me | Me | F | O | H |
| 25-40 | 3,4-$Cl_2$-isothiazol-5-yl | Me | Et | Me | H | Me | Me | F | O | H |
| 25-41 | 3,4-$Cl_2$-isothiazol-5-yl | Me | H | Me | Et | Me | Me | F | O | H |
| 25-42 | 34-$Cl_2$-isothiazol-5-yl | H | H | Et | H | Me | Me | F | O | H |
| 25-43 | 3,4-$Cl_2$-isothiazol-5-yl | Me | H | Me | Me | $-CH_2CF_3$ | Me | H | $CH_2$ | H |
| 25-44 | 3,4-$Cl_2$-isothiazol-5-yl | Me | H | Me | Me | $-CH_2CF_3$ | Me | H | O | H |
| 25-45 | 3-Me-isothiazol-5-yl | Me | H | Me | Me | Me | $CF_3$ | H | $CH_2$ | H |
| 25-46 | 3-Me-isothiazol-5-yl | Me | H | Me | Me | Me | $CF_3$ | H | O | H |
| 25-47 | 4-Me-1,2,3-thiadiazol-5-yl | Me | H | Me | Me | Me | $CHF_2$ | H | $CH_2$ | H |
| 25-48 | 3-Me-isothiazol-5-yl | Me | H | Me | Me | Me | $CHF_2$ | H | O | H |

(continued)

| No. | $R^1$ | $R^2$ | $R^3$ | $R^4$ | $R^5$ | $R^6$ | $R^7$ | $R^8$ | Z | Xm |
|---|---|---|---|---|---|---|---|---|---|---|
| 25-49 | 3-Me-4-Cl-isothiazol-5-yl | Me | H | Me | Me | Me | $CF_3$ | H | $CH_2$ | H |
| 25-50 | 3-Me-4-Cl-isothiazol-5-yl | Me | H | Me | Me | Me | $CF_3$ | H | O | H |
| 25-51 | 3-Me-4-Cl-isothiazol-5-yl | Me | H | Me | Me | Me | $CHF_2$ | H | $CH_2$ | H |

[Table 25-3]

| No. | $R^1$ | $R^2$ | $R^3$ | $R^4$ | $R^5$ | $R^6$ | $R^7$ | $R^8$ | Z | Xm |
|---|---|---|---|---|---|---|---|---|---|---|
| 25-52 | 3-Me-4-Cl-isothiazol-5-yl | Me | H | Me | Me | Me | $CHF_2$ | H | O | H |
| 25-53 | 4-Me-1,2,3-thiadiazol-5-yl | Me | H | Me | Me | Me | $CF_3$ | H | $CH_2$ | H |
| 25-54 | 4-Me-1,2,3-thiadiazol-5-yl | Me | H | Me | Me | Me | $CF_3$ | H | O | H |
| 25-55 | 3-Me-isothiazol-5-yl | Me | H | Me | Me | Me | $CHF_2$ | H | $CH_2$ | H |
| 25-56 | 4-Me-1,2,3-thiadiazol-5-l | Me | H | Me | Me | Me | $CHF_2$ | H | O | H |
| 25-57 | 4-Me-1,2,3-thiadiazol-5-yl | Me | H | Me | Me | Me | Me | F | $CH_2$ | H |
| 25-58 | 4-Me-1,2,3-thiadiazol-5-yl | Me | H | Me | Me | Me | Me | F | O | H |
| 25-59 | 4-$CF_3$-1,2,3-thiadiazol-5-yl | Me | H | Me | Me | Me | $CF_3$ | H | $CH_2$ | H |
| 25-60 | 4-$CF_3$-1,2,3-thiadiazol-5-yl | Me | H | Me | Me | Me | $CF_3$ | H | O | H |
| 25-61 | 4-$CF_3$-1,2,3-thiadiazol-5-l | Me | H | Me | Me | Me | $CHF_2$ | H | $CH_2$ | H |
| 25-62 | 4-$CF_3$-1,2,3-thiadiazol-5-yl | Me | H | Me | Me | Me | $CHF_2$ | H | O | H |
| 25-63 | 4-$CF_3$-1,2,3-thiadiazol-5-yl | Me | H | Me | Me | Me | Me | F | $CH_2$ | H |
| 25-64 | 4-$CF_3$-1,2,3-thiadiazol-5-yl | Me | H | Me | Me | Me | Me | F | O | H |
| 25-65 | 4-$CHF_2$-1,2,3-thiadiazol-5-yl | Me | H | Me | Me | Me | $CF_3$ | H | $CH_2$ | H |
| 25-66 | 4-$CHF_2$-1,2,3-thiadiazol-5-yl | Me | H | Me | Me | Me | $CF_3$ | H | O | H |
| 25-67 | 4-$CHF_2$-1,2,3-thiadiazol-5-yl | Me | H | Me | Me | Me | $CHF_2$ | H | $CH_2$ | H |
| 25-68 | 4-$CHF_2$-1,2,3-thiadiazol-5-yl | Me | H | Me | Me | Me | $CHF_2$ | H | O | H |
| 25-69 | 4-$CHF_2$-1,2,3-thiadiazol-5-yl | Me | H | Me | Me | Me | Me | F | $CH_2$ | H |
| 25-70 | 4-$CHF_2$-1,2,3-thiadiazol-5-yl | Me | H | Me | Me | Me | Me | F | O | H |
| 25-71 | 1,2,3-benzothiadiazol-7-yl | Me | H | Me | Me | Me | $CF_3$ | H | $CH_2$ | H |
| 25-72 | 1,2,3-benzothiadiazol-7-yl | Me | H | Me | Me | Me | $CF_3$ | H | O | H |
| 25-73 | 1,2,3-benzothiadiazol-7-yl | Me | H | Me | Me | Me | $CHF_2$ | H | $CH_2$ | H |
| 25-74 | 1,2,3-benzothiadiazol-7-yl | Me | H | Me | Me | Me | $CHF_2$ | H | O | H |
| 25-75 | 1,2,3-benzothiadiazol-7-yl | Me | H | Me | Me | Me | Me | F | $CH_2$ | H |
| 25-76 | 1,2,3-benzothiadiazol-7-yl | Me | H | Me | Me | Me | Me | F | O | H |
| 25-77 | 4-$CF_3$-2-Et-thiazole-5-yl | Me | H | Me | Me | Me | $CF_3$ | H | $CH_2$ | H |
| 25-78 | 4-$CF_3$-2-Et-thiazole-5-yl | Me | H | Me | Me | Me | $CF_3$ | H | O | H |
| 25-79 | 4-$CF_3$-2-Et-thiazole-5-yl | Me | H | Me | Me | Me | $CHF_2$ | H | $CH_2$ | H |

[Table 25-4]

| No. | $R^1$ | $R^2$ | $R^3$ | $R^4$ | $R^5$ | $R^6$ | $R^7$ | $R^8$ | Z | Xm |
|---|---|---|---|---|---|---|---|---|---|---|
| 25-80 | 4-$CF_3$-2-Et-thiazole-5-yl | Me | H | Me | Me | Me | $CHF_2$ | H | O | H |
| 25-81 | 4-$CF_3$-2-Et-thiazole-5-yl | Me | H | Me | Me | Me | Me | F | $CH_2$ | H |
| 25-82 | 4-$CF_3$-2-Et-thiazole-5-yl | Me | H | Me | Me | Me | Me | F | O | H |
| 25-83 | 4-$CHF_2$-2-Et-thiazole-5-yl | Me | H | Me | Me | Me | $CF_3$ | H | $CH_2$ | H |
| 25-84 | 4-$CHF_2$-2-Et-thiazole-5-yl | Me | H | Me | Me | Me | $CF_3$ | H | O | H |
| 25-85 | 4-$CHF_2$-2-Et-thiazole-5-yl | Me | H | Me | Me | Me | $CHF_2$ | H | $CH_2$ | H |
| 25-86 | 4-$CHF_2$-2-Et-thiazole-5-yl | Me | H | Me | Me | Me | $CHF_2$ | H | O | H |
| 25-87 | 4-$CHF_2$-2-Et-thiazole-5-yl | Me | H | Me | Me | Me | Me | F | $CH_2$ | H |
| 25-88 | 4-$CHF_2$-2-Et-thiazole-5-yl | Me | H | Me | Me | Me | Me | F | O | H |
| 25-89 | 4-$CF_3$-2-Me-thiazole-5-yl | Me | H | Me | Me | Me | $CF_3$ | H | $CH_2$ | H |
| 25-90 | 4-$CF_3$-2-Me-thiazole-5-yl | Me | H | Me | Me | Me | $CF_3$ | H | O | H |
| 25-91 | 4-$CF_3$-2-Me-thiazole-5-yl | Me | H | Me | Me | Me | $CHF_2$ | H | $CH_2$ | H |
| 25-92 | 4-$CF_3$-2-Me-thiazole-5-yl | Me | H | Me | Me | Me | $CHF_2$ | H | O | H |
| 25-93 | 4-$CF_3$-2-Me-thiazole-5-yl | Me | H | Me | Me | Me | Me | F | $CH_2$ | H |
| 25-94 | 4-$CF_3$-2-Me-thiazole-5-yl | Me | H | Me | Me | Me | Me | F | O | H |
| 25-95 | 4-$CHF_2$-2-Me-thiazole-5-yl | Me | H | Me | Me | Me | $CF_3$ | H | $CH_2$ | H |
| 25-96 | 4-$CHF_2$-2-Me-thiazole-5-yl | Me | H | Me | Me | Me | $CF_3$ | H | O | H |
| 25-97 | 4-$CHF_2$-2-Me-thiazole-5-yl | Me | H | Me | Me | Me | $CHF_2$ | H | $CH_2$ | H |
| 25-98 | 4-$CHF_2$-2-Me-thiazole-5-yl | Me | H | Me | Me | Me | $CHF_2$ | H | O | H |
| 25-99 | 4-$CHF_2$-2-Me-thiazole-5-yl | Me | H | Me | Me | Me | Me | F | $CH_2$ | H |
| 25-100 | 4-$CHF_2$-2-Me-thiazole-5-yl | Me | H | Me | Me | Me | Me | F | O | H |
| 25-101 | 4-$CF_3$-thiazole-5-yl | Me | H | Me | Me | Me | $CF_3$ | H | $CH_2$ | H |
| 25-102 | 4-$CF_3$-thiazole-5-yl | Me | H | Me | Me | Me | $CF_3$ | H | O | H |
| 25-103 | 4-$CF_3$-thiazole-5-yl | Me | H | Me | Me | Me | $CHF_2$ | H | $CH_2$ | H |
| 25-104 | 4-$CF_3$-thiazole-5-yl | Me | H | Me | Me | Me | $CHF_2$ | H | O | H |
| 25-105 | 4-$CF_3$-thiazole-5-yl | Me | H | Me | Me | Me | Me | F | $CH_2$ | H |
| 25-106 | 4-$CF_3$-thiazole-5-yl | Me | H | Me | Me | Me | Me | F | O | H |
| 25-107 | 4-$CHF_2$-thiazole-5-yl | Me | H | Me | Me | Me | $CF_3$ | H | $CH_2$ | H |

[Table 25-5]

| No. | $R^1$ | $R^2$ | $R^3$ | $R^4$ | $R^5$ | $R^6$ | $R^7$ | $R^8$ | Z | Xm |
|---|---|---|---|---|---|---|---|---|---|---|
| 25-108 | 4-$CHF_2$-thiazole-5-yl | Me | H | Me | Me | Me | $CF_3$ | H | O | H |
| 25-109 | 4-$CHF_2$-thiazole-5-yl | Me | H | Me | Me | Me | $CHF_2$ | H | $CH_2$ | H |
| 25-110 | 4-$CHF_2$-thiazole-5-yl | Me | H | Me | Me | Me | $CHF_2$ | H | O | H |
| 25-111 | 4-$CHF_2$-thiazole-5-yl | Me | H | Me | Me | Me | Me | F | $CH_2$ | H |
| 25-112 | 4-$CHF_2$-thiazole-5-yl | Me | H | Me | Me | Me | Me | F | O | H |
| 25-113 | 3,4-$Cl_2$-isoxazole-5-yl | Me | H | Me | Me | Me | $CHF_2$ | H | $CH_2$ | H |
| 25-114 | 3,4-$Cl_2$-isoxazole-5-yl | Me | H | Me | Me | Me | $CHF_2$ | H | O | H |

(continued)

| No. | $R^1$ | $R^2$ | $R^3$ | $R^4$ | $R^5$ | $R^6$ | $R^7$ | $R^8$ | Z | Xm |
|---|---|---|---|---|---|---|---|---|---|---|
| 25-115 | 3,4-$Cl_2$-isothiazol-5-yl | Me | H | Me | Me | Me | Me | Cl | O | H |
| 25-116 | 4-Me-1,2,3-thiadiazol-5-yl | Me | H | Me | Me | Me | Me | Cl | O | H |
| 25-117 | 4-$CHF_2$-1,2,3-thiadiazol-5-yl | Me | H | Me | Me | Me | Me | Cl | O | H |
| 25-118 | 1,2,3-benzothiadiazol-7-yl | Me | H | Me | Me | Me | Me | Cl | O | H |
| 25-119 | 4-$CHF_2$-thiazole-5-yl | Me | H | Me | Me | Me | Me | Cl | O | H |
| 25-120 | 4-$CF_3$-thiazole-5-yl | Me | H | Me | Me | Me | Me | Cl | O | H |
| 25-121 | 4-$CHF_2$-2-Me-thiazole-5-yl | Me | H | Me | Me | Me | Me | Cl | O | H |
| 25-122 | 4-$CF_3$-2-Me-thiazole-5-yl | Me | H | Me | Me | Me | Me | Cl | O | H |
| 25-123 | 2,3-$Cl_2$-thiophen-5-yl | Me | H | Me | Me | Me | Me | Cl | O | H |
| 25-124 | 3,4-$Cl_2$-isoxazole-5-yl | Me | H | Me | Me | Me | Me | Cl | O | H |
| 25-125 | 2,6-$Cl_2$-pyridin-4-yl | Me | H | Me | Me | Me | Me | Cl | O | H |

[Table 26-1]

Table 26

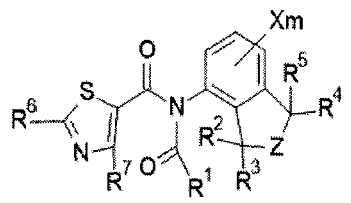

| No. | $R^1$ | $R^2$ | $R^3$ | $R^4$ | $R^5$ | $R^6$ | $R^7$ | Z | Xm |
|---|---|---|---|---|---|---|---|---|---|
| 26-1 | 3,4-$Cl_2$-isothiazol-5-yl | Me | H | Me | Me | H | $CF_3$ | $CH_2$ | H |
| 26-2 | 3,4-$Cl_2$-isothiazol-5-yl | Me | H | Me | Me | H | $CF_3$ | O | H |
| 26-3 | 3,4-$Cl_2$-isothiazol-5-yl | Me | H | Me | Me | H | $CHF_2$ | $CH_2$ | H |
| 26-4 | 3,4-$Cl_2$-isothiazol-5-yl | Me | H | Me | Me | H | $CHF_2$ | O | H |
| 26-5 | 3,4-$Cl_2$-isothiazol-5-yl | Me | H | Me | Me | Me | $CF_3$ | $CH_2$ | H |
| 26-6 | 3,4-$Cl_2$-isothiazol-5-yl | Me | H | Me | Me | Me | $CF_3$ | O | H |
| 26-7 | 3,4-$Cl_2$-isothiazol-5-yl | Me | Me | Me | H | Me | $CHF_2$ | $CH_2$ | H |
| 26-8 | 3,4-$Cl_2$-isothiazol-5-yl | Me | H | Me | Me | Me | $CHF_2$ | $CH_2$ | H |
| 26-9 | 3,4-$Cl_2$-isothiazol-5-yl | Me | Me | Me | Me | Me | $CHF_2$ | $CH_2$ | H |
| 26-10 | 3,4-$Cl_2$-isothiazol-5-yl | Me | Et | Me | H | Me | $CHF_2$ | $CH_2$ | H |
| 26-11 | 3,4-$Cl_2$-isothiazol-5-yl | Me | H | Me | Et | Me | $CHF_2$ | $CH_2$ | H |
| 26-12 | 3,4-$Cl_2$-isothiazol-5-yl | H | H | Et | H | Me | $CHF_2$ | $CH_2$ | H |
| 26-13 | 3,4-$Cl_2$-isothiazol-5-yl | Me | Me | Me | H | Me | $CHF_2$ | O | H |
| 26-14 | 3,4-$Cl_2$-isothiazol-5-yl | Me | H | Me | Me | Me | $CHF_2$ | O | H |
| 26-15 | 3,4-$Cl_2$-isothiazol-5-yl | Me | Me | Me | Me | Me | $CHF_2$ | O | H |
| 26-16 | 3,4-$Cl_2$-isothiazol-5-yl | Me | Et | Me | H | Me | $CHF_2$ | O | H |
| 26-17 | 3,4-$Cl_2$-isothiazol-5-yl | Me | H | Me | Et | Me | $CHF_2$ | O | H |
| 26-18 | 3,4-$Cl_2$-isothiazol-5-yl | H | H | Et | H | Me | $CHF_2$ | O | H |
| 26-19 | 3,4-$Cl_2$-isothiazol-5-yl | Me | Me | Me | H | Me | $CHF_2$ | $CH_2$ | 4-F |

(continued)

Table 26

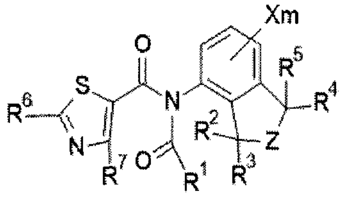

| No. | $R^1$ | $R^2$ | $R^3$ | $R^4$ | $R^5$ | $R^6$ | $R^7$ | Z | Xm |
|---|---|---|---|---|---|---|---|---|---|
| 26-20 | 3,4-$Cl_2$-isothiazol-5-yl | Me | H | Me | Me | Me | $CHF_2$ | $CH_2$ | 4-F |
| 26-21 | 3,4-$Cl_2$-isothiazol-5-yl | Me | Me | Me | Me | Me | $CHF_2$ | $CH_2$ | 4-F |
| 26-22 | 3,4-$Cl_2$-isothiazol-5-yl | Me | Et | Me | H | Me | $CHF_2$ | $CH_2$ | 4-F |
| 26-23 | 3,4-$Cl_2$-isothiazol-5-yl | Me | H | Me | Et | Me | $CHF_2$ | $CH_2$ | 4-F |

[Table 26-2]

| No. | $R^1$ | $R^2$ | $R^3$ | $R^4$ | $R^5$ | $R^6$ | $R^7$ | Z | Xm |
|---|---|---|---|---|---|---|---|---|---|
| 26-24 | 3,4-$Cl_2$-isothiazol-5-yl | H | H | Et | H | Me | $CHF_2$ | $CH_2$ | 4-F |
| 26-25 | 3,4-$Cl_2$-isothiazol-5-yl | Me | Me | Me | H | Me | $CHF_2$ | O | 4-F |
| 26-26 | 3,4-$Cl_2$-isothiazol-5-yl | Me | H | Me | Me | Me | $CHF_2$ | O | 4-F |
| 26-27 | 3,4-$Cl_2$-isothiazol-5-yl | Me | Me | Me | Me | Me | $CHF_2$ | O | 4-F |
| 26-28 | 3,4-$Cl_2$-isothiazol-5-yl | Me | Et | Me | H | Me | $CHF_2$ | O | 4-F |
| 26-29 | 3,4-$Cl_2$-isothiazol-5-yl | Me | H | Me | Et | Me | $CHF_2$ | O | 4-F |
| 26-30 | 3,4-$Cl_2$-isothiazol-5-yl | H | H | Et | H | Me | $CHF_2$ | O | 4-F |
| 26-31 | 3,4-$Cl_2$-isothiazol-5-yl | Me | H | Me | Me | Et | $CF_3$ | $CH_2$ | H |
| 26-32 | 3,4-$Cl_2$-isothiazol-5-yl | Me | H | Me | Me | Et | $CF_3$ | O | H |
| 26-33 | 3,4-$Cl_2$-isothiazol-5-yl | Me | H | Me | Me | Et | $CHF_2$ | $CH_2$ | H |
| 26-34 | 3,4-$Cl_2$-isothiazol-5-yl | Me | H | Me | Me | Et | $CHF_2$ | O | H |
| 26-35 | 3,4-$Cl_2$-isothiazol-5-yl | Me | H | Me | Me | Me | Me | $CH_2$ | H |
| 26-36 | 3,4-$Cl_2$-isothiazol-5-yl | Me | H | Me | Me | Me | Me | O | H |
| 26-37 | 3,4-$Cl_2$-isothiazol-5-yl | Me | H | Me | Me | $CF_3$ | Me | $CH_2$ | H |
| 26-38 | 3,4-$Cl_2$-isothiazol-5-yl | Me | H | Me | Me | $CF_3$ | Me | O | H |
| 26-39 | 3-Me-isothiazol-5-yl | Me | H | Me | Me | Me | $CF_3$ | $CH_2$ | H |
| 26-40 | 3-Me-isothiazol-5-yl | Me | H | Me | Me | Me | $CF_3$ | O | H |
| 26-41 | 3-Me-isothiazol-5-yl | Me | H | Me | Me | Me | $CHF_2$ | $CH_2$ | H |
| 26-42 | 3-Me-isothiazol-5-yl | Me | H | Me | Me | Me | $CHF_2$ | O | H |
| 26-43 | 3-Me-4-Cl-isothiazol-5-yl | Me | H | Me | Me | Me | $CF_3$ | $CH_2$ | H |
| 26-44 | 3-Me-4-Cl-isothiazol-5-yl | Me | H | Me | Me | Me | $CF_3$ | O | H |
| 26-45 | 3-Me-4-Cl-isothiazol-5-yl | Me | H | Me | Me | Me | $CHF_2$ | $CH_2$ | H |
| 26-46 | 3-Me-4-Cl-isothiazol-5-yl | Me | H | Me | Me | Me | $CHF_2$ | O | H |
| 26-47 | 4-Me-1,2,3-thiadiazol-5-l | Me | H | Me | Me | Me | $CF_3$ | $CH_2$ | H |
| 26-48 | 4-Me-1,2,3-thiadiazol-5-yl | Me | H | Me | Me | Me | $CF_3$ | O | H |
| 26-49 | 4-Me-1,2,3-thiadiazol-5-yl | Me | H | Me | Me | Me | $CHF_2$ | $CH_2$ | H |

(continued)

| No. | $R^1$ | $R^2$ | $R^3$ | $R^4$ | $R^5$ | $R^6$ | $R^7$ | Z | Xm |
|---|---|---|---|---|---|---|---|---|---|
| 26-50 | 4-Me-1,2,3-thiadiazol-5-yl | Me | H | Me | Me | Me | $CHF_2$ | O | H |
| 26-51 | 4-$CF_3$-1,2,3-thiadiazol-5-yl | Me | H | Me | Me | Me | $CF_3$ | $CH_2$ | H |

[Table 26-3]

| No. | $R^1$ | $R^2$ | $R^3$ | $R^4$ | $R^5$ | $R^6$ | $R^7$ | Z | Xm |
|---|---|---|---|---|---|---|---|---|---|
| 26-52 | 4-$CF_3$-1,2,3-thiadiazol-5-yl | Me | H | Me | Me | Me | $CF_3$ | O | H |
| 26-53 | 4-$CF_3$-1,2,3-thiadiazol-5-yl | Me | H | Me | Me | Me | $CHF_2$ | $CH_2$ | H |
| 26-54 | 4-$CF_3$-1,2,3-thiadiazol-5-yl | Me | H | Me | Me | Me | $CHF_2$ | O | H |
| 26-55 | 4-$CHF_2$-1,2,3-thiadiazol-5-yl | Me | H | Me | Me | Me | $CF_3$ | $CH_2$ | H |
| 26-56 | 4-$CHF_2$-1,2,3-thiadiazol-5-yl | Me | H | Me | Me | Me | $CF_3$ | O | H |
| 26-57 | 4-$CHF_2$-1,2,3-thiadiazol-5-yl | Me | H | Me | Me | Me | $CHF_2$ | $CH_2$ | H |
| 26-58 | 4-$CHF_2$-1,2,3-thiadiazol-5-yl | Me | H | Me | Me | Me | $CHF_2$ | O | H |
| 26-59 | 1,2,3-benzothiadiazol-7-yl | Me | H | Me | Me | Me | $CF_3$ | $CH_2$ | H |
| 26-60 | 1,2,3-benzothiadiazol-7-yl | Me | H | Me | Me | Me | $CF_3$ | O | H |
| 26-61 | 1,2,3-benzothiadiazol-7-yl | Me | H | Me | Me | Me | $CHF_2$ | $CH_2$ | H |
| 26-62 | 1,2,3-benzothiadiazol-7-yl | Me | H | Me | Me | Me | $CHF_2$ | O | H |
| 26-63 | 4-$CF_3$-2-Et-thiazole-5-yl | Me | H | Me | Me | Me | $CF_3$ | $CH_2$ | H |
| 26-64 | 4-$CF_3$-2-Et-thiazole-5-yl | Me | H | Me | Me | Me | $CF_3$ | O | H |
| 26-65 | 4-$CF_3$-2-Et-thiazole-5-yl | Me | H | Me | Me | Me | $CHF_2$ | $CH_2$ | H |
| 26-66 | 4-$CF_3$-2-Et-thiazole-5-yl | Me | H | Me | Me | Me | $CHF_2$ | O | H |
| 26-67 | 4-$CHF_2$-2-Et-thiazole-5-yl | Me | H | Me | Me | Me | $CF_3$ | $CH_2$ | H |
| 26-68 | 4-$CHF_2$-2-Et-thiazole-5-yl | Me | H | Me | Me | Me | $CF_3$ | O | H |
| 26-69 | 4-$CHF_2$-2-Et-thiazole-5-yl | Me | H | Me | Me | Me | $CHF_2$ | $CH_2$ | H |
| 26-70 | 4-$CHF_2$-2-Et-thiazole-5-vl | Me | H | Me | Me | Me | $CHF_2$ | O | H |
| 26-71 | 4-$CF_3$-2-Me-thiazole-5-yl | Me | H | Me | Me | Me | $CF_3$ | $CH_2$ | H |
| 26-72 | 4-$CF_3$-2-Me-thiazole-5-yl | Me | H | Me | Me | Me | $CF_3$ | O | H |
| 26-73 | 4-$CF_3$-2-Me-thiazole-5-yl | Me | H | Me | Me | Me | $CHF_2$ | $CH_2$ | H |
| 26-74 | 4-$CF_3$-2-Me-thiazole-5-yl | Me | H | Me | Me | Me | $CHF_2$ | O | H |
| 26-75 | 4-$CHF_2$-2-Me-thiazole-5-yl | Me | H | Me | Me | Me | $CF_3$ | $CH_2$ | H |
| 26-76 | 4-$CHF_2$-2-Me-thiazole-5-yl | Me | H | Me | Me | Me | $CF_3$ | O | H |
| 26-77 | 4-$CHF_2$-2-Me-thiazole-5-yl | Me | H | Me | Me | Me | $CHF_2$ | $CH_2$ | H |
| 26-78 | 4-$CHF_2$-2-Me-thiazole-5-yl | Me | H | Me | Me | Me | $CHF_2$ | O | H |
| 26-79 | 4-$CF_3$-thiazole-5-yl | Me | H | Me | Me | Me | $CF_3$ | $CH_2$ | H |

[Table 26-4]

| No. | $R^1$ | $R^2$ | $R^3$ | $R^4$ | $R^5$ | $R^6$ | $R^7$ | Z | Xm |
|---|---|---|---|---|---|---|---|---|---|
| 26-80 | 4-$CF_3$-thiazole-5-yl | Me | H | Me | Me | Me | $CF_3$ | O | H |

(continued)

| No. | $R^1$ | $R^2$ | $R^3$ | $R^4$ | $R^5$ | $R^6$ | $R^7$ | Z | Xm |
|---|---|---|---|---|---|---|---|---|---|
| 26-81 | 4-$CF_3$-thiazole-5-yl | Me | H | Me | Me | Me | $CHF_2$ | $CH_2$ | H |
| 26-82 | 4-$CF_3$-thiazole-5-yl | Me | H | Me | Me | Me | $CHF_2$ | O | H |
| 26-83 | 4-$CHF_2$-thiazole-5-yl | Me | H | Me | Me | Me | $CF_3$ | $CH_2$ | H |
| 26-84 | 4-$CHF_2$-thiazole-5-yl | Me | H | Me | Me | Me | $CF_3$ | O | H |
| 26-85 | 4-$CHF_2$-thiazole-5-yl | Me | H | Me | Me | Me | $CHF_2$ | $CH_2$ | H |
| 26-86 | 4-$CHF_2$-thiazole-5-yl | Me | H | Me | Me | Me | $CHF_2$ | O | H |
| 26-87 | 2,3-$Cl_2$-thiophen-5-yl | Me | H | Me | Me | Me | $CHF_2$ | $CH_2$ | H |
| 26-88 | 3-Cl-isoxazole-5-yl | Me | H | Me | Me | Me | $CHF_2$ | $CH_2$ | H |
| 26-89 | 2,6-$Cl_2$-pyridin-4-yl | Me | H | Me | Me | Me | $CHF_2$ | $CH_2$ | H |
| 26-90 | 2,3-$Cl_2$-thiophen-5-yl | Me | H | Me | Me | Me | $CHF_2$ | O | H |
| 26-91 | 3-Cl-isoxazole-5-yl | Me | H | Me | Me | Me | $CHF_2$ | O | H |
| 26-92 | 3,4-$Cl_2$-isoxazol-5-yl | Me | H | Me | Me | Me | $CHF_2$ | O | H |

[Table 27-1]

Table 27

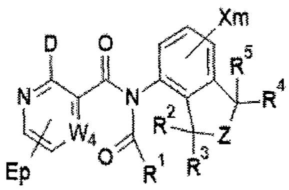

| No. | $R^1$ | $W^4$ | $R^2$ | $R^3$ | $R^4$ | $R^5$ | D | Ep | Z | Xm |
|---|---|---|---|---|---|---|---|---|---|---|
| 27-1 | 3,4-$Cl_2$-isothiazol-5-yl | CH | Me | H | Me | Me | $CF_3$ | H | $CH_2$ | H |
| 27-2 | 3,4-$Cl_2$-isothiazol-5-yl | CH | Me | H | Me | Me | $CF_3$ | H | O | H |
| 27-3 | 3,4-$Cl_2$-isothiazol-5-yl | CH | Me | H | Me | Me | $CHF_2$ | H | $CH_2$ | H |
| 27-4 | 3,4-$Cl_2$-isothiazol-5-yl | CH | Me | H | Me | Me | $CHF_2$ | H | O | H |
| 27-5 | 3-Me-isothiazol-5-yl | CH | Me | H | Me | Me | $CF_3$ | H | $CH_2$ | H |
| 27-6 | 3-Me-isothiazol-5-yl | CH | Me | H | Me | Me | $CF_3$ | H | O | H |
| 27-7 | 3-Me-isothiazol-5-yl | CH | Me | H | Me | Me | $CHF_2$ | H | $CH_2$ | H |
| 27-8 | 3-Me-isothiazol-5-yl | CH | Me | H | Me | Me | $CHF_2$ | H | O | H |
| 27-9 | 3-Me-4-Cl-isothiazol-5-yl | CH | Me | H | Me | Me | $CF_3$ | H | $CH_2$ | H |
| 27-10 | 3-Me-4-Cl-isothiazol-5-yl | CH | Me | H | Me | Me | $CF_3$ | H | O | H |
| 27-11 | 3-Me-4-Cl-isothiazol-5-yl | CH | Me | H | Me | Me | $CHF_2$ | H | $CH_2$ | H |
| 27-12 | 3-Me-4-Cl-isothiazol-5-yl | CH | Me | H | Me | Me | $CHF_2$ | H | O | H |
| 27-13 | 4-Me-1,2,3-thiadiazol-5-yl | CH | Me | H | Me | Me | $CF_3$ | H | $CH_2$ | H |
| 27-14 | 4-Me-1,2,3-thiadiazol-5-yl | CH | Me | H | Me | Me | $CF_3$ | H | O | H |
| 27-15 | 4-Me-1,2,3-thiadiazol-5-yl | CH | Me | H | Me | Me | $CHF_2$ | H | $CH_2$ | H |
| 27-16 | 4-Me-1,2,3-thiadiazol-5-yl | CH | Me | H | Me | Me | $CHF_2$ | H | O | H |
| 27-17 | 4-$CF_3$-1,2,3-thiadiazol-5-l | CH | Me | H | Me | Me | $CF_3$ | H | $CH_2$ | H |
| 27-18 | 4-$CF_3$-1,2,3-thiadiazol-5-yl | CH | Me | H | Me | Me | $CF_3$ | H | O | H |

(continued)

Table 27

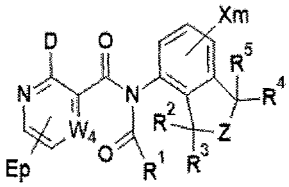

| No. | $R^1$ | $W^4$ | $R^2$ | $R^3$ | $R^4$ | $R^5$ | D | Ep | Z | Xm |
|---|---|---|---|---|---|---|---|---|---|---|
| 27-19 | 4-$CF_3$-1,2,3-thiadiazol-5-yl | CH | Me | H | Me | Me | $CHF_2$ | H | $CH_2$ | H |
| 27-20 | 4-$CF_3$-1,2,3-thiadiazol-5-yl | CH | Me | H | Me | Me | $CHF_2$ | H | O | H |
| 27-21 | 4-$CHF_2$-1,2,3-thiadiazol-5-l | CH | Me | H | Me | Me | $CF_3$ | H | $CH_2$ | H |
| 27-22 | 4-$CHF_2$-1,2,3-thiadiazol-5-yl | CH | Me | H | Me | Me | $CF_3$ | H | O | H |
| 27-23 | 4-$CHF_2$-1,2,3-thiadiazol-5-yl | CH | Me | H | Me | Me | $CHF_2$ | H | $CH_2$ | H |

[Table 27-2]

| No. | $R^1$ | $W^4$ | $R^2$ | $R^3$ | $R^4$ | $R^5$ | D | Ep | Z | Xm |
|---|---|---|---|---|---|---|---|---|---|---|
| 27-24 | 4-$CHF_2$-1,2,3-thiadiazol-5-yl | CH | Me | H | Me | Me | $CHF_2$ | H | O | H |
| 27-25 | 1,2,3-benzothiadiazol-7-yl | CH | Me | H | Me | Me | $CF_3$ | H | $CH_2$ | H |
| 27-26 | 1,2,3-benzothiadiazol-7-yl | CH | Me | H | Me | Me | $CF_3$ | H | O | H |
| 27-27 | 1,2,3-benzothiadiazol-7-yl | CH | Me | H | Me | Me | $CHF_2$ | H | $CH_2$ | H |
| 27-28 | 1,2,3-benzothiadiazol-7-yl | CH | Me | H | Me | Me | $CHF_2$ | H | O | H |
| 27-29 | 4-$CF_3$-2-Me-thiazole-5-yl | CH | Me | H | Me | Me | $CF_3$ | H | $CH_2$ | H |
| 27-30 | 4-$CF_3$-2-Me-thiazole-5-yl | CH | Me | H | Me | Me | $CF_3$ | H | O | H |
| 27-31 | 4-$CF_3$-2-Me-thiazole-5-yl | CH | Me | H | Me | Me | $CHF_2$ | H | $CH_2$ | H |
| 27-32 | 4-$CF_3$-2-Me-thiazole-5-yl | CH | Me | H | Me | Me | $CHF_2$ | H | O | H |
| 27-33 | 4-$CHF_2$-2-Me-thiazole-5-yl | CH | Me | H | Me | Me | $CF_3$ | H | $CH_2$ | H |
| 27-34 | 4-$CHF_2$-2-Me-thiazole-5-yl | CH | Me | H | Me | Me | $CF_3$ | H | O | H |
| 27-35 | 4-$CHF_2$-2-Me-thiazole-5-yl | CH | Me | H | Me | Me | $CHF_2$ | H | $CH_2$ | H |
| 27-36 | 4-$CHF_2$-2-Me-thiazole-5-yl | CH | Me | H | Me | Me | $CHF_2$ | H | O | H |
| 27-37 | 4-$CF_3$-thiazole-5-yl | CH | Me | H | Me | Me | $CF_3$ | H | $CH_2$ | H |
| 27-38 | 4-$CF_3$-thiazole-5-yl | CH | Me | H | Me | Me | $CF_3$ | H | O | H |
| 27-39 | 4-$CF_3$-thiazole-5-yl | CH | Me | H | Me | Me | $CHF_2$ | H | $CH_2$ | H |
| 27-40 | 4-$CF_3$-thiazole-5-yl | CH | Me | H | Me | Me | $CHF_2$ | H | O | H |
| 27-41 | 4-$CHF_2$-thiazole-5-yl | CH | Me | H | Me | Me | $CF_3$ | H | $CH_2$ | H |
| 27-42 | 4-$CHF_2$-thiazole-5-yl | CH | Me | H | Me | Me | $CF_3$ | H | O | H |
| 27-43 | 4-$CHF_2$-thiazole-5-yl | CH | Me | H | Me | Me | $CHF_2$ | H | $CH_2$ | H |
| 27-44 | 4-$CHF_2$-thiazole-5-yl | CH | Me | H | Me | Me | $CHF_2$ | H | O | H |
| 27-45 | 3,4-$Cl_2$-isothiazol-5-yl | N | Me | H | Me | Me | $CF_3$ | H | $CH_2$ | H |
| 27-46 | 3,4-$Cl_2$-isothiazol-5-yl | N | Me | H | Me | Me | $CF_3$ | H | O | H |
| 27-47 | 3,4-$Cl_2$-isothiazol-5-yl | N | Me | H | Me | Me | $CHF_2$ | H | $CH_2$ | H |
| 27-48 | 3,4-$Cl_2$-isothiazol-5-yl | N | Me | H | Me | Me | $CHF_2$ | H | O | H |

(continued)

| No. | $R^1$ | $W^4$ | $R^2$ | $R^3$ | $R^4$ | $R^5$ | D | Ep | Z | Xm |
|---|---|---|---|---|---|---|---|---|---|---|
| 27-49 | 3-Me-isothiazol-5-yl | N | Me | H | Me | Me | $CF_3$ | H | $CH_2$ | H |
| 27-50 | 3-Me-isothiazol-5-yl | N | Me | H | Me | Me | $CF_3$ | H | O | H |
| 27-51 | 3-Me-isothiazol-5-yl | N | Me | H | Me | Me | $CHF_2$ | H | $CH_2$ | H |

[Table 27-3]

| No. | $R^1$ | $W^4$ | $R^2$ | $R^3$ | $R^4$ | $R^5$ | D | Ep | Z | Xm |
|---|---|---|---|---|---|---|---|---|---|---|
| 27-52 | 3-Me-isothiazol-5-vl | N | Me | H | Me | Me | $CHF_2$ | H | O | H |
| 27-53 | 3-Me-4-Cl-isothiazol-5-yl | N | Me | H | Me | Me | $CF_3$ | H | $CH_2$ | H |
| 27-54 | 3-Me-4-Cl-isothiazol-5-yl | N | Me | H | Me | Me | $CF_3$ | H | O | H |
| 27-55 | 3-Me-4-Cl-isothiazol-5-yl | N | Me | H | Me | Me | $CHF_2$ | H | $CH_2$ | H |
| 27-56 | 3-Me-4-Cl-isothiazol-5-yl | N | Me | H | Me | Me | $CHF_2$ | H | O | H |
| 27-57 | 4-Me-1,2,3-thiadiazol-5-yl | N | Me | H | Me | Me | $CF_3$ | H | $CH_2$ | H |
| 27-58 | 4-Me-1,2,3-thiadiazol-5-yl | N | Me | H | Me | Me | $CF_3$ | H | O | H |
| 27-59 | 4-Me-1,2,3-thiadiazol-5-yl | N | Me | H | Me | Me | $CHF_2$ | H | $CH_2$ | H |
| 27-60 | 4-Me-1,2,3-thiadiazol-5-yl | N | Me | H | Me | Me | $CHF_2$ | H | O | H |
| 27-61 | 4-$CF_3$-1,2,3-thiadiazol-5-yl | N | Me | H | Me | Me | $CF_3$ | H | $CH_2$ | H |
| 27-62 | 4-$CF_3$-1,2,3-thiadiazol-5-yl | N | Me | H | Me | Me | $CF_3$ | H | O | H |
| 27-63 | 4-$CF_3$-1,2,3-thiadiazol-5-yl | N | Me | H | Me | Me | $CHF_2$ | H | $CH_2$ | H |
| 27-64 | 4-$CF_3$-1,2,3-thiadiazol-5-yl | N | Me | H | Me | Me | $CHF_2$ | H | O | H |
| 27-65 | 4-$CHF_2$-1,2,3-thiadiazol-5-yl | N | Me | H | Me | Me | $CF_3$ | H | $CH_2$ | H |
| 27-66 | 4-$CHF_2$-1,2,3-thiadiazol-5-yl | N | Me | H | Me | Me | $CF_3$ | H | O | H |
| 27-67 | 4-$CHF_2$-1,2,3-thiadiazol-5-yl | N | Me | H | Me | Me | $CHF_2$ | H | $CH_2$ | H |
| 27-68 | 4-$CHF_2$-1,2,3-thiadiazol-5-yl | N | Me | H | Me | Me | $CHF_2$ | H | O | H |
| 27-69 | 1,2,3-benzothiadiazol-7-yl | N | Me | H | Me | Me | $CF_3$ | H | $CH_2$ | H |
| 27-70 | 1,2,3-benzothiadiazol-7-yl | N | Me | H | Me | Me | $CF_3$ | H | O | H |
| 27-71 | 1,2,3-benzothiadiazol-7-yl | N | Me | H | Me | Me | $CHF_2$ | H | $CH_2$ | H |
| 27-72 | 1,2,3-benzothiadiazol-7-yl | N | Me | H | Me | Me | $CHF_2$ | H | O | H |
| 27-73 | 4-$CF_3$-2-Me-thiazole-5-yl | N | Me | H | Me | Me | $CF_3$ | H | $CH_2$ | H |
| 27-74 | 4-$CF_3$-2-Me-thiazole-5-yl | N | Me | H | Me | Me | $CF_3$ | H | O | H |
| 27-75 | 4-$CF_3$-2-Me-thiazole-5-yl | N | Me | H | Me | Me | $CHF_2$ | H | $CH_2$ | H |
| 27-76 | 4-$CF_3$-2-Me-thiazole-5-yl | N | Me | H | Me | Me | $CHF_2$ | H | O | H |
| 27-77 | 4-$CHF_2$-2-Me-thiazole-5-yl | N | Me | H | Me | Me | $CF_3$ | H | $CH_2$ | H |
| 27-78 | 4-$CHF_2$-2-Me-thiazole-5-yl | N | Me | H | Me | Me | $CF_3$ | H | O | H |
| 27-79 | 4-$CHF_2$-2-Me-thiazole-5-yl | N | Me | H | Me | Me | $CHF_2$ | H | $CH_2$ | H |

[Table 27-4]

| No. | $R^1$ | $W^4$ | $R^2$ | $R^3$ | $R^4$ | $R^5$ | D | Ep | Z | Xm |
|---|---|---|---|---|---|---|---|---|---|---|
| 27-80 | 4-$CHF_2$-2-Me-thiazole-5-yl | N | Me | H | Me | Me | $CHF_2$ | H | O | H |
| 27-81 | 4-$CF_3$-thiazole-5-yl | N | Me | H | Me | Me | $CF_3$ | H | $CH_2$ | H |
| 27-82 | 4-$CF_3$-thiazole-5-yl | N | Me | H | Me | Me | $CF_3$ | H | O | H |
| 27-83 | 4-$CF_3$-thiazole-5-yl | N | Me | H | Me | Me | $CHF_2$ | H | $CH_2$ | H |
| 27-84 | 4-$CF_3$-thiazole-5-yl | N | Me | H | Me | Me | $CHF_2$ | H | O | H |
| 27-85 | 4-$CHF_2$-thiazole-5-yl | N | Me | H | Me | Me | $CF_3$ | H | $CH_2$ | H |
| 27-86 | 4-$CHF_2$-thiazole-5-yl | N | Me | H | Me | Me | $CF_3$ | H | O | H |
| 27-87 | 4-$CHF_2$-thiazole-5-yl | N | Me | H | Me | Me | $CHF_2$ | H | $CH_2$ | H |
| 27-88 | 4-$CHF_2$-thiazole-5-yl | N | Me | H | Me | Me | $CHF_2$ | H | O | H |

[Table 28-1]

Table 28

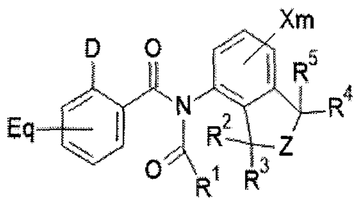

| No. | $R^1$ | $R^2$ | $R^3$ | $R^4$ | $R^5$ | D | Eq | Z | Xm |
|---|---|---|---|---|---|---|---|---|---|
| 28-1 | 3,4-$Cl_2$-isothiazol-5-yl | Me | H | Me | Me | $CF_3$ | H | $CH_2$ | H |
| 28-2 | 3,4-$Cl_2$-isothiazol-5-yl | Me | H | Me | Me | $CF_3$ | H | O | H |
| 28-3 | 3,4-$Cl_2$-isothiazol-5-yl | Me | H | Me | Me | $CHF_2$ | H | $CH_2$ | H |
| 28-4 | 3,4-$Cl_2$-isothiazol-5-yl | Me | H | Me | Me | $CHF_2$ | H | O | H |
| 28-5 | 3-Me-isothiazol-5-yl | Me | H | Me | Me | $CF_3$ | H | $CH_2$ | H |
| 28-6 | 3-Me-isothiazol-5-yl | Me | H | Me | Me | $CF_3$ | H | O | H |
| 28-7 | 3-Me-isothiazol-5-yl | Me | H | Me | Me | $CHF_2$ | H | $CH_2$ | H |
| 28-8 | 3-Me-isothiazol-5-yl | Me | H | Me | Me | $CHF_2$ | H | O | H |
| 28-9 | 3-Me-4-Cl-isothiazol-5-yl | Me | H | Me | Me | $CF_3$ | H | $CH_2$ | H |
| 28-10 | 3-Me-4-Cl-isothiazo1-5-yl | Me | H | Me | Me | $CF_3$ | H | O | H |
| 28-11 | 3-Me-4-Cl-isothiazol-5-yl | Me | H | Me | Me | $CHF_2$ | H | $CH_2$ | H |
| 28-12 | 3-Me-4-Cl-isothiazol-5-yl | Me | H | Me | Me | $CHF_2$ | H | O | H |
| 28-13 | 4-Me-1,2,3-thiadiazol-5-yl | Me | H | Me | Me | $CF_3$ | H | $CH_2$ | H |
| 28-14 | 4-Me-1,2,3-thiadiazol-5-l | Me | H | Me | Me | $CF_3$ | H | O | H |
| 28-15 | 4-Me-1,2,3-thiadiazol-5-yl | Me | H | Me | Me | $CHF_2$ | H | $CH_2$ | H |
| 28-16 | 4-Me-1,2,3-thiadiazol-5-yl | Me | H | Me | Me | $CHF_2$ | H | O | H |
| 28-17 | 4-$CF_3$-1,2,3-thiadiazol-5-l | Me | H | Me | Me | $CF_3$ | H | $CH_2$ | H |
| 28-18 | 4-$CF_3$-1,2,3-thiadiazol-5-yl | Me | H | Me | Me | $CF_3$ | H | O | H |
| 28-19 | 4-$CF_3$-1,2,3-thiadiazol-5-yl | Me | H | Me | Me | $CHF_2$ | H | $CH_2$ | H |
| 28-20 | 4-$CF_3$-1,2,3-thiadiazol-5-yl | Me | H | Me | Me | $CHF_2$ | H | O | H |
| 28-21 | 4-$CHF_2$-1,2,3-thiadiazol-5-yl | Me | H | Me | Me | $CF_3$ | H | $CH_2$ | H |

(continued)

| No. | $R^1$ | $R^2$ | $R^3$ | $R^4$ | $R^5$ | D | Eq | Z | Xm |
|---|---|---|---|---|---|---|---|---|---|
| 28-22 | 4-$CHF_2$-1,2,3-thiadiazol-5-yl | Me | H | Me | Me | $CF_3$ | H | O | H |
| 28-23 | 4-$CHF_2$-1,2,3-thiadiazol-5-yl | Me | H | Me | Me | $CHF_2$ | H | $CH_2$ | H |

[Table 28-2]

| No. | $R^1$ | $R^2$ | $R^3$ | $R^4$ | $R^5$ | D | Eq | Z | Xm |
|---|---|---|---|---|---|---|---|---|---|
| 28-24 | 4-$CHF_2$-1,2,3-thiadiazol-5-yl | Me | H | Me | Me | $CHF_2$ | H | O | H |
| 28-25 | 1,2,3-benzothiadiazol-7-yl | Me | H | Me | Me | $CF_3$ | H | $CH_2$ | H |
| 28-26 | 1,2,3-benzothiadiazol-7-yl | Me | H | Me | Me | $CF_3$ | H | O | H |
| 28-27 | 1,2,3-benzothiadiazol-7-yl | Me | H | Me | Me | $CHF_2$ | H | $CH_2$ | H |
| 28-28 | 1,2,3-benzothiadiazol-7-yl | Me | H | Me | Me | $CHF_2$ | H | O | H |
| 28-29 | 4-$CF_3$-2-Me-thiazole-5-yl | Me | H | Me | Me | $CF_3$ | H | $CH_2$ | H |
| 28-30 | 4-$CF_3$-2-Me-thiazole-5-yl | Me | H | Me | Me | $CF_3$ | H | O | H |
| 28-31 | 4-$CF_3$-2-Me-thiazole-5-yl | Me | H | Me | Me | $CHF_2$ | H | $CH_2$ | H |
| 28-32 | 4-$CF_3$-2-Me-thiazole-5-yl | Me | H | Me | Me | $CHF_2$ | H | O | H |
| 28-33 | 4-$CHF_2$-2-Me-thiazole-5-yl | Me | H | Me | Me | $CF_3$ | H | $CH_2$ | H |
| 28-34 | 4-$CHF_2$-2-Me-thiazole-5-yl | Me | H | Me | Me | $CF_3$ | H | O | H |
| 28-35 | 4-$CHF_2$-2-Me-thiazole-5-yl | Me | H | Me | Me | $CHF_2$ | H | $CH_2$ | H |
| 28-36 | 4-$CHF_2$-2-Me-thiazole-5-yl | Me | H | Me | Me | $CHF_2$ | H | O | H |
| 28-37 | 4-$CF_3$-thiazole-5-yl | Me | H | Me | Me | $CF_3$ | H | $CH_2$ | H |
| 28-38 | 4-$CF_3$-thiazole-5-yl | Me | H | Me | Me | $CF_3$ | H | O | H |
| 28-39 | 4-$CF_3$-thiazole-5-yl | Me | H | Me | Me | $CHF_2$ | H | $CH_2$ | H |
| 28-40 | 4-$CF_3$-thiazole-5-yl | Me | H | Me | Me | $CHF_2$ | H | O | H |
| 28-41 | 4-$CHF_2$-thiazole-5-yl | Me | H | Me | Me | $CF_3$ | H | $CH_2$ | H |
| 28-42 | 4-$CHF_2$-thiazole-5-yl | Me | H | Me | Me | $CF_3$ | H | O | H |
| 28-43 | 4-$CHF_2$-thiazole-5-yl | Me | H | Me | Me | $CHF_2$ | H | $CH_2$ | H |
| 28-44 | 4-$CHF_2$-thiazole-5-yl | Me | H | Me | Me | $CHF_2$ | H | O | H |

Next, although the production method of the imide derivative (1) of the present invention is described in detail below, the present invention is not limited to these methods. In this connection, as for the reactor, other than a magnetic stirrer or a mechanical stirrer, a reaction using a microwave synthesizer is also possible.

[Production Method 1]

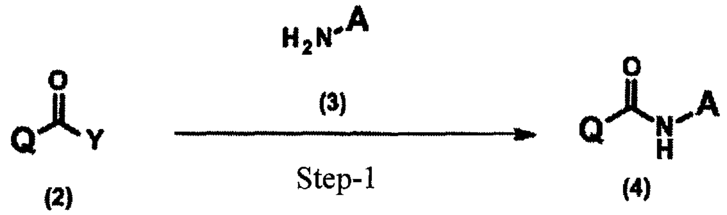


(2) (4)

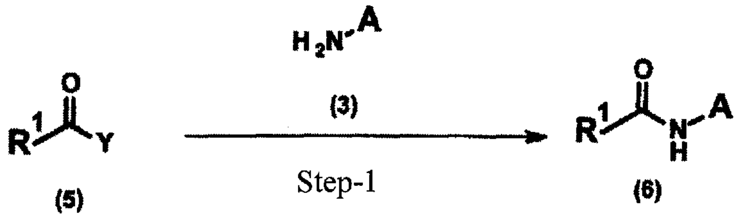


(5) (6)

(Q, A and $R^1$ represent the same meanings as above, Y represents a halogen atom, $R^{11}COO$, $R^{11}SO_3$ or a hydroxyl group, and $R^{11}$ represents a C1-C6 alkyl group, a phenyl group which may be substituted, *etc.*).

Step-1 is a step of reacting a carboxylic acid derivative represented by formula (2) or (5) with an amine derivative represented by formula (3) to produce an amide derivative represented by formula (4) or (6). The carboxylic acid derivatives represented by formulae (2) and (5) and the amine derivative represented by formula (3) can be easily produced from available reagents according to a known method described in *Lectures on Experimental Chemistry, Organic Synthesis, etc.*

In the case where Y is a halogen atom, $R^{11}COO$ or $R^{11}SO_3$, this reaction may be performed in the presence of a base. As the base, for example, an organic base such as triethylamine, diisopropylethylamine, tributylamine, N-methylmorpholine, N,N-dimethylaniline, N,N-diethylaniline, 4-tert-butyl-N,N-dimethylaniline, pyridine, picoline, lutidine and N-methylimidazole, and an alkali metal salt such as sodium carbonate, potassium carbonate, sodium hydrogencarbonate, potassium hydrogencarbonate, sodium hydroxide, potassium hydroxide, sodium acetate, potassium acetate, sodium methoxide, sodium ethoxide, potassium-tert-butoxide, sodium hydride, potassium hydride, sodium amide, n-butyllithium, sec-butyllithium, tert-butyllithium, lithium diisopropylamide, trimethylsilyl lithium and lithium hexamethyldisilazide can be used. Among these bases, a base such as triethylamine, sodium hydroxide and potassium hydroxide is preferred in view of good yield. The reaction is performed using the base in an amount of 0.1 to 5 equivalents relative to the substrate, and a target compound can thereby be obtained in good yield. The reaction substrate (3) is used usually in an amount of 1 to 5 equivalents relative to the substrate (2) or (5).

In the case where Y is a hydroxyl group, the reaction can also be performed in the co-presence of a dehydration condensation agent such as diethyl cyanophosphate (DEPC), carbonyldiimidazole (CDI), 1,3-dicyclohexylcarbodiimide (DCC), chlorocarbonic acid esters, chloro-1-methylpyridinium iodide, diisopropylcarbodiimide (DIC), 1-ethyl-3-(3-dimethylaminopropyl)carbodiimide hydrochloride (EDC), 4-(4,6-dimethoxy-1,3,5-triazin-2-yl)-4-methylmorpholinium chloride (DMT-MM), O-(7-azabenzotriazol-l-yl)-N,N,N',N'-tetramethyluronium hexafluorophosphate (HATU) and p-toluenesulfonic acid chloride. The dehydration condensation agent employed is used usually in an amount of 1 to 5 equivalents relative to the substrate (2) or (5).

This reaction is preferably performed in the presence of a solvent. As the solvent used, a solvent not adversely affecting the reaction can be used, and an aromatic hydrocarbon-based solvent such as benzene, toluene, xylene and chlorobenzene, an aliphatic hydrocarbon-based solvent such as pentane, hexane and octane, an ether-based solvent such as diethyl ether, diisopropyl ether, cyclopentylmethyl ether, tetrahydrofuran, dimethoxyethane and 1,4-dioxane, ketones such as acetone, methyl ethyl ketone and cyclohexanone, a halogen-based solvent such as chloroform, dichloromethane and carbon tetrachloride, a nitrile-based solvent such as acetonitrile and propionitrile, an ester-based solvent such as ethyl acetate, propyl acetate, butyl acetate and methyl propionate, an amide-based solvent such as N,N-dimethylformamide, N,N-dimethylacetamide and N-methylpyrrolidone, an alcohol-based solvent such as methanol, ethanol, 1-propanol, 2-propanol and tert-butanol, dimethylsulfoxide, water, or a mixed solvent thereof may be used. Furthermore, in order to promote the progress of the reaction, a phase transfer catalyst such as quaternary ammonium salt may also be added.

The reaction can be performed at a temperature appropriately selected from the range of -78°C to 200°C, although this varies depending on the reaction conditions. After the completion of the reaction, a target compound can be obtained by a normal post-treatment operation. If desired, purification by column chromatography or recrystallization,

*etc.* may also be performed.

[Production Method 2]

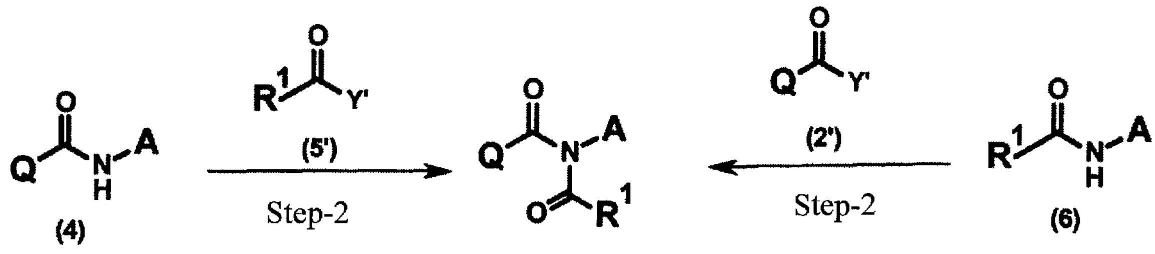


(Q, $R^1$ and A represent the same meanings as above, and Y' represents a leaving group such as a halogen atom, a methanesulfonyloxy group, a toluenesulfonyloxy group and a trifluoromethanesulfonyloxy group).

Step-2 is a step of reacting an amide derivative represented by formula (4) or (6) with a carboxylic acid derivative represented by formula (5') or (2') in the presence of a base to produce an imide derivative represented by formula (1). As the base, for example, an organic base such as triethylamine, diisopropylethylamine, tributylamine, N-methylmorpholine, N,N-dimethylaniline, N,N-diethylaniline, 4-tert-butyl-N,N-dimethylaniline, pyridine, picoline, lutidine and 4-dimethylaminopyridine, and an alkali metal salt such as sodium carbonate, potassium carbonate, sodium hydrogencarbonate, potassium hydrogencarbonate, sodium hydroxide, potassium hydroxide, sodium acetate, potassium acetate, sodium methoxide, sodium ethoxide, potassium-tert-butoxide, sodium hydride, potassium hydride, sodium amide, butyllithium, sec-butyllithium, tert-butyllithium, lithium diisopropylamide, trimethylsilyl lithium and lithium hexamethyldisilazide can be used. The reaction is performed using the base in an amount of 0.1 to 5 equivalents relative to the substrate, and a target compound can thereby be obtained in good yield. The reaction substrate (5') is used usually in an amount of 1 to 5 equivalents relative to the substrate (4). In addition, the reaction substrate (2') is used usually in an amount of 1 to 5 equivalents relative to the substrate (6).

This reaction is preferably conducted in the presence of a solvent. As the solvent used, a solvent which does not adversely affect the reaction can be used, and an aromatic hydrocarbon-based solvent such as benzene, toluene, xylene and chlorobenzene, an aliphatic hydrocarbon-based solvent such as pentane, hexane and octane, an ether-based solvent such as diethyl ether, diisopropyl ether, cyclopentylmethyl ether, tetrahydrofuran, dimethoxyethane and 1,4-dioxane, ketones such as acetone, methyl ethyl ketone and cyclohexanone, a halogen-based solvent such as chloroform, dichloromethane and carbon tetrachloride, a nitrile-based solvent such as acetonitrile and propionitrile, an ester-based solvent such as ethyl acetate, propyl acetate, butyl acetate and methyl propionate, an amide-based solvent such as N,N-dimethylformamide, N,N-dimethylacetamide and N-methylpyrrolidone, an alcohol-based solvent such as methanol, ethanol, 1-propanol, 2-propanol and tert-butanol, dimethylsulfoxide, water, or a mixed solvent thereof may be used.

The reaction can be performed at a temperature appropriately selected from the range of -78°C to 200°C, although this varies depending on bases used and the reaction conditions. After the completion of the reaction, a target compound can be obtained by a normal post-treatment operation. If desired, purification by column chromatography or recrystallization, *etc.* may also be performed.

The compound of the present invention can be analyzed, confirmed and identified by the melting point, infrared absorption spectrum, $^1H$-NMR, $^{13}C$-NMR, mass spectrometry, X-ray structural analysis, *etc.,* as needed.

In this connection, the compound of the present invention is not limited to the above-described production methods and can be produced by any organic synthesis technique.

The imide derivative (1) of the present invention can control diseases harmful to agricultural and horticultural crops with a low chemical dosage and is useful as an agricultural and horticultural fungicidal composition.

The imide derivative (1) of the present invention has a high control effect against Ascomycetes such as powdery mildew fungus in various crops, Basidiomycetes such as rust fungus and rice sheath blight fungus in various crops, Oomycetes such as downy mildew fungus in various crops and Phytophthora fungus in various crops, Deuteromycetes parasitic on various crops, such as rice blast fungus and gray mold fungus, and a wide range of other plant pathogenic fungi such as Plasmodiophoromycetes, and is useful as an agricultural and horticultural fungicide. In addition, the compound also has a control effect against bacterial diseases such as Pseudomonas disease, Xanthomonas disease and Erwinia. Furthermore, the compound also has a control effect against viral diseases caused by Tobacco mosaic virus, *etc.*

The plant disease that can be controlled by the present invention includes, more specifically, for example, rice: blast (*Pyricularia grisea*), brown spot (*Cochliobolus miyabeanus*), sheath blight (*Thanatephorus cucumeris*), bakanae disease (*Gibberella fujikuroi*), seedling blight (*Fusarium* fungus, *Rhizopus* fungus, *Pythium* fungus, *Trichoderma viride*), false smut (*Claviceps virens*), wheat: fusarium head blight (*Gibberella zeae, Fusarium avenaceum, Fusarium culmorum,*

*Monographella nivale*), snow mould (*Pythium* fungus, *Typhula* fungus, *Monographella nivalis, Myriosclerotinia borealis*), loose smut (*Ustilago nuda*), stinking smut (*Tilletia controversa*), eye spot (*Pseudocercosporella herpotrichoides*), leaf blotch (*Septoria tritici*), and glum blotch (*Phaeosphaeria nodorum*); citrus: melanose (*Diaporthe citri*), melanose-like blemish (*Diaporthe medusa, Alternaria citri*), scab (*Elsinoe fawcettii*), brown rot (*Phytophthora citrophthra*), green mold (*Penicillium digitatum*), and blue mold (*Penicillium italicum*); apple: blossom blight (*Monilinia mali*), scab (*Venturia inaequalis*), alternaria blotch (*Alternaria mali*), fruit spot (*Mycosphaerella pomi*), sooty blotch (*Gloeodes pomigena*), fly speck (*Zygophiala jamaicensis*), ring rot (*Botryosphaeria berengeriana*), blotch (*Diplocarpon mali*), rust (*Gymnosporangium yamadae*), and valsa canker (*Valsa ceratosperma*); pear: scab (*Venturia nashicola*), rust (*Gymnosporangium asiaticum*), ring rot (*Botryosphaeria berengeriana*), and phomopsis canker (*Phomopsis fukushii*); peach: leaf curl (*Taphrina deformans*), brown rot (*Monilinia fructicola, Monilinia fructigena*), scab (*Cladosporium carpophilum*), and phomopsis rot (*Phomopsis*); cherry: brown rot (*Monilinia fructicola, Monilinia fructigena*) and young fruit rot (*Monilinia kusanoi*); Japanese apricot: scab (*Cladosporium carpophilum*); grape: anthracnose (*Elsinoe ampelina*), ripe rot (*Colletotrichum acutatum, Glomerella cingulata*), leaf blight (*Pseudocercospora vitis*), and dead arm (*Phomopsis viticola*); persimmon: angular leaf spot (*Cercospora kaki*) and circular leaf spot (*Mycosphaerella nawae*); tea: gray blight (*Pestalotiopsis longiseta, Pestalotiopsis theae*), brown round spot (*Pseudocercospora ocellate, Cercospora chaae*), blister blight (*Exobasidium vexans*), and net blister blight (*Exobasidium reticulatum*); cucurbitaceous fruits: gummy stem blight (*Mycosphaerella melonis*), fusarium wilt (*Fusarium oxysporum*), scab (*Cladosporium cucumerinum*), and corynespora leaf spot (*Corynespora cassiicola*); tomato: leaf mold (*Fulvia fulva*) and early blight (*Alternaria solani*); eggplant: brown spot (*Phomopsis vexans*) and leaf mold (*Mycovellosiella nattrassii*); brassicaceous vegetables: white rust (*Albugo macrospora*) and white spot (*Cercosporella brassicae, Pseudocercosporella capsellae*); onion: gray-mold neck rot (*Botrytis allii*); strawberry: leaf spot (*Mycosphaerella fragariae*); potato: early blight (*Alternaria solani*); soy: stem rot (*Phytophthora sojae*) and purple stain (*Cercospora kikuchii*); adzuki bean: stem rot (*Phytophthora vignae*); peanut: brown leaf spot (*Mycoshaerella arachidis*); sugar beet: cercospora leaf spot (*Cercospora beticola*) and leaf blight (*Thanatephorus cucumeris*); lawn grass: curvularia leaf blight (*Curvularia* fungus), dollar spot (*Sclerotinia homoeocarpa*), and Helminthosporium leaf blight (*Cochliobolus* fungus); rose: black spot (*Diplocarpon rosae*); chrysanthemum: white rust (*Puccinia horiana*); various crops: downy mildew (*Peronospora* fungus, *Pseudoperonospora* fungus, *Plasmopara* fungus, *Bremia* fungus), blight (*Phytophthora* fungus), powdery mildew (*Erysiphe* fungus, *Blumeria* fungus, *Sphaerotheca* fungus, *Podosphaerea* fungus, *Phyllactinia* fungus, *Uncinula* fungus, *Oidiopsis* fungus), rust (*Puccinia* fungus, *Uromyces* fungus, *Physopella* fungus, *Phakopsora* fungus), anthracnose (*Glomerella* fungus, *Colletotrichum* fungus, *Gloeosporium* fungus), leaf spot (*Alternaria* fungus), gray mold (*Botrytis cinerea*), sclerotina rot (*Sclerotinia sclerotiorum*), white root rot (*Rosellinia necatrix*), violet root rot (*Helicobasidium mompa*), and southern blight (*Sclerotium rolfsii*); various other soil diseases (*Fusarium* fungus, *Rhizoctonia* fungus, *Pythium* fungus, *Aphanomyces* fungus, *Phoma* fungus, *Verticillium* fungus, *Plasmodiophora brassicae, etc.*); and bacterial diseases: diseases caused by *Pseudomonas,* such as cucumber bacterial spot (*Pseudomonas syringae pv. lachrymans*) and tomato bacterial wilt (*Pseudomonas solanacearum*), diseases caused by *Xanthomonas,* such as cabbage black rot (*Xanthomonas campestris*), rice bacterial leaf blight (*Xanthomonas oryzae*) and citrus canker (*Xanthomonas citri*), and diseases caused by *Erwinia,* such as cabbage soft rot (*Erwinia carotovora*). Of course, the plant pathogenic fungi as the target for the control of the present invention are not limited to those exemplified above.

In the case of using the imide derivative (1) of the present invention as an active ingredient of an agricultural and horticultural fungicide, the compound may be used alone but is preferably used in the form of a composition produced using common agricultural adjuvants. Although the form of the fungicide of the present invention is not particularly limited, for example, it is preferable to formulate it in the form of an emulsifiable concentrate, a suspension, a wettable powder, a water soluble powder, a liquid formulation, a sol (flowable formulation), a granular wettable powder, a dust, a fine granule, a granule, a tablet, an oil agent, a spray, a mist, an aerosol, a paste, *etc.* Furthermore, one kind or two or more kinds of imide derivatives (1) of the present invention can be blended, and in addition to adjuvants, other fungicidal components may also be incorporated as an active ingredient.

The component as an agricultural adjuvant used for the production of an agricultural and horticultural fungicide containing the imide derivative (1) of the present invention as an active ingredient includes a carrier, a surfactant, and other adjuvants.

The carrier contained in the agricultural and horticultural fungicide of the present invention may be either a liquid carrier or a solid carrier as long as it is an agriculturally and horticulturally usable carrier, and the carrier is not limited to specific carriers.

The liquid carrier includes water, alcohols such as isopropyl alcohol and ethylene glycol, ketones such as cyclohexanone and methyl ethyl ketone, ethers such as propylene glycol monomethyl ether and diethylene glycol mono-n-butyl ether, aliphatic hydrocarbons such as kerosene and gas oil, aromatic hydrocarbons such as xylene, trimethylbenzene, tetramethylbenzene, methylnaphthalene and solvent naphtha, amides such as N-methyl-2-pyrrolidone, esters such as glycerin ester of a fatty acid, and vegetable oils such as soybean oil and rapeseed oil. As the solid carrier, animal and plant powders such as starch, activated carbon, soybean flour, wheat flour, wood powder, fish powder and powdered milk, and mineral powders such as talc, kaolin, bentonite, zeolite, diatomaceous earth, white carbon, clay, alumina,

calcium carbonate, potassium chloride and ammonium sulfate, can be used. Two or more of these carriers can be used in combination.

The surfactant contained in the agricultural and horticultural fungicide of the present invention includes a nonionic surfactant, an anionic surfactant, a cationic surfactant, an amphoteric surfactant, *etc.* and, specifically, includes the followings.

The nonionic surfactant includes, for example, a polyoxyethylene alkyl ether, a polyoxyethylene alkylaryl ether, a polyoxyethylene styrylphenyl ether, a polyoxyethylene alkyl ester, a polyoxyethylene sorbitan alkylate, a polyoxyethylene phenyl ether polymer, a polyoxyethylene alkylene aryl phenyl ether, a polyoxyethylene alkylene glycol, a polyoxyethylene polyoxypropylene block copolymer, *etc.*

The anionic surfactant includes, for example, a lignin sulfonate, an alkyl aryl sulfonate, a dialkyl sulfosuccinate, a polyoxyethylene alkyl aryl ether sulfate, an alkyl naphthalene sulfonate, a polyoxyethylene styrylphenyl ether sulfate, *etc.*

The cationic surfactant includes, for example, an alkylamine salt, *etc.*

The amphoteric surfactant includes, for example, a quaternary ammonium salt alkylbetaine, an amine oxide, *etc.*

Note that the surfactant usable at the time of formulation is not limited to those described above. In addition, two or more kinds of surfactants can be used in combination.

Other adjuvants contained in the agricultural and horticultural fungicide of the present invention include, but are not limited to, a binder, a thickener, a fixing agent, an antiseptic/antifungal agent, a solvent, a stabilizer for agricultural active ingredients, an antioxidant, an anti-ultraviolet agent, an anti-crystallization agent, an antifoam agent, a property improver, a colorant, *etc.*

The binder, thickener and fixing agent are not particularly limited but include, for example, starch, dextrin, cellulose, methylcellulose, ethylcellulose, carboxymethylcellulose, hydroxyethylcellulose, hydroxypropylcellulose, hydroxypropylmethylcellulose, carboxymethyl starch, pullulan, sodium alginate, ammonium alginate, alginic acid propylene glycol ester, guar gum, locust bean gum, gum arabic, xanthan gum, gelatin, casein, polyvinyl alcohol, polyethylene oxide, polyethylene glycol, an ethylene/propylene block copolymer, sodium polyacrylate, polyvinylpyrrolidone, *etc.*

The agricultural and horticultural fungicide of the present invention can be produced according to the conventional formulation method depending on the intended dosage form except for blending the imide derivative (1) of the present invention.

The agricultural and horticultural fungicide of the present invention can be mixed with or used in combination with other fungicides (antifungal agent, bactericide, antiviral agent, plant resistance inducing agent), an insecticide, an acaricide, a nematicide, an insect growth regulator, an insect attractant, a herbicide, a plant growth regulator, a synergist, a safeners, a bird repellent, a fertilizer, a soil conditioner, *etc.* Furthermore, the imide derivative (1) of the present invention can be used by mixing it with these active ingredients at the time of formulation.

Although representative examples of the ingredient which can be mixed with or used in combination with the agricultural and horticultural fungicide of the present invention are described below, the present invention is not necessarily limited solely to these examples.

Although representative examples of the fungicide and insecticide/acaricide which can be mixed with or used in combination with the agricultural and horticultural fungicide of the present invention are described below, the present invention is not necessarily limited solely to these examples.

[Fungicide]

Amisulbrom, cyazofamid, fenpicoxamid, florylpicoxamid, dimethomorph, cymoxanil, fluopicolide, benthiavalicarb-isopropyl, metalaxyl, picarbutrazox, oxathiapiprolin, floxapiprolin, ametoctradin, acibenzolar, isotianil, tiadinil, probenazole, dichlobentiazox, carpropamid, diclocymet, fenoxanil, tricyclazole, pyroquilon, phthalide, tolprocarb, azoxystrobin, orysastrobin, kresoxim-methyl, trifloxystrobin, picoxystrobin, pyraclostrobin, famoxadone, metominostrobin, mandestrobin, pyriminostrobin, coumoxystrobin, enoxastrobin, fenaminstrobin, flufenoxystrobin, triclopyricarb, pyrametostrobin, pyraoxystrobin, methyltetraprole, tebufloquin, thiophanate-methyl, diethofencarb, thiabendazole, benomyl, pyridachlometyl, ipconazole, imibenconazole, oxpoconazole fumarate, difenoconazole, cyproconazole, simeconazole, tebuconazole, tetraconazole, triadimefon, triflumizole, triforine, bitertanol, fenarimol, fenbuconazole, prochloraz, propiconazole, hexaconazole, pefurazoate, myclobutanil, metconazole, epoxiconazole, prothioconazole, pyrisoxazole, ipfentrifluconazole, mefentrifluconazole, thifluzamide, bixafen, furametpyr, flutolanil, penflufen, penthiopyrad, boscalid, pyrapropoyne, fluindapyr, isoflucypram, pydiflumetofen, pyraziflumid, isofetamid, benzovindiflupyr, fluxapyroxad, sedaxane, isopyrazam, fluopyram, inpyrfluxam, aminopyrifen, mepronil, isoprothiolane, ferimzone, diclomedine, pencycuron, iprodione, procymidone, cyprodinil, mepanipyrim, pyrimethanil, fenhexamid, fenpyrazamine, quinofumelin, ipflufenoquin, fluazinam, fludioxonil, cyflufenamid, metrafenone, quinoxyfen, pyriofenone, valifenalate, flutianil, iminoctadine triacetate, iminoctadine albesilate, propamocarb hydrochloride, chlorothalonil (TPN), captan, fluoroimide, folpet, dithianon, chinomethionat, diflumetorim, triazine (anilazine), fosetyl-aluminium, amobam, ziram, thiadiazin (milneb), thiram, polycarbamate, mancozeb, maneb, flusulfamide, hydroxy isoxazole (hymexazol), echlomezole (etridiazole), iprobenfos (IBP),

edifenphos (EDDP), tolclofos-methyl, validamycin, blasticidin-S, polyoxins, polyoxin zinc salt (polyoxim), kasugamycin, oxytetracycline, streptomycin, oxolinic acid, tecloftalam, sulfur, sodium hydrogencarbonate, potassium hydrogencarbonate, metallic silver, copper oxychloride, basic copper sulfate, copper hydroxide, anhydrous copper sulfate, copper nonylphenol sulfonate, copper 8-hydroxyquinolate (oxine-copper), DBEDC, etc.

[Insecticide, Acaricide]

Acephate, isoxathion, ethylthiodemeton, cadusafos, chlorpyrifos, chlorpyrifos-methyl, cyanophos (CYAP), dichlofenthion (ECP), dimethoate, dichlorvos (DDVP), diazinon, trichlorfon (DEP), pyraclofos, pirimiphos-methyl, fenitrothion (MEP), fenthion (MPP), phenthoate (PAP), prothiofos, profenophos, phosalone, fostiazate, malathion, methidathion (DMTP), EPN, alanycarb, isoprocarb (MIPC), ethiofencarb, oxamyl, carbaryl (NAC), carbosulfan, thiodicarb, furathiocarb, benfuracarb, methomyl, fenobucarb (BPMC), XMC, acrinathrin, allethrin, etofenprox, cycloprothrin, cyhalothrin, cyfluthrin, cypermethrin, shilafluofen, tefluthrin, tralomethrin, bifenthrin, fenvalerate, fenpropathrin, flucythrinate, fluvalinate, permethrin, fenvalerate, cartap, thiocyclam, bensultap, acetamipirid, imidacloprid, clothianidin, dinotefuran, thiacloprid, thiamethoxam, nitenpyram, chlorfluazuron, diflubenzuron, teflubenzuron, novaluron, flufenoxuron, lufenuron, chromafenozide, tebfenozide, buprofenzin, methoxyfenozide, cyromazine, endosulfan, ethiprole, fipronil, indoxacarb MP, chlorfenapyr, diafenthiuron, tolfenpyrad, pymetrozine, pyridalyl, flubendiamide, chlorantraniliprole, flonicamid, abamectin, emamectin benzoate, spinosad, milbemectin, lepimectin, pyrethrins, starch, fatty acid glyceride, propylene glycol monofatty acid ester, petroleum oil, rapeseed oil, sodium oleate, BT, acequinocyl, amitraz, etoxazole, clofentezine, spirodichlofen, spiromeshifen, spirotetramat, tebufenpyrad, bifenazate, pyridaben, pyrimidifen, fenothiocarb, fenpyroximate, fenbutatin oxide, fluacrypyrim, hexythiazox, tyclopyrazoflor, flupyrimin, spiropidion, acynonapyr, benzpyrimoxan, chloroprallethrin, cyhalodiamide, fluxametamide, isocycloseram, epsilon-metofluthrin, epsilon-momfluorothrin, kappa-bifenthrin, broflanilide, dicloromezotiaz, kappa-tefluthrin, fluhexafon, tetraniliprole, triflumezopyrim, cyclaniliprole, momfluorothrin, heptafluthrin, afidopyropen, flometoquin, flupyradifurone, flufiprole, meperfluthrin, cyantraniliprole, oxazosulfyl, dimpropyridaz, *etc.*

In this connection, the production method of the agricultural and horticultural fungicide of the present invention is more specifically described later in Formulation Examples. Of course, the fungicide of the present invention is not limited to these Formulation Examples, and other various additives may be mixed in any ratio, or the formulation can be performed by mixing other fungicides, *etc.* at any ratio.

The method for applying the agricultural and horticultural fungicide (including a diluted one thereof) is not particularly limited and includes aerial application (for example, spraying, misting, atomizing, dusting, granule application, water surface application, or nursery box application), soil application (for example, mixing, irrigation, side row application, or in-ground application), surface application (for example, coating, dust coating, or covering), dipping, *etc.*

The application rate of the agricultural and horticultural fungicide of the present invention is not particularly limited and is appropriately selected from a wide range according to various conditions such as the concentration of active ingredient in fungicide, the form of preparation, the type of target disease or crop, the degree of damage due to disease, the location of application, the method of application, the timing of application, the types and usage amounts of formulations, fertilizers, *etc.* which are mixed or used in combination, and the meteorological phenomena. Usually, the fungicide is applied at a rate of, as the amount of the active ingredient compound of the present invention, approximately from 1 to 100,000 g, and preferably on the order of 10 to 10,000 g, per hectare

Furthermore, in the case where the agricultural and horticultural fungicide of the present invention is used in the form of a water-diluted formation such as liquid concentrate, emulsifiable concentrate, wettable powder, sol (flowable formulation) or granular wettable powder, the formulation is used at an application concentration of approximately from 0.1 to 10,000 ppm by mass, and preferably on the order of 1 to 500 ppm by mass. However, the present invention is not limited thereto.

## EXAMPLES

The present invention is described more specifically below by referring to Synthesis Examples, Formulation Examples and Test Examples of the compound of the present invention, however, the present invention is not limited thereto by any means.

[Synthesis Example 1]

N-{3-(Difluoromethyl)-1-methylpyrazole-4-carbonyl}-N-{2-(1,3-dimethylbutyl)phenyl}-4-methylthiadiazole-5-carboxamide (No. 1-146)

Thionyl chloride (619 mg, 5.20 mmol) and N,N-dimethylformamide (20 mg, 0.27 mmol) were added to a toluene

solution (10 mL) of 4-methylthiadiazole-5-carboxylic acid (250 mg, 1.73 mmol) and after heating under reflux for 2 hours, the reaction mixture solution was concentrated to obtain 4-methylthiadiazole-5-carbonyl chloride.

3-(Difluoromethyl)-N-{2-(1,3-dimethylbutyl)phenyl}-1-methyl-pyrazole-4-carboxamide (350 mg, 1.04 mmol) prepared in Reference Example 1 was added to a separate vessel containing sodium hydride (60%, 45.9 mg, 1.15 mmol) and tetrahydrofuran (2 mL), and the mixture was stirred at 25°C for 20 minutes. Thereto, a tetrahydrofuran solution (2 mL) of 4-methylthiadiazole-5-carbonyl chloride prepared above was added at 0°C, and the resulting mixture was stirred at 25°C for 1 hour. After confirming the completion of the reaction by TLC (thin layer chromatography), the reaction mixture solution was poured into saturated aqueous ammonium chloride under ice bath and extracted with chloroform. The extract was washed with saturated brine, dried over anhydrous sodium sulfate, and then concentrated under reduced pressure. The concentrate was purified by silica gel column chromatography (eluting solvent: ethyl acetate/n-hexane=1/1) to obtain the title compound (yielded: 350 mg, yield: 73%) as a white solid.

[Synthesis Example 2]

4-Difluoromethyl-N-{4-(difluoromethyl)-2-methyl-thiazole-5-carbonyl}-N-{2-(1,3-dimethylbutyl)phenyl}-2-methyl-thiazole-5-carboxamide (No. 7-238)

Thionyl chloride (620 mg, 5.12 mmol) and N,N-dimethylformamide (20 mg, 0.27 mmol) were added to a toluene solution (10 mL) of 4-(difluoromethyl)-2-methyl-thiazole-5-carboxylic acid (500 mg, 2.59 mmol) and after heating under reflux for 1 hour, the reaction mixture solution was concentrated to obtain 4-(difluoromethyl)-2-methyl-thiazole-5-carbonyl chloride.

4-(Difluoromethyl)-N-{2-(1,3-dimethylbutyl)phenyl}-2-methyl-thiazole-5-carboxamide (700 mg, 1.99 mmol) prepared in Reference Example 2 was added to a separate vessel containing sodium hydride (60%, 87.4 mg, 2.19 mmol) and tetrahydrofuran (2 mL), and the mixture was stirred at 25°C for 20 minutes. Thereto, a tetrahydrofuran solution (2 mL) of 4-(difluoromethyl)-2-methyl-thiazole-5-carbonyl chloride prepared above was added at 0°C, and the resulting mixture was stirred at 25°C for 1 hour. After confirming the completion of the reaction by TLC (thin layer chromatography), the reaction mixture solution was poured into saturated aqueous ammonium chloride under ice bath and extracted with ethyl acetate. The extract was washed with saturated brine, dried over anhydrous sodium sulfate, and then concentrated under reduced pressure. The concentrate was purified by silica gel column chromatography (eluting solvent: ethyl acetate/n-hexane=3/1) to obtain the title compound (yielded: 600 mg, yield: 57%) as a brown candy-like substance.

[Synthesis Example 3]

3,4-Dichloro-N-{2-(1,3-dimethylbutyl)phenyl}-N-(5-fluoro-1,3-dimethylpyrazole-4-carbonyl)isothiazole-5-carboxamide (No. 1-131)

Thionyl chloride (601 mg, 5.05 mmol) and N,N-dimethylformamide (20 mg, 0.27 mmol) were added to a toluene solution (10 mL) of 3,4-dichloroisothiazole-5-carboxylic acid (500 mg, 2.52 mmol) and after heating under reflux for 1 hour, the reaction mixture solution was concentrated to obtain 3,4-dichloroisothiazole-5-carbonyl chloride.

N-{2-(1,3-Dimethylbutyl)phenyl}-5-fluoro-1,3-dimethyl-pyrazole-4-carboxamide (700 mg, 2.21 mmol) was added to a separate vessel containing sodium hydride (60%, 97 mg, 2.43 mmol) and tetrahydrofuran (2 mL), and the mixture was stirred at 25°C for 20 minutes. Thereto, a tetrahydrofuran solution (2 mL) of 3,4-dichloroisothiazole-5-carbonyl chloride prepared above was added at 0°C, and the resulting mixture was stirred at 25°C for 1 hour. After confirming the completion of the reaction by TLC (thin layer chromatography), the reaction mixture solution was poured into saturated aqueous ammonium chloride under ice bath and extracted with ethyl acetate. The extract was washed with saturated brine, dried over anhydrous sodium sulfate, and then concentrated under reduced pressure. The concentrate was purified by silica gel column chromatography (eluting solvent: ethyl acetate/n-hexane=1/1) to obtain the title compound (yielded: 800 mg, yield: 73%) as a brown candy-like substance.

[Synthesis Example 4]

3,4-Dichloro-N-{2-(difluoromethyl)pyridine-3-carbonyl}-N-{2-(1,3-dimethylbutyl)phenyl}isothiazole-5-carboxamide (No. 13-2)

Thionyl chloride (430 mg, 3.62 mmol) and N,N-dimethylformamide (20 mg, 0.27 mmol) were added to a toluene solution (10 mL) of 3,4-dichloroisothiazole-5-carboxylic acid (358 mg, 1.81 mmol) and after heating under reflux for 1 hour, the reaction mixture solution was concentrated to obtain 3,4-dichloroisothiazole-5-carbonyl chloride.

2-(Difluoromethyl)-N-{2-(1,3-dimethylbutyl)phenyl}pyridine-3-carboxamide (500 mg, 1.50 mmol) prepared in

Reference Example 3 was added to a separate vessel containing sodium hydride (60%, 66 mg, 1.66 mmol) and tetrahydrofuran (2 mL), and the mixture was stirred at 25°C for 20 minutes. Thereto, a tetrahydrofuran solution (2 mL) of 3,4-dichloroisothiazole-5-carbonyl chloride prepared above was added at 0°C, and the resulting mixture was stirred at 25°C for 1 hour. After confirming the completion of the reaction by TLC (thin layer chromatography), the reaction mixture solution was poured into saturated aqueous ammonium chloride under ice bath and extracted with ethyl acetate. The extract was washed with saturated brine, dried over anhydrous sodium sulfate, and then concentrated under reduced pressure. The concentrate was purified by silica gel column chromatography (eluting solvent: ethyl acetate/n-hexane=3/1) to obtain the title compound (yielded: 620 mg, yield: 80%) as a white solid.

[Synthesis Example 5]

3,4-Dichloro-N-(5-chloro-1,3-dimethyl-pyrazole-4-carbonyl)-N-(1,3-dihydro-1,1,3-trimethylisobenzofuran-4-yl)-isothiazole-5-carboxamide (No. 25-115)

Thionyl chloride (571 mg, 4.81 mmol) and N,N-dimethylformamide (20 mg, 0.27 mmol) were added to a toluene solution (10 mL) of 3,4-dichloroisothiazole-5-carboxylic acid (476 mg, 2.40 mmol) and after heating under reflux for 1 hour, the reaction mixture solution was concentrated to obtain 3,4-dichloroisothiazole-5-carbonyl chloride.

5-Chloro-N-(1,3-dihydro-1,1,3-trimethylisobenzofuran-4-yl)-1,3-dimethylpyrazole-4-carboxamide (700 mg, 2.10 mmol) was added to a separate vessel containing sodium hydride (60%, 92 mg, 2.31 mmol) and tetrahydrofuran (2 mL), and the mixture was stirred at 25°C for 20 minutes. Thereto, a tetrahydrofuran solution (2 mL) of 3,4-dichloroisothiazole-5-carbonyl chloride prepared above was added at 0°C, and the resulting mixture was stirred at 25°C for 1 hour. After confirming the completion of the reaction by TLC (thin layer chromatography), the reaction mixture solution was poured into saturated aqueous ammonium chloride under ice bath and extracted with ethyl acetate. The extract was washed with saturated brine, dried over anhydrous sodium sulfate, and then concentrated under reduced pressure. The concentrate was purified by silica gel column chromatography (eluting solvent: ethyl acetate/n-hexane=2/1) to obtain the title compound (yielded: 780 mg, yield: 72%) as a white solid.

[Synthesis Example 6]

3,4-Dichloro-N-{3-(difluoromethyl)-1-methyl-pyrazole-4-carbonyl)-N-(2,3-dihydro-1,1,3-trimethyl-1H-inden-4-yl)-isothiazole-5-carboxamide (No. 25-4)

Thionyl chloride (541 mg, 4.54 mmol) and N,N-dimethylformamide (20 mg, 0.27 mmol) were added to a toluene solution (10 mL) of 3,4-dichloroisothiazole-5-carboxylic acid (450 mg, 2.27 mmol) and after heating under reflux for 1 hour, the reaction mixture solution was concentrated to obtain 3,4-dichloroisothiazole-5-carbonyl chloride.

3-(Difluoromethyl)-N-(2,3-dihydro-1,1,3-trimethyl-1H-inden-4-yl)-1-methylpyrazole-4-carboxamide (460 mg, 1.38 mmol) was added to a separate vessel containing sodium hydride (60%, 61 mg, 1.51 mmol) and tetrahydrofuran (2 mL), and the mixture was stirred at 25°C for 20 minutes. Thereto, a tetrahydrofuran solution (2 mL) of 3,4-dichloroisothiazole-5-carbonyl chloride prepared above was added at 0°C, and the resulting mixture was stirred at 25°C for 1 hour. After confirming the completion of the reaction by TLC (thin layer chromatography), the reaction mixture solution was poured into saturated aqueous ammonium chloride under ice bath and extracted with ethyl acetate. The extract was washed with saturated brine, dried over anhydrous sodium sulfate, and then concentrated under reduced pressure. The concentrate was purified by silica gel column chromatography (eluting solvent: ethyl acetate/n-hexane=2/1) to obtain the title compound (yielded: 500 mg, yield: 71%) as a white solid.

[Synthesis Example 7]

3,4-Dichloro-N-{2-(1,3-dimethylbutyl)-3-thienyl}-N-{1-methyl-3-(trifluoromethyl)pyrazole-4-carbonyl}isothiazole-5-carboxamide (No. 5-1)

Thionyl chloride (300 mg, 2.52 mmol) and N,N-dimethylformamide (20 mg, 0.27 mmol) were added to a toluene solution (10 mL) of 3,4-dichloroisothiazole-5-carboxylic acid (250 mg, 1.26 mmol) and after heating under reflux for 1 hour, the reaction mixture solution was concentrated to obtain 3,4-dichloroisothiazole-5-carbonyl chloride.

N-{2-(1,3-Dimethylbutyl)-3-thienyl}-1-methyl-3-(trifluoromethyl)pyrazole-4-carboxamide (350 mg, 0.97 mmol) was added to a separate vessel containing sodium hydride (60%, 43 mg, 1.07 mmol) and tetrahydrofuran (2 mL), and the mixture was stirred at 25°C for 20 minutes. Thereto, a tetrahydrofuran solution (2 mL) of 3,4-dichloroisothiazole-5-carbonyl chloride prepared above was added at 0°C, and the resulting mixture was stirred at 25°C for 1 hour. After confirming the completion of the reaction by TLC (thin layer chromatography), the reaction mixture solution was poured

into saturated aqueous ammonium chloride under ice bath and extracted with ethyl acetate. The extract was washed with saturated brine, dried over anhydrous sodium sulfate, and then concentrated under reduced pressure. The concentrate was purified by silica gel column chromatography (eluting solvent: ethyl acetate/n-hexane=2/1) to obtain the title compound (yielded: 270 mg, yield: 51%) as a brown candy-like substance.

[Synthesis Example 8]

N-(3,4-Dichloroisothiazol-5-yl)-N-(3',4'-difluorobiphen-2-yl)-3-(trifluoromethyl)pyrazine-2-carboxamide (No. 13-55)

Thionyl chloride (527 mg, 4.43 mmol) and N,N-dimethylformamide (20 mg, 0.27 mmol) were added to a toluene solution (10 mL) of 3,4-dichloroisothiazole-5-carboxylic acid (439 mg, 2.22 mmol) and after heating under reflux for 1 hour, the reaction mixture solution was concentrated to obtain 3,4-dichloroisothiazole-5-carbonyl chloride.

N-(3',4'-Difluorobiphenyl-2-yl)-3-(trifluoromethyl)pyrazine-2-carboxamide (700 mg, 1.84 mmol) was added to a separate vessel containing sodium hydride (60%, 81 mg, 2.03 mmol) and tetrahydrofuran (2 mL), and the mixture was stirred at 25°C for 20 minutes. Thereto, a tetrahydrofuran solution (2 mL) of 3,4-dichloroisothiazole-5-carbonyl chloride prepared above was added at 0°C, and the resulting mixture was stirred at 25°C for 1 hour. After confirming the completion of the reaction by TLC (thin layer chromatography), the reaction mixture solution was poured into saturated aqueous ammonium chloride under ice bath and extracted with ethyl acetate. The extract was washed with saturated brine, dried over anhydrous sodium sulfate, and then concentrated under reduced pressure. The concentrate was purified by silica gel column chromatography (eluting solvent: ethyl acetate/n-hexane=2/1) to obtain the title compound (yielded: 270 mg, yield: 51%) as a white solid.

[Synthesis Example 9]

N-(3,4-Dichloroisothiazole-5-carbonyl)-4-(difluoromethyl)-N-{2-(1,3-dimethylbutyl)phenyl}-2-methyl-thiazole-5-carboxamide (No. 7-38)

Thionyl chloride (732 mg, 6.15 mmol) and N,N-dimethylformamide (40 mg, 0.54 mmol) were added to a toluene solution (10 mL) of 4-(difluoromethyl)-2-methyl-thiazole-5-carboxylic acid (594 mg, 3.08 mmol) and after heating under reflux for 1 hour, the reaction mixture solution was concentrated to obtain 4-(difluoromethyl)-2-methyl-thiazole-5-carbonyl chloride.

3,4-Dichloro-N-{2-(1,3-dimethylbutyl)phenyl}isothiazole-5-carboxamide (1,000 mg, 2.80 mmol) prepared in Reference Example 4 was added to a separate vessel containing sodium hydride (60%, 123 mg, 3.08 mmol) and tetrahydrofuran (2 mL), and the mixture was stirred at 25°C for 20 minutes. Thereto, a tetrahydrofuran solution (2 mL) of 4-(difluoromethyl)-2-methyl-thiazole-5-carbonyl chloride prepared above was added at 0°C, and the resulting mixture was stirred at 25°C for 1 hour. After confirming the completion of the reaction by TLC (thin layer chromatography), the reaction mixture solution was poured into saturated aqueous ammonium chloride under ice bath and extracted with ethyl acetate. The extract was washed with saturated brine, dried over anhydrous sodium sulfate, and then concentrated under reduced pressure. The concentrate was purified by silica gel column chromatography (eluting solvent: ethyl acetate/n-hexane=3/1) to obtain the title compound (yielded: 990 mg, yield: 66%) as a brown candy-like substance.

[Reference Example 1]

3-(Difluoromethyl)-N-{2-(1,3-dimethylbutyl)phenyl}-1-methyl-pyrazole-4-carboxamide

Oxalyl chloride (3.20 g, 24.5 mmol) and N,N-dimethylformamide (0.10 g, 1.40 mmol) were added to a chloroform solution (44 mL) of 3-(difluoromethyl)-1-methyl-pyrazole-4-carboxylic acid (4.00 g, 22.7 mmol) and after stirring at 25°C for 1 hour, the reaction mixture solution was concentrated to obtain 3-(difluoromethyl)-1-methyl-pyrazole-4-carbonyl chloride.

2-(1,3-Dimethylbutyl)aniline (4.00 g, 22.7 mmol), triethylamine (2.50 g, 24.5 mmol), and chloroform (44 mL) were added to a separate vessel. Thereto, a chloroform solution (5 mL) of 3-(difluoromethyl)-1-methyl-pyrazole-4-carbonyl chloride prepared above was added at 0°C, and the resulting mixture was stirred at 25°C for 1 hour. After confirming the completion of the reaction by TLC (thin layer chromatography), water was poured into the reaction mixture solution, and the solution was extracted with chloroform. The extract was washed with saturated brine, dried over anhydrous sodium sulfate, and then concentrated under reduced pressure. The concentrate was purified by silica gel column chromatography (eluting solvent: ethyl acetate/n-hexane=1/1) to obtain the title compound (yielded: 6.50 g, yield: 85%) as a white solid.

[Reference Example 2]

4-(Difluoromethyl)-N-{2-(1,3-dimethylbutyl)phenyl}-2-methyl-thiazole-5-carboxamide

Thionyl chloride (550 mg, 4.66 mmol) and N,N-dimethylformamide (100 mg, 1.40 mmol) were added to a toluene solution (10 mL) of 4-(difluoromethyl)-2-methyl-thiazole-5-carboxylic acid (450 mg, 2.33 mmol) and after heating under reflux for 1 hour, the reaction mixture solution was concentrated to obtain 4-(difluoromethyl)-2-methyl-thiazole-5-carbonyl chloride.

2-(1,3-Dimethylbutyl)aniline (430 mg, 2.45 mmol), triethylamine (260 mg, 2.56 mmol), and chloroform (10 mL) were added to a separate vessel. Thereto, a chloroform solution (1 mL) of 4-(difluoromethyl)-2-methyl-thiazole-5-carbonyl chloride prepared above was added at 0°C, and the resulting mixture was stirred at 25°C for 1 hour. After confirming the completion of the reaction by TLC (thin layer chromatography), water was poured into the reaction mixture solution, and the solution was extracted with chloroform. The extract was washed with saturated brine, dried over anhydrous sodium sulfate, and then concentrated under reduced pressure. The concentrate was purified by silica gel column chromatography (eluting solvent: ethyl acetate/n-hexane=3/1) to obtain the title compound (yielded: 550 mg, yield: 67%) as a white solid.

[Reference Example 3]

2-(Difluoromethyl)-N-{2-(1,3-dimethylbutyl)phenyl}pyridine-3-carboxamide

Thionyl chloride (646 mg, 5.43 mmol) and N,N-dimethylformamide (20 mg, 0.27 mmol) were added to a toluene solution (10 mL) of 2-(difluoromethyl)pyridine-3-carboxylic acid (470 mg, 2.71 mmol) and after heating under reflux for 1 hour, the reaction mixture solution was concentrated to obtain 2-(difluoromethyl)pyridine-3-carbonyl chloride.

2-(1,3-Dimethylbutyl)aniline (505 mg, 2.85 mmol), triethylamine (302 mg, 2.99 mmol), and chloroform (10 mL) were added to a separate vessel. Thereto, a chloroform solution (1 mL) of 2-(difluoromethyl)pyridine-3-carbonyl chloride prepared above was added at 0°C, and the resulting mixture was stirred at 25°C for 1 hour. After confirming the completion of the reaction by TLC (thin layer chromatography), water was poured into the reaction mixture solution, and the solution was extracted with chloroform. The extract was washed with saturated brine, dried over anhydrous sodium sulfate, and then concentrated under reduced pressure. The concentrate was purified by silica gel column chromatography (eluting solvent: ethyl acetate/n-hexane=3/1) to obtain the title compound (yielded: 800 mg, yield: 87%) as a white solid.

[Reference Example 4]

3,4-Dichloro-N-{2-(1,3-dimethylbutyl)phenyl}isothiazole-5-carboxamide

Thionyl chloride (671 mg, 5.64 mmol) and N,N-dimethylformamide (20 mg, 0.27 mmol) were added to a toluene solution (10 mL) of 3,4-dichloroisothiazole-5-carboxylic acid (614 mg, 3.10 mmol) and after heating under reflux for 1 hour, the reaction mixture solution was concentrated to obtain 3,4-dichloroisothiazole-5-carbonyl chloride.

2-(1,3-Dimethylbutyl)aniline (500 mg, 2.82 mmol), triethylamine (314 mg, 3.10 mmol), and chloroform (10 mL) were added to a separate vessel. Thereto, a chloroform solution (1 mL) of 3,4-dichloroisothiazole-5-carbonyl chloride prepared above was added at 0°C, and the resulting mixture was stirred at 25°C for 1 hour. After confirming the completion of the reaction by TLC (thin layer chromatography), water was poured into the reaction mixture solution, and the solution was extracted with chloroform. The extract was washed with saturated brine, dried over anhydrous sodium sulfate, and then concentrated under reduced pressure. The concentrate was purified by silica gel column chromatography (eluting solvent: ethyl acetate/n-hexane=3/1) to obtain the title compound (yielded: 820 mg, yield: 81%) as a white solid.

The $^1H$ NMR spectra ($CDCl_3$), $\sigma$ (ppm) values, melting points (°C), *etc.* of the compound of the present invention produced based on these Synthesis Examples and the Production Methods above are shown in Table 29. The $^1H$ NMR data was measured by JNM-ECS400 Spectrometer (manufactured by JEOL Ltd.).

[Table 29-1]

Table 29

| No. | $^1$HNMR Spectrum ($CDCl_3$) σ ppm: | Shape | Melting Point (°C) |
|---|---|---|---|
| 1-29 | 7.90-7.45(1H, m), 7.43-7.36(1H, m), 7.30-7.23(1H, m), 7.10-6.90(2H, m), 6.79-6.70(1H, m), 3.80(1.3H, s), 3.77(1.7H, s), 1.71-1.64(1H, m), 1.16-1.10 (1H, m), 0.94-0.66(3H, m), 0.45-0.19(2H, m), 0.10-0.01(2H, m) | brown candy-like | |
| 1-34 | 7.47(t, 0.5H, 7.6Hz), 7.46(t, 0.5H, 7.6Hz), 7.40(d, 0.5H, 7.6Hz), 7.39(d, 0.5H, 7.6Hz), 7.33(1H, m), 7.27-7.23(1H, m), 7.07(1H, t, 54Hz), 6.59(1H, s), 3.76 (3H, s), 2.60-2.42(2H, m), 1.61-1.53(1H, m), 1.50-1.37(2H, m), 0.88(3H, d, 6.6Hz), 0.87(3H, d, 6.6Hz) | brown candy-like | - |
| 1-35 | 7.56-7.10(4H, m), 7, .08(0.6H, t, 54Hz), 7.06(0.4H, t, 54Hz), 6.50(0.4H, s), 6.50(0.6H, s), 3.77(1.2H, s), 3.74(1.8H, s), 2.94-2.78(1H, m), 1.58-1.23(3H, m), 1.10(1.8H, d, 6.7Hz), 0.94(1.2H, d, 6.7Hz), 0.83(1.8H, d, 6.2Hz), 0.77 (1.2H, d, 6.2Hz), 0.74(1.8H, d, 6.4Hz), 0.72(1.2H, d, 6.4Hz) | white solid | 75.9-79.4 |
| 1-61 | 7.60-7.52(2H, m), 7.47-7.40(2H, m), 7.37-7.32(3H, m), 7.12-7.07(2H, m), 6.74(1H, s), 6.46(1H, t, 54Hz), 3.79(3H, s) | white solid | 120.9-125.9 |
| 1-65 | 7.61-7.55(2H, m), 7.50-7.46(1H, m), 7.38-7.34(1H, m), 6.90-6.88(1H, m), 6.80-6.73(2H, m), 6.74(1H, t, 54Hz), 3.82(3H, s) | brown candy-like | - |
| 1-122 | 7.26-7.18(1H, m), 7.07(1H, t, 54Hz), 6.97(1H, d, 8.2Hz), 6.83(1H, d, 8.3Hz), 6.78(1H, s), 4.39-4.32(1H, m), 3.78(3H, s), 2.11(3H, s), 1.82-1.58(1H, m), 1.32(3H, t, 6.2Hz), 1.25(3H, t, 7.3Hz), 0.98(1.5H, t, 7.6Hz), 0.97(1.5H, t, 7.6Hz) | brown candy-like | - |
| 1-131 | 7.47-7.41(1H, m), 7.40-7.35(1H, m), 7.28-7.13(2H, m), 3.38(1.4H, d, 1.4Hz), 3.63(1.6H, d, 1.4Hz), 2.92-2.82(1H, m), 2.41(1.4H, s), 2.38(1.6H, s), 1.65-1.22(3H, m), 1.10(1.4H, d, 6.9Hz), 1.03(1.6H, d, 6.9Hz), 0.85-0.79(3H, m), 0.75(1.6H, d, 6.4Hz), 0.66(1.4H, d, 6.4Hz) | brown candy-like | - |
| 1-136 | 7.54-7.45(1H, m), 7.44-7.37(1H, m), 7.35-7.30(1H, m), 7.28-6.91(4H, m), 3.79(1.1H, s), 3.74(1.9H, s), 2.89-2.80(1H, m), 2.54(1.9H, s), 2.53(1.1H, s), 1.60-1.15(3H, m), 1.10(1.1H, d, 6.9Hz), 0.92(1.9H, d, 6.9Hz), 0.90-0.75 (4.9H, m), 0.74-0.69(1.1H, d, 6.4Hz) | brown candy-like | - |
| 1-140 | 7.55-7.46(1H, m), 7.44-7.38(1H, m), 7.35-7.24(2H, m), 7.24-6.91(2H, m), 3.77(1.1H, s), 3.74(1.9H, s), 2.98-2.82(1H, m), 2.49(1.9H, s), 2,48(1.1H, s), 1.62-1.58(1H, m), 1.60(3H, m), 1.58-1.54(1H, m), 1.56(3H, m), 1.54-1.40 (1H, m), 1.48-1.32(3H, m) | brown candy-like | - |
| 1-145 | 7.47(1H, m), 7.41-7.37(1H, m), 7.35-7.30(1H, m), 7.24-7.20(1H, m), 6.98 (1H, t, 54Hz), 7.03(1H, s), 3.85(3H, s), 2.91(3H, s), 2.50-2.44(2H, m), 1.65-1.48(3H, m), 0.86(6H, d, 6.4Hz) | brown candy-like | - |
| 1-146 | 7.56-7.49(1H, m), 7.45-7.38(1H, m), 7.37-7.29(1H, m), 7.25-7.20(1 H, m), 7.15-6.80(2H, m),3.88(1.5H, s), 3.82(1.5H, s), 2.91(1.5H, s), 2.90(1.5H, s), 2.86-2.72(1H, m), 1.47-1.36(1H, m), 1.29-1.21(2H, m), 0.98(3H, d, 6.9Hz), 0.82-0.78(3H, m), 0.73-0.69(3H, m) | white solid | 130.4-135.5 |

[Table 29-2]

| No. | $^1$HNMR Spectrum ($CDCl_3$) σ ppm: | Shape | Melting Point (°C) |
|---|---|---|---|
| 1-158 | 7.52-7.45(1H, m) 7.42-7.37(1H, m) 7.29-7.24(1H, m) 7.13(1H, t, 8.8Hz) 3.71(3H, s) 2.95(3H, s), 2.92-2.79(1H, m) 2.43(3H, s) 1.61-1.47(1H, m) 1.39-1.12(2H, m), 1.04(1.5H, d, 6.9Hz), 1.01(1.5H, d, 6.9Hz), 0.80(1.5H, d, 6.4Hz), 0.77(1.5H, 6.4Hz), 0.71(1.5H, d, 6.2Hz), 0.64(1.5H, d, 6.2Hz) | brown candy-like | - |

(continued)

| No. | $^1$HNMR Spectrum ($CDCl_3$) σ ppm: | Shape | Melting Point (°C) |
|---|---|---|---|
| 1-237 | 7.59-7.51(1H, m), 7.46-7.30(3H, m), 7.32(0.4H, t, 53.2Hz), 7.30(0.6H, t, 53.2Hz), 6.96(0.6H, t, 54.1Hz), 6.94(0.4H, t, 53.1Hz), 6.52(0.4H, s), 6.47 (0.6H, s), 3.77(1.2H, s), 3.73(1.8H, s), 2.82-2.72(1H, m), 1.50-1.28(3H, m), 1.05(1.2H, d, 6.9Hz), 0.84(1.8H, d, 6.9Hz), 0.83(1.8H, d, 6.4Hz), 0.78(1.8H, d, 6.9Hz), 0.72(1.2H, d, 6.4Hz), 0.67(1.2H, d, 6.9Hz) | brown candy-like | |
| 1-258 | 7.56-7.47(1H, m), 7.43-7.39(1.5H, m), 7.32-7.22(2H, m), 7.15(0.5H, d, 7.8Hz), 3.70(1.5H, s), 3.66(1.5H, s), 2.86-2.79(1H, m), 2.41(1.5H, s), 2.39 (1.5H, s), 1.59-1.17(3H, m), 1.00(3H, d, 6.9Hz), 0.77(1.5H, d, 6.4Hz), 0.76 (1.5H, d, 6.4Hz), 0.72(1.5H, d, 6.4Hz), 0.61(1.5H, d, 6.4Hz) | brown candy-like | - |
| 1-266 | 7.35-7.33(4H, m), 7.19-7.14(1H, m), 7.00-6.94(1H, m), 3.67-3.64(3H, m), 2.94-2.87(1H, m), 2.41-2.39(3H, m), 1.67-1.61(1H, m), 1.39-1.35(1H, m), 1.28-1.24(1H, m), 1.20(1.5H, d, 6.9Hz), 1.04(1.5H, d, 6.9Hz), 0.95(1.5H, d, 6.6Hz), 0.87(1.5H, d, 6.6Hz), 0.84(1.5H, d, 6.4Hz), 0.63(1.5H, d, 6.4Hz) | white solid | 66.6-68.9 |
| 1-279 | 8.73(0.5H, d, 8.2Hz), 8.69(0.5H, d, 8.2Hz), 7.71(0.5H, d, 7.3Hz), 7.55(0.5H, d, 7.3Hz), 7.54-7.44(1H, m), 7.41-7.30(2H, m), 7.19-7.05(1H, m), 7.06-6.98 (1H, m), 3.58(1.5H, s), 3.58(1.5H, s), 3.02-2.86(1H, m), 2.43(1.5H, s), 2.41 (1.5H, s), 1.64-1.48(2H, m), 1.44-1.32(1H, m), 1.10(1.5H, d, 6.9Hz), 0.88 (1.5H, d, 6.9Hz), 0.84(1.5H, d, 6.4Hz), 0.81(1.5H, d, 6.4Hz), 0.67(1.5H, d, 6.4Hz), 0.62(1.5H, d, 6.4Hz) | white solid | 136.3-140.8 |
| 1-292 | 7.44-7.33(2.3H, m), 7.25-7.18(1.6H, m), 7.12-7.06(1.1H, m), 3.68(1.5H, s), 3.66(1.5H, s), 2.94(2H, q, 7.6Hz), 2.92-2.82(1H, m), 2.41(3H, s), 1.67-1.50 (2H, m), 1.41-1.32(1H, m), 1.30(1.5H, q, 7.6Hz), 1.27(1.5H, q, 7.6Hz), 1.09 (1.5H, d, 6.7Hz), 1.05(1.5H, d, 6.7Hz), 0.86(1.5H, d, 6.7), 0.82(1.5H, d, 6.7Hz), 0.78(1.5H, d, 6.6Hz), 0.63(1.5H, d, 6.6Hz) | brown candy-like | - |
| 1-300 | 7.54-7.45(1H, m), 7.44-7.20(3H, m), 7.19-6.88(1H, m),6.44-6.39(1H, m), 3.75(1H, s), 3.71(2H, s), 2.86-2.75(1H, m), 2.77(2H, s), 2.75(1H, s), 1.65-1.32(3H, m), 1.19-1.09(1H, m), 0.88-0.79(6H, m), 0.75-0.68(2H, m), | brown candy-like | - |
| 1-307 | 7.37-7.28(2H, m), 7.21-7.09(2H, m), 3.66(1.5H, s), 3.57(1.5H, s), 2.94-2.78 (1H, m), 2.68(1.5H, s,) 2.62(1.5H, s) 2.39(1.5H, s) 2.33(1.5H, s), 1.66-1.31 (3H, m) 1.15(1.5H, d, 6.6Hz), 0.95(1.5H, d, 6.6Hz), 0.91(1.5H, d, 6.6Hz), 0.84(1.5H, d, 6.6Hz), 0.80(1.5H, d, 6.4Hz), 0.59(1.5H, d, 6.4Hz) | brown candy-like | - |
| 1-313 | 7.45-7.39(1H, m), 7.35-7.34(1H, m), 7.32-7.26(1H, m), 7.24-7.19(1H, m), 7.09(1H, t, 54Hz), 7.04(1H, t, 54Hz), 7.00(1H, s), 3.82(3H, s), 2.69(3H, s), 2.53-2.45(2H, m), 1.61-1.49(1H, m), 1.46-1.38(2H, m), 0.86(6H, d, 6.4Hz) | white solid | 94.2-97.6 |

[Table 29-3]

| No. | $^1$HNMR Spectrum ($CDCl_3$) σ ppm: | Shape | Melting Point (°C) |
|---|---|---|---|
| 1-314 | 7.51-7.44(1H, m), 7.42-7.35(1H, m), 7.35-6.86(5H, m), 3.85(1.5H, s), 3.78 (1.5H, s), 2.89-2.78(1H, m), 2.73(1.5H, s), 2.69(1.5H, s), 1.59-1.48(1H, m), 1.47-1.36(1H, m), 1.35-1.20(1H, m), 1.03(1.5H, d, 6.9Hz), 0.94(1.5H, d, 6.9Hz), 0.83(1.5H, d, 6.6Hz), 0.79(1.5H, d, 6.6Hz), 0.74(1.5H, d, 6.4Hz), 0.71(1.5H, d, 6.4Hz) | brown candy-like | - |
| 1-325 | 7.44-7.35(2H, m), 7.24-7.19(2H, m), 7.11-7.07(1H, m), 3.68-3.66(3H, m), 2.91-2.83(1H, m), 2.63-2.62(3H, m), 2.41(3H, s), 1.40-1.28(3H, m), 1.10-1.04(3H, m), 0.87-0.77(4H, m), 0.64-0.62(2H, m) | brown candy-like | - |

(continued)

| No. | $^1$HNMR Spectrum ($CDCl_3$) σ ppm: | Shape | Melting Point (°C) |
|---|---|---|---|
| 1-333 | 8.87(0.6H, s), 8.84(0.4H, s), 7.55-6.87(5H, m), 6.41(0.6H, s), 6.33(0.4H, s), 3.72(1H, s), 3.69(2H, s), 2.88-2.77(1H, m), 1.57-1.20(3H, m), 1.13-1.08 (1H, m), 0.91-0.78(6H, m), 0.75-0.65(2H, m), | brown candy-like | - |
| 1-336 | 8.03(0.5H, s), 8.72(0.5H, s), 7.38-7.28(2H, m), 7.23- 7.08(2H, m), 3.68 (1.5H, s), 3.59(1.5H, s), 2.97-2.79(1H, m), 2.42(1.5H, s), 2.35(1.5H, s), 1.68-1.55(1H, m), 1.54-1.19(2H, m), 1.16(1.5H, d, 6.7Hz), 0.96(1.5H, 6.7Hz), 0.93(1.5H, d, 6.4Hz), 0.86(1.5H, d, 6.4Hz), 0.81(1.5H, d, 6.9Hz), 0.60(1.5H, d, 6.9Hz) | brown candy-like | - |
| 1-341 | 8.86(0.6H, s) 8.83(0.4H, s), 7.52-7.46(1H, m), 7.42-7.37(1H, m), 7.35-7.30 (1H, m), 7.19-7.01(1H, m), 7.06-6.70(3H, m), 3.84(1.2H, s), 3.78(1.8H, s), 2.91-2.78 (1H, m), 1.47-1.16 (3H, m), 1.03(1.2H, d, 6.9Hz), 0.92(1.8H, d, 6.9Hz), 0.84(1.8H, d, 6.7Hz), 079(1.2H, d, 6.7Hz), 0.76(1.8H, d, 6.6Hz), 0.70(1.2H, d, 6.6Hz) | brown candy-like | |
| 1-346 | 8.77(0.4H, s), 8.74(0.6H, s), 7.60-7.34(2.4H, m), 7.31-7.27(0.6H), 7.24-7.18(1H, m), 7.16-7.08(1H, m), 3.69(1.7H, s), 3.67(1.3H, s), 2.93-2.82 (1H, m), 2.42(2H, s) 2.41(1H, s), 1.66-1.50(1H, m), 1.41-1.23(2H, m), 1.07 (1.7H, d, 6.9Hz), 1.04(1.3H, d, 6.9Hz), 0.86(1.3H, d, 6.4Hz), 0.82(1.7H, d, 6.4Hz), 0.77(1.3H, d, 6.6Hz), 0.63(1.7H, d, 6.6Hz) | brown candy-like | |
| 1-349 | 8.73(0.4H, d, 5.0Hz), 8.72(0.6H, d, 5.0Hz), 7.87(0.6H, d, 8.0Hz), 7.80(0.4H, d, 8.0Hz), 7.56-7.44(3H, m), 7.42-7.36(2H, m), 6.97(1H, t, 54Hz), 6.83 (0.6H, d, 54Hz), 6.81(0.4H, d, 54Hz), 6.31(0.6H, s), 6.03(0.4H, s), 3.69(3H, s), 2.94-2.76(1H, m), 1.66-1.37(3H, m), 1.17(1.2H, d, 6.4Hz), 0.90(1.8H, d, 6.4Hz), 0.85(1.8H, d, 6.9Hz), 0.83(1.2H, d, 6.9Hz), 0.71(1.2H, d, 6.9Hz), 0.70(1.8H, d, 6.9Hz) | white solid | 143.5-147.9 |
| 1-354 | 7.51-7.41(2H, m), 7.33-7.17(2H, m), 6.87(1H, t, 54Hz), 6.82(1H, t, 54Hz), 6.44(0.7H, s), 6.3(0.3H, s), 3.88(3H, s), 3.69(3H, s), 2.95-2.78(1H, m), 1.61-1.31(3H, m), 1.13(1H, d, 6.6Hz), 0.89(2H, d, 6.6Hz), 0.84(2H, d, 6.4Hz), 0.82(1H, d, 6.4Hz), 0.75(2H, d, 5.7Hz), 0.71(1H, d, 5.7Hz) | brown candy-like | - |
| 1-359 | 7.53-7.46(1H, m), 7.43-7.38(1H, m), 7.34-7.26(1.5H, m), 7.22-7.18(1.5H, m), 7.16-6.87(2H, m), 3.90(1.5H, s), 3.87(1.5H, s), 2.92-2.82(1H, m), 1.62-1.36(2H, m), 1.33-1.15(1H, m), 1.04(1.5H, d, 6.9Hz), 1.03(1.5H, d, 6.9Hz), 0.80(3H, d, 6.4Hz), 0.72(1.5H, d, 6.6Hz), 0.68(1.5H, d, 6.6Hz) | brown candy-like | - |

[Table 29-4]

| No. | $^1$HNMR Spectrum ($CDCl_3$) σ ppm: | Shape | Melting Point (°C) |
|---|---|---|---|
| 1-362 | 7.50-7.45(1H, m), 7.43-7.38(1H, m), 7.28-7.23(1H, m), 7.17-7.13(1H, m), 6.88(0.5H,s), 6.85(0.5H, s), 3.68(3H, s), 2.96-2.86(1H, m), 2.42(1.5H, s), 2.40(1.5H, s), 1.60-1.48(1H, m), 1.42-1.18(2H, m), 1.09(1.5H, d, 4.6Hz), 1.07(1.5H, d, 4.6Hz), 0.81(1.5H, d, 6.4Hz), 0.80(1.5H, d. 6.4Hz), 0.77 (1.5H, d, 6.9Hz), 0.63(1.5H, d, 6.9Hz) | brown candy-like | - |
| 1-368 | 7.62-7.55(2H, m) 7.47(1H, m) 7.43(1H, s) 7.30(1H, m) 6.80-6.70(2H, m), 6.76(1H, t, 54Hz), 3.91(3H, s) 2.58(3H, s) | white solid | 201.6-202.5 |
| 1-369 | 8.73(1H, d, 8.2Hz), 7.68(1H, d, 7.3Hz), 7.61-7.48(4H, m), 7.32-7.25(2H, m), 6.75(1H, t, 54Hz), 6.72-6.64(2H, m), 3.83(3H, s) | white solid | 168.5-176.9 |

(continued)

| No. | $^1$HNMR Spectrum ($CDCl_3$) σ ppm: | Shape | Melting Point (°C) |
|---|---|---|---|
| 1-370 | 8.81(1H, s), 7.62-7.52(2H, m), 7.51-7.47(1H, m), 7.34-7.31(1H, m), 7.19 (1H, s), 6.83-6.79(2H, m), 6.78(1H, t, 54Hz), 6.73(1H, t, 54Hz), 3.85 (3H, s). | white solid | 173.0-176.0 |
| 1-371 | 8.65(1H, s), 7.67-7.55(3H, m), 7.33-7.37(1H, m), 6.79-6.69(3H, m), 6.64 (1H, t, 54Hz), 3.78(3H, s) | white solid | 223.5-224.1 |
| 1-372 | 7.58-7.52(2H, m), 7.46-7.42(1H, m), 7.35-7.30(1H, m), 6.85-6.80(3H, m), 6.77(1H, t, 54Hz), 6.71(1H, t, 54H), 3.86(3H, s), 2.68(3H, s) | white solid | 190-195 |
| 1-373 | 8.58(1H, d, 8.2Hz), 7.69(1H, d, 7.3Hz), 7.61-7.48(4H, m), 7.32-7.25(1H, m), 6.75(1H, t, 54Hz), 6.70-6.65(1H, m), 3.83(3H, s) | white solid | 170.0-179.1 |
| 1-374 | 7.61-7.54(2H, m), 7.45-7.35(2H, m), 6.92-6.61(4H, m), 3.82(3H, s) | white solid | 160.4-163.1 |
| 1-375 | 7.60-7.57(2H, m),7.44-7.42(1H, m), 7.37-7.35(1H, m), 7.16(2H, s), 6.89 (1H, s), 6.79-6.75(2H, m), 6.72(1H, t, 54Hz), 3.84(3H, s). | white solid | 187.9-189.8 |
| 1-376 | 7.48-7.36(2H, m), 7.25-7.19(1H, m), 7.12-7.06(1H, m), 6.40(0.5H, s), 6.27 (0.5H, s), 3.69(1.5H, s), 3.67(1.5H, s), 2.97-2.85(1H, m), 2.42(3H, s), 1.62-1.53(1H, m), 1.51-1.38(1H, m), 1.37-1.29(1H, m), 1.13(1.5H, d, 6.7Hz), 1.08(1.5H, d, 6.7Hz), 0.86-0.79(3H, m). 0.77(1.5H, d, 6.4Hz), 0.65 (1.5H, 6.4Hz) | brown candy-like | - |
| 1-377 | 7.50-7.35(2H, m), 7.20-7.12(2H, m), 3.74(1.7H, s), 3.70(1.3H, s), 2.91-2.80(1H, m), 2.44(1.7H, s). 2.38(1.3H, s), 2.36(1.7H, s), 2.32(1.3H, s), 1.68-1.20(3H, m), 1.10-1.04(3H, m), 0.86-0.80(3H, m), 0.77-0.68(3H, m) | brown candy-like | - |
| 1-378 | 7.49-7.42(1H, m), 7.41-7.36(1H, m), 7.29-7.22(1H, m), 7.19-7.15(1H, m), 3.79(1.8H, s), 3.78(1.2H, s), 2.97-2.82(1H, s), 2.41(1.8H, s), 2.39(1.2H, s), 1.64-1.33(3H, m), 1.08(1.2H, d, 6.7Hz), 1.07(1.8H, d, 6.7Hz), 0.81 (1.8H, d, 6.4Hz), 0.80(1.2H, d, 6.4Hz), 0.76(1.2H, d, 6.6Hz), 0.66(1.8H, d, 6.6Hz) | brown candy-like | - |
| 1-379 | 7.56-7.46(1H, m), 7.45-7.36(1H, m), 7.35-7.20(2H, m), 6.84(0.3H, t, 54Hz), 6.83(0.7H, t, 54Hz), 3.93(3H, s), 2.92-2.77(1H, m), 1.64-1.46(2H, m), 1.23-1.13(1H, m), 1.11(1H, d, 6.4Hz), 1.01(2H, d, 6.4Hz), 0.85(2H, d, 6.4Hz), 0.79(1H, d, 6.4Hz), 0.74(1H, d, 6.4Hz), 0.73(2H, d, 6.4Hz) | brown candy-like | - |
| 5-1 | 7.30(1H, d, 5.5Hz), 7.20(1H, s), 6.86(1H, d, 5.5Hz), 3.88(3H, s), 2.99-2.92 (1H, m), 1.61-1.20(3H, m), 1.02(3H, d, 6.9Hz), 0.81(3H, d, 6.4Hz), 0.78 (3H, d, 6.4Hz) | brown candy-like | - |

[Table 29-5]

| No. | $^1$HNMR Spectrum ($CDCl_3$) σ ppm: | Shape | Melting Point (°C) |
|---|---|---|---|
| 5-5 | 7.30(1H, d, 5.7Hz), 7.05(1H, t, 54Hz), 6.89(1H, d, 5.7Hz), 6.82(1H, s), 3.84(3H, s), 3.01-2.93(1H, m), 1.64-1.32(3H, m), 1.03(3H, d, 6.9Hz), 0.77(6H, d, 6.4Hz) | brown candy-like | - |
| 5-19 | 7.33(1H, d, 6.4Hz), 7.17(1H, d, 6.4Hz), 6.82(1H, d, 5.5Hz), 3.96(3H, s), 2.95-2.85 (1H, m), 2.90(3H, s), 1.54-1.22(3H, m), 1.02(3H, d, 6.9Hz), 0.79(3H, d, 6.4Hz), 0.74(3H, d, 6.4Hz) | white solid | 121.1-125.4 |
| 5-23 | 7.32(1H, d, 5.3Hz), 7.18(1H, s), 6.94(1H, t, 54Hz), 6.84(1H, d, 5.3Hz), 3.91(3H, s), 2.97-2.83(1H, m), 2.90(3H, s), 1.53-1.22(3H, m), 1.03(3H, d, 6.9Hz), 0.77(3H, d, 6.4Hz), 0.74(3H, d, 6.4Hz) | brown candy-like | - |

(continued)

| No. | $^1$HNMR Spectrum ($CDCl_3$) σ ppm: | Shape | Melting Point (°C) |
|---|---|---|---|
| 7-37 | 7.47(1H, t, 7.3Hz), 7.42-7.38(1H, m), 7.33-7.25(1H, m), 7.21-7.17(1H, m), 7.14 (1H, t, 54Hz), 2.66(3H, s), 2.57-2.51(2H, m), 1.66-1.53(1H, m), 1.52-1.44(2H, m), 0.90(3H, d, 6.4Hz), 0.89(3H, d, 6.4Hz) | brown candy-like | - |
| 7-38 | 7.56-7.48(1H, m), 7.46-7.39(1H, m), 7.34-7.26(1H, m), 7.24-7.18(1H, m), 7.16 (0.6H, t, 54Hz), 7.14(0.4H, t, 54Hz), 2.68(1.1H, s), 2.65(1.9H, s), 2.89-2.75(1H, m), 1.65-1.50(1H, m), 1.43-1.35(1H, m), 1.33-1.20(1H, m), 1.08(1H, d, 6.8Hz), 1.05(2H, d, 6.8Hz) ,0.92-0.80(3H, m), 0.77(1H, d, 6.4Hz), 0.70(2H, d, 6.4Hz) | brown candy-like | - |
| 7-68 | 7.75(0.5H, d, 1.4Hz), 7.73(0.5H, d, 1.4Hz), 7.45-7.29(3H, m), 7.26(2H, s), 7.10 (1H, t, 54Hz), 2.68(3H, s) | brown candy-like | - |
| 7-125 | 7.23-7.17(1H, m), 7.14(1H, t, 54Hz), 6.96(1H, d, 8.2Hz), 6.76(1H, d, 7.8Hz), 4.44-4.30(1H, m), 2.66(3H, s), 2.12(3H, s), 1.83-1.58(2H, m), 1.33(1.5H, d, 6.0Hz), 1.31(1.5H, d, 6.0Hz), 0.99(1.5H, t, 7.6Hz), 0.97(1.5H, t, 7.6Hz) | brown candy-like | - |
| 7-129 | 7.56(2H, s), 7.11(1H, t, 54Hz), 2.73(3H, s) | brown candy-like | - |
| 7-132 | 7.55-7.47(1H, m), 7.46-7.38(1H, m), 7.34-7.26(1H, m), 7.23-7.18(1H, m), 7.15 (0.7H, t, 54Hz), 7.14(0.3H, t, 54Hz), 3.04-2.93(2H, m), 2.91-2.76(1H, m), 1.68-1.50(2H, m), 1.44-1.22(1H, m), 1.33(1.2H, t, 7.6Hz), 1.30(1.8H, t, 7.6Hz), 1.08(1.2H, d, 6.6Hz), 1.05(1.8H, d, 6.6Hz), 0.84(1.2H, d, 6.4Hz), 0.83(1.8H, d, 6.4Hz), 0.78(1.2H, d, 6.4Hz), 0.70(1.8H, d, 6.4Hz) | brown candy-like | - |
| 7-134 | 7.51-7.45(1H, m), 7.41-7.36(1H, m), 7.31-6.90(4H, m), 2.90-2.75(1H, m), 2.67 (1.1H, s), 2.63(1.9H, s), 2.53(1.9H, s), 2.51(1.1H, s), 1.63-1.46(1H, m), 1.42-1.15 (1H, m), 1.07(1.1H, d, 6.4Hz), 1.01(1.9H, d, 6.4Hz), 0.90-0.80(4H, m), 0.76(1.1H, d, 6.4Hz), 0.71(1.9H, d, 6.4Hz) | brown candy-like | - |
| 7-141 | 7.52-7.44(1H, m), 7.42-7.36(1H, m), 7.31-7.26(1H, m), 7.28-6.98(1H, m), 7.22-7.17(1H, m), 2.89-2.79(1H, m), 2.66(1.1H, s), 2.63(1.9H, s), 2.48(1.9H, s) 2.47(1.1H, s), 1.63-1.46(1H, m), 1.42-1.20(2H, m), 1.06(1.1H, d, 6.9Hz), 1.04 (1.9H, d, 6.9Hz), 0.90-0.80(3H, m), 0.76(1.1H, d, 6.4Hz), 0.68(1.9H, d, 6.4Hz) | brown candy-like | - |
| 7-151 | 7.52-7.46(1H, m), 7.42-7.37(1H, m), 7.36-7.30(1H, m), 7.22-7.17(1H, m), 7.05 (1H, t, 54Hz), 2.91(3H, s), 2.73(3H, s), 2.56-2.42(2H, m), 1.61-1.52(1H, m), 1.50-1.372H, m), 0.88(6H, d, 6.9Hz) | brown candy-like | - |

[Table 29-6]

| No. | $^1$HNMR Spectrum ($CDCl_3$) σ ppm: | Shape | Melting Point (°C) |
|---|---|---|---|
| 7-152 | 7.59-7.51(1H, m), 7.46-7.38(1H, m), 7.37-7.31(1H, m), 7.25-7.19(1H, m), 7.07(0.5H, t, 54Hz), 7.00(0.5H, t, 54Hz), 2.91(3H, s), 2.82-2.68(1H, m), 2.76(1.5H, s), 2.73(1.5H, s), 1.47-1.38(1H, m), 1.35-1.10(2H, m), 1.06 (1.5H, d, 6.9Hz), 0.95(1.5H, d, 6.9Hz), 0.84(1.5H, d, 6.9Hz), 0.78(1.5H, d, 6.9Hz), 0.76(1.5H, d, 6.4Hz), 0.69(1.5H, d, 6.4Hz) | white solid | 100.1-103.1 |
| 7-176 | 7.59-7.53(1H, m), 7.46-7.29(3H, m), 7.33(0.4H, t, 54Hz), 7.32(0.6H, t, 54Hz), 7.03(0.6H, t, 54Hz), 7.00(0.4H, t, 54Hz), 2.77-2.70(1H, m), 2.68 (1.2H, s), 2.63(1.8H, s), 1.52-1.12(3H, m), 1.02(1.2H, d, 6.9Hz), 0.91(1.8H, d, 6.9Hz), 0.83(1.8H, d, 6.7Hz), 0.78(3H, d, 6.4Hz), 0.72(1.2H, d, 6.7Hz) | brown candy-like | - |

(continued)

| No. | $^1$HNMR Spectrum ($CDCl_3$) σ ppm: | Shape | Melting Point (°C) |
|---|---|---|---|
| 7-188 | 7.52-7.49(1H, m), 7.43-7.41(3H, m), 7.33-7.25(2H, m), 7.11(1H, t, 54Hz), 2.81-2.78(1H, m), 2.68(1.2H, s), 2.66(1.8H, s), 1.63-1.41(1H, m), 1.35-1.20(2H, m), 1.11(1.2H, d, 6.6Hz), -1.03(1.8H, d, 6.6Hz), 0.88-0.86 (3H, m), 0.80-0.75(3H, m). | brown candy-like | - |
| 7-200 | 8.84(0.5H, d, 8.2Hz), 8.81(0.5H, d, 8.2Hz), 7.73-7.61(1H, m), 7.52-7.36 (2H, m), 7.31-7.14(3H, m), 7.16(0.5H, t, 54Hz), 7.13(0.5H, t, 54Hz), 2.95-2.85(1H, m), 2.69(1.5H, s), 2.67(1.5H, s), 1.62-1.38(3H, m), 1.10 (1.5H, d, 6.9Hz), 0.87(1.5H, d, 6.9Hz), 0.86(1.5H, d, 6.6Hz), 0.85(1.5H, d, 6.6Hz), 0.75(1.5H, d, 6.0Hz), 0.47(1.5H, d, 6.0Hz) | brown candy-like | |
| 7-216 | 7.52-7.45(m, 1H), 7.41-7.36(1H, m), 7.32-7.25(1H, m), 7.22-6.90(3H, m), 3.04(q, 1H, 7.6Hz), 3.00(1H, q, 7.6Hz), 2.85-2.76(1H, m), 2.72(1.5H, s), 2.68(1.5H, s), 1.62-1.41(2H, m), 1.38(1.5H, t, 7.6Hz), 1.33(1.5H, t, 7.6Hz), 1.31-1.19(1H, m), 1.03(1.5H, d, 6.4Hz), 1.02(1.5H, d, 6.4Hz), 0.84(3H, d, 6.4Hz), 0.73(3H, d, 6.4Hz) | brown candy-like | - |
| 7-226 | 7.52-7.46(1H, m), 7.41-6.96(4H, m), 2.85-2.76(1H, m), 2.78(2H, s), 2.74 (1H, s), 2.66(1H, s). 2.61(2H, s), 1.62-1.42(1H, m), 1.39-1,35(1H, m), 1.33-1.21(1H, m), 1.09(1H, d, 6.4Hz), 0.93-0.84(4H, m), 0.83-0.77(3H, m), 0.76-0.72(1H, m) | brown candy-like | - |
| 7-237 | 7.47-7.46(1H, m), 7.38-7.34(1H, m), 7.32-7.17(2H, m), 7.10(2H, t, 54Hz), 2.70(6H, s), 2.57-2.47(2H, m), 1.61-1.52(1H, m), 1.49-1.42(2H, m), 0.88 (6H, d, 6.4Hz) | brown candy-like | - |
| 7-238 | 7.52-7.40(1H, m), 7.42-7.37(1H, m), 7.32-7.25(1H, m), 7.23-6.92(3H, m), 2.86-2.75(1H, m), 2.71(3H, s), 2.66(3H, s), 1.62-1.52(1H, m), 1.50-1.40 (1H, m), 1.33-1.21(1H, m), 1.03(3H, d, 6.4Hz), 0.84(3H, d, 6.4Hz), 0.74 (3H, d, 6.8Hz) | brown candy-like | - |
| 7-250 | 8.92(0.6H, s), 8.87(0.4H, s), 7.54-7.48(1H, m), 7.42-6.95(4H, m), 2.84-2.71(1H, m), 2.65(1H, s), 2.61(2H, s), 1.71-1.40(3H, m), 1.16-1.08 (1H, m), 0.92-0.78(7H, m), 0.74(1H, d, 6.4Hz), | brown candy-like | - |
| 7-262 | 8..88(0.5H, s), 8.83(0.5H, s), 7.53-7.47(1H, m), 7.41-7.37(1H, m), 7.35-7.28(1H, m), 7.25-6.90(3H, m), 2.85-2.77(1H, m), 2.72(1.5H, s), 2.67 (1.5H, s), 1.61-1.54(1H, m), 1.58-1.39(1H, m), 1.34-1.20(1H, m), 1.01 (1.5H, d, 6.2Hz), 0.99(1.5H, d, 6.2Hz), 0.85(1.5H, d, 6.6Hz), 0.83(1.5H, d, 6.6Hz), 0.76(1.5H, d, 6.4Hz), 0.74(1.5H, d, 6.4Hz) | brown candy-like | - |

[Table 29-7]

| No. | $^1$HNMR Spectrum ($CDCl_3$) σ ppm: | Shape | Melting Point (°C) |
|---|---|---|---|
| 7-270 | 8.75(0.4H, d, 4.8Hz), 8.74(0.6H, d, 4.8Hz), 7.89(0.6H, d, 7.9Hz), 7.83(0.4H, d, 7.9Hz), 7.66-7.26(5H, m), 7.05(0.4H, d, 54Hz), 7.03(0.6H, d, 54Hz), 6.86 (0.4H, d, 54Hz), 6.81(0.6H, d, 54Hz), 2.84-2.76(1H, m), 2.60(1.2H, s), 2.58 (1.8H, s), 1.62-1.34(3H, m), 1.16(1.2H, d, 6.7Hz), 0.91(1.8H, d, 6.7Hz), 0.88(1.2H, d, 6.4Hz), 0.85(1.8H, d, 6.4Hz), 0.78(1.2H, d, 6.7Hz), 0.73(1.8H, d, 6.7Hz) | brown candy-like | - |
| 7-272 | 7.51-7.34(2H, m), 7.30-6.70(4H, m), 3.91(3H, s), 2.92-2.74(1H, m), 2.62 (1H, s), 2.60(2H, s), 1.68-1.52(1.5H, m), 1.38-1.32(1.5H, m), 1.11(1H, d, 6.7Hz), 0.98(2H, d, 6.7Hz), 0.86-0.82(3H, m), 0.77(1H, d, 6.4Hz), 0.70(2H, d, 6.4Hz) | brown candy-like | - |

(continued)

| No. | $^1$HNMR Spectrum ($CDCl_3$) σ ppm: | Shape | Melting Point (°C) |
|---|---|---|---|
| 7-274 | 7.54-7.48(1H, m), 7.45-7.40(1H, m), 7.36-7.27(1H, m), 7.2-7.15(1H, m), 7.10-6.88(2H, m), 2.91-2.80(1H, m), 2.75(1.5H, s), 2.73(1.5H, s), 1.60-1.32 (2H, m), 1.29-1.17(1H, m), 1.09(1.5H, d, 6.9Hz), 1.02(1.5H, d, 6.9Hz), 0.84 (1.5H, d, 6.4Hz), 0.81(1.5H, d, 6.4Hz), 0.74(1.5H, d, 6.4Hz), 0.73(1.5H, d, 6.4Hz) | brown candy-like | - |
| 7-277 | 7.54-7.47(1H, m), 7.46-7.38(1H, m), 7.32-6.90(3H, m), 2.89-2.78(1H, m), 2.66(1H, s), 2.64(2H, s), 1.57-1.20(3H, m), 1.10(1H, d, 6.9Hz), 1.06(2H, d, 6.9Hz), 0.84(3H, d, 6.9Hz), 0.78(1H, d, 6.4Hz), 0.70(2H, d, 6.4Hz) | brown candy-like | - |
| 7-278 | 7.54-7.47(1H, m), 7.46-7.39(1H, m), 7.32-7.28(1H, m), 7.18-6.99(2H, m), 6.81(0.6H, s), 6.92(0.4H, s), 2.90-2.81(1H, m), 2.69(1.2H, s), 2.66(1.8H, s), 1.66-1.35(2H, m), 1.33-1,20(1H, m), 1.12-1,05(3H, m), 0.91-0.80(3H, m), 0.77(1.2H, d, 6.4Hz), 0.68(1.8H, d, 6.9Hz) | brown candy-like | - |
| 7-279 | 7.56(2H, s), 2.76(3H, s) | brown candy-like | - |
| 7-280 | 7.56(2H, s) 2.91(3H, s) 2.79(3H, s) | white solid | 97.0-98.2 |
| 7-281 | 7.58(2H, s), 7.28(1H, t, 54Hz), 2.74(3H, s) | white solid | 96.7-102.7 |
| 7-282 | 8.80(1H, d, 8.2Hz), 7.94(1H, d, 7.3Hz), 7.65-7.58(1H, m). 7.54(2H, s), 2.67 (3H, s) | white solid | 172.5-188.5 |
| 7-283 | 8.87 (1H, s), 7.55 (2H, s), 7.12 (1H, t, 53.4 Hz), 2.78 (3H, s). | white solid | 166.0-167.0 |
| 7-284 | 8.87(1H, s), 7.75(2H, s), 2.72(3H, s) | white solid | 151.0-159.0 |
| 7-285 | 7.56(2H, s), 7.07(1H, t, 54Hz), 2.77(3H, s), 2.70(3H, s) | white solid | 143.0-146.0 |
| 7-286 | 7.54(2H, s) 2.73(6H, s) | brown candy-like | - |
| 7-287 | 7.55(2H, s), 7.08(2H, t, 54Hz), 2.75(6H, s) | white solid | 145.0-150.0 |

[Table 29-8]

| No. | $^1$HNMR Spectrum ($CDCl_3$) σ ppm: | Shape | Melting Point (°C) |
|---|---|---|---|
| 11-8 | 7.30(1H, d, 5.0Hz), 7.14(1H, t, 54Hz), 6.83(1H, d, 5.0Hz), 3.02-2.90(1H, m), 2.71(3H, s), 1.60-1.25(3H, m), 1.27(3H, d, 6.9Hz), 0.82(3H, d, 6.2Hz), 0.78(3H, d, 6.2Hz) | brown candy-like | - |
| 11-20 | 7.33(1H, d, 5.3Hz), 7.15(1H, t, 54Hz), 6.84(1H, d, 5.3Hz), 2.95-2.85(1H, m), 2.91(3H, s), 2.76(3H, s), 1.50-1.20(3H, m), 1.05(3H, d, 6.9Hz), 0.88 (3H, d, 5.6Hz), 0.76(3H, d, 5.6Hz) | brown candy-like | - |
| 13-1 | 8.83(1H, d, 5.0Hz), 7.89(0.5H, d, 6.9Hz), 7.82(0.5H, d, 6.9Hz), 7.66-7.53 (2H, m), 7.48-7.33(3H, m), 2.88-2.78(1H, m), 1.70-1.58(1H, m), 1.57-1.46 (1H, m), 1.45-1.39(1H, m), 1.19(1.5H, d, 6.8Hz), 0.96(1.5H, d, 6.8Hz), 0.92 (1.5H, d, 6.4Hz), 0.89(1.5H, d, 6.4Hz), 0.77(1.5H, d, 6.4Hz), 0.73(1.5H, d, 6.4Hz) | brown candy-like | - |
| 13-2 | 8.75(1H, d, 4.2Hz), 7.89(0.5H, d, 8.1Hz), 7.83(0.5H, d, 8.1Hz) 7.57-7.33 (5H, m), 6.89(0.5H, t, 54Hz), 6.84(0.5H, t, 54Hz), 2.92-2.77(1H, m), 1.69-1.36(3H, m), 1.18(1.5H, d, 6.9Hz), 0.97(1.5H, d, 6.9Hz), 0.92(1.5H, d, 6.4Hz), 0.89(1.5H, d, 6.4Hz, 0.78(1.5H, d, 6.7Hz), 0.72(1.5H, d, 6.7Hz) | white solid | 111.0-115.6 |

(continued)

| No. | $^1$HNMR Spectrum ($CDCl_3$) σ ppm: | Shape | Melting Point (°C) |
|---|---|---|---|
| 13-10 | 8.77(0.5H, d, 4.8Hz), 8.76(0.5H, d, 4.8Hz), 7.89(0.5H, d, 7.8Hz), 7.83(0.5H, d, 7.8Hz), 7.66-7.51(2H, m), 7.49-7.36(3H, m), 6.83(0.5H, t, 54Hz), 6.80 (0.5H, t, 54Hz), 2.81-2.72(1H, m), 2.79(3H, s), 1.64-1.34(3H, m), 1.17(1.5H, d, 6.7Hz), 0.92(1.5H, d, 6.7Hz), 0.87(1.5H, d, 6.9Hz), 0.83(1.5H, d, 6.9Hz), 0.68(1.5H, d, 6.4Hz), 0.64(1.5H, d, 6.4Hz) | brown candy-like | |
| 13-16 | 8.75-7.71(1.3H, m), 8.66(0.7H, m), 7.99(0.7H, d, 7.8Hz), 7.92(0.3H, d, 7.8Hz), 7.80(0.3H, d, 7.3Hz), 7.72(0.7H, d, 7.3Hz), 7.60-7.49(2H, m), 7.47-7.29(4H, m), 6.96(0.3H, t, 54Hz), 6.87(0.7H, t, 54Hz), 2.94-2.86(0.3H, m), 2.74-2.63(0.7H, m), 1.52-1.20(3H, m), 1.18(0.9H, d, 6.6Hz), 0.92(2.1H, d, 6.6Hz), 0.84(2.1H, d, 6.4Hz), 0.59(0.9H, d, 6.4Hz), 0.46(0.9H, d, 6.4Hz), 0.36(2.1H, d, 6.4Hz) | white solid | 61.8-76.0 |
| 13-20 | 8.75(0.4H, d, 4.8Hz), 8.74(0.6H, d, 4.8Hz), 7.89(0.6H, d, 7.9Hz), 7.83(0.4H, d, 7.9Hz), 7.66-7.26(5H, m), 7.05(0.4H, d, 54Hz), 7.03(0.6H, d, 54Hz), 6.86 (0.4H, t, 54Hz), 6.81(0.6H, t, 54Hz), 2.84-2.76(1H, m), 2.60(1.2H, s), 2.58 (1.8H, s), 1.62-1.34(3H, m), 1.16(1.2H, d, 6.7Hz), 0.91(1.8H, d, 6.7Hz), 0.88(1.2H, d, 6.4Hz), 0.85(1.8H, d, 6.4Hz), 0.78(1.2H, d, 6.7Hz), 0.73(1.8H, d, 6.7Hz) | brown candy-like | - |
| 13-49 | 8.77-8.72(1H, m), 7.92-7.83(1H, m), 7.51-7.20(5H, m), 6.95(0.5H, t, 54Hz), 6.91(0.5H, t, 54Hz), 3.02-2.86(1H, m), 1.73-1.60(1H, m), 1.58-1.36(2H, m), 1.20(1.5H, d, 7.1Hz), 1.08(1.5H, d, 7.1Hz), 0.94(1.5H, d, 6.4Hz), 0.89(1.5H, d, 6.9Hz), 0.87(1.5H, d, 6.9Hz), 0.76(1.5H, d, 6.4Hz), | white solid | 126.4-129.2 |
| 13-50 | 8.76-8.72(1H, m), 7.88-7.81(1H, m), 7.61-7.37(5H, m), 6.84(0.5H, t, 54Hz), 6.83(0.5H, t, 54Hz), 6.81(0.5H, s), 6.77(0.5H, s), 2.90-2.81(1H, m), 1.62-1.36(3H, m), 1.18(1.5H, d, 6.6Hz), 0.93(1.5H, d, 6.6Hz), 0.90(1.5H, d, 6.4Hz), 0.84(1.5H, d, 6.4Hz), 0.74(1.5H, d, 7.1Hz), 0.72(1.5H, d, 7.1Hz), | brown candy-like | - |

[Table 29-9]

| No. | $^1$HNMR Spectrum ($CDCl_3$) σ ppm: | Shape | Melting Point (°C) |
|---|---|---|---|
| 13-51 | 8.40-8.35(1H, m), 8.20-8.13(1H, m), 7.55-7.49(1H, m), 7.45-7.41 (1H, m), 7.40-7.29(2H, m), 7.19-7.13(2H, m), 2.98-2.72(1H, m), 1.10(1.3H, d, 6.6Hz), 1.07(1.7H, d, 6.9Hz), 0.84(1.7H, d, 6.9Hz), 0.81(1.3H, d, 6.6Hz), 0.77(1.3H, d, 6.4Hz), 0.72(1.7H, d, 6.4Hz) | brown candy-like | - |
| 13-52 | 8.59-8.56(0.5H, m), 8.54-8.51(0.5H, m), 7.76-7.69(1H, m), 7.50-7.22(3H, m), 7.20-7.16(1H, m), 7.16-7.13(0.5H, m), 7.09-7.04 (0.5H, m), 2.97-2.84(1H, m), 2.75(1.5H, s), 2.73(1.5H, s), 1.74-1.36 (3H, m), 1.11(1.5H, d, 6.4Hz), 1.06(1.5H, d, 6.4Hz), 0.92(1.5H, d, 6.6Hz), 0.87(1.5H, d, 6.6Hz), 0.84(1.5H, d, 6.4Hz), 0.76(1.5H, d, 6.4Hz) | brown candy-like | - |
| 13-53 | 8.53-8.50(0.7H, m), 8.50-8.47(0.3H, m), 7.90-7.84(1H, m), 7.57-7.50(1H, m), 7.47-7.33(4H, m), 2.96-2.79(1H, m), 1.62-1.37 (3H, m), 1.17(1H, d, 6.6Hz), 0.99(2H, d, 6.6Hz), 0.90(2H, d, 6.9Hz), 0.84(2H, d, 6.9Hz), 0.78(1H, d, 6.4Hz), 0.74(1H, d, 6.4Hz) | brown candy-like | - |
| 13-54 | 8.54-8.46(1H, m), 7.90-7.84(1H, m), 7.58-7.50(1H, m), 7.48-7.33 (4H, m), 2.96-2.79(1H, m), 1.67-1.44(3H, m), 1.17(1H, d, 6.6Hz), 0.99(2H, d, 6.6Hz), 0.90(2H, d, 6.9Hz), 0.84(2H, d, 6.9Hz), 0.78 (1H, d, 6.4Hz), 0.74(1H, d, 6.4Hz) | brown candy-like | - |

(continued)

| No. | $^1$HNMR Spectrum ($CDCl_3$) σ ppm: | Shape | Melting Point (°C) |
|---|---|---|---|
| 13-55 | 8.76(1H, s), 8.68(1H, s), 7.65-7.56(3H, m), 7.43-7.37(1H, m), 7.22-7.14(1H, m), 7.04-6.97(1H, m), 6.93-6.87(1H, m) | white solid | 136.1-139.2 |
| 13-56 | 8.82(0.5H, s), 8.81(0.5H, s), 8.27(0.5H, s), 8.25(0.5H, s), 7.53-7.48 (1H, m), 7.43-7.39(2H, m), 7.32-7.26(1H, m), 7.25-7.21(1H, m), 4.88(1H, d, 18.1Hz), 4.76(1H, d, 18.1Hz), 2.93-2.83(1H, m), 1.58-1.32(3H, m), 1.24(3H, d, 6.9Hz), 0.78(3H, d, 6.4Hz), 0.71(3H, d, 6.0Hz) | brown candy-like | - |
| 13-57 | 8.82-8.78(1H, m), 8.26-8.18(1H, m), 8.75-8.34(3H, m), 7.32-7.23 (1H, m), 7.20-7.12(1H, m), 5.11-5.03(0.4H, m), 4.90-4.77(0.6H, m), 2.95-2.85(0.4H, m), 2.82-2.67(0.6H, m), 1.72-1.35(6H, m), 1.35-1.07(3H, m), 0.92-0.62(6H, m) | brown candy-like | - |
| 18-2 | 8.75(1H, d, 4.1Hz), 7.88(1H, d, 8.4Hz), 7.58-7.48(1H, m), 7.34(1H, d, 5.0Hz), 6.99(1H, d, 5.0Hz), 6.79(1H, t, 54Hz), 3.05-2.90(1H, m), 1.63-1.35(3H, m), 1.19(1.5H, d, 6.2Hz), 1.02(1.5H, d, 6.2Hz), 0.92 (1.5H, d, 6.4Hz), 0.88(1.5H, d, 6.4Hz), 0.76(3H, d, 5.5Hz) | brown candy-like | - |
| 18-8 | 8.75(1H, d, 4.1Hz), 7.86(1H, d, 8.0Hz), 7.60-7.55(1H, m), 7.40(1H, d, 5.3Hz), 7.00(1H, d, 5.3Hz), 6.77(0.4H, t, 54Hz), 6.75(0.6H, t, 54Hz), 2.96-2.78(1H, m), 2.80(3H, s), 1.64-1.34(3H, m), 1.22(1.5H, d, 6.6Hz), 0.91(1.5H, d, 6.6Hz), 0.88(1.5H, d, 7.1Hz), 0.86(1.5H, d, 7.1Hz), 0.68(1.5H, d, 6.2Hz), 0.65(1.5H, d, 6.2Hz) | brown candy-like | - |
| 19-2 | 7.77-7.72(1H, m), 7.61-7.51(2H, m), 7.50-7.35(3H, m), 7.31-7.18 (2H, m), 7.17(0.5H, t, 54Hz), 7.13(0.5H, t, 54Hz), 3.02-2.92(1H, m), 1.75-1.38(3H, m), 1.18(1.5H, d, 6.4Hz), 1.06(1.5H, d, 6.9Hz), 0.94(1.5H, d, 6.9Hz), 0.89(1.5H, d, 6.4Hz), 0.83(1.5H, d, 6.4Hz), 0.72(1.5H, d, 6.9Hz), | colorless transparent candy-like | - |

[Table 29-10]

| No. | $^1$HNMR Spectrum ($CDCl_3$) σ ppm: | Shape | Melting Point (°C) |
|---|---|---|---|
| 25-4 | 7.40(0.7H, t, 7.6Hz), 7.32-6.88(3.6H, m), 6.28(0.7H, s), 3.84(0.8H, s), 3.69 (2.2H, s), 3.20-3.10(0.3H, m), 2.87-2.76(0.7H, m), 2.09-2.00(1H, m), 1.67-1.54(1H, m), 1.39(0.7H, s), 1.37(2.3H, s), 1.24(3H, d, 6.9Hz), 1.14 (0.7H, s), 0.94(2.3H, s) | white solid | 154.3-155.6 |
| 25-10 | 7.52-7.44(0.6H, m), 7.42-7.30(0.4H, m), 7.26-6.54(4H, m), 5.22-5.13(0.4H, m), 5.02-4.93(0.6H, m), 3.84(1H, s), 3.75(2H, s), 1.59(3H, s), 1.43(3H, d, 5.0Hz), 1.31(3H, s) | white solid | 145.5-148.6 |
| 25-47 | 7.46(0.5H, m), 7.40-7.33(1H, m), 7.31-7.27(1H, m), 7.25-6.90(2H, m), 6.48 (0.5H, s), 3.99(1.5H, s), 3.74(1.5H, s), 2.92-2.77(1H, m), 2.92(1.5H, s), 2.89 (1.5H, s), 2.11-2.02(1H, m), 1.63-1.55(1H, m), 1.39(1.5H, s), 1.36(1.5H, s), 1.29-1.22(3H, m), 0.99(3H, s) | white solid | 159.3-167.2 |
| 25-56 | 7.57-7.40(1.5H, m), 7.29-7.24(1.5H, m), 7.18-7.14(1H, m), 6.91(1H, t, 54Hz), 5.09-4.87(1H, m), 3.92(1.5H, s), 3.83(1.5H, s), 2.91(3H, s), 1.59(3H, s), 1.50-1.40(3H, m), 1.33(3H, s) | white solid | 128.1-129.9 |
| 25-68 | 7.52-7.18(4H, m), 7.08-6.92(1H, m), 6.79(0.3H, s), 6.52(0.7H, s), 5.11(0.3H, m), 4.90(0.7H, m), 3.82(0.9H, s), 3.72(2.1H, s), 1.57(3H, s), 1.41(3H, d, 6.4Hz), 1.27(3H, s) | white solid | 116.0-124.0 |

(continued)

| No. | $^1$HNMR Spectrum ($CDCl_3$) σ ppm: | Shape | Melting Point (°C) |
|---|---|---|---|
| 25-73 | 8.82(0.7H, d, 7.8Hz), 8.74(0.3H, d, 7.8Hz), 8.12-7.90(1H, m), 7.82-7.49(2H, m), 7.39-6.53(4H, m), 3.86(1H, s), 3.73(2H, s), 2.94-2.82(0.7H, m), 2.72-2.62(0.3H, m), 2.15-1.90(1H, m), 1.70-1.45(1H, m), 1.34(2H, s), 1.31 (1H, s), 1.86(1H, d, 7.3Hz), 1.26(2H, d, 7.3Hz), 0.99(2H, s), 0.93(1H, s) | white solid | 127.8-134.7 |
| 25-74 | 8.86-8.74(1H, m), 8.10-7.50(2H, m), 7.44-7.37(1H, m), 7.26-7.05(3H, m), 6.98(1H, t, 54Hz), 5.15-4.65(1H, m), 3.82(3H, s), 1.59(3H, s), 1.49(3H, d, 6.4Hz), 1.32(3H, m) | white solid | 114.0-119.7 |
| 25-85 | 7.42-7.28(2H, m), 7.25-6.81(4H, m), 3.91(1.2H, s), 3.71(1.8H, s), 3.06(1H, q, 7.6Hz), 2.95(1H, q, 7.6Hz), 2.94-2.80(1H, m), 2.13-2.00(1H, m), 1.62-1.55(1H, m), 1.39(1.8H, t, 7.6Hz), 1.36(3H, s), 1.28(1.2H, t, 7.6Hz), 1.27(1.2H, s), 1.25(1.8H, s), 1.01(1.2H, s), 0.95(1.8H, s) | brown candy-like | - |
| 25-92 | 7.49(1H, m) 7.23-6.87(3H, m) 6.54(1H, m) 4.89(1H, m) 3.73(3H, s) 2.78 (3H, s) 1.57(3H, s) 1.43(3H, d, 6.4Hz) 1.26(3H, m) | brown candy-like | - |
| 25-97 | 7.40-6.43(5.5H, m), 6.44(0.5H, s), 3.90(1.2H, s), 3.71(1.8H, s), 3.00-2.90 (0.4H, m), 2.89-2.78(0.6H, m), 2.74(1.8H, s), 2.63(1.2H, s), 2.14-2.01(1H, m), 1.62-1.53(1H, m), 1.37(3H, s), 1.26(3H, t, 7.1Hz), 1.03(1.2H, s), 0.95 (1.8H, s) | brown candy-like | - |
| 25-103 | 8.91(1H, s), 7.41(1H, t, 7.6Hz), 7.28-6.89(3H, m), 6.18(1H, s), 3.66(3H, s), 2.84-2.74(1H, m), 2.06-1.99(1H, m), 1.60-1.51(1H, m), 1.37(3H, s), 1.24 (3H, d, 6.9Hz), 0.90(3H, s) | white solid | 126.0-135.9 |
| 25-109 | 8.88(0.7H, s), 8.77(0.3H, s), 7.42-7.29(1.7H, m), 7.25-6.80(3.6H, m), 6.39 (0.7H, s), 3.91(1H, s), 3.70(2H, s), 2.95-2.90(0.3H, m), 2.88-2.80(0.7H, m), 2.10-2.01(1H, m), 1.63-1.52(1H, m), 1.36(3H, s), 1.28-1.23(3H, m), 1.00 (1H, s), 0.94(2H, s) | white solid | 160.0-167.3 |

[Table 29-11]

| No. | $^1$HNMR Spectrum ($CDCl_3$) σ ppm: | Shape | Melting Point (°C) |
|---|---|---|---|
| 25-110 | 8.88(0.6H, s), 8.82(0.4H, s), 7.50-7.30(2H, m), 7.25-7.10(2H, m), 7.05-6.70(2H, m), 5.10-4.92(1H, m), 3.90(1.3H, s), 3.78(1.7H, s), 1.54 (3H, s), 1.46(3H, d, 6.4Hz), 1.32(3H, s) | white solid | 63.0-76.0 |
| 25-113 | 7.39(0.7H, t, 7.5Hz), 7.30-7.21(1.7H, m), 7.15-7.07(1.3H, m), 6.97-6.95 (0.3H, m), 6.70(0.3H, s), 6.32(0.7H, s), 3.78(0.9H, s), 3.68(2.1H, s), 3.24-3.14(0.3H, m), 2.88-2.78(0.7H, m), 2.27-2.20(0.3H, m), 2.08-2.00 (0.7H, m), 1.68-1.54(1H, m), 1.41(1H, s), 1.37(2H, s), 1.33(1H, d, 6.8Hz), 1.23(2H, d, 6.8Hz), 0.95(3H, s) | brown candy-like | - |
| 25-114 | 7.52-7.42(0.6H, m), 7.38-7.29(0.4H, m), 7.28-5.14(2H, m), 7.06-6.63 (2H, m), 5.20-5.11(0.4H, m), 5.04-4.92(0.6H, m), 3.80(1.3H, s), 3.75 (1.7H, s), 1.63(1.3H, s), 1.57(1.7H, s), 1.55-1.40(4.3H, m), 1.31(1.7H, s) | brown candy-like | - |
| 25-115 | 7.43-7.33(1H, m), 7.24-7.18(1H, m), 7.17-7.12(1H, m), 5.22-5.14(1H, m), 3.80(3H, s), 2.41(3H, s), 1.58(3H, s), 1.47(3H, d, 6.0Hz), 1.38(3H, s) | white solid | 165.5-170.3 |
| 25-116 | 7.41(1H, m) 7.23(1H, m) 7.12(1H, m) 5.30-4.85(1H, m) 3.83(3H, s) 2.94 (3H, s) 2.44(3H, s) 1.58(3H, s) 1.46(3H, d, 6.4Hz) 1.35(3H, m) | brown candy-like | - |

(continued)

| No. | $^1$HNMR Spectrum ($CDCl_3$) σ ppm: | Shape | Melting Point (°C) |
|---|---|---|---|
| 25-117 | 7.41(1H, brs), 7.40(1H, t, 53.4Hz), 7.26(1H, d, 6.9Hz), 7.15(1H, d, 7.8Hz), 5.10(1H, m), 3.81(3H, s), 2.41(3H, s), 1.58(3H, s), 1.43(3H, d, 6.4Hz), 1.37(3H, s) | white solid | 147.0-155.0 |
| 25-118 | 8.78-8.66(1H, m), 7.90-7.53(1H, m), 7.50-7.33(2H, m), 7.28-7.08(2H, m), 5.30-5.05(0.4H, m), 4.75-4.53(0.6H, m), 3.71(3H, s), 2.44(3H, s), 1.56(3H, s), 1.44(3H, s), 1.18(3H, s) | white solid | 227.2-232.9 |
| 25-119 | 8.78(1H, s), 7.43-7.39(1H, m), 7.28-7.26(1H, m), 7.20-7.10 (2H, m), 5.11 (1H, m), 3.81(3H, s), 2.43(3H, s), 1.55(3H, s), 1.48(3H, brs), 1.35(3H, brs). | white solid | 82.0-86.0 |
| 25-120 | 8.80-8.75(1H, s), 7.34-7.28(1H, m), 7.14-7.06(2H, m), 5.35-5.05(1H, m), 3.78(3H, s), 2.40(3H, s), 1.56(3H, s), 1.47(3H, s), 1.33(3H, s) | white solid | 67.5-79.7 |
| 25-121 | 7.37-7.09(4H, m), 5.15-5.10(1H, m), 3.81(3H, s), 2.63(3H, s), 2.42(3H, s), 1.77(3H, d, 6.4Hz), 1.46(3H, s), 1.36(3H, s) | white solid | 68.9-72.4 |
| 25-122 | 7.31(1H, t, 7.2Hz) 7.12-7.09(2H, m) 5.25(1H, m) 3.78(3H, s) 2.65(3H, s) 2.40(3H, s) 1.56(3H, s) 1.55(3H, s) 1.34(3H, s) | white solid | 197.8-199.0 |
| 25-123 | 7.49-7.38(0.8H, m), 7.25-6.95(2.4H, m), 6.86(0.8H, m), 5.29-5.15(0.3H, m), 5.13-5.00(0.7H, m), 3.81(3H, s), 2.41(3H, s), 1.60(3H, s), 1.31(3H, d, 5.0Hz), 1.37(3H, s) | white solid | 156.3-166.1 |
| 25-124 | 7.37-7.31(1H, m), 7.17-7.12(1H, m), 7.11-7.03(1H, m), 5.23-5.20(1H, m), 3.77(3H, s), 2.39(3H, s), 1.58(3H, s), 1.49(3H, d, 6.0Hz), 1.41(3H, s) | brown candy-like | - |
| 25-125 | 7.40-7.30(3H, m), 7.13(1H, d, 6.9Hz), 7.02-7.00(1H, m), 5.13(1H, m), 3.77(3H, s), 2.39(3H, s), 1.57(3H, s), 1.49(3H, brs), 1.36(3H, s). | white solid | 165.0-168.5 |
| 26-8 | 7.40-7.34(0.7H, m), 7.33-6.95(3.3H, m), 3.18-3.06(0.3H, m), 2.98-2.87 (0.7H, m), 2.72(1H, s), 2.61(2H, s), 2.20-2.04(1H, m), 1.69-1.53(1H, m), 1.40(1H, s), 1.37(2H, s), 1.31(1H, d, 6.9Hz), 1.24(2H, d, 6.9Hz), 1.12 (1H, s), 0.98(2H, s) | white solid | 124.9-126.3 |
| 26-14 | 7.50-7.32(1H, m), 7.30-7.22(1H, m), 7.21-6.80(2H, m), 5.17-5.07(1H, m), 2.71(1.2H, s), 2.65(1.8H, s), 1.64-1.52(3H, m), 1.49-1.40(3H, m), 1.37-1.23(3H, m) | white solid | 128.3-135.9 |

[Table 29-12]

| No. | $^1$HNMR Spectrum ($CDCl_3$) σ ppm: | Shape | Melting Point (°C) |
|---|---|---|---|
| 26-49 | 7.42-7.25(2H, m), 7.13-6.97(1H, m), 6.96(1H, t, 54Hz), 2.92(3H, s), 2.76 (3H, s), 2.18-2.02(1H, m), 1.67-1.52(1H, m), 1.40-1.32(1H, m), 1.36-1.36 (3H, m), 1.29-1.22(3H, m), 1.10-0.99(3H, m) | brown candy-like | - |
| 26-50 | 7.52-7.39(1H, m), 7.30-7.26(1H, m), 7.22-7.04(1H, m), 6.97(1H, t, 54Hz), 5.18-5.10(0.5H, m), 4.95-4.85(0.5H, m), 2.92(3H, s), 2.77(1.5H, s), 2.73 (1.5H, s), 1.62-1.57(3H, m), 1.49-1.43(3H, m), 1.42-1.32(3H, m) | brown candy-like | - |
| 26-57 | 7.47-7.29(2.7H, m), 7.20(1H, d, 6.4Hz), 7.12-7.09(0.3H, m), 7.06(0.7H, t, 54Hz), 6.94(0.3H, t, 54Hz), 2.98-2.84(1H, m), 2.72(1H, s), 2.59(2H, s), 2.11-2.04(1H, m), 1.62-1.55(1H, m), 1.38(1H, s), 1.36(2H, s), 1.24(1H, d, 6.9Hz), 1.23(2H, d, 6.9Hz), 1.05(1H, s), 0.96(2H, s) | brown candy-like | - |

(continued)

| No. | $^1$HNMR Spectrum ($CDCl_3$) σ ppm: | Shape | Melting Point (°C) |
|---|---|---|---|
| 26-61 | 8.85(0.5H, d, 8.2Hz), 8.76(0.5H, d, 8.2Hz), 8.05(0.5H, m), 7.79-7.68(1H, m), 7.89-7.51(0.5H, m), 7.36-6.94(4H, m), 3.05-2.93(1H, m), 2.72(1.5H, s), 2.65(1.5H, s), 2.18-2.08(0.5H, m), 1.98-1.90(0.5H, m), 1.70-1.60(0.5H, m), 1.57-7.48(0.5H, m), 1.39(1.5H, s), 1.34(1.5H, s), 1.32(1.5H, d, 7.1Hz), 1.30(1.5H, d, 7.1Hz), 1.05(1.5H, s), 0.91(1.5H, s) | white solid | 82.0-92.0 |
| 26-62 | 8.83(1H, d, 9.2Hz), 8.07-7.97(0.5H, m), 7.88-7.78(0.5H, m), 7.76-7.66 (0.5H, m), 7.65-7.55(0.5H, m), 7.45-7.32(1H, m), 7.28-6.94(3H, m), 5.20-5.10(0.5H, m), 4.83-4.71(0.5H, m), 2.72(1.5H, s), 2.68(1.5H, s), 1.59 (3H, s), 1.50(3H, d, 6.4Hz), 1.39(1.5H, s), 1.27(1.5H, s) | white solid | 112.1-126.0 |
| 26-70 | 7.43-7.37(1H, m), 7.30-6.85(4H, m), 5.08-5.02(1H, m), 3.08-2.95(2H, m), 2.74(1.5H, s), 2.67(1.5H, s), 1.59(3H, s), 1.49(1.5H, s), 1.47(1.5H, s), 1.35 (3H, s), 1.26(3H, t, 7.3Hz) | brown candy-like | - |
| 26-73 | 7.39(1H, t, 7.6Hz) 7.28-7.02(2H, m), 7.06(1H, t, 54Hz), 2.87-2.69(1H, m), 2.79(3H, s), 2.58(3H, s) 2.08-2.00(1H, m), 1.64-1.52(1H, m), 1.35(3H, s) 1.23(3H, d, 6.9Hz), 0.93(3H, s) | white solid | 102.5-104.1 |
| 26-77 | 7.35-7.23(2H, m), 7.17-7.14(1H, m), 7.07-6.86(2H, m), 2.99-2.89(1H, m), 2.74(3H, s), 2.63(3H, s), 2.13-2.06(1H, m), 1.63-1.56(1H, m), 1.37(3H, s), 1.27(2H, d, 6.8Hz), 1.01(3H, s) | white solid | 130.0-132.0 |
| 26-78 | 7.42-7.38(1H, m), 7.30-7.22(1H, m), 7.17-6.90(3H, m), 5.08-5.03(1H, m), 2.74(3H,s), 2.68(3H, s), 1.60(3H, s), 1.48(3H, d, 6.4Hz), 1.36(3H, s) | white solid | 138.0-141.0 |
| 26-82 | 8.94(0.7H, s), 0.86(0.3H, s), 7.53-7.46(0.7H, m), 7.71-7.34(0.3H, m), 7.28-7.20(2H, m), 7.08(0.7H, t, 54Hz), 7.05(0.3H, t, 54Hz), 5.26-5.18(0.3H, m), 5.00-4.93(0.7H, m), 2.69(1H, s), 2.63(2H, s), 1.62-1.56(3H, m), 1.48 (1H, d, 6.4Hz), 1.45(2H, d, 6.4Hz), 1.42-1.28(3H, m) | brown candy-like | - |
| 26-85 | 8.88(1H, s), 8.02-7.82(1H, m), 7.80-7.72(1H, m), 7.30-7.22(1H, m), 7.23 (1H, t, 54Hz), 7.06-7.01(1H, m), 3.42-3.32(1H, m), 2.77(3H, s), 2.29-2.21 (1H, m), 1.71-1.64(1H, m), 1.36(3H, s), 1.31(3H, d, 6.9Hz), 1.25(3H, s) | white solid | 105.0-111.0 |
| 26-87 | 7.40-7.35(0.7H, m), 7.32-7.27(1.3H, m), 7.20-6.83(3H, m), 3.09-3.02(0.3H, m), 2.97-2.89(1H, m), 2.75(1.8H, s), 2.70(1.2H, s), 2.14-2.05(1H, m), 1.67-1.57(1H, m), 1.39(3H, s), 1.31(1.2H, d, 7.3Hz), 1.25(1.8H, d, 7.3Hz), 1.12(1.2H, s), 1.03(1.8H, s) | white solid | 131.6-136.0 |

[Table 29-13]

| No. | $^1$HNMR Spectrum ($CDCl_3$) σ ppm: | Shape | Melting Point (°C) |
|---|---|---|---|
| 26-88 | 7.38-7.31(0.8H, m), 7.29-7.13(1.8H, m), 7.07-6.57(2.4H, m), 3.08-2.92 (1H, m), 2.69(1.3H, s), 2.62(1.7H, s), 2.22-2.06(1H, m), 1.68-1.54(1H, m), 1.42-1.31(3H, m), 1.39-1.22(3H, m), 1.14(1.3H, s), 1.01(1.7H, s) | brown candy-like | - |
| 26-89 | 7.43(1.3H, s), 7.40-7.36(0.6H, m), 7.34(0.7H, s), 7.30-7.27(0.8H, m), 7.24-7.22(0.7H, m), 7.13-7.08(0.6H, m), 7.05-7.02(0.6H, m),6.99-6.95 (0.7H, m), 3.04-2.98(0.4H, m), 2.94-2.85(0.6H, m), 2.71(1.0H, s), 2.61 (2H, s), 2.15-2.06(1H, m), 1.66-1.58(1H, m), 1.39(1H, s), 1.37(2H, s), 1.32(1H, d, 6.9Hz), 1.24(2H, d, 6.9Hz), 1.08(1H, s), 0.99(2H, s) | white solid | 146.9-149.8 |
| 26-90 | 7.50-7.33(1H, m), 7.26-6.84(4H, m), 5.18-5.08(0.4H, m), 5.05-4.95 (0.6H, m), 2.75(3H, s), 1.60(3H, s), 1.56(3H, s), 1.47(1.2H, s), 1.36(1.8H, s) | white solid | 128.3-136.8 |

(continued)

| No. | $^1$HNMR Spectrum ($CDCl_3$) σ ppm: | Shape | Melting Point (°C) |
|---|---|---|---|
| 26-91 | 7.46-7.40(0.5H, m), 7.37-7.31(0.5H, m), 7.30-7.21(1H, m), 7.18-7.10 (1H, m), 7.03-6.80(2H, m), 5.15-5.05(1H, m), 2.70(1.5H, s), 2.67(1.5H, s), 1.64(1.5H, s), 1.59(1.5H, s), 1.57-1.52(2H, m), 1.46-1.34(4H, m) | brown candy-like | - |
| 26-92 | 7.49-7.21(2H, m), 7.19-6.94(2H, m), 5.15-5.07(1H, m), 2.69(1.5H, s), 2.65(1.5H, s), 1.60-1.55(3H, m), 1.51-1.42(6H, m) | brown candy-like | - |
| 27-3 | 8.26(1H, d, 3.2Hz), 7.92(1H, d, 8.0Hz), 7.60-7.55(1H, m), 7.46-7.40(1H, m), 7.35-7.28(2H, m), 6.81(1H, t, 54Hz), 3.04-2.94(1H, m), 2.13-2.04 (1H, m), 1.66-1.56(1H, m), 1.38(3H, s), 1.33(3H, d, 6.9Hz), 0.98(3H, s) | white solid | 118.0-124.7 |
| 27-4 | 8.82(1H, d, 3.7Hz), 7.92(0.7H, d, 6.9Hz), 7.82(0.3H, d, 6.9Hz), 7.60-7.36 (3H, m), 7.30-7.19(1H, m), 6.88(0.3H, t, 54Hz), 6.83(0.7H, t, 54Hz), 5.40-5.30(0.3H, m), 5.10-5.00(0.7H, m), 1.57(3H, s), 1.52(2H, d, 6.4Hz), 1.47(1H, d, 6.4Hz), 1.44(1H, s), 1.31(2H, s) | white solid | 127.1-134.0 |
| 27-16 | 8.77(1H, d, 4.1Hz), 7.91(0.7H, d, 7.1Hz), 7.79(0.3H, d, 7.1Hz), 7.62-7.52 (2H, m), 7.46-7.41(1H, m), 7.37-7.28(1H, m), 6.83(0.3H, t, 54Hz), 6.78 (0.7H, t, 54Hz), 5.42-5.34(0.3H, m), 4.85-4.78(0.7H, m), 2.80(2.1H, s), 2.79(0.9H, s), 1.59(3H, s), 1.50(2H, d, 6.4Hz), 1.41(1H, d, 6.4Hz), 1.26 (3H, s) | brown candy-like | - |
| 27-28 | 8.75(1H, d, 4.6Hz), 8.68(1H, d, 6.9Hz), 8.06-8.00(1H, m), 7.70-7.36(5H, m), 7.14(1H, d, 7.8Hz), 6.84(1H, t, 54Hz), 5.42-5.16(0.3H, m), 4.42-4.33 (0.7H, m), 1.49(3H, s), 1.45(3H, d, 5.0Hz), 1.01(3H, s) | white solid | 176.6-184.2 |
| 27-36 | 8.76(1H, d, 4.6Hz), 7.90(0.7H, d, 7.6Hz), 7.77(0.3H, d, 7.6Hz), 7.59-7.43 (2H, m), 7.43-7.35(1H, m), 7.28-7.22(1H, m), 7.02(0.7H, t, 54Hz), 7.01 (0.3H, t, 54Hz), 6.81(1H, t, 54Hz), 5.36-5.25(0.3H, m), 4.98-4.88(0.7H, m), 2.62(1H, s), 2.59(2H, s), 1.63(1H, s), 1.58(2H, s), 1.53(1H, d, 6.7Hz), 1.49(2H, d, 6.7Hz), 1.27(3H, s) | white solid | 69.4-70.4 |
| 28-4 | 7.79-7.73(1H, m), 7.65-7.47(3H, m), 7.44-7.10(2H, m), 7.14(1H, t, 54Hz), 5.30-5.22(1H, m), 5.16-5.08(1H, m), 1.62-1.53(6H, m), 1.37-1.30 (3H, m) | white solid | 1372.-139.1 |
| 1-380 | 7.56-7.49(1H, m), 7.48-7.41(1H, m), 7.38-7.31(1H, m), 7.25-7.18(1H, m), 7.03-6.99(2H, m), 7.01(1H, t, 54Hz), 6.46(0.6H, s), 6.41(0.4H, s), 3.77(1.2H, s), 3.75(1.8H, s), 2.90-2.76(1H, m), 1.60-1.32(3H, m), 1.12 (1.2H, d, 6.9Hz), 0.94(1.8H, d, 6.9Hz), 0.85(1.8H, d, 6.4Hz), 0.78(1.2H, d, 6.4Hz), 0.77-0.73(3H, m) | colorless transparent candy-like | - |

[Table 29-14]

| No. | $^1$HNMR Spectrum ($CDCl_3$) σ ppm: | Shape | Melting Point (°C) |
|---|---|---|---|
| 7-288 | 7.54-7.47(1H, m), 7.42-7.38(1H, m), 7.34-7.25(1H, m), 7.17-7.12(1H, m), 7.11(1H, t, 54Hz), 7.04-7.01(2H, m), 2.84-2.73(1H, m), 2.68(1.2H, s), 2.65 (1.8H, s), 1.68-1.28(3H, m), 1.09(1.2H, d, 6.9Hz), 1.04(1.8H, d, 6.9Hz), 0.87-0.81(3H, m), 0.78(1.2H, d, 6.4Hz), 0.73(1.8H, d, 6.4Hz) | colorless transparent candy-like | - |
| 13-58 | 8.78-8.72(1H, m), 7.93-7.91(0.6H, m), 7.86-7.72(0.4H, m), 7.57-7.52(0.6H, m), 7.49-7.44(0.4H, m), 7.44-7.23(4H, m), 6.91(0.8H, s), 6.90(0.4H, t, 54Hz), 6.87(0.6H, t, 54Hz), 6.86(1.2H, s), 2.92-2.84(0.4H, m), 2.82-2.72 (0.6H, m), 1.75-1.51(1.5H, m), 1.46-1.33(1.1H, m), 1.17(1.2H, d, 6.9Hz), 1.13-1.05(0.4H, m), 0.99-0.83(7.8H, m) | colorless transparent candy-like | - |

The method for formulating the compound of the present invention as an agricultural and horticultural fungicide is described more specifically by the following Formulation Examples. However, the agricultural and horticultural fungicide is not limited only to these Formulation Examples, and the compound may also be formulated by mixing it with other various additives at any ratio.

Formulation Example-1 [Wettable Powder]

A wettable powder containing 20 wt% of an active ingredient can be obtained by uniformly mixing and pulverizing a mixture of compound (1-146) (20 parts by weight) of Synthesis Example 1, sodium alkylbenzenesulfonate (3 parts by weight), polyoxyethylene nonylphenyl ether (5 parts by weight), and terra alba (72 parts by weight). Furthermore, wettable powders can be obtained by the same method by using respective compounds shown in Tables 1 to 28.

Formulation Example-2 [Emulsifiable Concentrate]

An emulsifiable concentrate containing 30 wt% of an active agent can be obtained by mixing and dissolving compound (1-146) (30 parts by weight) of Synthesis Example 1, methyl ethyl ketone (40 parts by weight), and polyoxyethylene nonylphenyl ether (30 parts by weight). Furthermore, emulsifiable concentrates can be obtained by the same method by using respective compounds shown in Tables 1 to 28.

Formulation Example-3 [Flowable Formulation]

A sol containing 25 wt% of an active agent can be obtained by uniformly mixing a mixture of compound (1-146) (25 parts by weight) of Synthesis Example 1, polyoxyethylene alkyl ether (1 part by weight), sodium alkylnaphthalenesulfonate (1 part by weight), carboxymethylcellulose (1 part by weight), and water (72 parts by weight). Furthermore, flowable formulations can be obtained by the same method by using respective compounds shown in Tables 1 to 28.

Formulation Example-4 [Granule]

A granule containing 5 wt% of an active agent can be obtained by further adding water (15 parts by weight) to a mixture of compound (1-146) (5 parts by weight) of Synthesis Example 1, sodium laurylsulfate (1 part by weight), calcium ligninsulfonate (5 parts by weight), bentonite (30 parts by weight), and clay (59 parts by weight) and subjecting the mixture to kneading in a kneader, granulating in a granulator, and drying in a fluidized drier. Furthermore, granules can be obtained by the same method by using respective compounds shown in Tables 1 to 28.

Next, in order to demonstrate the fungicidal effect of the imide derivative of the present invention, Test Examples are described.

With respect to the compound of the present invention and the agricultural and horticultural fungicide containing the compound as an active ingredient, their usefulness is demonstrated below by referring to Test Examples 1 to 10. However, the usefulness of the present invention is not limited to the usefulness demonstrated by these Test Examples.

Test Example 1 [Test of Control Effect Against Cucumber Gray Mold by Foliage Application]

A diluted solution (100 ppm) of a wettable powder prepared according to Formulation Example-1 was sprayed at 10 mL per pot on a 1.5-leaf-stage cucumber (variety: Sagami Hanjiro) seedling grown in a plastic pot having a size of 6 cm in diameter within a greenhouse (foliage application). On the day after treatment with the formulation, a fungi-containing agar disc (diameter: 5 mm) of cucumber gray mold-causing fungus (*Botrytis cinerea*) grown in advance on a potato decoction medium was inoculated on a first leaf of the formulation-treated cucumber and placed in a greenhouse at 20°C. Four days after the inoculation, the lesion diameter (cm) was measured, and the control value (%) was calculated according to the following formula (a). Then, the control value was converted to an evaluation value according to Table 30. This test was performed with three replications, each consisting of one plot and one pot, per one formulation concentration, and its average evaluation value of control effect was determined.

$$\text{Control value (\%)} = [(\text{lesion diameter in unsprayed plot} - \text{lesion diameter in sprayed plot}) / \text{lesion diameter in unsprayed plot}] \times 100 \ldots \text{(a)}$$

[Table 30]

Table 30

| Evaluation Value of Control Effect | Control Value |
|---|---|
| 5 | from 95 to 100% |
| 4 | from 80 to less than 95% |
| 3 | from 60 to less than 80% |
| 2 | from 40 to less than 60% |
| 1 | from 20 to less than 40% |
| 0 | less than 20% |

As a result, the following compounds showed an evaluation value of 4. In this connection, with all of the following compounds, phytotoxicity on cucumber was not observed.
Compound No.: 1-29, 1-34, 1-35, 1-61, 1-136, 1-140, 1-145, 1-146, 1-237, 1-300, 1-314, 1-354, 1-359, 1-369, 1-377, 1-378, 1-379, 1-380, 5-5, 5-23, 7-37, 7-38, 7-141, 7-151, 7-152, 7-216, 7-238, 11-8, 11-20, 13-2, 13-52, 13-53, 13-54, 13-55, 13-56, 18-2, 18-8, 25-68, 25-115

Test Example 2 [Test of Control Effect Against Barley Powdery Mildew by Foliage Application]

A diluted solution (100 ppm) of a wettable powder prepared according to Formulation Example-1 was sprayed at 10 mL per pot on a 1.5-leaf-stage barley (variety: Mikamo Golden) seedling grown in a plastic pot having a size of 6 cm in diameter within a greenhouse (foliage application). On the day after the treatment with the formulation, a conidia spore suspension (spore concentration: $5 \times 10^5$ spores/mL) of barley powdery mildew fungus (*Erysiphe graminis*) formed in advance on a separate barley leaf was inoculated into the formulation-applied pot by spraying at 5 mL per pot, and the disease development was monitored in an artificial weather chamber at 20°C. Seven days after the inoculation, the percentage (%) of the area of barley powdery mildew lesions on the first leaf was examined, and the control value (%) was calculated according to the following formula (b). Then, the control value was converted to an evaluation value according to Table 30. This test was performed with three replications, each consisting of one plot and one pot, per one formulation concentration, and its average evaluation value of control effect was determined.

$$\text{Control value (\%)} = [1 - (\text{lesion area percentage in sprayed plot} / \text{lesion area percentage in unsprayed plot})] \times 100 \ldots \text{(b)}$$

As a result, the following compounds showed an evaluation value of 4. In this connection, with all of the following compounds, phytotoxicity on barley was not observed.
Compound No.: 1-29, 1-34, 1-61, 1-131, 1-136, 1-146, 1-158, 1-258, 1-266, 1-307, 1-313, 1-314, 1-325, 1-333, 1-336, 1-341, 1-346, 1-349, 1-359, 1-368, 1-369, 1-370, 1-372, 1-373, 1-374, 1-375, 1-376, 1-377, 1-379, 7-37, 7-68, 7-125, 7-129, 7-132, 7-134, 7-141, 7-151, 7-188, 7-200, 7-216, 7-226, 7-237, 7-250, 7-262, 7-270, 7-272, 7-274, 7-278, 7-279, 7-280, 7-281, 7-283, 7-286, 7-288, 13-16, 13-20, 13-50, 13-51, 13-52, 13-53, 13-54, 13-56, 19-2, 25-4, 25-10, 25-47, 25-56, 25-68, 25-74, 25-85, 25-92, 25-97, 25-103, 25-109, 25-110,25-113, 25-114, 25-115, 25-116, 25-124, 25-125, 26-14, 26-49, 26-50, 26-57, 26-62, 26-73, 26-77, 26-78, 26-88, 26-89, 26-91, 27-4, 27-16
The following compounds showed an evaluation value of 5. In this connection, with all of the following compounds, phytotoxicity on barley was not observed.
Compound No.: 1-35, 1-65, 1-140, 1-145, 1-237, 1-300, 1-354, 1-380, 5-1, 5-5, 5-19, 5-23, 7-38, 7-152, 7-176, 7-238, 7-277, 11-8, 11-20, 13-1, 13-2, 13-10, 13-49, 13-55, 13-58, 18-2, 18-8, 25-73, 26-8, 26-85, 27-3

Test Example 3 [Test of Control Effect Against Rice Blast by Foliage Application]

A diluted solution (100 ppm) of a flowable prepared according to Formulation Example-3 was sprayed at 10 mL per pot on a 3-leaf-stage paddy rice (variety: Asahi) seedling (5 seedlings/pot) grown in a plastic pot having a size

of 6 cm in diameter within a greenhouse (foliage application). On the day after the treatment with the formulation, a conidia spore suspension (spore concentration: $2\times10^6$ spores/mL) of rice blast fungus (*Pyricularia grisea*) formed in advance on a separate paddy rice foliage was inoculated into the formulation-applied pot by spraying at 5 mL per pot and after standing still for 24 hours in an inoculation box (relative humidity = 100%) at 24°C, disease development was promoted in an artificial weather chamber at 24°C. Seven days after the inoculation, the number of rice blast lesions on the third leaf was examined, and the control value (%) was calculated according to the following formula (c). Then, the control value was converted to an evaluation value according to Table 30. This test was performed with three replications, each consisting of one plot and one pot, per one formulation concentration, and its average evaluation value of control effect was determined.

$$\text{Control value (\%)} = [1 - (\text{number of lesions in sprayed plot} / \text{number of lesions in unsprayed plot})] \times 100 \ldots \text{(c)}$$

As a result, the following compounds showed an evaluation value of 4. In this connection, with all of the following compounds, phytotoxicity on paddy rice was not observed.
Compound No.: 1-29, 1-35, 1-131, 1-146, 1-237, 1-313, 1-336, 1-346, 1-368, 1-370, 1-371, 1-374, 1-376, 1-377, 1-378, 1-379, 5-5, 5-19, 5-23, 7-134, 7-141, 7-216, 7-226, 7-238, 7-262, 7-272, 7-279, 7-286, 13-2, 13-50, 13-54, 13-55, 13-56, 13-57, 13-58, 19-2, 25-56, 25-68, 25-97, 25-110, 25-115, 25-119, 25-120, 25-122, 26-49, 26-50, 26-73, 26-82, 26-85, 26-89, 28-4
The following compounds showed an evaluation value of 5. In this connection, with all of the following compounds, phytotoxicity on paddy rice was not observed.
Compound No.: 1-34, 1-145, 1-380, 7-37, 7-38, 7-125, 7-151, 7-152, 7-176, 7-188, 7-237, 7-277, 7-278, 7-288, 11-8, 11-20, 13-49, 18-2, 18-8,25-10, 25-114, 26-8, 26-14, 26-57,26-88,26-91

Test Example 4 [Test of Control Effect Against Rice Sheath Blight by Foliage Application (Flowable Formulation)]

A diluted solution (100 ppm) of a flowable prepared according to Formulation Example-3 was sprayed at 10 mL per pot on a 7-leaf-stage paddy rice (variety: Asahi) seedling (pot) grown in a plastic pot having a size of 6 cm in diameter within a greenhouse (foliage application). On the day after the treatment with the formulation, a culture of rice sheath blight fungus *(Thanatephorus cucumeris)* grown in advance on a rice straw medium was inoculated into the root of the formulation-applied pot, and disease development was promoted in an artificial weather chamber at 26°C (relative humidity = 100%). Seven days after the inoculation, the rice sheath blight lesion height (height from the root) was examined, and the control value (%) was calculated according to the following formula (d). Then, the control value was converted to an evaluation value according to Table 30. This test was performed with three replications, each consisting of one plot and one pot, per one formulation concentration, and its average evaluation value of control effect was determined.

$$\text{Control value (\%)} = [1 - (\text{lesion height in sprayed plot} / \text{lesion height in unsprayed plot})] \times 100 \ldots \text{(d)}$$

As a result, the following compounds showed an evaluation value of 4. In this connection, with all of the following compounds, phytotoxicity on paddy rice was not observed.
Compound No.: 1-279, 1-292, 1-341, 1-362, 1-371, 1-378, 5-1, 5-5, 5-19, 5-23, 7-152, 7-274, 11-8, 13-1, 13-50, 13-51, 13-56, 13-57, 25-92, 25-103, 25-118, 25-120, 25-121, 25-122, 25-123, 26-61, 26-873
The following compounds showed an evaluation value of 5. In this connection, with all of the following compounds, phytotoxicity on paddy rice was not observed.
Compound No.: 1-29, 1-34, 1-35, 1-61, 1-65, 1-122, 1-131, 1-136, 1-140, 1-145, 1-146, 1-158, 1-237, 1-258, 1-266, 1-300, 1-307, 1-313, 1-314, 1-325, 1-333, 1-336, 1-346, 1-349, 1-354, 1-359, 1-368, 1-369, 1-370, 1-372, 1-373, 1-374, 1-375, 1-376, 1-377, 1-379, 1-380, 7-37, 7-38, 7-68, 7-125, 7-129, 7-132, 7-134, 7-141, 7-151, 7-176, 7-188, 7-200, 7-216, 7-226, 7-237, 7-238, 7-250, 7-262, 7-270, 7-272, 7-277, 7-278, 7-279, 7-280, 7-281, 7-282, 7-283, 7-284, 7-285, 7-286, 7-287, 7-288, 11-20, 13-2, 13-10, 13-16, 13-20, 13-49, 13-52, 13-53, 13-54, 13-55, 13-58, 18-2, 18-8, 19-2, 25-4, 25-10, 25-47, 25-56, 25-68, 25-73, 25-74, 25-85, 25-97, 25-109, 25-110, 25-113, 25-114, 25-115, 25-116, 25-117, 25-119, 25-124, 25-125, 26-8, 26-14, 26-49, 26-50, 26-57, 26-62, 26-70, 26-73, 26-77, 26-78, 26-82, 26-85, 26-88, 26-89, 26-90, 26-91, 26-92, 27-3, 27-4, 27-16, 27-28, 27-36, 28-4

Test Example 5 [Test of Control Effect Against Rice Blast by Box Treatment (Flowable Formulation)]

By irrigation, 50 mL of a diluted solution (5,000 ppm) of a flowable prepared according to Formulation Example-3 was applied to a 2.5-leaf-stage paddy rice (variety: Asahi) seedling raised using a nursery box (1/10 the area of standard nursery box in size: 12 cm×15 cmxheight 4 cm) within a greenhouse. The formulation-treated paddy rice seedling was immediately transplanted at 4 seedlings/pot into a pot (1/10000 are in size) filled with paddy soil (flooded). After growing for 3 weeks within a greenhouse, the conidia spore of rice blast fungus (*Pyricularia grisea*) formed in advance on a separate paddy rice foliage were inoculated into the formulation-treated paddy rice and after standing still for 24 hours in an inoculation box (relative humidity = 100%) at 24°C, disease development was promoted in an artificial weather chamber at 24°C. Seven days after the inoculation, the number of rice blast lesions on the second leaf from the top at the inoculation into paddy rice was examined to determine an average number of lesions, and the control value (%) was calculated according to formula (c). Then, the control value was converted to an evaluation value according to Table 30. This test was performed with three replications, each consisting of one plot and one pot, per one formulation plot, and its average evaluation value of control effect was determined.

As a result, the following compounds showed an evaluation value of 4. In this conneciton, with all of the following compounds, phytotoxicity on paddy rice was not observed.

Compound No.: 1-65, 1-122, 1-131, 1-140, 1-266, 1-279, 1-336, 1-354, 1-362, 1-368, 1-369, 1-371, 1-373, 7-68, 7-129, 7-279, 7-282, 7-284, 25-4, 25-47, 25-103, 25-113, 25-115, 25-116, 25-119, 25-120, 25-123, 25-124, 25-125, 26-8, 26-61, 26-73, 26-77, 26-82, 26-88, 26-89, 27-3

The following compounds showed an evaluation value of 5. In this conneciton, with all of the following compounds, phytotoxicity on paddy rice was not observed.

Compound No.: 1-29, 1-34, 1-35, 1-136, 1-145, 1-146, 1-158, 1-237, 1-258, 1-313, 1-314, 1-333, 1-341, 1-346, 1-359, 1-370, 1-374, 1-375, 1-376, 1-377, 1-378, 1-379, 1-380, 5-1, 5-5, 5-19, 5-23, 7-37, 7-38, 7-125, 7-132, 7-134, 7-141, 7-151, 7-152, 7-176, 7-188, 7-200, 7-216, 7-226, 7-237, 7-238, 7-250, 7-262, 7-270, 7-274, 7-277, 7-278, 7-280, 7-281, 7-283, 7-288, 11-8, 13-1, 13-2, 13-10, 13-16, 13-20, 13-49, 13-50, 13-51, 13-52, 13-53, 13-54, 13-55, 13-56, 13-57, 13-58, 18-2, 18-8, 19-2, 25-10, 25-56, 25-68, 25-73, 25-74, 25-109, 25-110, 25-114, 25-117, 25-118, 26-14, 26-49, 26-50, 26-57, 26-62, 26-70, 26-78, 26-85, 26-87, 26-90, 26-91, 26-92, 27-4, 27-16, 27-28, 27-36, 28-4

Test Example 6 [Test of Control Effect Against Rice Sheath Blight by Box Treatment (Flowable Formulation)]

By irrigation, 50 mL of a diluted solution (5,000 ppm) of a flowable prepared according to Formulation Example-3 was applied to a 2.5-leaf-stage paddy rice (variety: Asahi) seedling raised using a nursery box (1/10 the area of standard nursery box in size: 12 cm×15 cm×height 4 cm) within a greenhouse. The formulation-treated paddy rice seedling was immediately transplanted at 5 seedlings/pot into a pot (1/10000 are in size) filled with paddy soil (flooded). After growing for 3 weeks within a greenhouse, a culture of rice sheath blight fungus *(Thanatephorus cucumeris)* grown in advance on a rice straw medium was inoculated into the root of the formulation-treated paddy rice, and disease development was promoted in an artificial weather chamber at 26°C (relative humidity = 100%). Seven days after the inoculation, the rice sheath blight lesion height (height from the root) was examined, and the control value (%) was calculated according to formula (d). Then, the control value was converted to an evaluation value according to Table 30. This test was performed with three replications, each consisting of one plot and one pot, per one formulation plot, and its average evaluation value of control effect was determined.

As a result, the following compounds showed an evaluation value of 4. In this conneciton, with all of the following compounds, phytotoxicity on paddy rice was not observed.

Compound No.: 1-158, 1-266, 1-307, 1-325, 1-346, 1-349, 1-371, 5-1, 7-129, 7-200, 7-282, 7-285, 7-287, 11-8, 13-1, 13-50, 25-73, 25-103, 25-109, 25-119, 25-120, 25-121, 26-49, 26-87

The following compounds showed an evaluation value of 5. In this conneciton, with all of the following compounds, phytotoxicity on paddy rice was not observed.

Compound No.: 1-29, 1-34, 1-35, 1-65, 1-122, 1-131, 1-136, 1-140, 1-145, 1-146, 1-237, 1-258, 1-279, 1-292, 1-300, 1-313, 1-314, 1-333, 1-341, 1-354, 1-359, 1-368, 1-369, 1-370, 1-372, 1-373, 1-374, 1-375, 1-376, 1-380, 5-5, 5-23, 7-37, 7-38, 7-134, 7-141, 7-151, 7-152, 7-176, 7-188, 7-216, 7-226, 7-237, 7-238, 7-250, 7-262, 7-270, 7-274, 7-277, 7-278, 7-279, 7-280, 7-281, 7-283, 7-284, 7-286, 7-288, 13-2, 13-10, 13-16, 13-20, 13-49, 13-53, 13-54, 13-55, 13-58, 18-2, 18-8, 25-4, 25-10, 25-47, 25-56, 25-68, 25-74, 25-85, 25-92, 25-97, 25-110, 25-113, 25-114, 25-115, 25-116, 25-117, 25-124, 26-8, 26-14, 26-50, 26-57, 26-62, 26-70, 26-73, 26-77, 26-78, 26-82, 26-85, 26-88, 26-89, 26-90, 26-91, 26-92, 27-3, 27-4, 27-16, 27-28, 27-36, 28-4

Test Example 7 [Test of Control Effect Against Rice Blast by Box Treatment (Granule)]

From the top of a nursery box, 5 g of a granule prepared according to Formulation Example-4 was applied to

a 2.5-leaf-stage paddy rice (variety: Asahi) seedling raised using a nursery box (1/10 the area of standard nursery box in size: 12 cm×15 cmxheight 4 cm) within a greenhouse. The formulation-treated paddy rice seedling was immediately transplanted at 4 seedlings/pot into a pot (1/10000 are in size) filled with paddy soil (flooded). After growing for 3 weeks within a greenhouse, the conidia spore of rice blast fungus (*Pyricularia grisea*) formed in advance on a separate paddy rice foliage were inoculated into the formulation-treated paddy rice and after standing still for 24 hours in an inoculation box (relative humidity = 100%) at 24°C, disease development was promoted in an artificial weather chamber at 24°C. Seven days after the inoculation, the number of rice blast lesions on the second leaf from the top at the inoculation into paddy rice was examined to determine an average number of lesions, and the control value (%) was calculated according to formula (c). Then, the control value was converted to an evaluation value according to Table 30. This test was performed with three replications, each consisting of one plot and one pot, per one formulation plot, and its average evaluation value of control effect was determined.

As a result, the following compounds showed an evaluation value of 4. In this conneciton, with all of the following compounds, phytotoxicity on paddy rice was not observed.

Compound No.: 1-65, 1-122, 1-131, 1-140, 1-266, 1-279, 1-336, 1-354, 1-362, 1-368, 1-369, 1-371, 1-373, 7-68, 7-129, 7-279, 7-282, 7-284, 25-4, 25-47, 25-103, 25-113, 25-115, 25-116, 25-119, 25-120, 25-123, 25-124, 25-125, 26-8, 26-61, 26-73, 26-77, 26-82, 26-88, 26-89, 27-3

The following compounds showed an evaluation value of 5. In this conneciton, with all of the following compounds, phytotoxicity on paddy rice was not observed.

Compound No.: 1-29, 1-34, 1-35, 1-136, 1-145, 1-146, 1-158, 1-237, 1-258, 1-313, 1-314, 1-333, 1-341, 1-346, 1-359, 1-370, 1-374, 1-375, 1-376, 1-377, 1-378, 1-379, 1-380, 5-1, 5-5, 5-19, 5-23, 7-37, 7-38, 7-125, 7-132, 7-134, 7-141, 7-151, 7-152, 7-176, 7-188, 7-200, 7-216, 7-226, 7-237, 7-238, 7-250, 7-262, 7-270, 7-274, 7-277, 7-278, 7-280, 7-281, 7-283, 7-288, 11-8, 13-1, 13-2, 13-10, 13-16, 13-20, 13-49, 13-50, 13-51, 13-52, 13-53, 13-54, 13-55, 13-56, 13-57, 13-58, 18-2, 18-8, 19-2, 25-10, 25-56, 25-68, 25-73, 25-74, 25-109, 25-110, 25-114, 25-117, 25-118, 26-14, 26-49, 26-50, 26-57, 26-62, 26-70, 26-78, 26-85, 26-87, 26-90, 26-91, 26-92, 27-4, 27-16, 27-28, 27-36, 28-4

Test Example 8 [Test of Control Effect Against Rice Sheath Blight by Box Treatment (Granule)]

From the top of a nursery box, 5 g of a granule prepared according to Formulation Example-4 was applied to a 2.5-leaf-stage paddy rice (variety: Asahi) seedling raised using a nursery box (1/10 the area of standard nursery box in size: 12 cm×15 cmxheight 4 cm) within a greenhouse. The formulation-treated paddy rice seedling was immediately transplanted at 5 seedlings/pot into a pot (1/10000 are in size) filled with paddy soil (flooded). After growing for 3 weeks within a greenhouse, a culture of rice sheath blight fungus *(Thanatephorus cucumeris)* grown in advance on a rice straw medium was inoculated into the root of the formulation-treated paddy rice, and disease development was promoted in an artificial weather chamber at 26°C (relative humidity = 100%). Seven days after the inoculation, the rice sheath blight lesion height (height from the root) was examined, and the control value (%) was calculated according to formula (d). Then, the control value was converted to an evaluation value according to Table 30. This test was performed with three replications, each consisting of one plot and one pot, per one formulation plot, and its average evaluation value of control effect was determined.

As a result, the following compounds showed an evaluation value of 4. In this connection, with all of the following compounds, phytotoxicity on paddy rice was not observed.

Compound No.: 1-158, 1-266, 1-307, 1-325, 1-346, 1-349, 1-371, 5-1, 7-129, 7-200, 7-282, 7-285, 7-287, 11-8, 13-1, 13-50, 25-73, 25-103, 25-109, 25-119, 25-120, 25-121, 26-49, 26-87

The following compounds showed an evaluation value of 5. In this connection, with all of the following compounds, phytotoxicity on paddy rice was not observed.

Compound No.: 1-29, 1-34, 1-35, 1-65, 1-122, 1-131, 1-136, 1-140, 1-145, 1-146, 1-237, 1-258, 1-279, 1-292, 1-300, 1-313, 1-314, 1-333, 1-341, 1-354, 1-359, 1-368, 1-369, 1-370, 1-372, 1-373, 1-374, 1-375, 1-376, 1-380, 5-5, 5-23, 7-37, 7-38, 7-134, 7-141, 7-151, 7-152, 7-176, 7-188, 7-216, 7-226, 7-237, 7-238, 7-250, 7-262, 7-270, 7-274, 7-277, 7-278, 7-279, 7-280, 7-281, 7-283, 7-284, 7-286, 7-288, 13-2, 13-10, 13-16, 13-20, 13-49, 13-53, 13-54, 13-55, 13-58, 18-2, 18-8, 25-4, 25-10, 25-47, 25-56, 25-68, 25-74, 25-85, 25-92, 25-97, 25-110, 25-113, 25-114, 25-115, 25-116, 25-117, 25-124, 26-8, 26-14, 26-50, 26-57, 26-62, 26-70, 26-73, 26-77, 26-78, 26-82, 26-85, 26-88, 26-89, 26-90, 26-91, 26-92, 27-3, 27-4, 27-16, 27-28, 27-36, 28-4

Test Example 9 [Test of Control Effect Against Rice Blast by Water Surface Application or Treatment (Flowable Formulation)]

By dripping treatment in paddy water, 1 mL of a diluted solution (1,000 ppm) of a flowable prepared according to Formulation Example-3 was applied to a 5-leaf-stage paddy rice (variety: Asahi) seedling (4 seedlings/stub, one stub/pot) grown in a pot (1/10000 are in size) filled with paddy soil (flooded) within a greenhouse. After growing for 1

week within a greenhouse, the conidia spore of rice blast fungus (*Pyricularia grisea*) formed in advance on a separate paddy rice foliage were inoculated into the formulation-treated paddy rice and after standing still for 24 hours in an inoculation box at 24°C, disease development was promoted in an artificial weather chamber at 24°C. Seven days after the inoculation, the number of rice blast lesions on the second leaf from the top at the inoculation into paddy rice was examined to determine an average number of lesions, and the control value (%) was calculated according to formula (c). Then, the control value was converted to an evaluation value according to Table 30. This test was performed with three replications, each consisting of one plot and one pot, per one formulation plot, and its average evaluation value of control effect was determined.

As a result, the following compounds showed an evaluation value of 4. In this conneciton, with all of the following compounds, phytotoxicity on paddy rice was not observed.

Compound No.: 1-65, 1-122, 1-131, 1-140, 1-266, 1-279, 1-336, 1-354, 1-362, 1-368, 1-369, 1-371, 1-373, 7-68, 7-129, 7-279, 7-282, 7-284, 25-4, 25-47, 25-103, 25-113, 25-115, 25-116, 25-119, 25-120, 25-123, 25-124, 25-125, 26-8, 26-61, 26-73, 26-77, 26-82, 26-88, 26-89, 27-3

The following compounds showed an evaluation value of 5. In this connection, with all of the following compounds, phytotoxicity on paddy rice was not observed.

Compound No.: 1-29, 1-34, 1-35, 1-136, 1-145, 1-146, 1-158, 1-237, 1-258, 1-313, 1-314, 1-333, 1-341, 1-346, 1-359, 1-370, 1-374, 1-375, 1-376, 1-377, 1-378, 1-379, 1-380, 5-1, 5-5, 5-19, 5-23, 7-37, 7-38, 7-125, 7-132, 7-134, 7-141, 7-151, 7-152, 7-176, 7-188, 7-200, 7-216, 7-226, 7-237, 7-238, 7-250, 7-262, 7-270, 7-274, 7-277, 7-278, 7-280, 7-281, 7-283, 7-288, 11-8, 13-1, 13-2, 13-10, 13-16, 13-20, 13-49, 13-50,13-51, 13-52, 13-53, 13-54, 13-55, 13-56, 13-57, 13-58, 18-2, 18-8, 19-2, 25-10, 25-56, 25-68, 25-73, 25-74, 25-109, 25-110, 25-114, 25-117, 25-118, 26-14, 26-49, 26-50, 26-57, 26-62, 26-70, 26-78, 26-85, 26-87, 26-90, 26-91, 26-92, 27-4, 27-16, 27-28, 27-36, 28-4

Test Example 10 [Test of Control Effect Against Rice Sheath Blight by Water Surface Application or Treatment (Flowable Formulation)]

By dripping treatment in paddy water, 1 mL of a diluted solution (1,000 ppm) of a flowable prepared according to Formulation Example-3 was applied to a 5-leaf-stage paddy rice (variety: Asahi) seedling (5 seedlings/stub, one stub/pot) grown in a pot (1/10000 are in size) filled with paddy soil (flooded) within a greenhouse. After growing for 1 week within a greenhouse, a culture of rice sheath blight fungus *(Thanatephorus cucumeris)* grown in advance on a rice straw medium was inoculated into the root of the formulation-treated paddy rice, and disease development was promoted in an artificial weather chamber at 26°C (relative humidity = 100%). Seven days after the inoculation, the rice sheath blight lesion height (height from the root) was examined, and the control value (%) was calculated according to formula (d). Then, the control value was converted to an evaluation value according to Table 30. This test was performed with three replications, each consisting of one plot and one pot, per one formulation plot, and its average evaluation value of control effect was determined.

As a result, the following compounds showed an evaluation value of 4. In this connection, with all of the following compounds, phytotoxicity on paddy rice was not observed.

Compound No.: 1-158, 1-266, 1-307, 1-325, 1-346, 1-349, 1-371, 5-1, 7-129, 7-200, 7-282, 7-285, 7-287, 11-8, 13-1, 13-50, 25-73, 25-103, 25-109, 25-119, 25-120, 25-121, 26-49,26-87

The following compounds showed an evaluation value of 5. In this connection, with all of the following compounds, phytotoxicity on paddy rice was not observed.

Compound No.: 1-29, 1-34, 1-35, 1-65, 1-122, 1-131, 1-136, 1-140, 1-145, 1-146, 1-237, 1-258, 1-279, 1-292, 1-300, 1-313, 1-314, 1-333, 1-341, 1-354, 1-359, 1-368, 1-369, 1-370, 1-372, 1-373, 1-374, 1-375, 1-376, 1-380, 5-5, 5-23, 7-37, 7-38, 7-134, 7-141, 7-151, 7-152, 7-176, 7-188, 7-216, 7-226, 7-237, 7-238, 7-250, 7-262, 7-270, 7-274, 7-277, 7-278, 7-279, 7-280, 7-281, 7-283, 7-284, 7-286, 7-288, 13-2, 13-10, 13-16, 13-20, 13-49, 13-53, 13-54, 13-55, 13-58, 18-2, 18-8, 25-4, 25-10, 25-47, 25-56, 25-68, 25-74, 25-85, 25-92, 25-97, 25-110, 25-113, 25-114, 25-115, 25-116, 25-117, 25-124, 26-8, 26-14, 26-50, 26-57, 26-62, 26-70, 26-73, 26-77, 26-78, 26-82, 26-85, 26-88, 26-89, 26-90, 26-91, 26-92, 27-3, 27-4, 27-16, 27-28, 27-36, 28-4

INDUSTRIAL APPLICABILITY

According to the present invention, a novel imide derivative offering an excellent fungicidal effect in agricultural and horticultural situations is provided.

While the invention has been described in detail and with reference to specific embodiments thereof, it will be apparent to one skilled in the art that various changes and modifications can be made therein without departing from the spirit and scope of the invention.

This application is based on Japanese Patent Application (Patent Application No. 2018-035759) filed on February 28, 2018 and Japanese Patent Application (Patent Application No. 2018-124041) filed on June 29, 2018, and the

contents of which are incorporated herein by way of reference.

**Claims**

**1.** An imide derivative represented by formula (1):

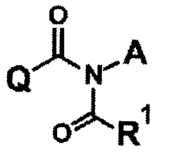

(1)

wherein in formula (1), $R^1$ represents (1a) to (1j) shown below:

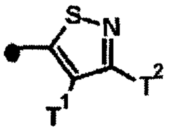

(1a)

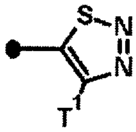

(1b)

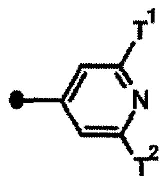

(1c)

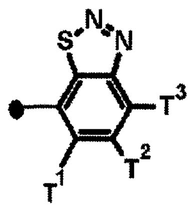

(1d)

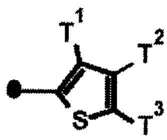

(1e)

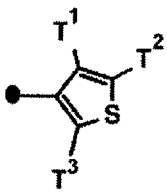

(1f)

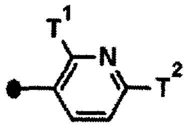

(1g)

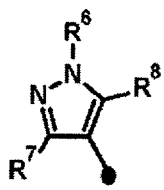

(1h)

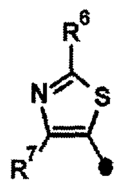

(1i)

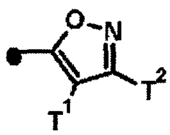

(1j)

wherein each of $T^1$, $T^2$ and $T^3$ represents a hydrogen atom, a halogen atom, a C1-C6 alkyl group, or a C1-C6 haloalkyl group; $T^1$, $T^2$ and $T^3$ may be the same or different; each of $R^6$ and $R^7$ represents a hydrogen atom, a C1-C6 alkyl group, or a C1-C6 haloalkyl group; and $R^8$ represents a hydrogen atom, a halogen atom, a C1-C6 alkyl group, or a C1-C6 haloalkyl group,

A represents (1k) to (1n) shown below:

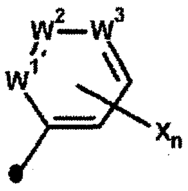 (1 k) 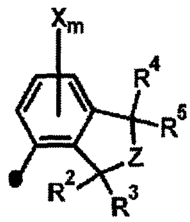 (1 l) 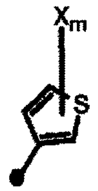 (1 m)

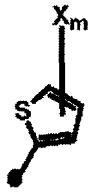 (1 n)

wherein X in formula (1k), (11), (1m), or (In) represents a hydrogen atom, a halogen atom, a C1-C16 alkyl group which may be substituted (the group may be monosubstituted or polysubstituted by the same or different groups selected from the group consisting of a C1-C6 trialkylsilyl group wherein the alkyl groups may be the same or different, and a cyano group), a C1-C16 haloalkyl group, a C2-C16 alkenyl group which may be substituted (the group may be monosubstituted or polysubstituted by the same or different halogen atoms), a C2-C16 haloalkenyl group, a C2-C16 alkynyl group, a C2-C16 haloalkynyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkoxy C1-C6 alkyl group, a C1-C6 haloalkoxy C1-C6 alkyl group, a C1-C6 alkoxy C1-C6 alkoxy C1-C6 alkyl group, a C1-C6 alkylthio C1-C6 alkyl group, a C1-C6 haloalkylthio C1-C6 alkyl group, a C3-C6 cycloalkylthio C1-C6 alkyl group, a C1-C6 alkylsulfinyl C1-C6 alkyl group, a C1-C6 alkylsulfonyl C1-C6 alkyl group, a C1-C6 alkylamino C1-C6 alkyl group, a C1-C6 alkoxycarbonyl C1-C6 alkyl group, a C1-C6 alkylcarbonyl group, a C1-C6 haloalkylcarbonyl group, a C3-C6 cycloalkylcarbonyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a benzenecarbonyl group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), a C1-C6 alkylaminocarbonyl group, a di C1-C6 alkylaminocarbonyl group (the alkyl moieties of the group may be the same or different and may also combine with each other through an alkylene group to form a 3-membered ring, a 4-membered ring, a 5-membered ring, or a 6-membered ring), a C1-C6 alkylsulfonyl group, a C1-C6 haloalkylsulfonyl group, a C1-C6 alkylaminosulfonyl group, a di C1-C6 alkylaminosulfonyl group (the alkyl moieties of the group may be the same or different and may also combine with each other through an alkylene group to form a 3-membered ring, a 4-membered ring, a 5-membered ring, or a 6-membered ring), a C1-C6 alkoxycarbonyl group, a C7-C12 aralkyl group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), a heterocyclic C1-C6 alkyl group, a C1-C6 alkoxy group, a C1-C6 haloalkoxy group, a C1-C6 alkylthio group, C1-C6 haloalkylthio group, a C3-C6 cycloalkyloxy group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C3-C6 cycloalkyl C1-C6 alkoxy group, a C1-C6 alkylamino group, a di C1-C6 alkylamino group (the alkyl moieties of the group may be the same or different and may also combine with each other through an alkylene group to form a 3-membered ring, a 4-membered ring, a 5-membered ring, or a 6-membered ring), a C1-C6 alkylthio C1-C6 alkoxy group, a C1-C6 alkoxy C1-C6 alkoxy group, a phenyl group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), a heterocyclic ring which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), a phenoxy group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), a C7-C12 aralkyloxy group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), or a phenylthio group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group);
n in formula (1k) represents an integer of 1 to 5 and when n is an integer of 2 to 5, X may be the same or different;
$W^1$, $W^2$ and $W^3$ in formula (1k) all represent a carbon atom or represent two carbon atoms and one nitrogen atom whereinwhen any of $W^1$, $W^2$ and $W^3$ is a nitrogen atom, n cannot be 5;
m in formulae (11), (1m), and (In) represents an integer of 1 to 3, and when m is an integer of 2 to 3, X may be the same or different;
each of $R^2$, $R^3$, $R^4$ and $R^5$ in formula (11) represents a hydrogen atom or a C1-C6 alkyl group; and
Z in formula (11) represents a methylene group or an oxygen atom, and
Q represents formula (1h), (1i), (1o), or (1p) shown below:

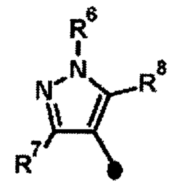 (1 h) 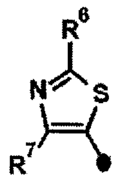 (1 i) 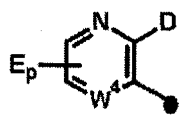 (1 o)

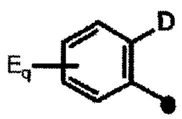 (1 p)

wherein each of $R^6$ and $R^7$ in formula (1h) or (1i) represents a hydrogen atom, a C1-C6 alkyl group, or a C1-C6 haloalkyl group, and $R^8$ in formula (1h) represents a hydrogen atom, a halogen atom, a C1-C6 alkyl group, or a C1-C6 haloalkyl group;
D in formula (1o) or (1p) represents a halogen atom, a C1-C6 alkyl group, or a C1-C6 haloalkyl group; E in formula (1o) or (1p) represents a hydrogen atom, a halogen atom, or a C1-C6 alkyl group; $W^4$ in formula (1o) represents a carbon atom or a nitrogen atom; and p represents an integer of 1 to 3 wherein when $W^4$ is a nitrogen atom, p cannot be 3, and when p is 2 to 3, E may be the same or different; and q represents an integer of 1 to 4, and when q is 2 to 4, E may be the same or different.

**2.** The imide derivative according to claim 1, wherein
$R^1$ is formula (1a), (1b), (1c), (1d), (1i) or (1j) shown below:

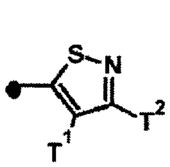 (1 a) 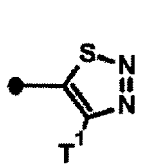 (1 b) 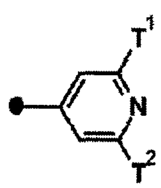 (1 c)

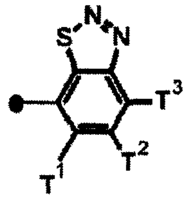 (1 d) 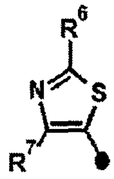 (1 i) 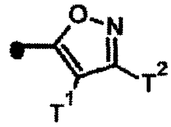 (1 j)

wherein each of $T^1$, $T^2$ and $T^3$ in formula (1a), (1b), (1c), (1d) or (1j) is a hydrogen atom, a halogen atom, a C1-C6 alkyl group, or a C1-C6 haloalkyl group wherein$T^1$, $T^2$ and $T^3$ may be the same or different,
$R^6$ in formula (1i) is a hydrogen atom or a C1-C6 alkyl group, and
group, $R^7$ in formula (1i) is a hydrogen atom, a C1-C6 alkyl group, or a C1-C6 haloalkyl
Q is formula (1h) shown below:

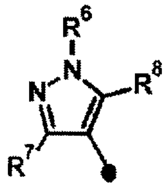 (1 h)

wherein $R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group, and
A is formula (1ka) shown below:

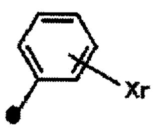 (1 k a)

wherein X in formula (1ka) is a hydrogen atom, a halogen atom, a C1-C8 alkyl group, a C1-C8 haloalkyl group, a C2-C8 alkenyl group which may be substituted (the group may be monosubstituted or polysubstituted by the same or different halogen atoms), a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkoxy C1-C6 alkyl group, a C1-C6 alkylthio C1-C6 alkyl group, a C1-C6 haloalkylthio C1-C6 alkyl group, a C1-C6 alkoxy group, a C1-C6 haloalkoxy group, a C1-C6 alkylthio group, a C1-C6 haloalkylthio group, a C3-C6 cycloalkyloxy group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkyl amino group, a di C1-C6 alkylamino group (the alkyl moieties of the group may be the same or different and may also combine with each other through an alkylene group to form a 3-membered ring, a 4-membered ring, a 5-membered ring, or a 6-membered ring), a phenyl group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), or a phenoxy group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), and
r is an integer of 1 to 5 and when r is an integer of 2 to 5, X may be the same or different.

3. The imide derivative according to claim 1, wherein

$R^1$ is formula (1a), (1b), (1c), (1d), (1i) or (1j) shown below:

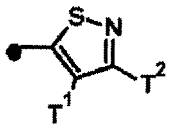 (1a) 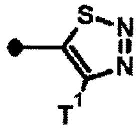 (1b) 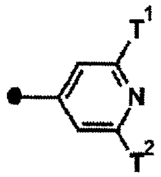 (1c)

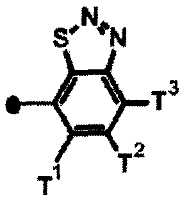 (1d) 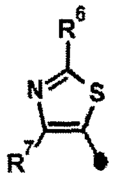 (1i) 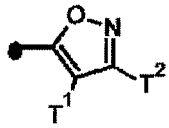 (1j)

wherein each of $T^1$, $T^2$ and $T^3$ in formula (1a), (1b), (1c), (1d) or (1j) is a hydrogen atom, a halogen atom, a C1-C6 alkyl group, or a C1-C6 haloalkyl group wherein$T^1$, $T^2$ and $T^3$ may be the same or different,
$R^6$ in formula (1i) is a hydrogen atom or a C1-C6 alkyl group, and
$R^7$ in formula (1i) is a hydrogen atom, a C1-C6 alkyl group, or a C1-C6 haloalkyl group,
Q is formula (1h) shown below:

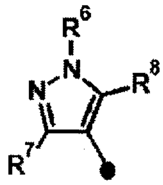 (1h)

$R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group, and
A is formula (11) shown below:

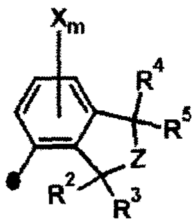 (1l)

wherein X in formula (11) is a hydrogen atom, a halogen atom, or a C1-C6 alkyl group,
m is an integer of 1 to 3, and when m is an integer of 2 to 3, X may be the same or different,
each of $R^2$, $R^3$, $R^4$ and $R^5$ in formula (11) is a hydrogen atom or a C1-C6 alkyl group, and
Z is a methylene group or an oxygen atom.

**4.** The imide derivative according to claim 1, wherein
$R^1$ is formula (1a), (1b), (1c), (1d), (1i) or (1j) shown below:

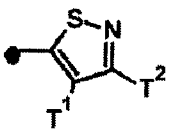 (1a) 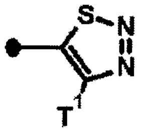 (1b) 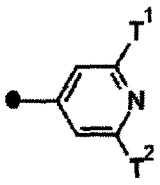 (1c)

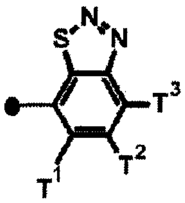 (1d) 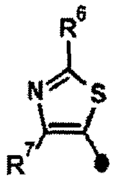 (1i) 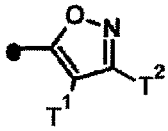 (1j)

each of $T^1$, $T^2$ and $T^3$ in formula (1a), (1b), (1c), (1d) or (1j) is a hydrogen atom, a halogen atom, a C1-C6 alkyl group, or a C1-C6 haloalkyl group ($T^1$, $T^2$ and $T^3$ may be the same or different),
$R^6$ in formula (1i) is a hydrogen atom or a C1-C6 alkyl group, and
$R^7$ in formula (1i) is a hydrogen atom, a C1-C6 alkyl group, or a C1-C6 haloalkyl group,
Q is formula (1h) shown below:

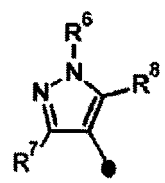 (1h)

$R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group, and
A is formula (1m) shown below:

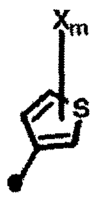 (1m)

X in formula (1m) is a hydrogen atom, a halogen atom, a C1-C8 alkyl group, a C1-C8 haloalkyl group, a C2-C8 alkenyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), or a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), and
m is an integer of 1 to 3, and when m is an integer of 2 to 3, X may be the same or different.

**5.** The imide derivative according to claim 1, wherein
$R^1$ is formula (1a), (1b), (1c), (1d), (1i) or (1j) shown below:

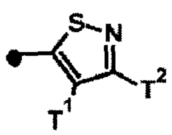 (1a) 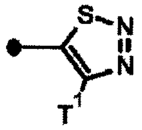 (1b) 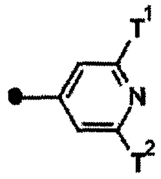 (1c)

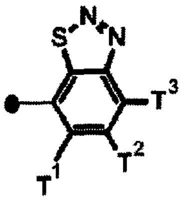 (1d) 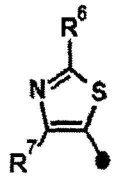 (1i) 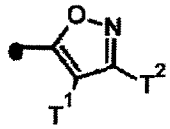 (1j)

wherein each of $T^1$, $T^2$ and $T^3$ in formula (1a), (1b), (1c), (1d) or (1j) is a hydrogen atom, a halogen atom, a C1-C6 alkyl group, or a C1-C6 haloalkyl group wherein $T^1$, $T^2$ and $T^3$ may be the same or different,
$R^6$ in formula (1i) is a hydrogen atom or a C1-C6 alkyl group, and
$R^7$ in formula (1i) is a hydrogen atom, a C1-C6 alkyl group, or a C1-C6 haloalkyl group,
Q is formula (1i) shown below:

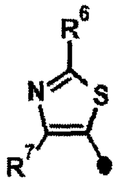 (1i)

wherein $R^6$ in formula (1i) is a hydrogen atom or a C1-C6 alkyl group, and
$R^7$ in formula (1i) is a hydrogen atom, a C1-C6 alkyl group, or a C1-C6 haloalkyl group, and
A is formula (1ka) shown below:

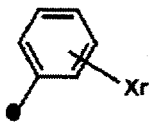 (1ka)

wherein X in formula (1ka) is a hydrogen atom, a halogen atom, a C1-C8 alkyl group, a C1-C8 haloalkyl group, a C2-C8 alkenyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkoxy C1-C6 alkyl group, a C1-C6 alkylthio C1-C6 alkyl group, a C1-C6 haloalkylthio C1-C6 alkyl group, a C1-C6 alkoxy group, a C1-C6 haloalkoxy group, a C1-C6 alkylthio group, a C1-C6 haloalkylthio group, a C3-C6 cycloalkyloxy group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkylamino group, a di C1-C6 alkylamino group (the groups may be the same or different and may also combine with each other through an alkylene group to form a 3-membered ring, a 4-membered ring, a 5-membered ring, or a 6-membered ring), a phenyl group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group, wherein the case where Q and $R^1$ in formula (1) are formula (li), $R^6$ in formula (1i) is a methyl group, and $R^7$ is a difluoromethyl group is excluded), or a phenoxy group which may be substituted (the group may be monosubstituted or polysubstituted by the

same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), and
r is an integer of 1 to 5 and when r is an integer of 2 to 5, X may be the same or different.

**6.** The imide derivative according to claim 1, wherein
$R^1$ is formula (1a), (1b), (1c), (1d), (1i) or (1j) shown below:

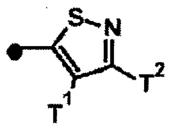
(1a)
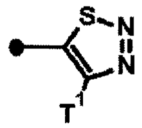
(1b)
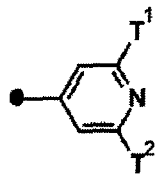
(1c)
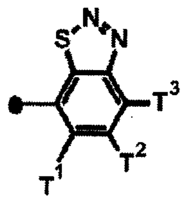
(1d)
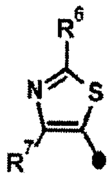
(1i)
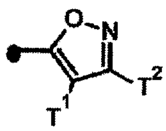
(1j)

wherein each of $T^1$, $T^2$ and $T^3$ in formula (1a), (1b), (1c), (1d) or (1j) is a hydrogen atom, a halogen atom, a C1-C6 alkyl group, or a C1-C6 haloalkyl group wherein $T^1$, $T^2$ and $T^3$ may be the same or different,
$R^6$ in formula (1i) is a hydrogen atom or a C1-C6 alkyl group, and
$R^7$ in formula (1i) is a hydrogen atom, a C1-C6 alkyl group, or a C1-C6 haloalkyl group,
Q is formula (1i) shown below:

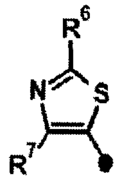
(1i)

wherein $R^6$ in formula (1i) is a hydrogen atom or a C1-C6 alkyl group, and
$R^7$ in formula (1i) is a hydrogen atom, a C1-C6 alkyl group, or a C1-C6 haloalkyl group, and
A is formula (11) shown below:

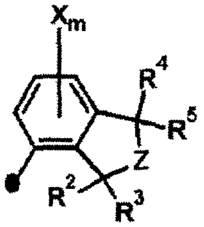
(1l)

wherein X in formula (11) is a hydrogen atom, a halogen atom, or a C1-C6 alkyl group,
m is an integer of 1 to 3, and when m is an integer of 2 to 3, X may be the same or different,
each of $R^2$, $R^3$, $R^4$ and $R^5$ in formula (11) is a hydrogen atom or a C1-C6 alkyl group, and
Z is a methylene group or an oxygen atom.

**7.** The imide derivative according to claim 1, wherein
$R^1$ is formula (1a), (1b), (1c), (1d), (1i) or (1j) shown below:

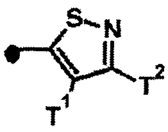 (1 a) 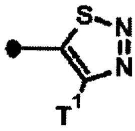 (1 b) 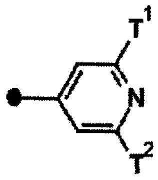 (1 c)

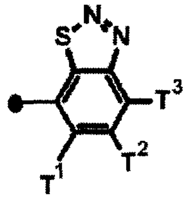 (1 d) 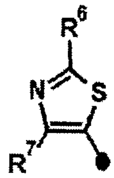 (1 i) 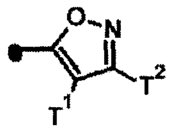 (1 j)

wherein each of $T^1$, $T^2$ and $T^3$ in formula (1a), (1b), (1c), (1d) or (1j) is a hydrogen atom, a halogen atom, a C1-C6 alkyl group, or a C1-C6 haloalkyl group wherein $T^1$, $T^2$ and $T^3$ may be the same or different,
$R^6$ in formula (1i) is a hydrogen atom or a C1-C6 alkyl group, and
$R^7$ in formula (1i) is a hydrogen atom, a C1-C6 alkyl group, or a C1-C6 haloalkyl group,
Q is formula (1i) shown below:

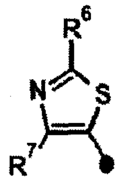 (1 i)

wherein $R^6$ in formula (1i) is a hydrogen atom or a C1-C6 alkyl group, and
$R^7$ in formula (1i) is a hydrogen atom, a C1-C6 alkyl group, or a C1-C6 haloalkyl group, and
A is formula (1m) shown below:

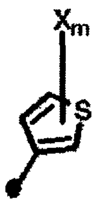 (1 m)

wherein X in formula (1m) is a hydrogen atom, a halogen atom, a C1-C8 alkyl group, a C1-C8 haloalkyl group, a C2-C8 alkenyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), or a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), and
m is an integer of 1 to 3, and when m is an integer of 2 to 3, X may be the same or different.

**8.** The imide derivative according to claim 1, wherein
$R^1$ is formula (1a), (1b), (1c), (1d), (1i) or (1j) shown below:

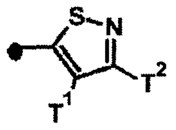 (1a) 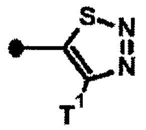 (1b) 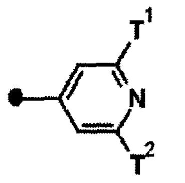 (1c)

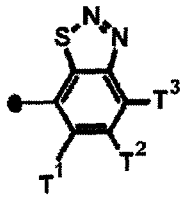 (1d) 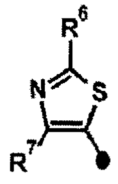 (1i) 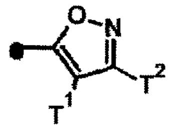 (1j)

wherein each of $T^1$, $T^2$ and $T^3$ in formula (1a), (1b), (1c), (1d) or (1j) is a hydrogen atom, a halogen atom, a C1-C6 alkyl group, or a C1-C6 haloalkyl group wherein $T^1$, $T^2$ and $T^3$ may be the same or different,
$R^6$ in formula (1i) is a hydrogen atom or a C1-C6 alkyl group, and
$R^7$ in formula (1i) is a hydrogen atom, a C1-C6 alkyl group, or a C1-C6 haloalkyl group,
Q is formula (1o) shown below:

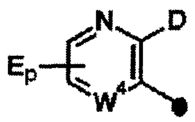 (1o)

wherein D in formula (1o) is a halogen atom, a C1-C6 alkyl group, or a C1-C6 haloalkyl group,
E in formula (1o) is a hydrogen atom, a halogen atom, or a C1-C6 alkyl group,
$W^4$ in formula (1o) is a carbon atom or a nitrogen atom, and
p is an integer of 1 to 3 wherein when $W^4$ is a nitrogen atom, p cannot be 3, and when p is 2 to 3, E may be the same or different,
A is formula (1ka) shown below:

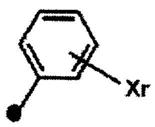 (1ka)

wherein X in formula (1ka) is a hydrogen atom, a halogen atom, a C1-C8 alkyl group, a C1-C8 haloalkyl group, a C2-C8 alkenyl group, a C3-C6 cycloalkyl group which may be substituted (group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkoxy C1-C6 alkyl group, a C1-C6 alkylthio C1-C6 alkyl group, a C1-C6 haloalkylthio C1-C6 alkyl group, a C1-C6 alkoxy group, a C1-C6 haloalkoxy group, a C1-C6 alkylthio group, a C1-C6 haloalkylthio group, a C3-C6 cycloalkyloxy group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkylamino group, a di C1-C6 alkylamino group (the groups may be the same or different and may also combine with each other through an alkylene group to form a 3-membered ring, a 4-membered ring, a 5-membered ring, or a 6-membered ring), a phenyl group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), or a phenoxy group which may be substituted (the group may be monosubstituted or polysubstituted by the same or different groups selected from the group consisting

of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), and
r is an integer of 1 to 5 and when r is an integer of 2 to 5, X may be the same or different.

**9.** The imide derivative according to claim 1, wherein
$R^1$ is formula (1a), (1b), (1c), (1d), (1i) or (1j) shown below:

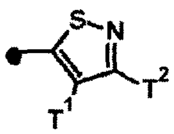 (1a) 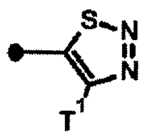 (1b) 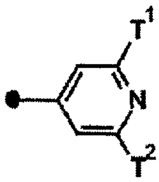 (1c)

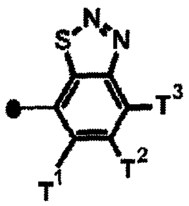 (1d) 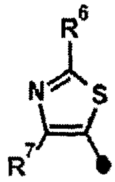 (1i) 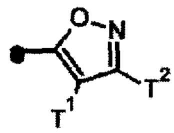 (1j)

wherein each of $T^1$, $T^2$ and $T^3$ in formula (1a), (1b), (1c), (1d) or (1j) is a hydrogen atom, a halogen atom, a C1-C6 alkyl group, or a C1-C6 haloalkyl group wherein $T^1$, $T^2$ and $T^3$ may be the same or different,
$R^6$ in formula (1i) is a hydrogen atom or a C1-C6 alkyl group, and
$R^7$ in formula (1i) is a hydrogen atom, a C1-C6 alkyl group, or a C1-C6 haloalkyl group,
Q is formula (1o) shown below:

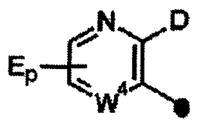 (1o)

wherein D in formula (1o) is a halogen atom, a C1-C6 alkyl group, or a C1-C6 haloalkyl group,
E in formula (1o) is a hydrogen atom, a halogen atom, or a C1-C6 alkyl group,
$W^4$ in formula (1o) is a carbon atom or a nitrogen atom, and
p is an integer of 1 to 3 wherein when $W^4$ is a nitrogen atom, p cannot be 3, and when p is 2 to 3, E may be the same or different, and
A is formula (11) shown below:

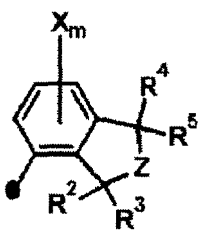 (1 l)

wherein X in formula (11) is a hydrogen atom, a halogen atom, or a C1-C6 alkyl group,
m is an integer of 1 to 3, and when m is an integer of 2 to 3, X may be the same or different,
each of $R^2$, $R^3$, $R^4$ and $R^5$ in formula (11) is a hydrogen atom or a C1-C6 alkyl group, and
Z is a methylene group or an oxygen atom.

**10.** The imide derivative according to claim 1, wherein
$R^1$ is formula (1a), (1b), (1c), (1d), (1i) or (1j) shown below:

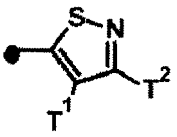 (1 a) 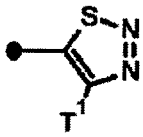 (1 b) 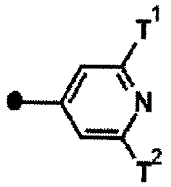 (1 c)

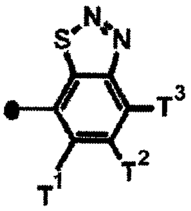 (1 d) 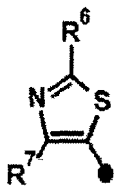 (1 i) 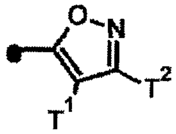 (1 j)

wherein each of $T^1$, $T^2$ and $T^3$ in formula (1a), (1b), (1c), (1d) or (1j) is a hydrogen atom, a halogen atom, a C1-C6 alkyl group, or a C1-C6 haloalkyl group wherein $T^1$, $T^2$ and $T^3$ may be the same or different,
$R^6$ in formula (1i) is a hydrogen atom or a C1-C6 alkyl group, and
$R^7$ in formula (1i) is a hydrogen atom, a C1-C6 alkyl group, or a C1-C6 haloalkyl group,
Q is formula (1o) shown below:

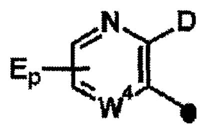 (1 o)

wherein D in formula (1o) is a halogen atom, a C1-C6 alkyl group, or a C1-C6 haloalkyl group,
E in formula (1o) is a hydrogen atom, a halogen atom, or a C1-C6 alkyl group,
$W^4$ in formula (1o) is a carbon atom or a nitrogen atom, and
p is an integer of 1 to 3 wherein when $W^4$ is a nitrogen atom, p cannot be 3, and when p is 2 to 3, E may be the same or different, and
A is formula (1m) shown below:

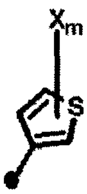 (1 m)

wherein X in formula (1m) is a hydrogen atom, a halogen atom, a C1-C8 alkyl group, a C1-C8 haloalkyl group, a C2-C8 alkenyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), or a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), and
m is an integer of 1 to 3, and when m is an integer of 2 to 3, X may be the same or different.

**11.** The imide derivative according to claim 1, wherein
$R^1$ is formula (1a), (1b), (1c), (1d), (1i) or (1j) shown below:

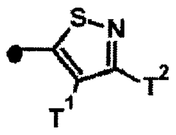 (1a) 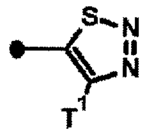 (1b) 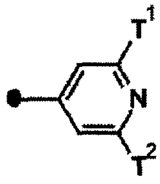 (1c)

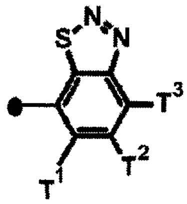 (1d) 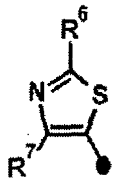 (1i) 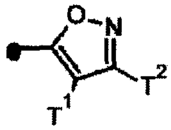 (1j)

wherein each of $T^1$, $T^2$ and $T^3$ in formula (1a), (1b), (1c), (1d) or (1j) is a hydrogen atom, a halogen atom, a C1-C6 alkyl group, or a C1-C6 haloalkyl group wherein $T^1$, $T^2$ and $T^3$ may be the same or different,
$R^6$ in formula (li) is a hydrogen atom or a C1-C6 alkyl group, and
$R^7$ in formula (1i) is a hydrogen atom, a C1-C6 alkyl group, or a C1-C6 haloalkyl group,
Q is formula (1p) shown below:

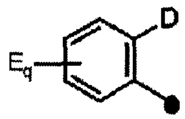 (1p)

wherein D in formula (1p) is a halogen atom, a C1-C6 alkyl group, or a C1-C6 haloalkyl group,
E in formula (1p) is a hydrogen atom, a halogen atom, or a C1-C6 alkyl group, and
q is an integer of 1 to 4, and when q is 2 to 4, E may be the same or different, and
A is formula (1ka) shown below:

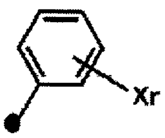 (1ka)

wherein X in formula (1ka) is a hydrogen atom, a halogen atom, a C1-C8 alkyl group, a C1-C8 haloalkyl group, a C2-C8 alkenyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkoxy C1-C6 alkyl group, a C1-C6 alkylthio C1-C6 alkyl group, a C1-C6 haloalkylthio C1-C6 alkyl group, a C1-C6 alkoxy group, a C1-C6 haloalkoxy group, a C1-C6 alkylthio group, a C1-C6 haloalkylthio group, a C3-C6 cycloalkyloxy group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkylamino group, a di C1-C6 alkylamino group (the groups may be the same or different and may also combine with each other through an alkylene group to form a 3-membered ring, a 4-membered ring, a 5-membered ring, or a 6-membered ring), a phenyl group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), or a phenoxy group which may be substituted (the group may be monosubstituted or polysubstituted by the same or different groups selected

from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), and
r is an integer of 1 to 5 and when r is an integer of 2 to 5, X may be the same or different.

**12.** The imide derivative according to claim 1, wherein
$R^1$ is formula (1a), (1b), (1c), (1d), (1i) or (1j) shown below:

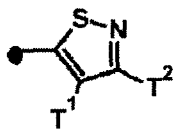
(1 a)
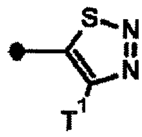
(1 b)
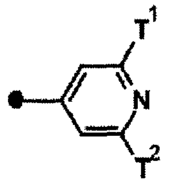
(1 c)

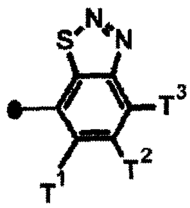
(1 d)
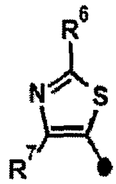
(1 i)
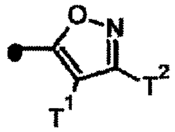
(1 j)

wherein each of $T^1$, $T^2$ and $T^3$ in formula (1a), (1b), (1c), (1d) or (1j) is a hydrogen atom, a halogen atom, a C1-C6 alkyl group, or a C1-C6 haloalkyl group wherein $T^1$, $T^2$ and $T^3$ may be the same or different,
$R^6$ in formula (1i) is a hydrogen atom or a C1-C6 alkyl group, and
$R^7$ in formula (1i) is a hydrogen atom, a C1-C6 alkyl group, or a C1-C6 haloalkyl group,
Q is formula (1p) shown below:

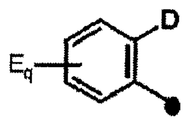
(1 p)

wherein D in formula (1p) is a halogen atom, a C1-C6 alkyl group, or a C1-C6 haloalkyl group,
E in formula (1p) is a hydrogen atom, a halogen atom, or a C1-C6 alkyl group, and
q is an integer of 1 to 4, and when q is 2 to 4, E may be the same or different, and
A is formula (11) shown below:

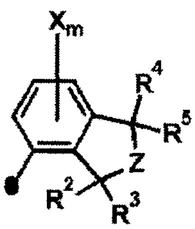
(1 l)

wherein X in formula (11) is a hydrogen atom, a halogen atom, or a C1-C6 alkyl group,
m is an integer of 1 to 3, and when m is an integer of 2 to 3, X may be the same or different,
each of $R^2$, $R^3$, $R^4$ and $R^5$ in formula (11) is a hydrogen atom or a C1-C6 alkyl group, and
Z is a methylene group or an oxygen atom.

**13.** The imide derivative according to claim 1, wherein
$R^1$ is formula (1a), (1b), (1c), (1d), (1i) or (1j) shown below:

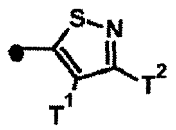 (1a) 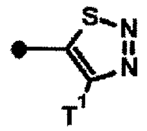 (1b) 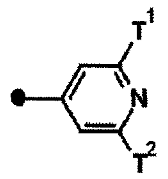 (1c)

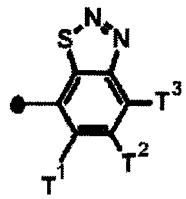 (1d) 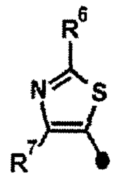 (1i) 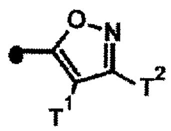 (1j)

wherein each of $T^1$, $T^2$ and $T^3$ in formula (1a), (1b), (1c), (1d) or (1j) is a hydrogen atom, a halogen atom, a C1-C6 alkyl group, or a C1-C6 haloalkyl group wherein $T^1$, $T^2$ and $T^3$ may be the same or different,
$R^6$ in formula (1i) is a hydrogen atom or a C1-C6 alkyl group, and
$R^7$ in formula (1i) is a hydrogen atom, a C1-C6 alkyl group, or a C1-C6 haloalkyl group,
Q is formula (1p) shown below:

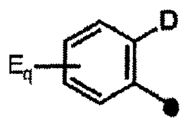 (1p)

wherein D in formula (1p) is a halogen atom, a C1-C6 alkyl group, or a C1-C6 haloalkyl group,
E in formula (1p) is a hydrogen atom, a halogen atom, or a C1-C6 alkyl group, and
q is an integer of 1 to 4, and when q is 2 to 4, E may be the same or different, and
A is formula (1m) shown below:

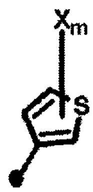 (1m)

X in formula (1m) is a hydrogen atom, a halogen atom, a C1-C8 alkyl group, a C1-C8 haloalkyl group, a C2-C8 alkenyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), or a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), and
m is an integer of 1 to 3, and when m is an integer of 2 to 3, X may be the same or different.

**14.** The imide derivative according to claim 1, wherein
$R^1$ is formula (1e), (1f), (1g) or (1h) shown below:

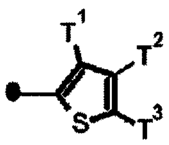
(1 e)
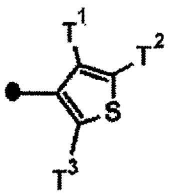
(1 f)
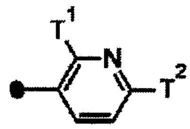
(1 g)

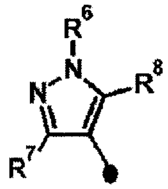
(1 h)

wherein each of $T^1$, $T^2$ and $T^3$ in formula (1e), (1f) or (1g) is a hydrogen atom, a halogen atom, a C1-C6 alkyl group, or a C1-C6 haloalkyl group wherein $T^1$, $T^2$ and $T^3$ may be the same or different,
$R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group,
Q is formula (1h) shown below:

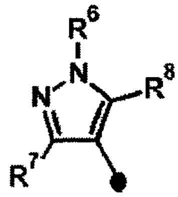
(1 h)

wherein $R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group, and
A is formula (1ka) shown below:

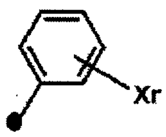
(1 k a)

X in formula (1ka) is a hydrogen atom, a halogen atom, a C1-C8 alkyl group, a C1-C8 haloalkyl group, a C2-C8 alkenyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkoxy C1-C6 alkyl group, a C1-C6 alkylthio C1-C6 alkyl group, a C1-C6 haloalkylthio C1-C6 alkyl group, a C1-C6 alkoxy group, a C1-C6 haloalkoxy group, a C1-C6 alkylthio group, a C1-C6 haloalkylthio group, a C3-C6 cycloalkyloxy group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkylamino group, a di C1-C6 alkylamino group (the groups may be the same or different and may also combine with each other through an alkylene group to form a 3-membered ring, a 4-membered ring, a 5-membered ring, or a 6-membered ring), a phenyl group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), or a phenoxy group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), and r is an integer of 1 to 5 and when r is an integer of 2 to 5, X may be the same or different.

**15.** The imide derivative according to claim 1, wherein $R^1$ is formula (1e), (1f), (1g) or (1h) shown below:

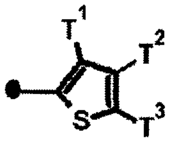
(1e)
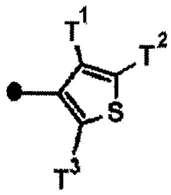
(1f)
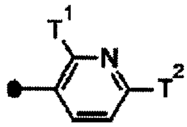
(1g)

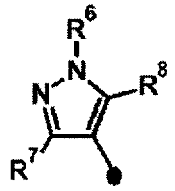
(1h)

wherein each of $T^1$, $T^2$ and $T^3$ in formula (1e), (1f) or (1g) is a hydrogen atom, a halogen atom, a C1-C6 alkyl group, or a C1-C6 haloalkyl group wherein $T^1$, $T^2$ and $T^3$ may be the same or different,
$R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group,
Q is formula (1h) shown below:

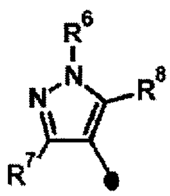
(1h)

wherein $R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group, and
A is formula (11) shown below:

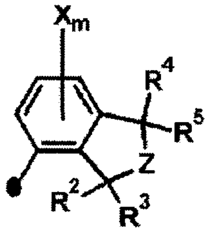
(1l)

wherein X in formula (11) is a hydrogen atom, a halogen atom, or a C1-C6 alkyl group,
m is an integer of 1 to 3, and when m is an integer of 2 to 3, X may be the same or different,
each of $R^2$, $R^3$, $R^4$ and $R^5$ in formula (11) is a hydrogen atom or a C1-C6 alkyl group, and
Z is a methylene group or an oxygen atom.

**16.** The imide derivative according to claim 1, wherein $R^1$ is formula (1e), (1f), (1g) or (1h) shown below:

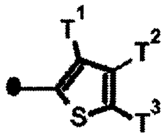
(1e)
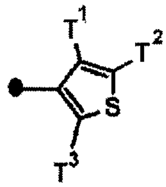
(1f)
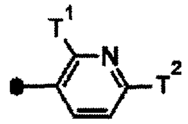
(1g)

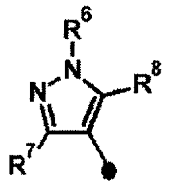
(1h)

wherein each of $T^1$, $T^2$ and $T^3$ in formula (1e), (1 f) or (1g) is a hydrogen atom, a halogen atom, a C1-C6 alkyl group, or a C1-C6 haloalkyl group wherein $T^1$, $T^2$ and $T^3$ may be the same or different,
$R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group,
Q is formula (1h) shown below:

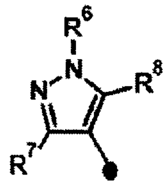
(1h)

wherein $R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group, and
A is formula (1m) shown below:

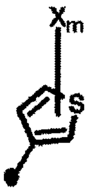
(1m)

wherein X in formula (1m) is a hydrogen atom, a halogen atom, a C1-C8 alkyl group, a C1-C8 haloalkyl group, a C2-C8 alkenyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), or a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), and
m is an integer of 1 to 3, and when m is an integer of 2 to 3, X may be the same or different.

**17.** The imide derivative according to claim 1, wherein $R^1$ is formula (1e), (1f), (1g) or (1h) shown below:

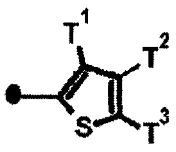 (1 e) 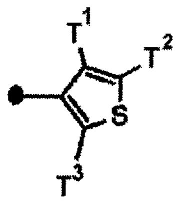 (1 f) 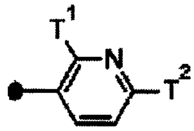 (1 g)

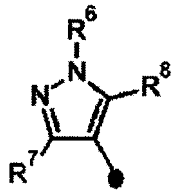 (1 h)

wherein each of $T^1$, $T^2$ and $T^3$ in formula (1e), (1f) or (1g) is a hydrogen atom, a halogen atom, a C1-C6 alkyl group, or a C1-C6 haloalkyl group wherein $T^1$, $T^2$ and $T^3$ may be the same or different,
$R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group,
Q is formula (1i) shown below:

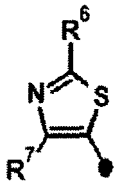 (1 i)

$R^6$ in formula (1i) is a hydrogen atom or a C1-C6 alkyl group, and
$R^7$ in formula (1i) is a hydrogen atom, a C1-C6 alkyl group, or a C1-C6 haloalkyl group, and
A is formula (1ka) shown below:

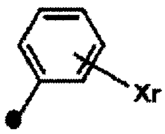 (1 k a)

wherein X in formula (1ka) is a hydrogen atom, a halogen atom, a C1-C8 alkyl group, a C1-C8 haloalkyl group, a C2-C8 alkenyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkoxy C1-C6 alkyl group, a C1-C6 alkylthio C1-C6 alkyl group, a C1-C6 haloalkylthio C1-C6 alkyl group, a C1-C6 alkoxy group, a C1-C6 haloalkoxy group, a C1-C6 alkylthio group, a C1-C6 haloalkylthio group, a C3-C6 cycloalkyloxy group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkylamino group, a di C1-C6 alkylamino group (the groups may be the same or different and may also combine with each other through an alkylene group to form a 3-membered ring, a 4-membered ring, a 5-membered ring, or a 6-membered ring), a phenyl group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group, wherein the case where Q and $R^1$

in formula (1) are formula (1i), $R^6$ in formula (1i) is a methyl group, and $R^7$ is a difluoromethyl group is excluded), or a phenoxy group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), and
r is an integer of 1 to 5 and when r is an integer of 2 to 5, X may be the same or different.

**18.** The imide derivative according to claim 1, wherein
$R^1$ is formula (1e), (1f), (1g) or (1h) shown below:

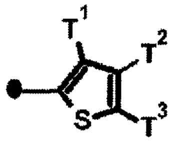
(1 e)
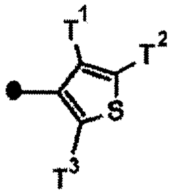
(1 f)
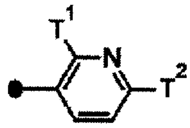
(1 g)

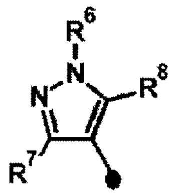
(1 h)

wherein each of $T^1$, $T^2$ and $T^3$ in formula (1e), (1f) or (1g) is a hydrogen atom, a halogen atom, a C1-C6 alkyl group, or a C1-C6 haloalkyl group wherein $T^1$, $T^2$ and $T^3$ may be the same or different,
$R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group,
Q is formula (1 i) shown below:

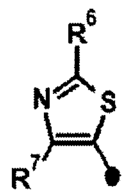
(1 i)

wherein $R^6$ in formula (1i) is a hydrogen atom or a C1-C6 alkyl group, and
$R^7$ in formula (1i) is a hydrogen atom, a C1-C6 alkyl group, or a C1-C6 haloalkyl group, and
A is formula (11) shown below:

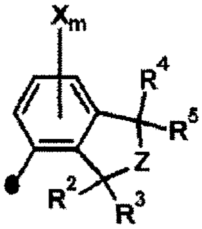
(1 l)

wherein X in formula (11) is a hydrogen atom, a halogen atom, or a C1-C6 alkyl group,
m is an integer of 1 to 3, and when m is an integer of 2 to 3, X may be the same or different,
each of $R^2$, $R^3$, $R^4$ and $R^5$ in formula (11) is a hydrogen atom or a C1-C6 alkyl group, and
Z is a methylene group or an oxygen atom.

**19.** The imide derivative according to claim 1, wherein $R^1$ is formula (1e), (1f), (1g) or (1h) shown below:

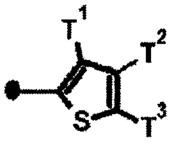 (1 e) 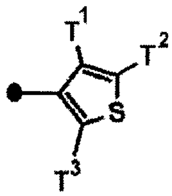 (1 f) 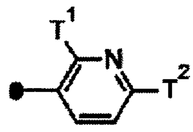 (1 g)

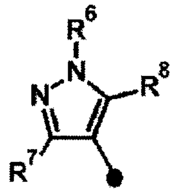 (1 h)

wherein each of $T^1$, $T^2$ and $T^3$ in formula (1e), (1f) or (1g) is a hydrogen atom, a halogen atom, a C1-C6 alkyl group, or a C1-C6 haloalkyl group wherein $T^1$, $T^2$ and $T^3$ may be the same or different,
$R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group,
Q is formula (1i) shown below:

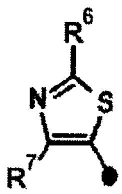 (1 i)

wherein $R^6$ in formula (1i) is a hydrogen atom or a C1-C6 alkyl group, and
$R^7$ in formula (1i) is a hydrogen atom, a C1-C6 alkyl group, or a C1-C6 haloalkyl group, and
A is formula (1m) shown below:

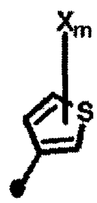 (1 m)

wherein X in formula (1m) is a hydrogen atom, a halogen atom, a C1-C8 alkyl group, a C1-C8 haloalkyl group, a C2-C8 alkenyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), or a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), and
m is an integer of 1 to 3, and when m is an integer of 2 to 3, X may be the same or different.

**20.** The imide derivative according to claim 1, wherein $R^1$ is formula (1e), (1f), (1g) or (1h) shown below:

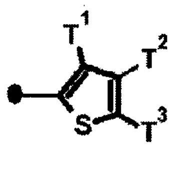 (1e) 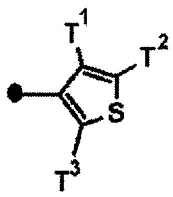 (1f) 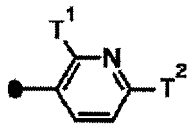 (1g)

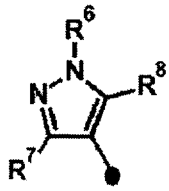 (1h)

wherein each of $T^1$, $T^2$ and $T^3$ in formula (1e), (1f) or (1g) is a hydrogen atom, a halogen atom, a C1-C6 alkyl group, or a C1-C6 haloalkyl group wherein $T^1$, $T^2$ and $T^3$ may be the same or different,
$R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group,
Q is formula (1o) shown below:

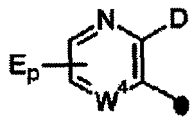 (1o)

D in formula (1o) is a halogen atom, a C1-C6 alkyl group, or a C1-C6 haloalkyl group,
E in formula (1o) is a hydrogen atom, a halogen atom, or a C1-C6 alkyl group,
$W^4$ in formula (1o) is a carbon atom or a nitrogen atom, and
p is an integer of 1 to 3 wherein when $W^4$ is a nitrogen atom, p cannot be 3, and when p is 2 to 3, E may be the same or different, and
A is formula (1ka) shown below:

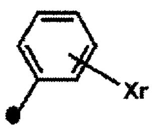 (1ka)

X in formula (1ka) is a hydrogen atom, a halogen atom, a C1-C8 alkyl group, a C1-C8 haloalkyl group, a C2-C8 alkenyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkoxy C1-C6 alkyl group, a C1-C6 alkylthio C1-C6 alkyl group, a C1-C6 haloalkylthio C1-C6 alkyl group, a C1-C6 alkoxy group, a C1-C6 haloalkoxy group, a C1-C6 alkylthio group, a C1-C6 haloalkylthio group, a C3-C6 cycloalkyloxy group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkylamino group, a di C1-C6 alkylamino group (the groups may be the same or different and may also combine with each other through an alkylene group to form a 3-membered ring, a 4-membered ring, a 5-membered ring, or a 6-membered ring), a phenyl group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), or a phenoxy group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), and
r is an integer of 1 to 5 and when r is an integer of 2 to 5, X may be the same or different.

**21.** The imide derivative according to claim 1, wherein
$R^1$ is formula (1e), (1f), (1g) or (1h) shown below:

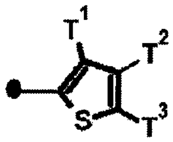
(1 e)
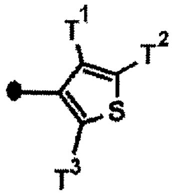
(1 f)
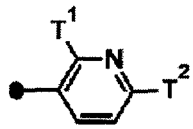
(1 g)

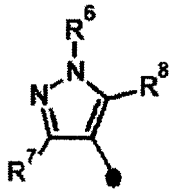
(1 h)

wherein each of $T^1$, $T^2$ and $T^3$ in formula (1e), (1f) or (1g) is a hydrogen atom, a halogen atom, a C1-C6 alkyl group, or a C1-C6 haloalkyl group wherein $T^1$, $T^2$ and $T^3$ may be the same or different,
$R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group,
Q is formula (1o) shown below:

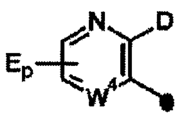
(1 o)

wherein D in formula (1o) is a halogen atom, a C1-C6 alkyl group, or a C1-C6 haloalkyl group,
E in formula (1o) is a hydrogen atom, a halogen atom, or a C1-C6 alkyl group,
$W^4$ in formula (1o) is a carbon atom or a nitrogen atom, and
p is an integer of 1 to 3 wherein when $W^4$ is a nitrogen atom, p cannot be 3, and when p is 2 to 3, E may be the same or different, and
A is formula (11) shown below:

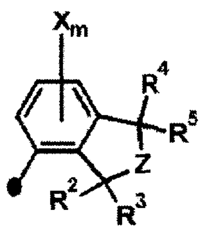
(1 l)

wherein X in formula (11) is a hydrogen atom, a halogen atom, or a C1-C6 alkyl group,
m is an integer of 1 to 3, and when m is an integer of 2 to 3, X may be the same or different,
each of $R^2$, $R^3$, $R^4$ and $R^5$ in formula (11) is a hydrogen atom or a C1-C6 alkyl group, and
Z is a methylene group or an oxygen atom.

**22.** The imide derivative according to claim 1, wherein
$R^1$ is formula (1e), (1f), (1g) or (1h) shown below:

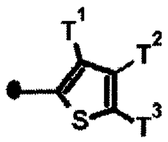
(1 e)
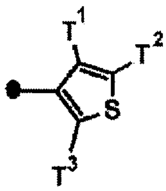
(1 f)
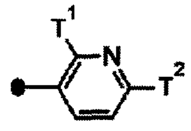
(1 g)

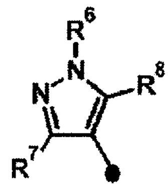
(1 h)

wherein each of $T^1$, $T^2$ and $T^3$ in formula (1e), (1f) or (1g) is a hydrogen atom, a halogen atom, a C1-C6 alkyl group, or a C1-C6 haloalkyl group wherein $T^1$, $T^2$ and $T^3$ may be the same or different,
$R^6$ in formula (1h) is a C1-C6 alkyl group,
$R^7$ in formula (1h) is a hydrogen atom, a C1-C6 alkyl group, or a C1-C6 haloalkyl group,
$R^8$ in formula (1h) is a hydrogen atom, a halogen atom, a C1-C6 alkyl group, or a C1-C6 haloalkyl group,
Q is formula (1o) shown below:

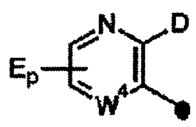
(1 o)

wherein D in formula (1o) is a halogen atom, a C1-C6 alkyl group, or a C1-C6 haloalkyl group,
E in formula (1o) is a hydrogen atom, a halogen atom, or a C1-C6 alkyl group,
$W^4$ in formula (1o) is a carbon atom or a nitrogen atom, and
p is an integer of 1 to 3 wherein when $W^4$ is a nitrogen atom, p cannot be 3, and when p is 2 to 3, E may be the same or different, and
A is formula (1m) shown below:

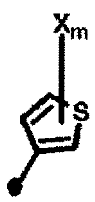
(1 m)

wherein X in formula (1m) is a hydrogen atom, a halogen atom, a C1-C8 alkyl group, a C1-C8 haloalkyl group, a C2-C8 alkenyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), or a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), and
m is an integer of 1 to 3, and when m is an integer of 2 to 3, X may be the same or different.

**23.** The imide derivative according to claim 1, wherein
$R^1$ is formula (1e), (1f), (1g) or (1h) shown below:

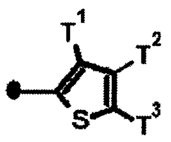 (1e) 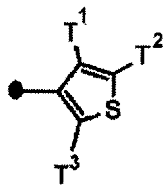 (1f) 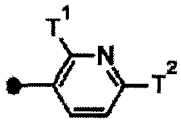 (1g)

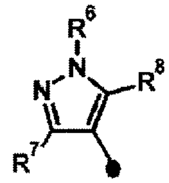 (1h)

wherein each of $T^1$, $T^2$ and $T^3$ in formula (1e), (1f) or (1g) is a hydrogen atom, a halogen atom, a C1-C6 alkyl group, or a C1-C6 haloalkyl group wherein $T^1$, $T^2$ and $T^3$ may be the same or different,
$R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group,
Q is formula (1p) shown below:

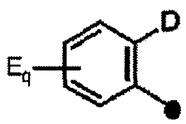 (1p)

wherein D in formula (1p) is a halogen atom, a C1-C6 alkyl group, or a C1-C6 haloalkyl group,
E in formula (1p) is a hydrogen atom, a halogen atom, or a C1-C6 alkyl group, and
q is an integer of 1 to 4, and when q is 2 to 4, E may be the same or different, and
A is formula (1ka) shown below:

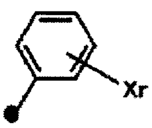 (1ka)

wherein X in formula (1ka) is a hydrogen atom, a halogen atom, a C1-C8 alkyl group, a C1-C8 haloalkyl group, a C2-C8 alkenyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkoxy C1-C6 alkyl group, a C1-C6 alkylthio C1-C6 alkyl group, a C1-C6 haloalkylthio C1-C6 alkyl group, a C1-C6 alkoxy group, a C1-C6 haloalkoxy group, a C1-C6 alkylthio group, a C1-C6 haloalkylthio group, a C3-C6 cycloalkyloxy group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), a C1-C6 alkylamino group, a di C1-C6 alkylamino group (the groups may be the same or different and may also combine with each other through an alkylene group to form a 3-membered ring, a 4-membered ring, a 5-membered ring, or a 6-membered ring), a phenyl group which may be substituted (the group may be monosubstituted or polysubstituted by the same or different groups selected from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), or a phenoxy group which may be substituted (the group may be monosubstituted or polysubstituted by the same or different groups selected

from the group consisting of a halogen atom, a cyano group, a nitro group, a C1-C6 alkyl group, a C1-C6 alkoxy group, a C1-C6 haloalkyl group, a C1-C6 haloalkoxy group, a C1-C6 alkylthio group, and a C1-C6 haloalkylthio group), and
r is an integer of 1 to 5 and when r is an integer of 2 to 5, X may be the same or different.

**24.** The imide derivative according to claim 1, wherein
$R^1$ is formula (1e), (1f), (1g) or (1h) shown below:

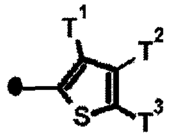
(1 e)
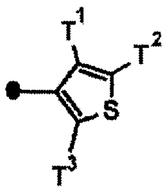
(1 f)
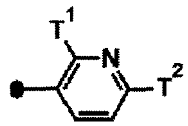
(1 g)

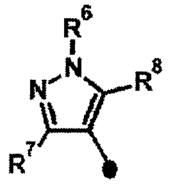
(1 h)

wherein each of $T^1$, $T^2$ and $T^3$ in formula (1e), (1f) or (1g) is a hydrogen atom, a halogen atom, a C1-C6 alkyl group, or a C1-C6 haloalkyl group wherein $T^1$, $T^2$ and $T^3$ may be the same or different,
$R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group,
Q is formula (1p) shown below:

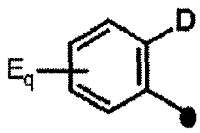
(1 p)

wherein D in formula (1p) is a halogen atom, a C1-C6 alkyl group, or a C1-C6 haloalkyl group,
E in formula (1p) is a hydrogen atom, a halogen atom, or a C1-C6 alkyl group, and
q is an integer of 1 to 4, and when q is 2 to 4, E may be the same or different, and
A is formula (11) shown below:

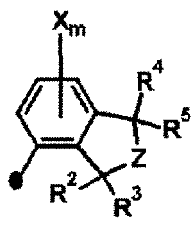
(1 l)

wherein X in formula (11) is a hydrogen atom, a halogen atom, or a C1-C6 alkyl group,
m is an integer of 1 to 3, and when m is an integer of 2 to 3, X may be the same or different,
each of $R^2$, $R^3$, $R^4$ and $R^5$ in formula (11) is a hydrogen atom or a C1-C6 alkyl group, and
Z is a methylene group or an oxygen atom.

**25.** The imide derivative according to claim 1, wherein
$R^1$ is formula (1e), (1f), (1g) or (1h) shown below:

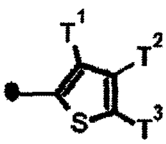 (1 e) 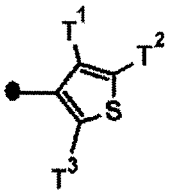 (1 f) 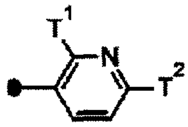 (1 g)

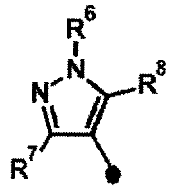 (1 h)

wherein each of $T^1$, $T^2$ and $T^3$ in formula (1e), (1f) or (1g) is a hydrogen atom, a halogen atom, a C1-C6 alkyl group, or a C1-C6 haloalkyl group wherein $T^1$, $T^2$ and $T^3$ may be the same or different,
$R^6$ in formula (1h) is a C1-C6 alkyl group,
$R^7$ in formula (1h) is a hydrogen atom, a C1-C6 alkyl group, or a C1-C6 haloalkyl group, and
$R^8$ in formula (1h) is a hydrogen atom, a halogen atom, a C1-C6 alkyl group, or a C1-C6 haloalkyl group,
Q is formula (1p) shown below:

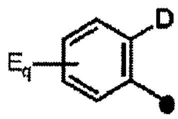 (1 p)

wherein D in formula (1p) is a halogen atom, a C1-C6 alkyl group, or a C1-C6 haloalkyl group,
E in formula (1p) is a hydrogen atom, a halogen atom, or a C1-C6 alkyl group, and
q is an integer of 1 to 4, and when q is 2 to 4, E may be the same or different, and
A is formula (1m) shown below:

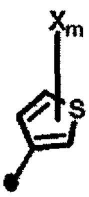 (1 m)

X in formula (1m) is a hydrogen atom, a halogen atom, a C1-C8 alkyl group, a C1-C8 haloalkyl group, a C2-C8 alkenyl group, a C3-C6 cycloalkyl group which may be substituted (the group may be monosubstituted or polysubstituted by the same or different groups selected from the group consisting of a halogen atom, a C1-C6 alkyl group, a C1-C6 alkoxy group, a C1-C6 haloalkyl group, and a C3-C6 cycloalkyl group), or a C3-C6 cycloalkyl C1-C6 alkyl group which may be substituted (the group may be monosubstituted or polysubstituted by the same or different groups selected from the group consisting of a halogen atom, a C1-C6 alkyl group, a C1-C6 alkoxy group, and a C1-C6 haloalkyl group), and
m is an integer of 1 to 3, and when m is an integer of 2 to 3, X may be the same or different.

**26.** An agricultural and horticultural fungicide comprising, as an active ingredient, the imide derivative according to any one of claims 1 to 25.

## INTERNATIONAL SEARCH REPORT

International application No.
PCT/JP2019/007874

A. CLASSIFICATION OF SUBJECT MATTER
Int.Cl. C07D417/12(2006.01)i, A01N43/80(2006.01)i, A01N43/828(2006.01)i, A01P3/00(2006.01)i

According to International Patent Classification (IPC) or to both national classification and IPC

B. FIELDS SEARCHED

Minimum documentation searched (classification system followed by classification symbols)
Int.Cl. C07D417/12, A01N43/80, A01N43/828, A01P3/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2007-308470 A (NIHON NOHYAKU CO., LTD.) 29 November 2007, claims, compounds I-544-546 & WO 2007/020986 A1; claims, compounds I-544-546 & CA 2618803 A & AU 2006280672 A & TW 200738642 A & AR 57974 A & EP 1925613 A1 & KR 10-2008-0048031 A & CN 101243049 A & US 2009/0105325 A1 & RU 2008109214 A & ZA 200802228 A & KR 10-2010-0099356 A & EG 25684 A | 1, 2, 14, 17<br>1-26 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 May 2019 (17.05.2019) | 04 June 2019 (04.06.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2019/007874

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JP 2007-537192 A (BASF AG.) 20 December 2007, claims, formulas II, IIB, compounds IIB-3 to 5 & WO 2005/110089 A2; claims, formulas II, IIB, compounds IIB-3 to 5 & CA 2563814 A & AU 2005244421 A & UY 28900 A & TW 200614916 A & AR 49171 A & NO 20065543 A & KR 10-2007-0011598 A & MX PA06012071 A & EP 1755398 A1 & CN 1972595 A & EA 200602022 A & US 2007/0197556 A1 & CR 8708 A & ZA 200610327 A | 1, 5, 26<br>1-26 |
| X | NORMAN, Mark H., JOURNAL OF HETEROCYCLIC CHEMISTRY, 1993, 30(3), pp. 771-779 | 1 |
| X | JP 2010-536926 A (BASF SE) 02 December 2010, claims, formula I, compound 131 & WO 2009/027393 A2 claims, formula I, compound 131 & AU 2008292238 A & CA 2695733 A & TW 200917962 A & AR 68063 A & MX 2010001462 A & CR 11297 A & KR 10-2010-0065340 A & EP 2197280 A1 & CN 101835377 A & US 2011/0059973 A1 & RU 2010111359 A & US 2014/0068820 A1 | 1 |
| Y | JP 2004-519464 A (SYNGENTA PARTICIPATIONS AG.) 02 July 2004, claims & WO 2002/059086 A1; claims & CA 2433819 A & KR 10-2003-0076621 A & CZ 20032018 A & AR 32113 A & EP 1355879 A1 & BR 206678 A & US 2004/0138265 A1 | 1-26 |
| Y | JP 8-176112 A (MITSUI TOATSU CHEMICALS, INC.) 09 July 1996, claims (Family: none) | 1-26 |
| P, X | WO 2018/047670 A1 (SUMITOMO CHEMICAL CO., LTD.) 15 March 2018, claims, formula I (Family: none) | 1, 5, 26 |
| P, X | CN 108117529 A (HUNAN RESEARCH INSTITUTE OF CHEMICAL INDUSTRY CO., LTD.) 05 June 2018, table 1, compound 3 (Family: none) | 1, 5, 26 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2002059086 A
- JP H08176112 A
- WO 2005042480 A
- WO 2005074686 A
- WO 2007068375 A
- JP S53132536 A